# EUROPEAN PATENT SPECIFICATION

(11) **EP 1 653 950 B1**
(45) Date of publication and mention of the grant of the patent: **09.01.2008**
(21) Application number: 04766269.7
(22) Date of filing: 20.07.2004
(51) Int. Cl.: A61K 31/4178

(54) **NITROOXY DERIVATIVES OF LOSARTAN, VALSATAN, CANDESARTAN, TELMISARTAN, EPROSARTAN AND OLMESARTAN AS ANGIOTENSIN-II RECEPTOR BLOCKERS FOR THE TREATMENT OF CARDIOVASCULAR DISEASES**
NITROOXYDERIVATE VON LOSARTAN, VALSATAN, CANDESARTAN, TELMISARTAN, EPROSARTAN AND OLMESARTAN ALS ANGIOTENSIN-II-REZEPTOR-BLOCKER ZUR BEHANDLUNG VON HERZ-KREISLAUF-ERKRANKUNGEN
DERIVES NITROOXY DU LOSARTAN ET D'AUTRES INHIBITEURS DU RECEPTEUR ANGIOTENSIN-II SIMILAIRES POUR LE TRAITEMENT DES MALADIES CARDIO-VASCULAIRES

(30) Priority: 31.07.2003 EP 03102379
(43) Date of publication of application: 10.05.2006
(73) Proprietor: NicOx S.A., 06560 Sophia Antipolis - Valbonne (FR)
(72) Inventor: ALMIRANTE, Nicoletta, I-20155 Milano (IT); DEL SOLDATO, Piero, I-20052 Monza (IT); ONGINI, Ennio, I-20090 Segrate (IT)
(74) Representative: Barchielli, Giovanna
(86) International application number: PCT/EP2004/051550
(87) International publication number: WO 2005/011646

(56) References cited:
- EP-A- 0 955 294
- EP-A- 1 312 379
- WO-A-00/61537
- WO-A-03/013499
- MCINTYRE ET AL.: "Losartan, an Orally Active Angiotensin (AT1) Receptor Antagonist: A Review of Its Efficacy and Safety in Essential Hypertension" PHARMACOL. THER., vol. 74, no. 2, 1997, pages 181-194, XP002262197
- HEDNER T ET AL: "A COMPARISON OF THE ANGIOTENSIN II ANTAGONISTS VALSARTAN AND LOSARTAN IN THE TREATMENT OF ESSENTIAL HYPERTENSION" AMERICAN JOURNAL OF HYPERTENSION, NEW YORK, NY, US, vol. 12, no. 4, April 1999 (1999-04), pages 414-417, XP000866169
- "TELMISARTAN" DRUGS OF THE FUTURE, BARCELONA, ES, vol. 22, no. 10, 1997, pages 1112-1116, XP009008343 ISSN: 0377-8282
- "OLMESARTAN MEDOXOMIL CS-866 BENEVAS ANTIHYPERTENSIVE ANGIOTENSIN AT ANTAGONIST" DRUGS OF THE FUTURE, BARCELONA, ES, vol. 25, no. 11, November 2000 (2000-11), pages 1217-1218, XP009030725 ISSN: 0377-8282
- HEDNER T: "THE CLINICAL PROFILE OF THE ANGIOTENSIN II RECEPTOR BLOCKER EPROSARTAN" JOURNAL OF HYPERTENSION, CURRENT SCIENCE, PHILADELPHIA, PA, US, vol. 20, no. SUPPL 5, 2002, pages S33-S38, XP008011504 ISSN: 0263-6352

## Description

The present invention relates to Angiotensin II Receptor Blocker (ARB) derivatives. More particularly, the present invention relates to ARB nitroderivatives, pharmaceutical compositions containing them and their use for the treatment of cardiovascular, renal and chronic liver diseases, inflammatory processes and metabolic syndromes.

With the angiotensin II receptor blockers a class of compounds is intended, comprising as main components Losartan, EXP3174, Candesartan, Telmisartan, Valsartan, Eprosartan, Irbesartan and Olmesartan.

ARBs are approved only for the treatment of hypertension, the antihypertensive activity is due mainly to selective blockade of AT₁ receptors and the consequent reduced pressor effect of angiotensin II. Angiotensin II stimulates the synthesis and secretion of aldosterone and raises blood pressure via a potent direct vasoconstrictor effect.

Now, it has been reported that angiotensin II receptor blockers have side-effects such as for example hypotension, hyperkalaemia, myalgia, respiratory-tract disorders, renal disorders, back pain, gastrointestinal disturbances, fatigue, and neutropenia (Martindale, Thirty-third edition, p. 921) .

It was now object of the present invention to provide new derivatives of ARBs able not only to eliminate or at least reduce the side effects associated with their parent compounds, but also having an improved pharmacological activity. It has been so surprisingly found that angiotensin II receptor blocker nitroderivatives have a significantly improved overall profile as compared to native compounds both in term of wider pharmacological activity and enhanced tolerability.

In particular, it has been recognized that the angiotensin II receptor blocker nitroderivatives of the present invention exhibit a strong anti-inflammatory, antithrombotic and antiplatelet activity and can be furthermore employed for treating or preventing heart failure, myocardial infarction, ischemic stroke, atherosclerosis, ocular and pulmonary hypertension, hypertension, diabetic nephropathy, peripheral vascular diseases, left ventricular dysfunction and hypertrophy, liver fibrosis, portal hypertension and metabolic syndromes.

Object of the present invention are, therefore, Angiotensin II Receptor Blocker nitroderivatives of general formula (I) and pharmaceutically acceptable salts or stereoisomers thereof:

R-(Y-ONO₂)ₛ (I)

wherein:
s is an integer equal to 1 or 2;
R is selected from the following Angiotensin II Receptor Blocker residues of formula (II) or (III): 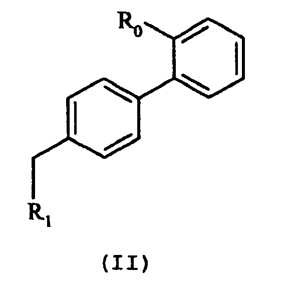 wherein:
R₀ is 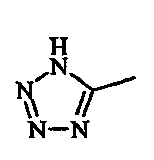 or -N₀ which is a group capable to bind to Y, having one of the following meaning:
   -COO-, -O-, -CONH-, -OCO-, -OCOO- or 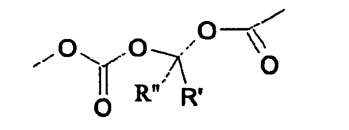 wherein R' and R" are the same or different, and are H or straight or branched C₁-C₄ alkyl;
R₁ is selected from the group consisting of: 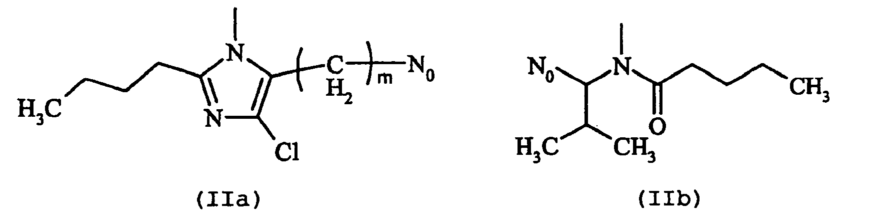 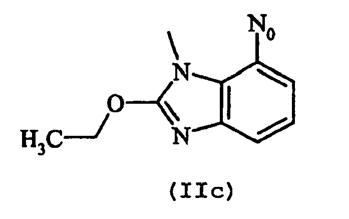 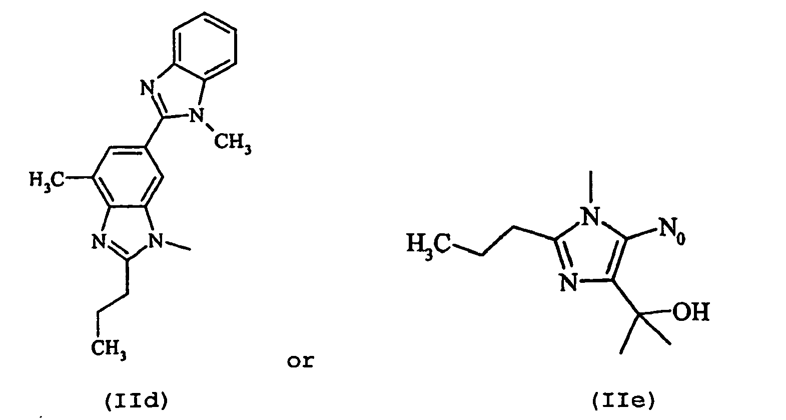 wherein m is an integer equal to 0 or 1 and N₀ is as above defined; 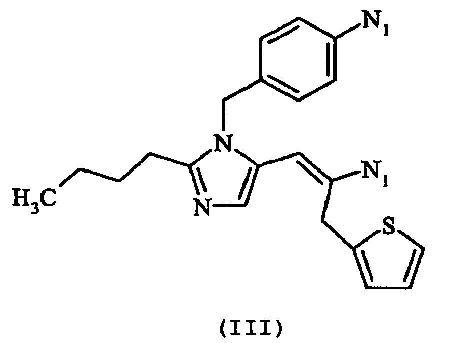 wherein N₁ has the same meaning as N₀ or is equal to -COOH; with the proviso that at least one of the groups N₁ is equal to -COO- or -CONH-, i.e. it is a group capable to bind to Y;
Y is a bivalent radical having the following meaning:
   a)
      - straight or branched C₁-C₂₀ alkylene, preferably C₁-C₁₀, being optionally substituted with one or more of the substituents selected from the group consisting of: halogen atoms, hydroxy, -ONO₂ or T₀, wherein T₀ is
      - OC(O) (C₁-C₁₀ alkyl) -ONO₂ or -O(C₁-C₁₀ alkyl)-ONO₂;
      - cycloalkylene with 5 to 7 carbon atoms into cycloalkylene ring, the ring being optionally substituted with side chains T, wherein T is straight or branched alkyl with from 1 to 10 carbon atoms, preferably CH₃;
   b) 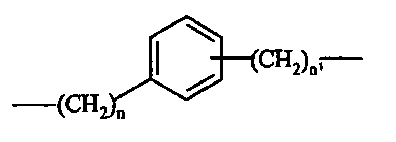
   c) 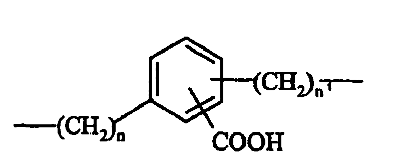 wherein n is an integer from 0 to 20, and n¹ is an integer from 1 to 20;
   d) 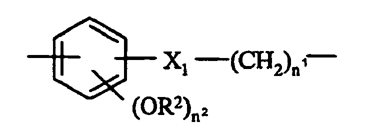 wherein:
      n¹ is as defined above and n² is an integer from 0 to 2;
      X₁ = -OCO- or -COO- and R² is H or CH₃;
   e) 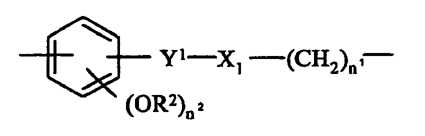 wherein:
      n¹, n², R² and X₁ are as defined above;
      Y¹ is -CH₂-CH₂- or -CH=CH-(CH₂)n²-;
   f) 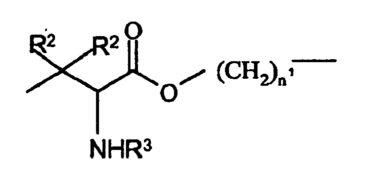 wherein:
      n¹ and R² are as defined above, R³ is H or -COCH₃;
      with the proviso that when Y is selected from the bivalent radicals mentioned under b)-f), the -ONO₂ group is linked to a - (CH₂)n¹ group;
   g) 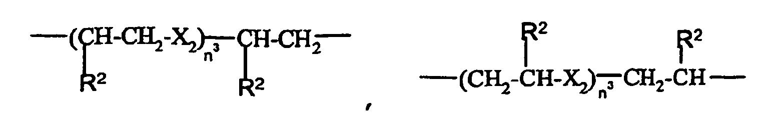 wherein X₂ is -O- or -S-, n³ is an integer from 1 to 6, preferably from 1 to 4, R² is as defined above;
   h) 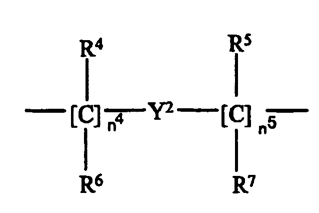 wherein:
      n⁴ is an integer from 0 to 10;
      n⁵ is an integer from 1 to 10;
      R⁴, R⁵, R⁶, R⁷ are the same or different, and are H or straight or branched C₁-C₄ alkyl, preferably R⁴, R⁵, R⁶, R⁷ are H;
      wherein the -ONO₂ group is linked to 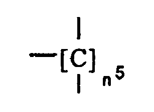 wherein n⁵ is as defined above;
      Y² is an heterocyclic saturated, unsaturated or aromatic 5 or 6 members ring, containing one or more heteroatoms selected from nitrogen, oxygen, sulfur,
      and is selected from

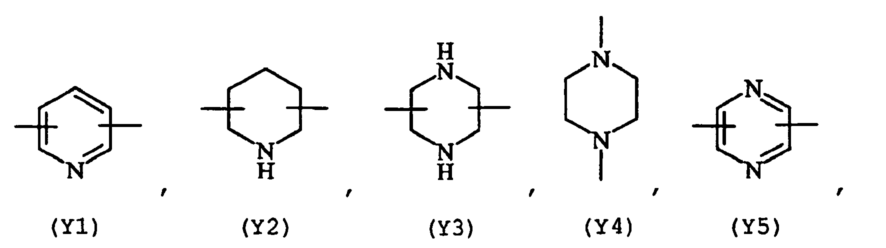

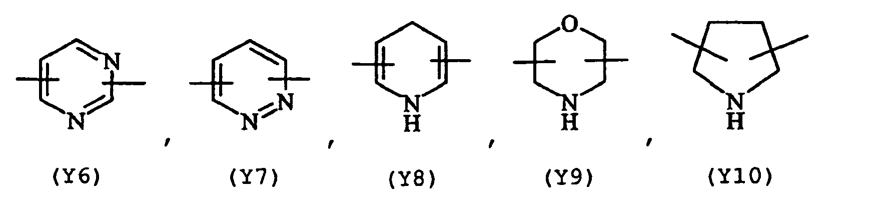

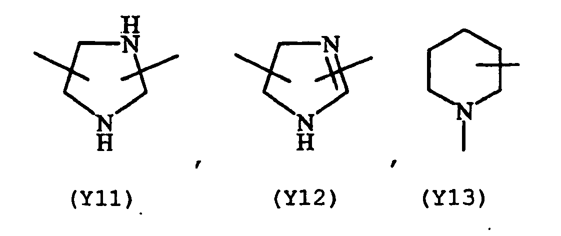

The term "C₁-C₂₀ alkylene" as used herein refers to branched or straight chain C₁-C₂₀ hydrocarbon, preferably having from 1 to 10 carbon atoms such as methylene, ethylene, propylene, isopropylene, n-butylene, pentylene, n-hexylene and the like.

The term "C₁-C₁₀ alkyl" as used herein refers to branched or straight chain alkyl groups comprising one to ten carbon atoms, including methyl, ethyl, n-propyl, isopropyl, n-butyl, isobutyl, t-butyl, pentyl, hexyl, octyl and the like.

The term "cycloalkylene" as used herein refers to ring having from 5 to 7 carbon atoms including, but not limited to, cyclopentylene, cyclohexylene optionally substituted with side chains such as straight or branched (C₁-C₁₀)-alkyl, preferably CH₃.

The term "heterocyclic" as used herein refers to saturated, unsaturated or aromatic 5 or 6 members ring, containing one or more heteroatoms selected from nitrogen, oxygen, sulphur, such as for example pyridine, pyrazine, pyrimidine, pyrrolidine, morpholine, imidazole and the like.

Another aspect of the present invention provides the use of the compounds of formula (I) in combination with at least a compound used to treat cardiovascular disease selected from the group consisting of: ACE inhibitors, HMGCoA reductase inhibitors, beta-adrenergic blockers, calcium channel blockers, diuretics, antithrombotics such as aspirin, nitrosated ACE inhibitors, nitrosated HMGCoA reductase inhibitors, nitrosated beta-adrenergic blockers, nitrosated aspirin and nitrosated diuretics.

Suitable ACE inhibitors, HMGCoA reductase inhibitors, beta-adrenergic blockers, calcium channel blockers, antithrombotics and diuretics are described in the literature such as The Merck Index (13^{th} edition).

Suitable nitrosated compounds are disclosed in WO 98/21193, WO 97/16405 and WO 98/09948.

The administration of the compounds above reported can be carried out simultaneously or successively.

The present invention also provides pharmaceutical kits comprising one or more containers filled with one or more of the compounds and/or compositions of the present invention and one or more of the compounds used to treat cardiovascular diseases reported above.

As stated above, the invention includes also the pharmaceutically acceptable salts of the compounds of formula (I) and stereoisomers thereof.

Examples of pharmaceutically acceptable salts are either those with inorganic bases, such as sodium, potassium, calcium and aluminium hydroxides, or with organic bases, such as lysine, arginine, triethylamine, dibenzylamine, piperidine and other acceptable organic amines.

The compounds according to the present invention, when they contain in the molecule one salifiable nitrogen atom, can be transformed into the corresponding salts by reaction in an organic solvent such as acetonitrile, tetrahydrofuran with the corresponding organic or inorganic acids.

Examples of organic acids are: oxalic, tartaric, maleic, succinic, citric acids. Examples of inorganic acids are: nitric, hydrochloric, sulphuric, phosphoric acids. Salts with nitric acid are preferred.

The compounds of the invention which have one or more asymmetric carbon atoms can exist as optically pure enantiomers, pure diastereomers, enantiomers mixtures, diastereomers mixtures, enantiomer racemic mixtures, racemates or racemate mixtures. Within the object of the invention are also all the possible isomers, stereoisomers and their mixtures of the compounds of formula (I).

Preferred compounds are those of formula (I) wherein:
s and R are as above defined;
Y is a bivalent radical having the following meaning:
   a)
      - straight or branched C₁-C₁₀ alkylene, being optionally substituted with T₀, wherein T₀ is as above defined;
   b) 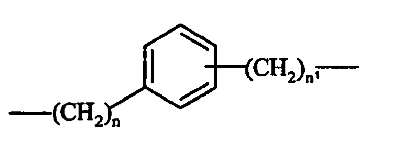 wherein n is an integer equal to 0 or 1, and n¹ is an integer equal to 1; with the proviso the -ONO₂ group is linked to a - (CH2)ₙ¹ group;
   g) 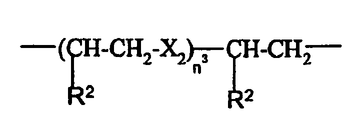 wherein X₂ is -O- or -S-, n³ is an integer equal to 1 and R² is H;

The following are preferred compounds according to the present invention:

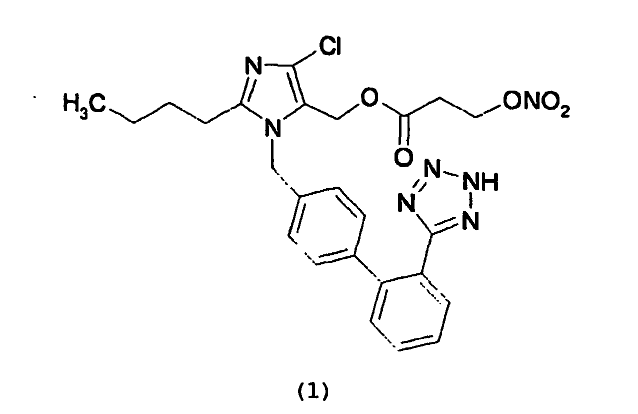

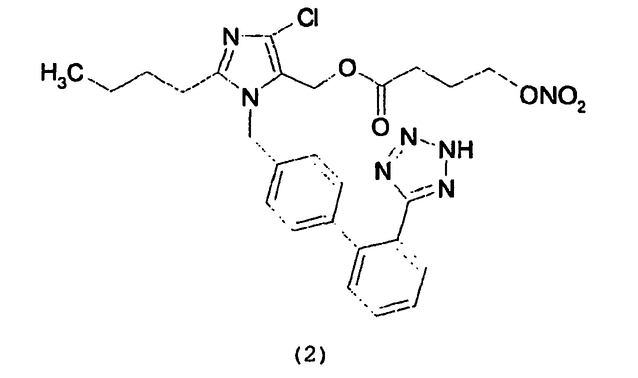

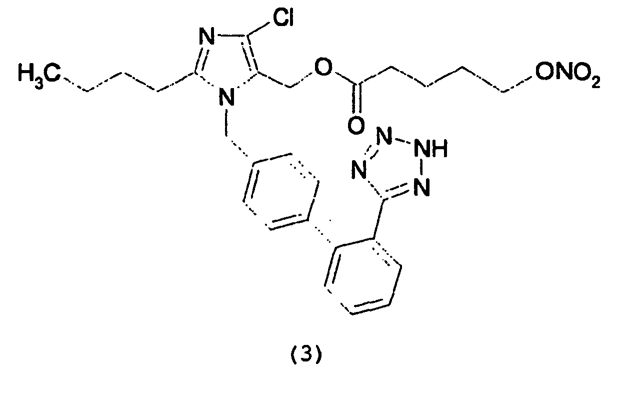

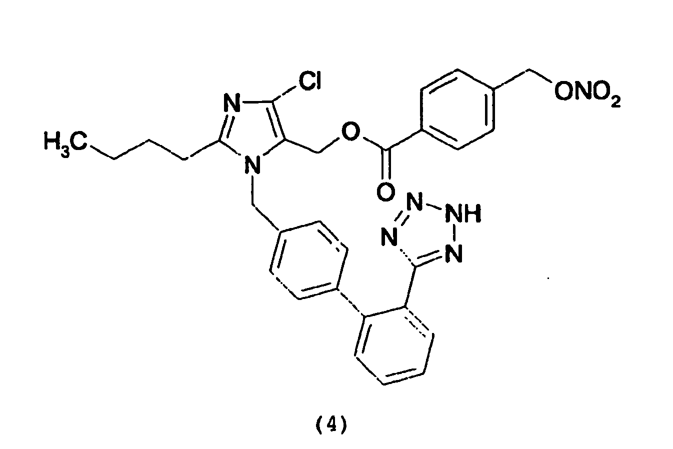

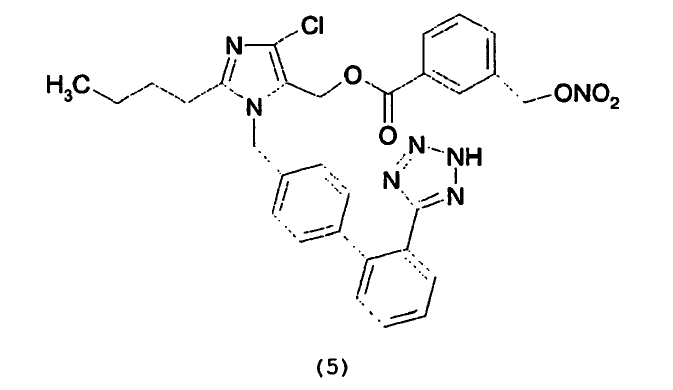

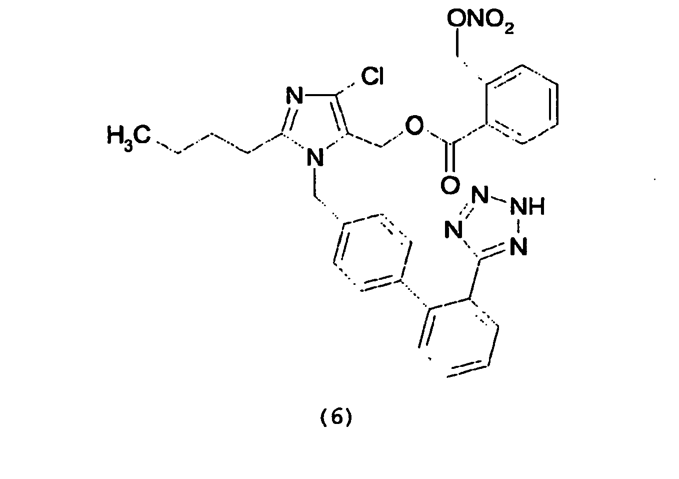

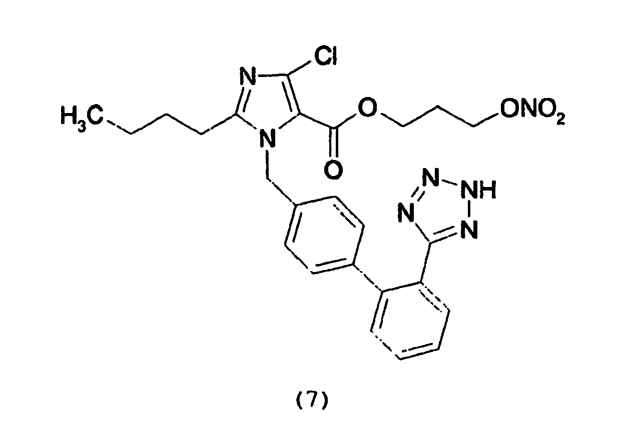

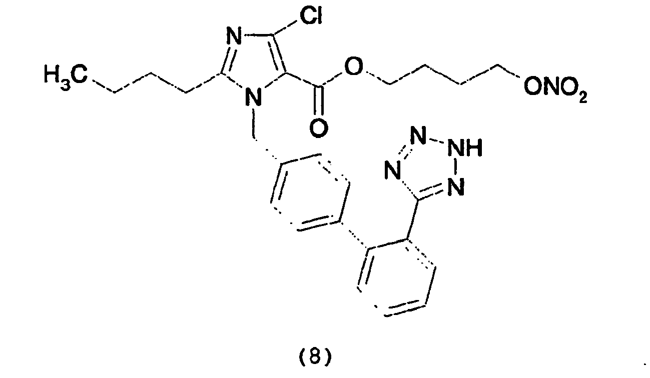

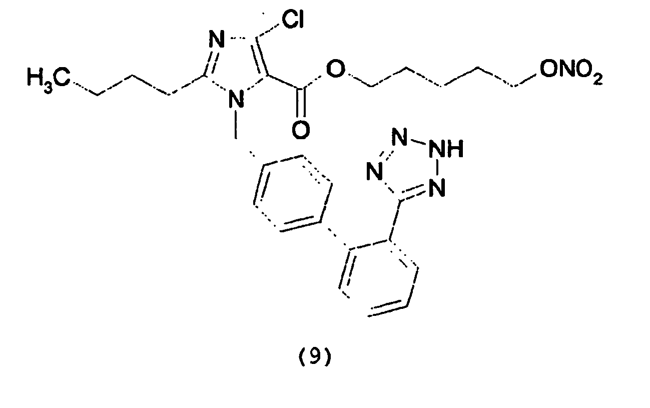

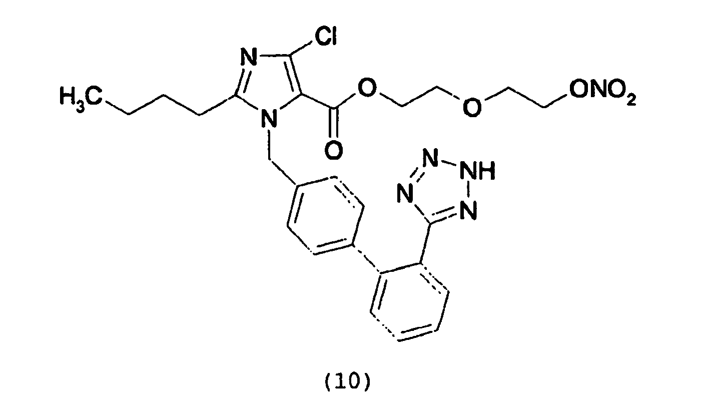

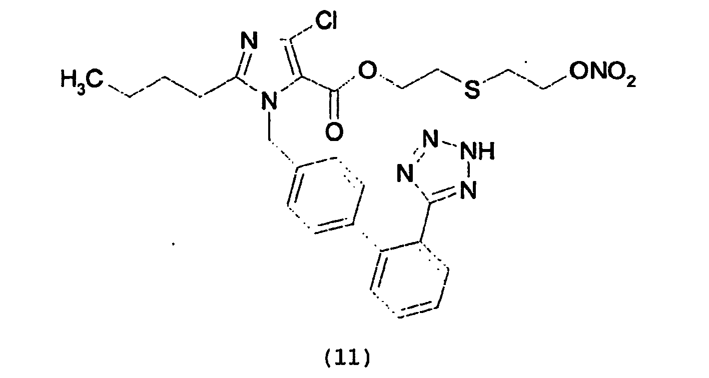

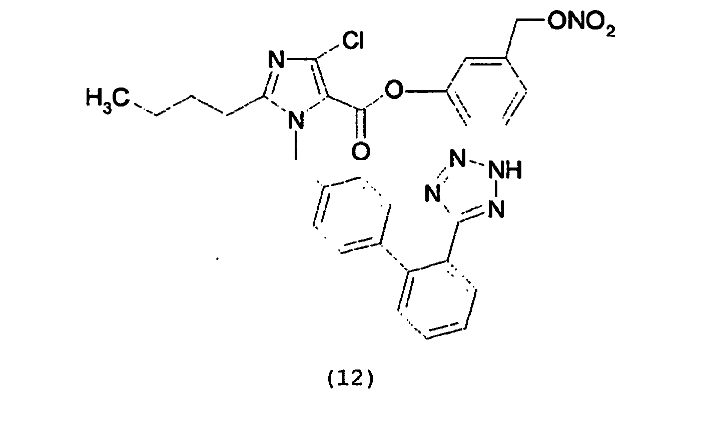

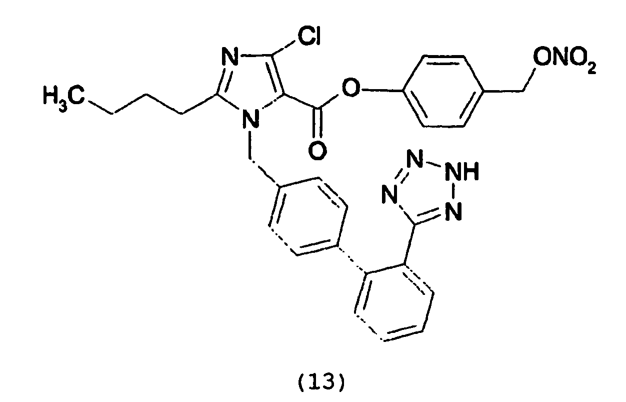

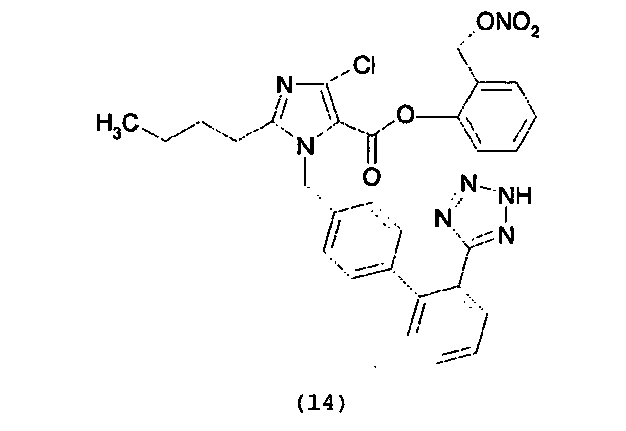

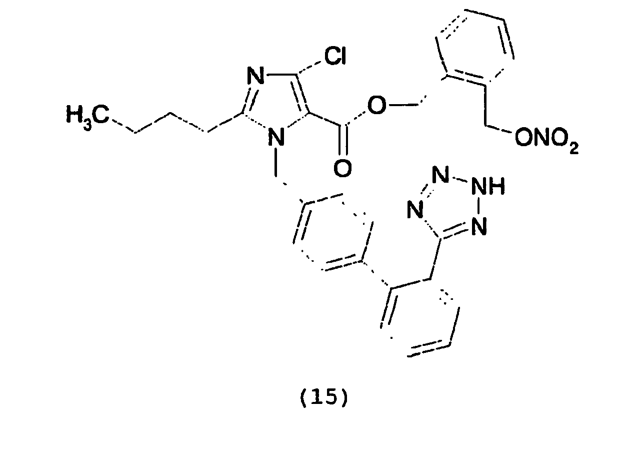

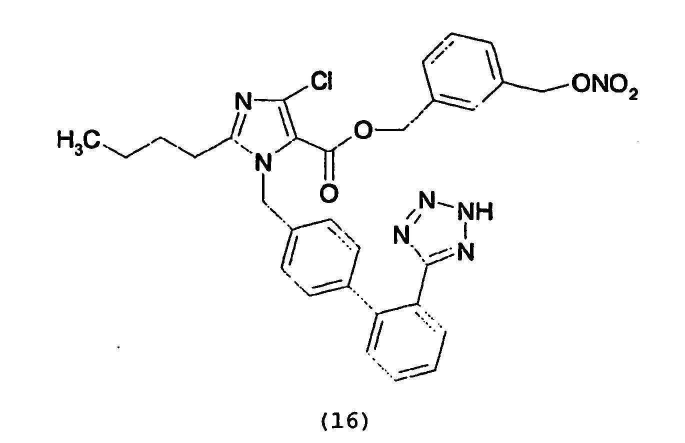

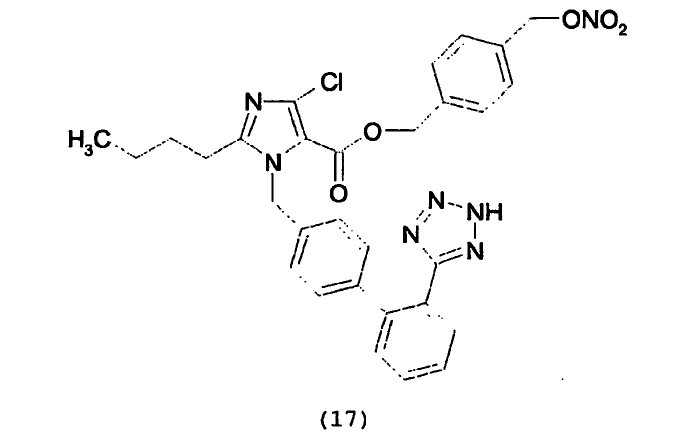

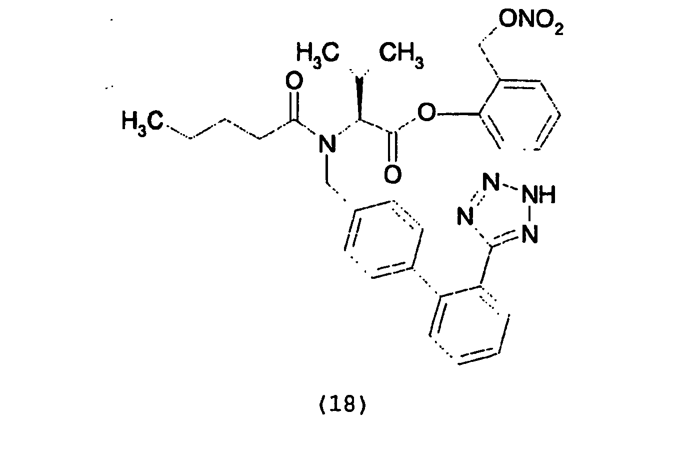

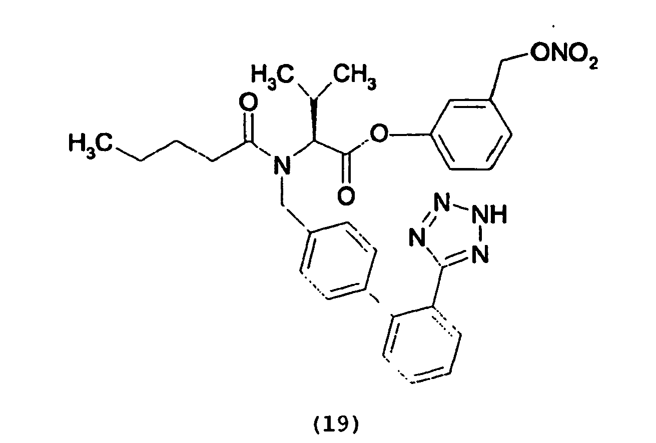

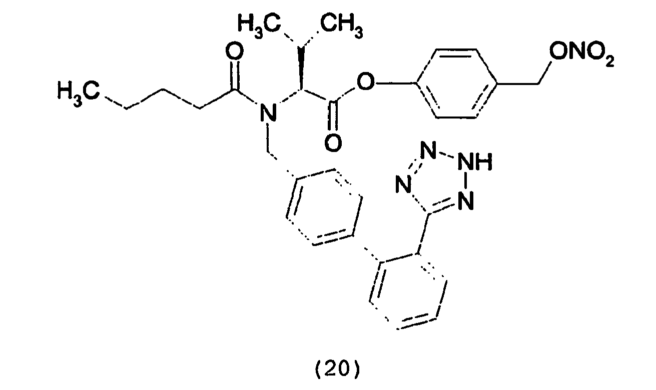

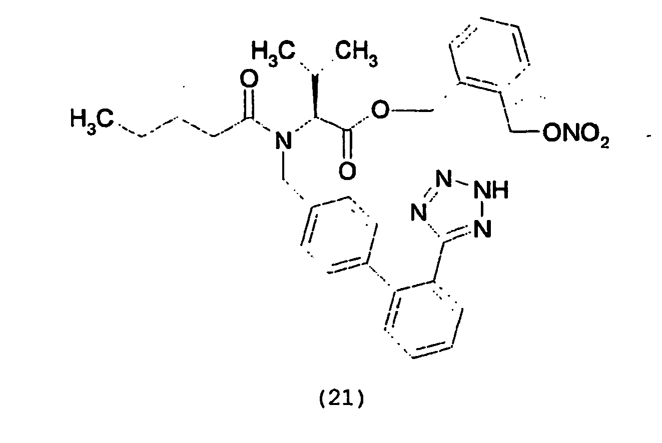

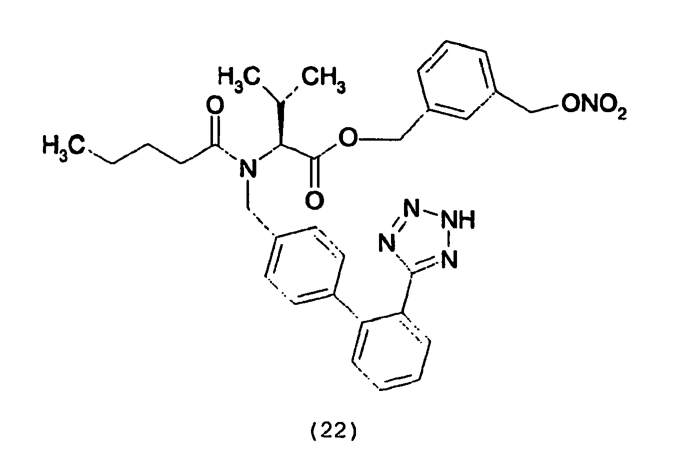

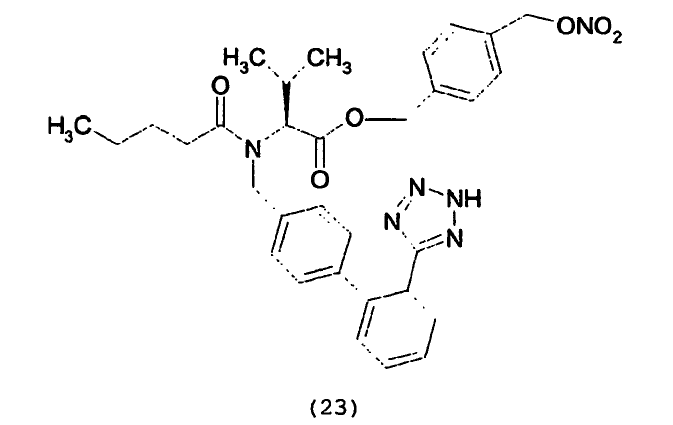

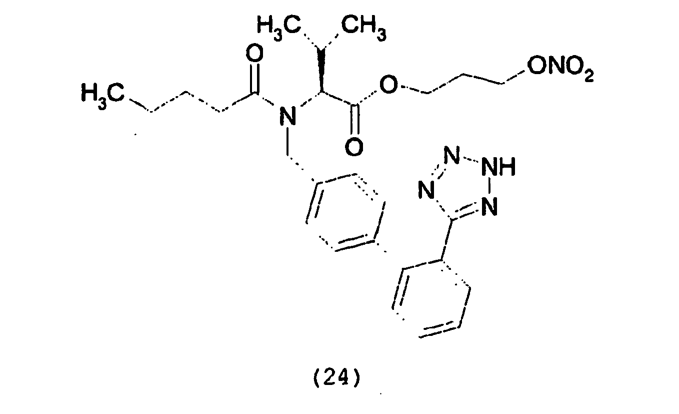

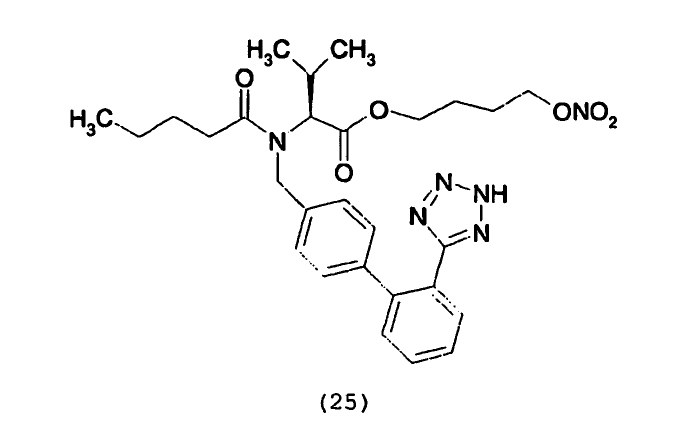

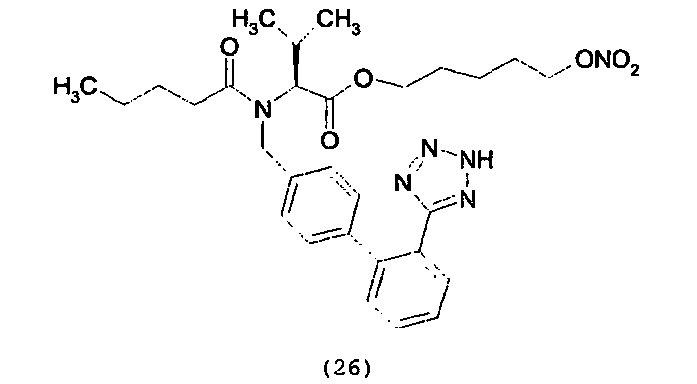

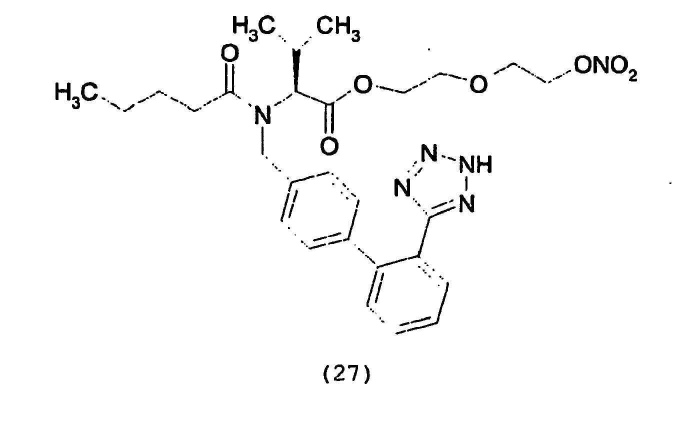

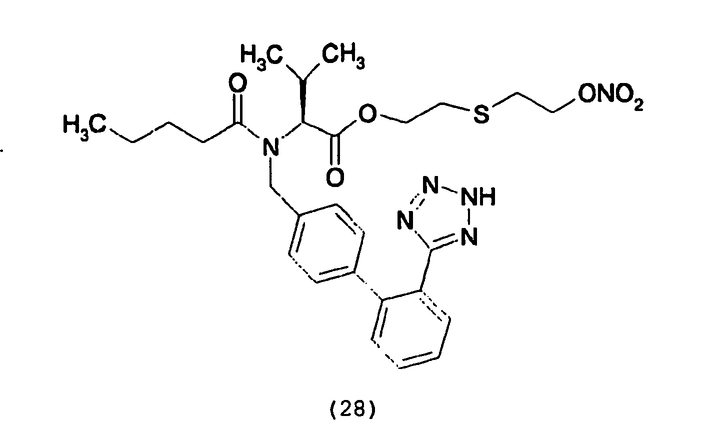

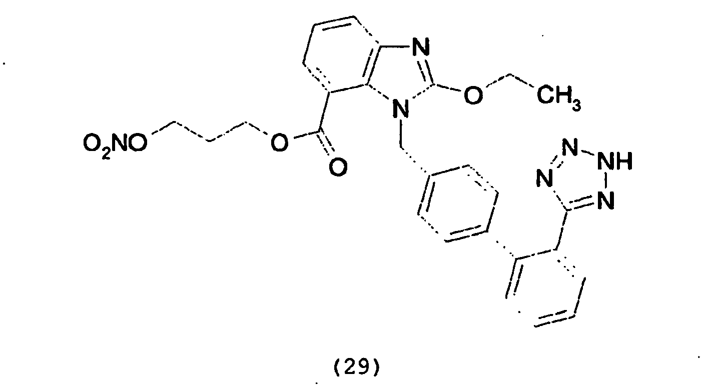

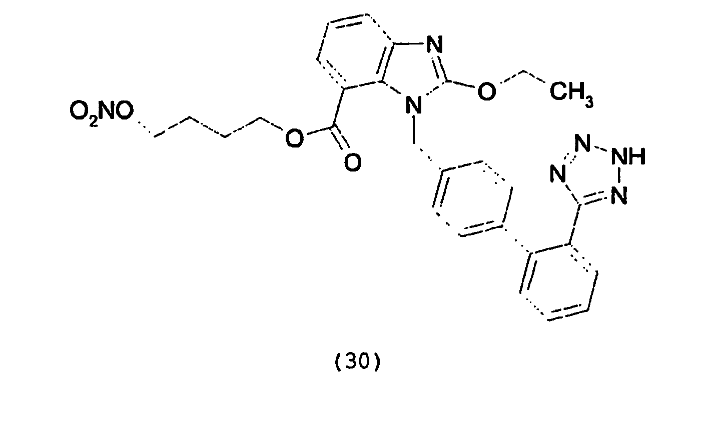

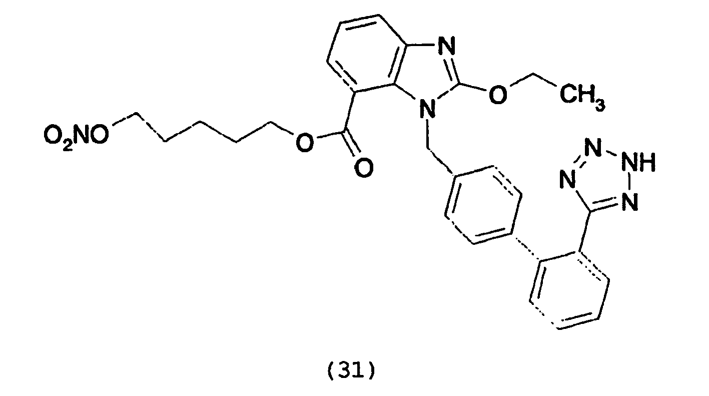

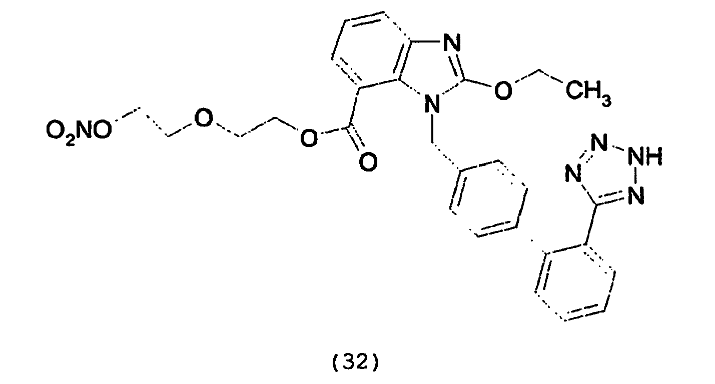

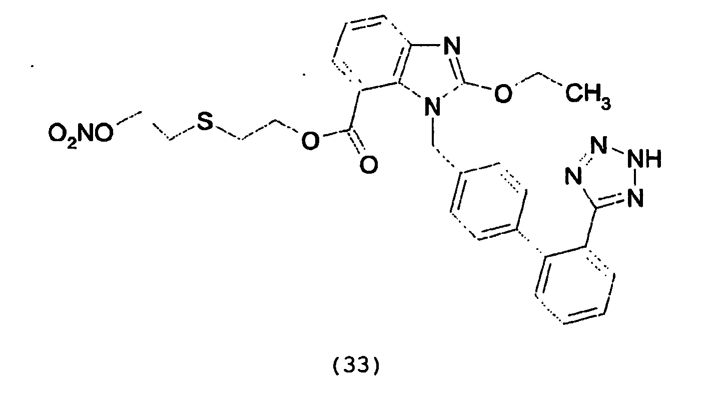

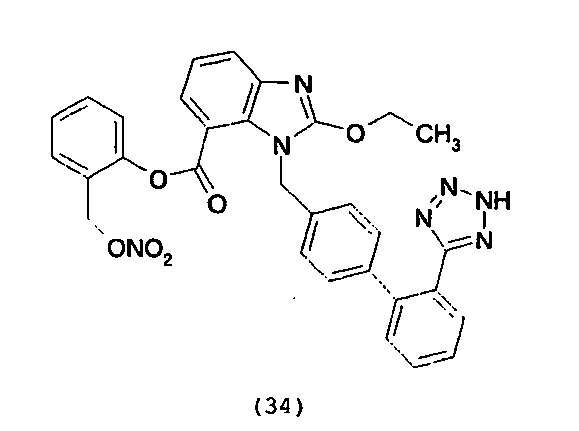

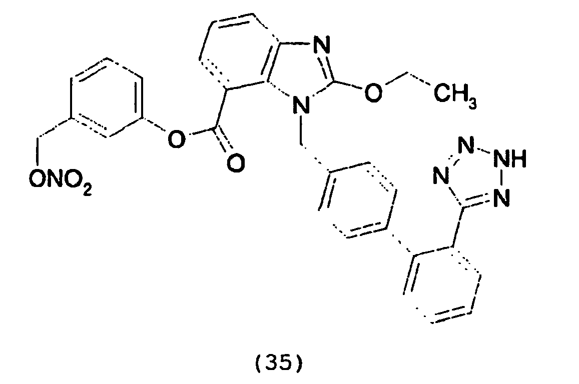

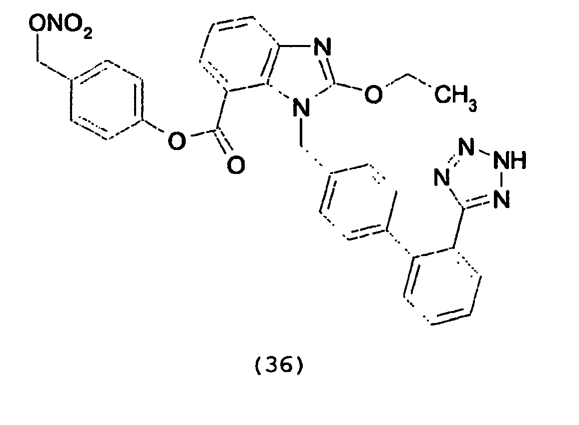

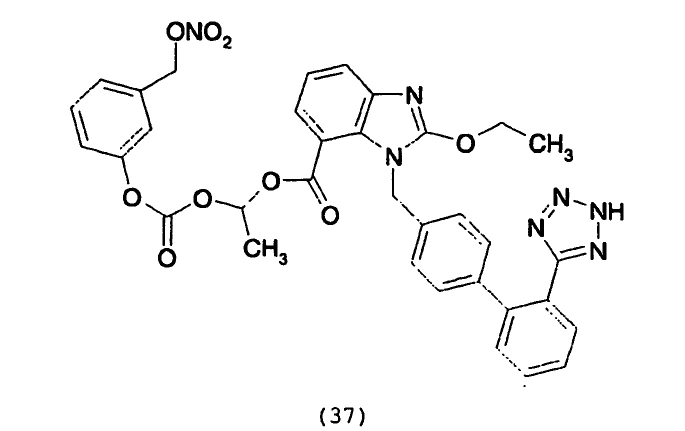

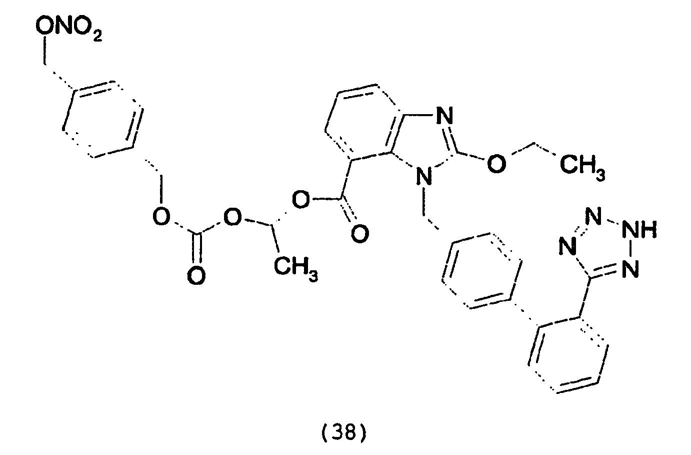

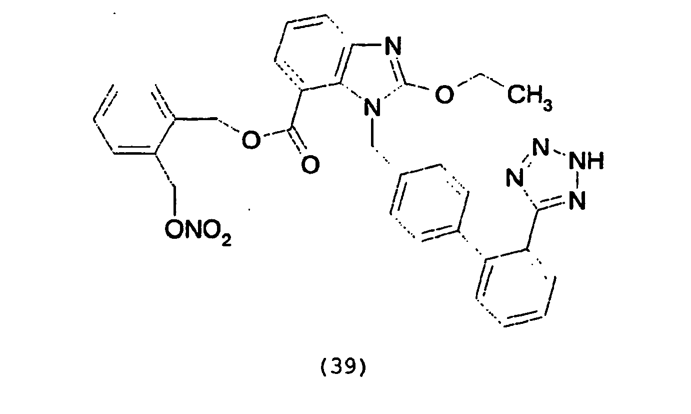

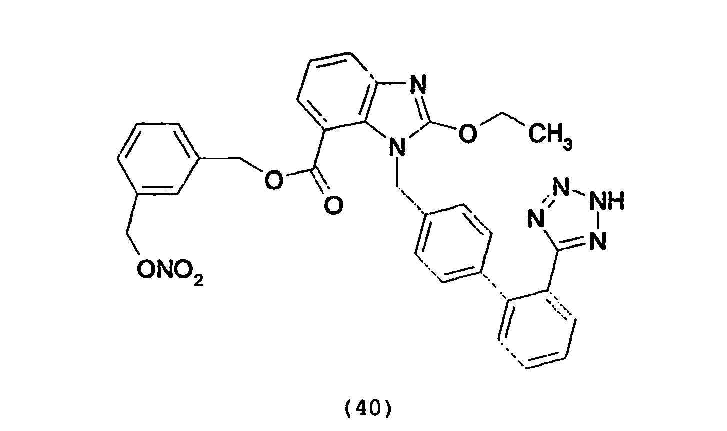

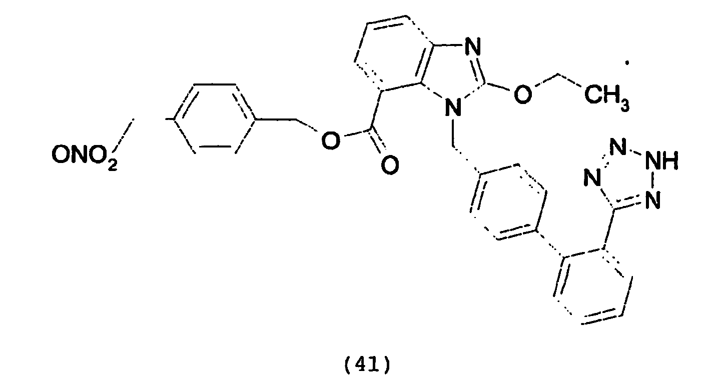

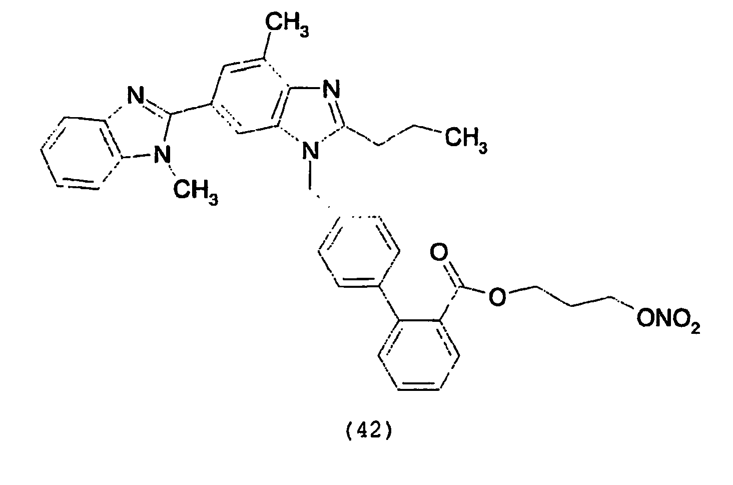

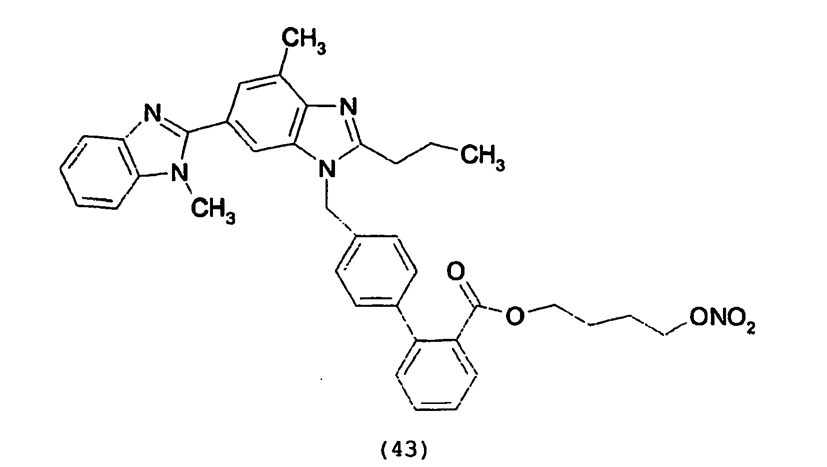

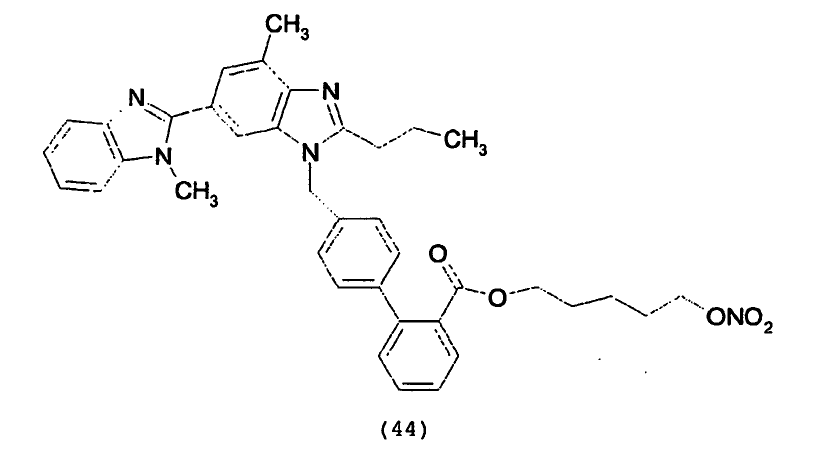

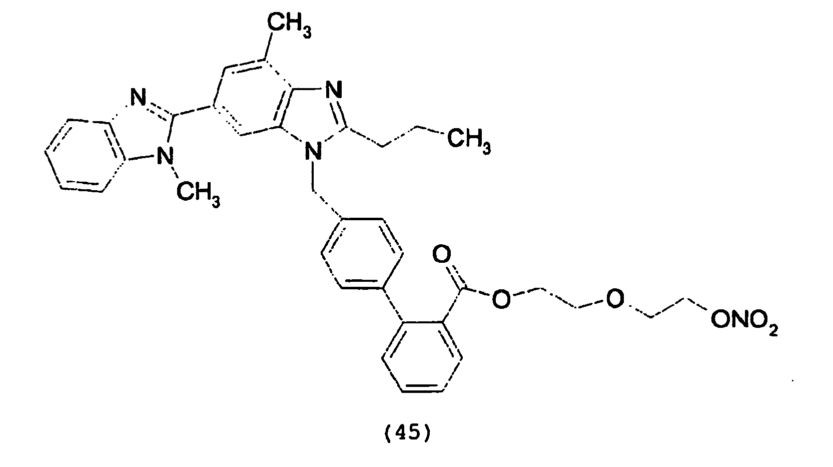

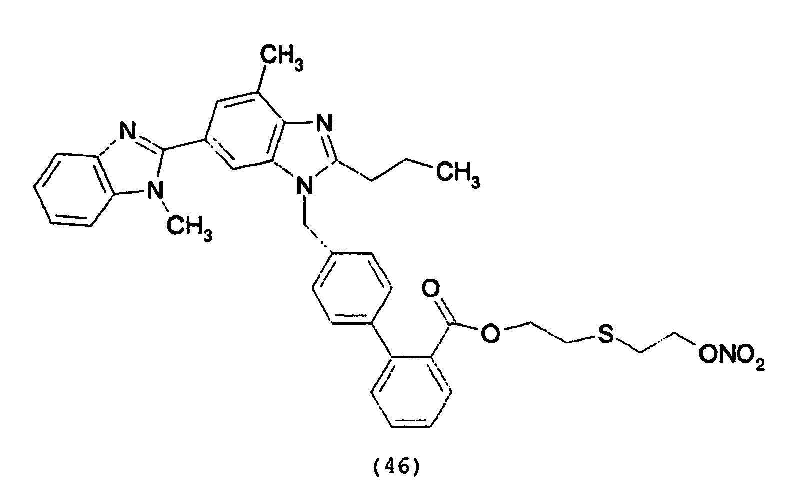

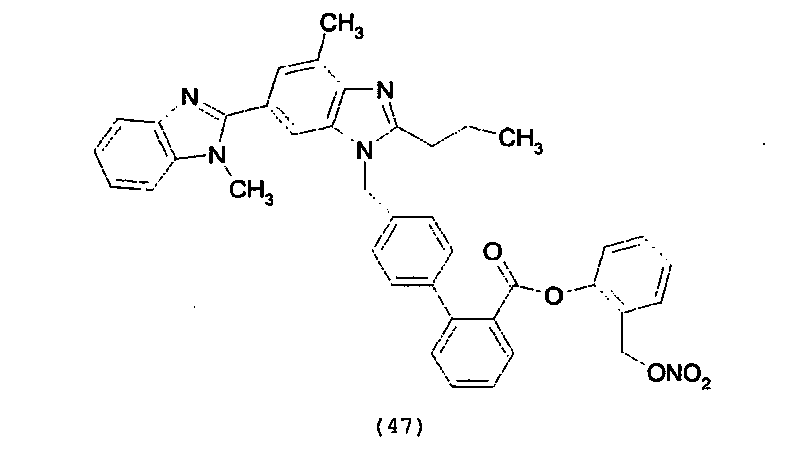

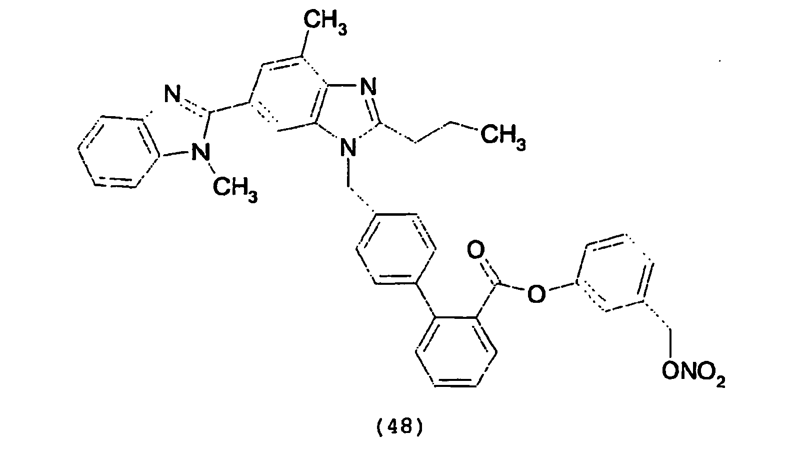

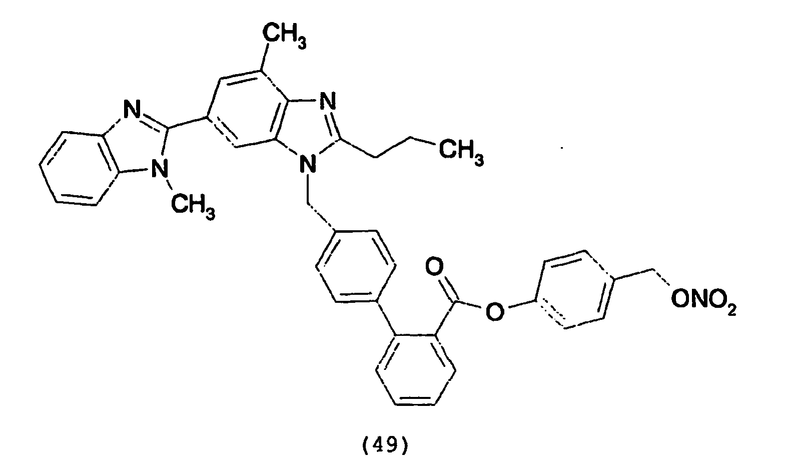

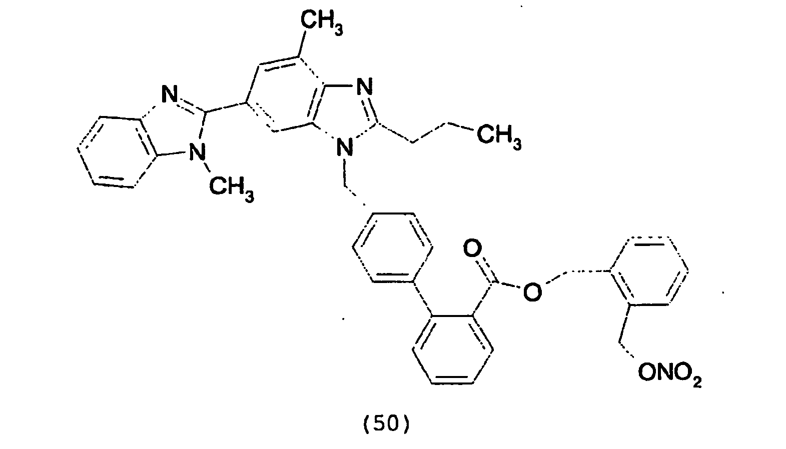

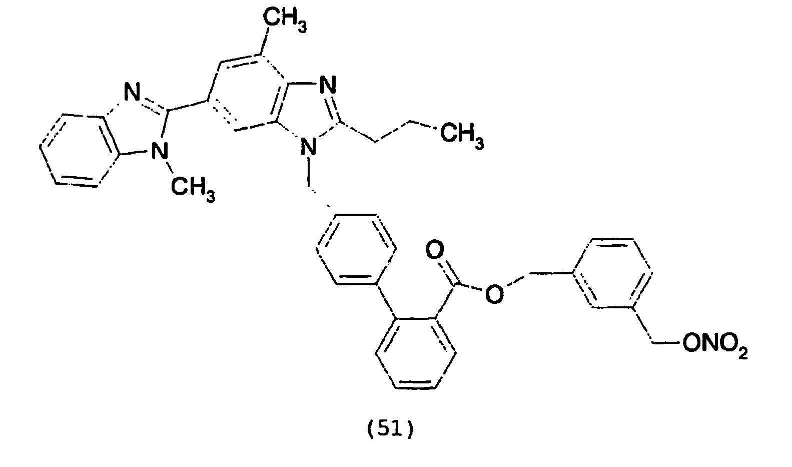

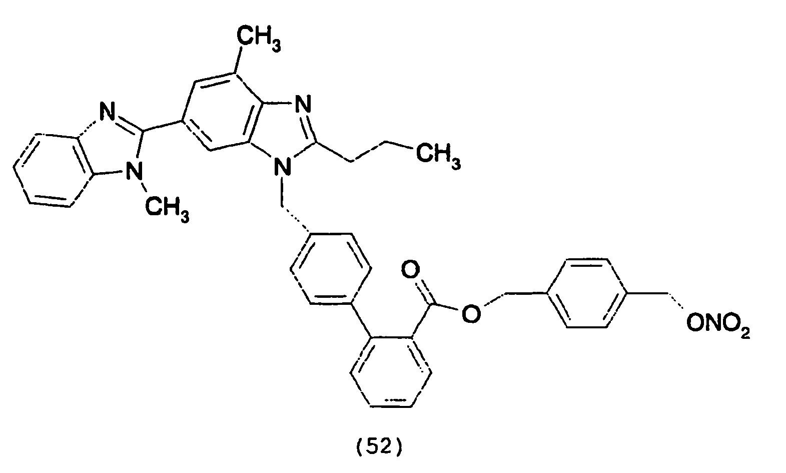

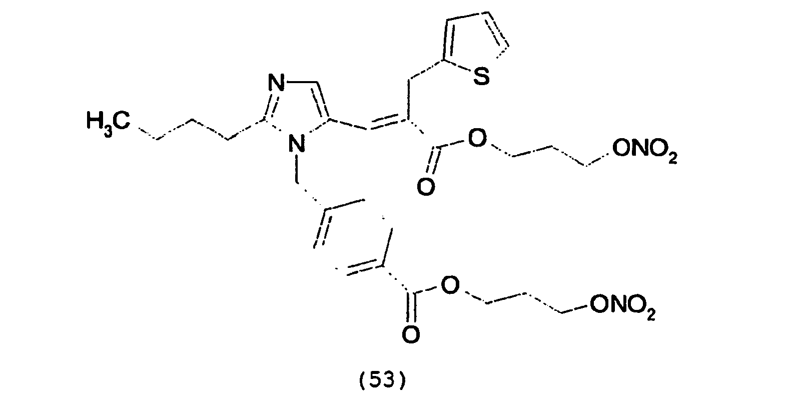

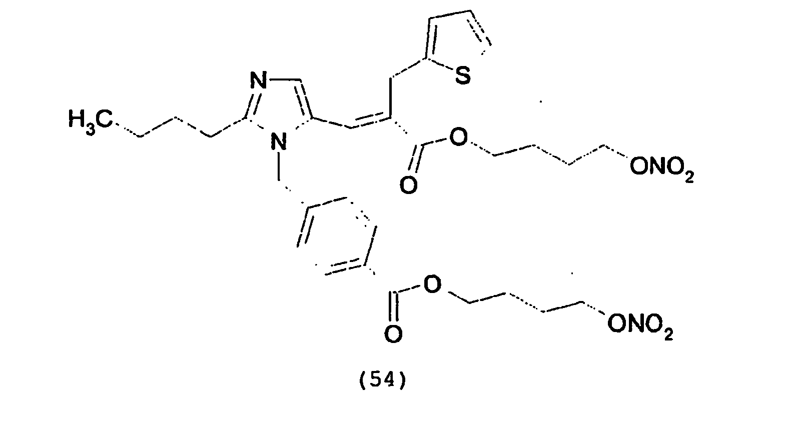

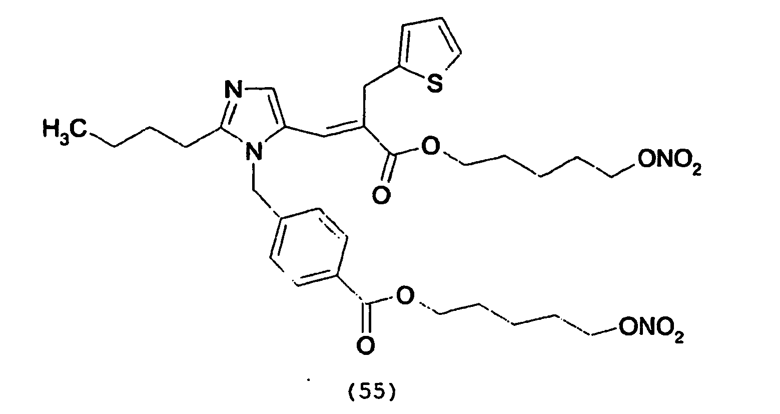

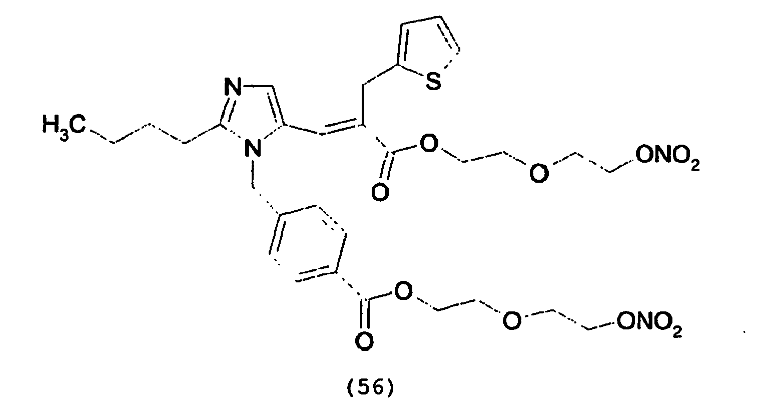

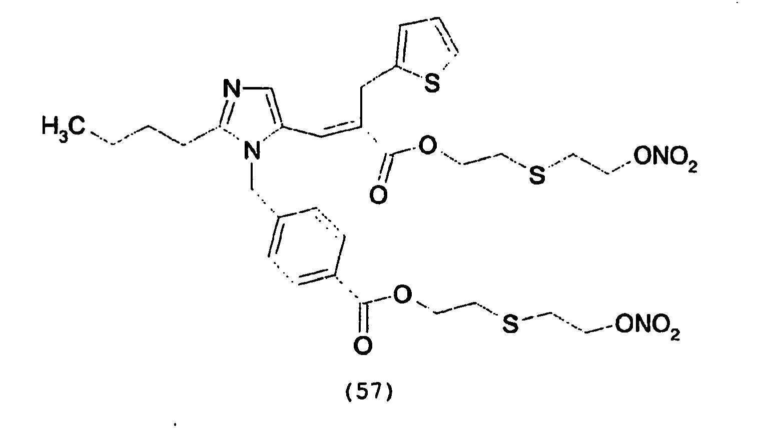

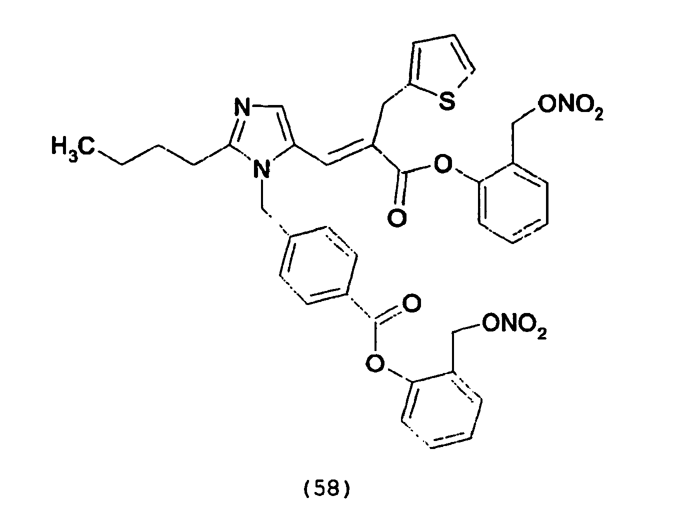

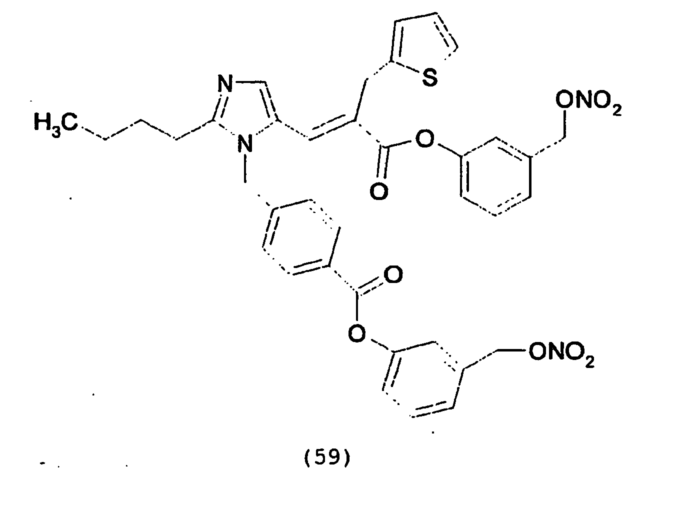

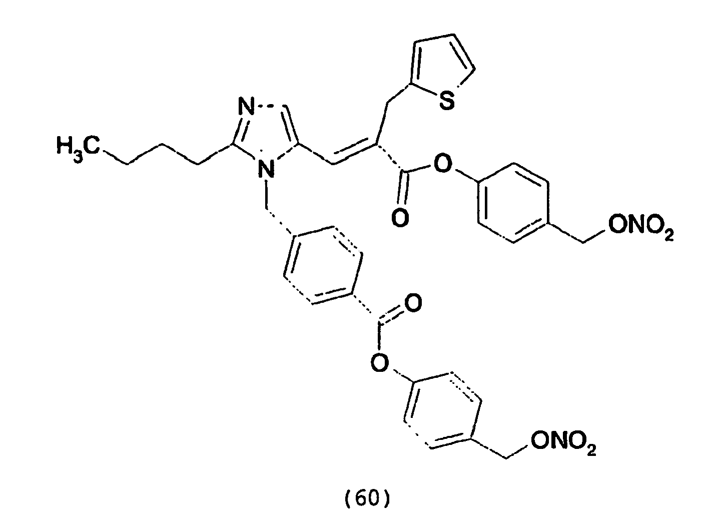

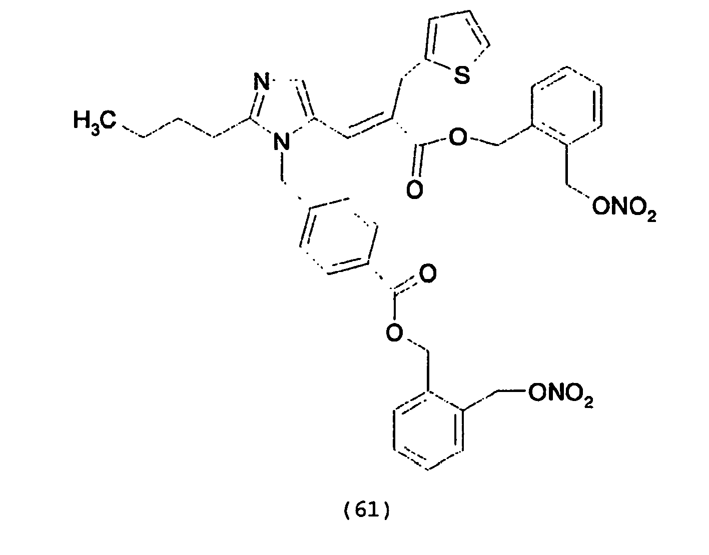

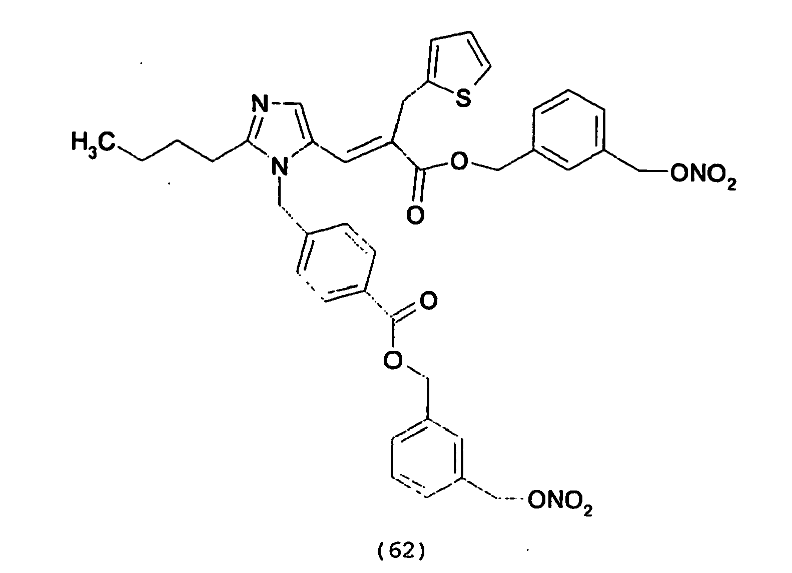

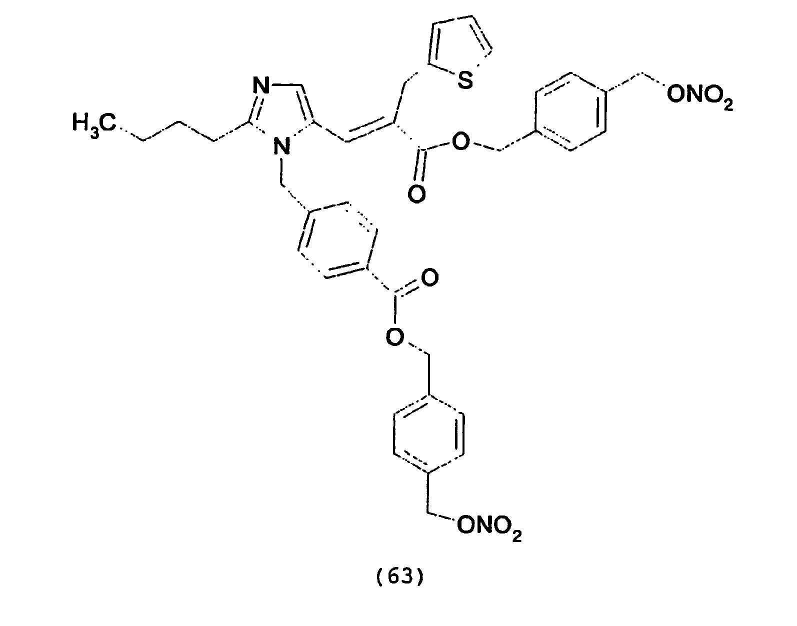

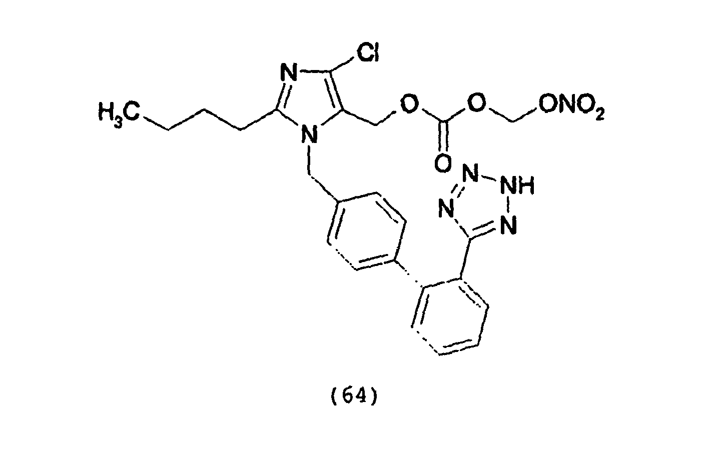

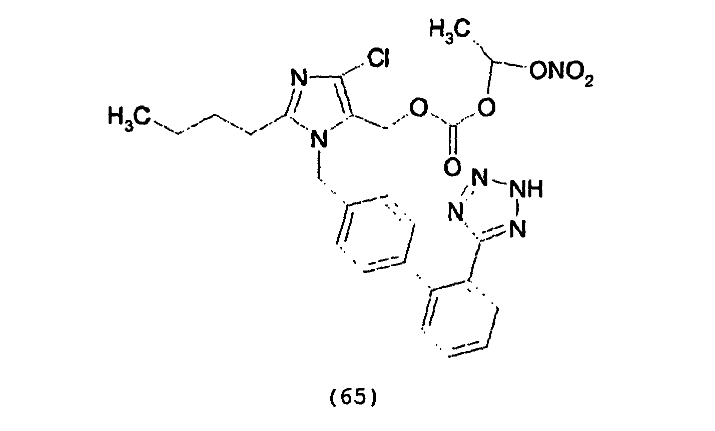

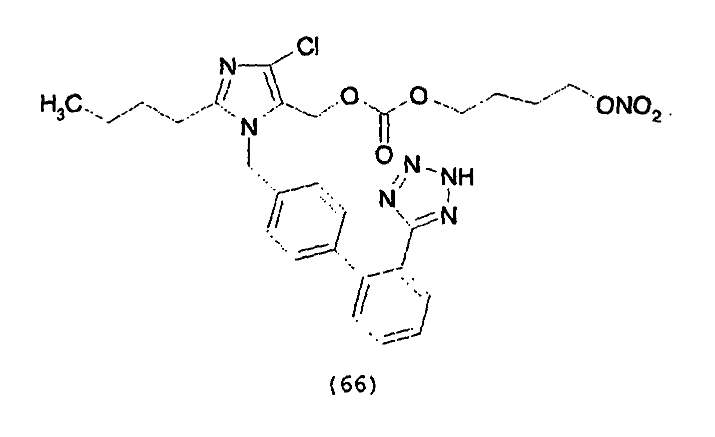

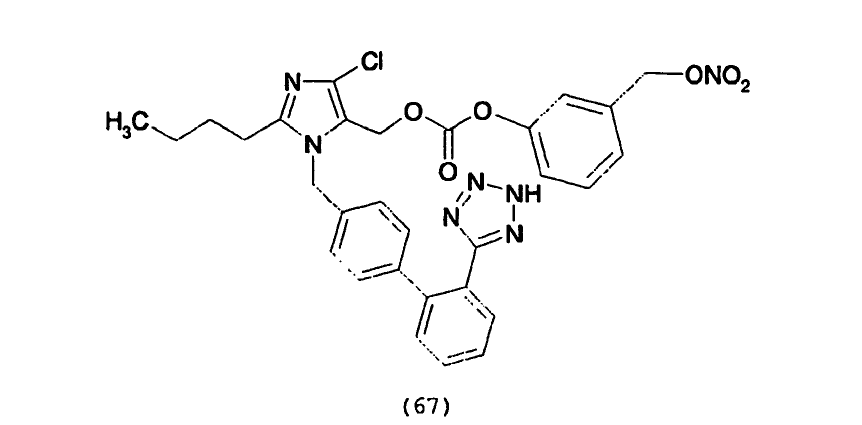

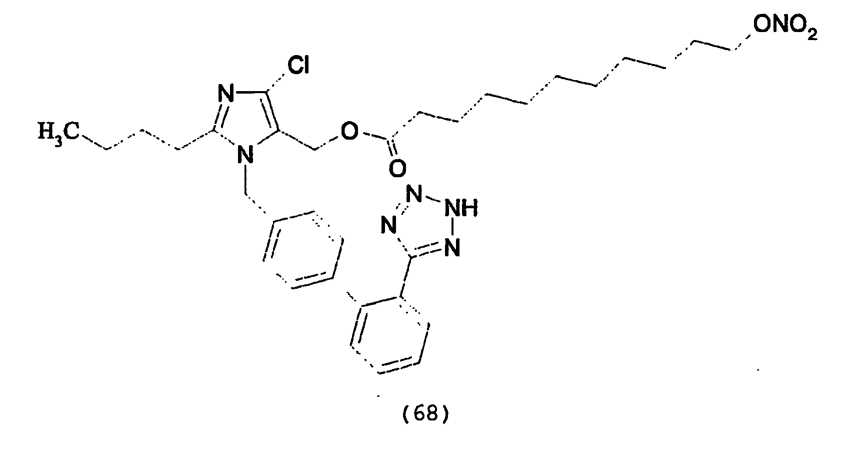

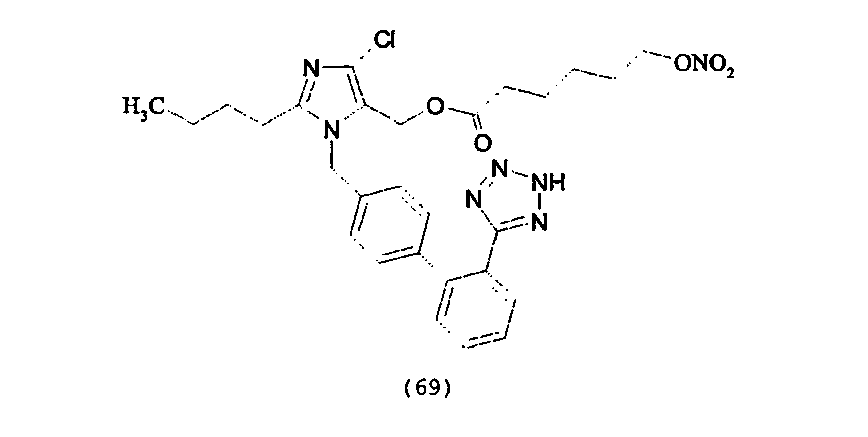

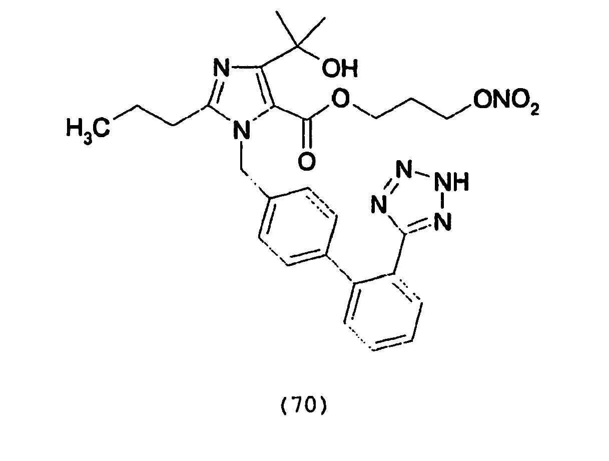

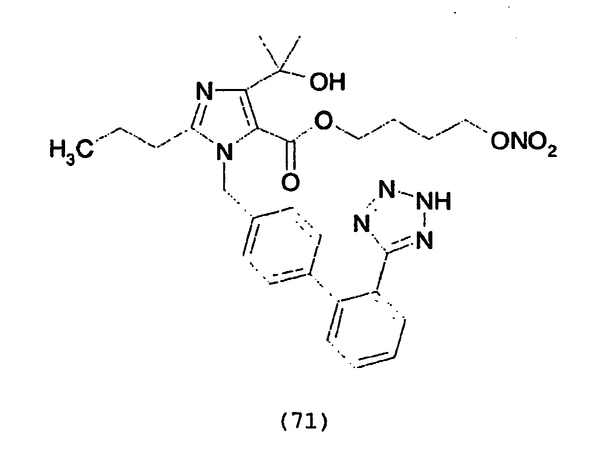

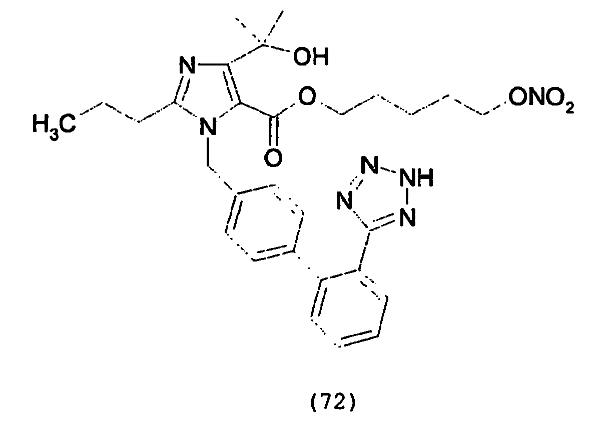

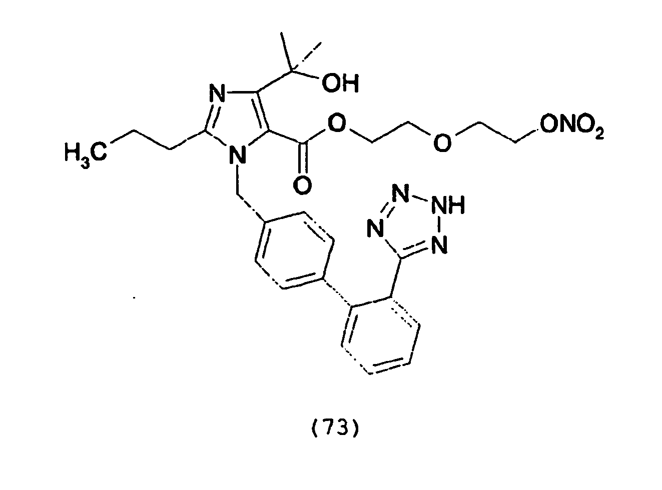

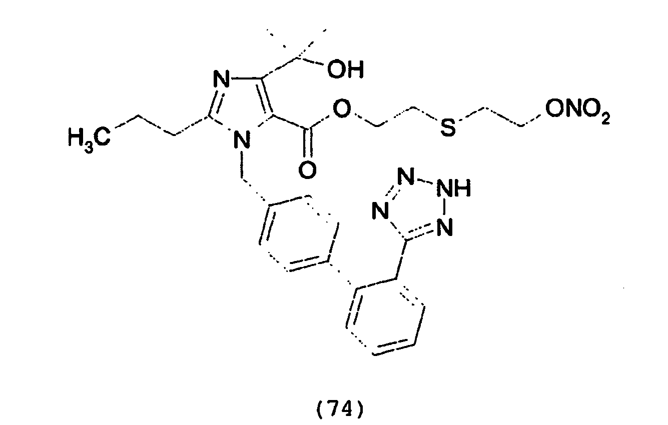

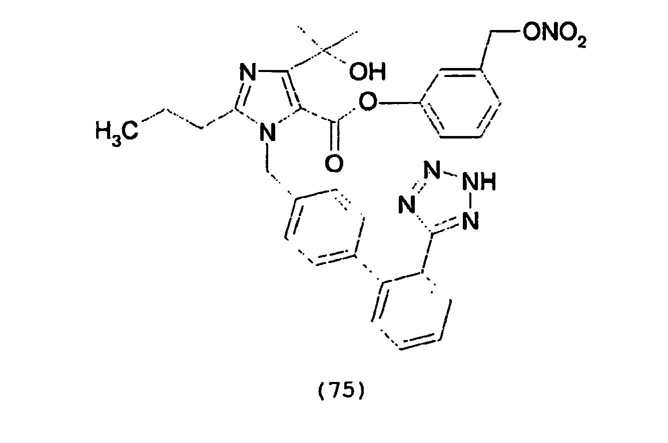

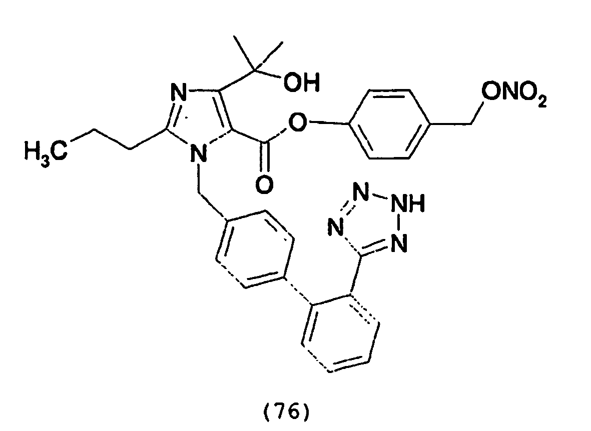

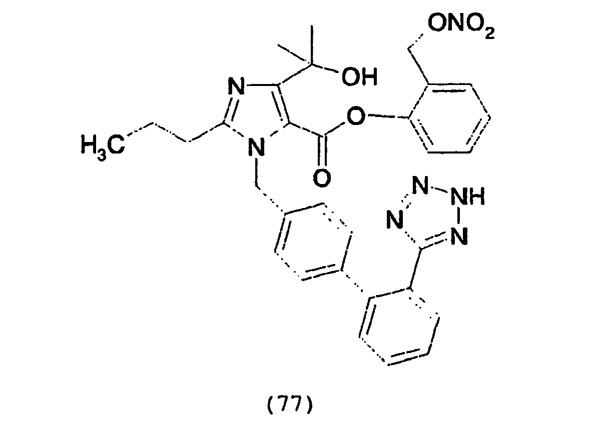

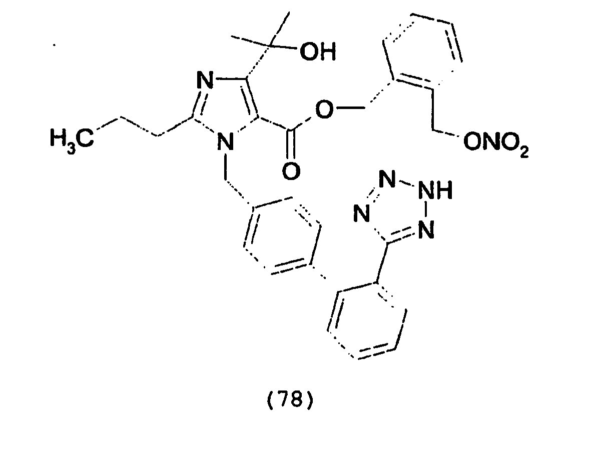

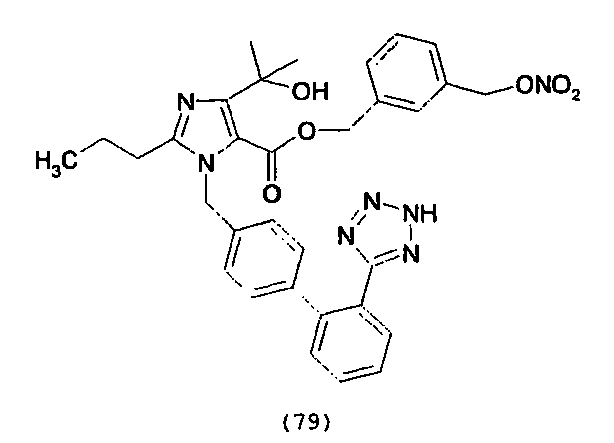

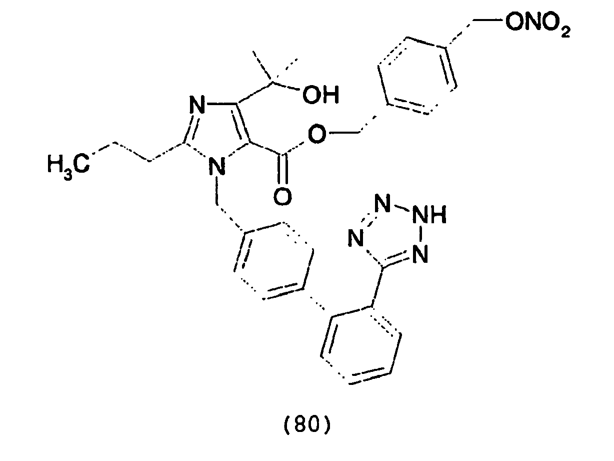

As mentioned above, object of the present invention are also pharmaceutical compositions containing at least a compound of the present invention of formula (I) together with non toxic adiuvants and/or carriers usually employed in the pharmaceutical field.

The daily dose of active ingredient that should be administered can be a single dose or it can be an effective amount divided into several smaller doses that are to be administered throughout the day. Usually, total daily dose may be in amounts preferably from 50 to 500 mg. The dosage regimen and administration frequency for treating the mentioned diseases with the compound of the invention and/or with the pharmaceutical compositions of the present invention will be selected in accordance with a variety of factors, including for example age, body weight, sex and medical condition of the patient as well as severity of the disease, route of administration, pharmacological considerations and eventual concomitant therapy with other drugs. In some instances, dosage levels below or above the aforesaid range and/or more frequent may be adequate, and this logically will be within the judgment of the physician and will depend on the disease state.

The compounds of the invention may be administered orally, parenterally, rectally or topically, by inhalation or aerosol, in formulations eventually containing conventional non-toxic pharmaceutically acceptable carriers, adjuvants and vehicles as desired. Topical administration may also involve the use of transdermal administration such as transdermal patches or iontophoresis devices. The term "parenteral" as used herein, includes subcutaneous injections, intravenous, intramuscular, intrasternal injection or infusion techniques.

Injectable preparations, for example sterile injectable aqueous or oleaginous suspensions may be formulated according to known art using suitable dispersing or wetting agents and suspending agents. The sterile injectable preparation may also be a sterile injectable solution or suspension in a non-toxic parenterally acceptable diluent or solvent. Among the acceptable vehicles and solvents are water, Ringer's solution and isotonic sodium chloride. In addition, sterile, fixed oils are conventionally employed as a solvent or suspending medium. For this purpose any bland fixed oil may be employed including synthetic mono or diglycerides, in addition fatty acids such as oleic acid find use in the preparation of injectables.

Suppositories for rectal administration of the drug can be prepared by mixing the active ingredient with a suitable non-irritating excipient, such as cocoa butter and polyethylene glycols.

Solid dosage forms for oral administration may include capsules, tablets, pills, powders, granules and gels. In such solid dosage forms, the active compound may be admixed with at least one inert diluent such as sucrose, lactose or starch. Such dosage forms may also comprise, as in normal practice, additional substances other than inert diluents, e.g. lubricating agents such as magnesium stearate. In the case of capsules, tablets and pills, the dosage forms may also comprise buffering agents. Tablets and pills can additionally be prepared with enteric coatings.

Liquid dosage forms for oral administration may include pharmaceutically acceptable emulsions, solutions, suspensions, syrups and elixirs containing inert diluents commonly used in the art, such as water. Such compositions may also comprise adjuvants, such as wetting agents, emulsifying and suspending agents, and sweetening, flavouring and the like.
The compounds of the present invention can be synthesized as follows.
A) The compound of general formula (I) or a pharmaceutically acceptable salt, as above defined:

   R-(Y-ONO₂)ₛ (I)

   when R is the residue of formula (II), can be obtained by a process comprising:
   i) reacting a compound of formula (IV):

      R₂-(Y-Hal)ₛ (IV)

      wherein s = 1 and R₂ is the residue of formula (IIA): 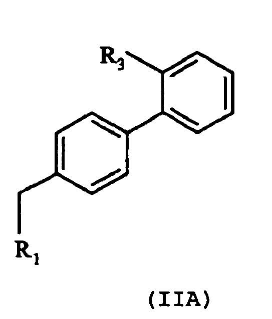 wherein R₃ is the group of formula (VA): 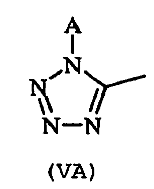 wherein A = H or W, W being a tetrazole protecting group such as trityl, tert-butoxycarbonyl (BOC) and ethyloxycarbonyl or R₃ is -COO-, a group capable to bind Y; R₁ is selected from the groups (IIa)-(IIe), as above defined, wherein No is a group capable to bind Y; Y is as above defined and Hal is an halogen atom preferably Cl, Br or I;
      with AgNO₃ in a suitable organic solvent such as acetonitrile or tetrahydrofuran (THF) under nitrogen in the dark at temperatures range between 20°-80°C; alternatively the reaction with AgNO₃ can be performed under microwave irradiation in solvents such acetonitrile or THF at temperatures in the range between 100-180°C for short time (1-60 min) and
   ii) optionally acid hydrolysing the tetrazole protecting group W, as well known in the art, for example as described in T. W. Greene "Protective groups in organic synthesis", Harvard University Press, 1980 and
   iii) if desired, converting the resulting compound of general formula (I) into a pharmaceutically acceptable salt thereof.

- The compound of formula (IV) can be obtained by reacting a compound of formula (V): 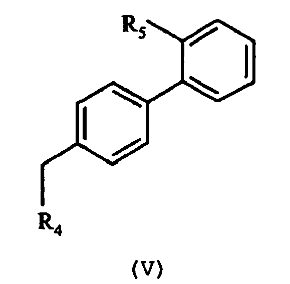 wherein R₅ is the group of formula (VA) as above defined or -COOH and R₄ has the same meaning as R₁ with N₀ = -COOH or -OH,
i.1) when R₅ is the group (VA), R₄ = R₁ and R₁ is the group (IIa) wherein m = 1 and N₀ = -OH, with a compound of formula (VI) or (VII) :

   Hal-Y-COAct (VI)

   Hal-Y-OCOAct (VII)

   wherein Hal and Y are as above defined and Act is Hal or a carboxylic acid activating group used in peptide chemistry as:

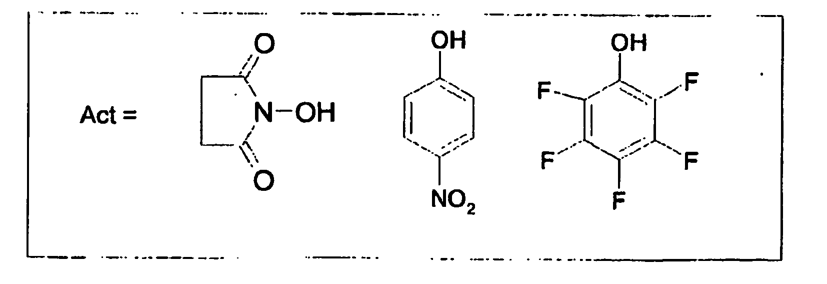

The reaction is generally carried out in presence of a inorganic or organic base in an aprotic polar/non-polar solvent such as DMF, THF or CH₂Cl₂ at temperatures range between 0°-65°C or in a double phase system H₂O/Et₂O at temperatures range between 20°- 40°C;

The compounds of formula (VI) where Act is = Hal are commercially available or can be obtained from the corresponding acids of formula (VIII):

Hal-Y-COOH (VIII)

by well known reactions, for example by reaction with thionyl or oxalyl chloride, halides of P^{III} or P^{V} in solvents inert such as toluene, chloroform, DMF, etc. The corresponding acids are commercially available compounds. The compounds of formula (VI) where Act is not Hal can be obtained from the corresponding compounds of formula (VI) where Act is Hal by reacting with N-Hydroxysuccinimide or with the appropriate substituded phenols in the presence of a base as known in the literature.

The compounds of formula (VII) where Act is = Hal are commercially available or can be obtained from the corresponding alcohols of formula (IX): Hal-Y-OH (IX) by reaction with triphosgene in presence of an organic base; the compounds of formula (VII) where Act is not = Hal can be obtained from the corresponding compound (VII) where Act is Hal by reacting with N-Hydroxysuccinimide or with the appropriate substituded phenols in the presence of a base as known in the literature.

Alternatively, the compound of formula (IV) can be obtained by reacting a compound of formula (V) as defined in i.1), with a compound of formula (VIII), as above defined and commercially available, in presence of a condensing agent like dicyclohexylcarbodiimide (DCC), EDAC in the presence of a catalytic amount of DMAP or activating agent as N,N'-carbonyldiimidazole (CDI) in solvent such as DMF, THF, chloroform at a temperature in the range from -5°C to 50°C; i.2) when R₅ is the group (VA) or -COOH, R₄ = R₁ and R₁ is selected from the groups (IIa) - (IId) wherein m = 0 and No = -COOH, with a compound of formula (IX), as above defined, in presence of a condensing agent like dicyclohexylcarbodiimide (DCC), EDAC in the presence of a catalytic amount of DMAP or activating the carboxylic group with agent as N,N'-carbonyldiimidazole(CDI) in solvent such as DMF, THF, chloroform at a temperature in the range from -5°C to 50°C.

The compounds of formula (IX) are commercially available.

Alternatively, transforming the group -COOH into an activated acyl chloride or into another group suitable for esterification, according to methods well known in the literature, and carrying out the esterification in presence of a organic or inorganic base in an aprotic polar/non-polar solvent such as DMF, THF or CH₂Cl₂ at temperatures range between 0°-65°C or in a double phase system H₂O/Et₂O at temperatures range between 20°- 40°C;
Al) Alternatively, the compounds of formula (I) as above defined, when R is the residue of formula (II), can be obtained by reacting compounds of formula (V) as above defined:
   i.1.1) when R₅ is the group (VA), R_{4 =} R₁ and R₁ is the group (IIa) wherein m = 1 and No = -OH, with a compound of formula (X) :

      O₂NO-Y-COZ (X)
   where Y is as previously defined and Z is OH or the group Act already defined, with the best suitable synthetical path, for example in presence of a condensing agent like dicyclohexylcarbodiimide (DCC)or EDAC or activating with N,N'-carbonyldiimidazole(CDI) in solvent such as DMF, THF, chloroform at a temperature in the range from -5°C to 50°C and/or in the presence of a organic or inorganic base. The compounds of formula (X) can be obtained from the corresponding alcohols by reaction with nitric acid and acetic anhydride in a temperature range from -50°C to 0°C or reacting the corresponding halogen derivatives of formula (VI) or (VIII) with AgNO₃ as already described. i.2.1) when R₅ is the group (VA) or -COOH, R₄ = R₁ and R₁ is selected from the groups (IIa)-(IId) wherein m = 0 and No = -COOH, with a compound of formula (XI):

   O₂NO-Y-O (XI)

   wherein Y is as above defined; in presence of a condensing agent like dicyclohexylcarbodiimide (DCC) or EDAC or an activating agent as N,N'-carbonyldiimidazole(CDI) in solvent such as DMF, THF, chloroform at a temperature in the range from -5°C to 50°C.

The compound of formula (XI) can be obtained by reacting a compound of formula (IX) with AgNO₃ in a suitable organic solvent such as acetonitrile or THF under nitrogen at temperatures range between 20°-80°C;
alternatively the reaction with AgNO₃ can be performed under microwave irradiation in solvents such acetonitrile or THF at temperatures in the range between 100-180°C for short time (1-60 min).

Alternatively when R₅ is the group (VA) or -COOH, R₄ = R₁ and R₁. is selected from the groups (IIa)-(IIe) wherein m = 0 and No = -COOH, with a compound of formula (XI.1):

O₂NO-Y-Hal (XI.1)

where Y and Hal are as previously defined by reacting in the presence of an inorganic or organic base able to salify the carboxylic group.
B) The compound of general formula (I), when R is the residue of formula (III), can be obtained by reacting a compound of formula (XII):

   R₆-(Y-Hal)ₛ (XII)

   wherein s = 2, R₆ is the residue (III) and N₁ is -COO-, Y and Hal are as above defined, with AgNO₃ as already described.

Compounds of formula (XII) are obtained by reacting a compound of formula (XIII): 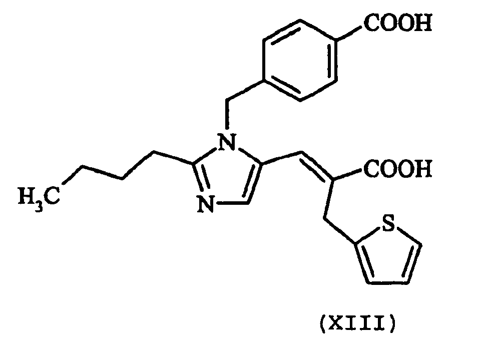 with compounds of formula (IX), as above defined, in presence of a condensing agent like dicyclohexylcarbodiimide (DCC) or EDAC or an activating agent as N,N'-carbonyldiimidazole(CDI) in solvent such as DMF, THF, chloroform at a temperature in the range from - 5°C to 50°C as already described.

Alternatively, transforming the group -COOH into an activated acyl chloride or into another group suitable for esterification, according to methods well known in the literature, and carrying out the esterification in presence of a organic or inorganic base in an aprotic polar/non-polar solvent such as THF or CH₂Cl₂ at a temperature in the range between 0°-65°C or in a double phase system.
B1) Alternatively, the compounds of general formula (I) as above defined, when R is the residue of formula (III), can be obtained by reacting the compound of formula (XIII) with a compound of formula (XI), as above defined, in presence of a condensing or activating agent as already described.

Alternatively, transforming the group -COOH into a salt with an inorganic or organic base according to methods well known in the literature, and reacting with:

O₂NO-Y-Hal (XI.1)

as known in the literature.
C) The compounds of formula (I), as above defined, when s = 1 and R is the residue of formula (II), wherein R₀ is the tetrazole group and R₁ is the group (IIa) wherein m = 1 and No is 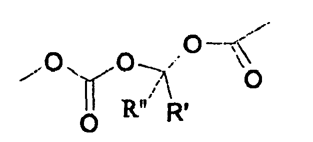
wherein R' and R" are as above defined, can be obtained by reacting a compound of formula (IVa):

R₂-(CR'R"-Hal)ₛ (IVa)

wherein s =1, R₂ and Hal are as above defined, R₃ is the group (VA), R₁ is the group (IIa) wherein m = 1 and N₀ is -OCOO-,
with a compound of formula (X) as above defined, in presence of an organic or inorganic base in a polar solvent as DMF, THF, acetonitrile at a temperature in the range from -5°C to 60°C or in a double phase system as already known in the literature.

The compounds (IVa) can be obtained by reacting a compound of formula (V) as above defined, wherein R₅ is the group (VA), R₄ = R₁ and R₁ is the group (IIa) wherein m = 1 and N₀ = -OH, with a compound of formula (VIIa):

Hal-CR'R"-OCOAct x (IVa)

where Act as the same meaning above described for (VII), in the same manner already described for the compounds (IV); and optionally acid hydrolysing the tetrazole protecting group as above described.
D) The compounds of formula (I), as above defined, when s =1 and R is the residue of formula (II), wherein R₀ is the tetrazole group and R₁ selected from the groups (IIa)-(IIc) wherein m = 0 and N₀ is 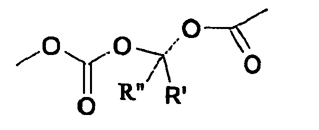
wherein R' and R" are as above defined, can be obtained by reacting a compound of formula (V), wherein R₅ is the group (VA), R₄ = R₁ and R₁ is the group (IIc) wherein N₀ = -COOH, with a compound of formula (XIV):

Hal-CR'R"-OCOO-Y-ONO₂ (XIV)

wherein Hal, Y, R' and R" are as above defined, in presence of an organic or inorganic base in a polar solvent as DMF, THF, acetonitrile at a temperature in the range from -5°C to 60°C or in a double phase system as already known in the literature.

Compounds of formula (XIV) can be obtained by reacting compounds (XI) with compounds (VIIa) as above defined.

The reaction is generally carried out in presence of a base in an aprotic polar/non-polar solvent such as THF or CH₂Cl₂ at temperatures range between 0°-65°C or in a double phase system H₂O/Et₂O at temperatures range between 20°- 40°C; and optionally acid hydrolysing the tetrazole protecting group as above described.
E) the compounds of formula (I), as above defined, when s = 1 and R is the residue of formula (II), wherein R₀ is the tetrazole group and R₁ is selected from the groups (IIa)-(IIc) can also be obtained reacting compound of formula (XV) with a compound of formula (XVI) commercially available: 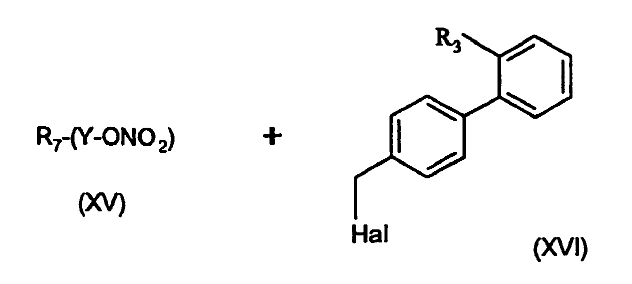
where R₇ is the residue (IIa) - (IIc), R₃ is the group (VA) and Hal is as already defined. The reaction is generally carried out in presence of a base in an aprotic polar/non-polar solvent such as DMF, THF or CH₂Cl₂ at temperatures range between -15°-+80°C or in a double phase system H₂O/Et₂O at temperatures range between 20°- 40°C; and eventually acid hydrolysing the tetrazole protecting group as above described.

Compounds of formula (XV) can be obtained by reacting compounds of formula (XVII):

R₈-(Y-Hal) (XVII)

wherein R₈ is the residue of formula (IIa.1), (IIb.1) or (IIc.1) : 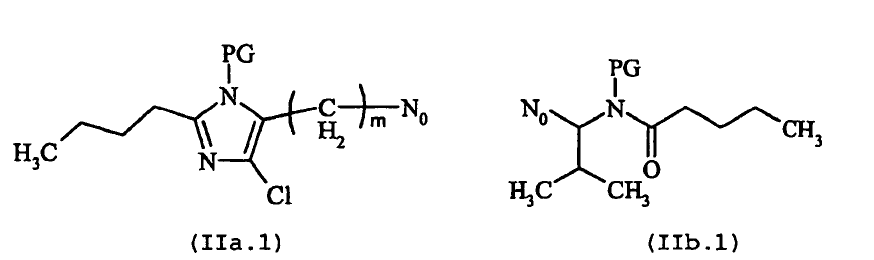 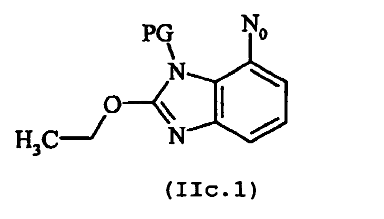 wherein PG is a N-protecting group such as BOC or trityl, with AgNO₃ as already described and optionally hydrolysing the N-protective group.

Compounds (XVII) where R₈ is (IIa.1) wherein m =1 and N₀ = -OCO- can be obtained from the corresponding alcohols by reaction with a compound of formula (VI) or (VII) as already described.

The alcohols above defined, are obtained by known protection and reduction reactions from commercially available compounds of formula (IIa.2): 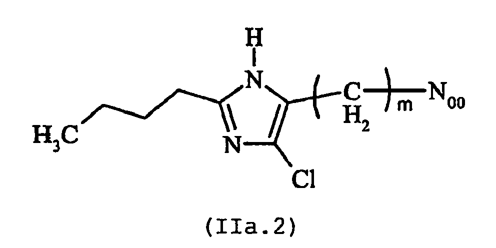 wherein m is 0 and N₀₀ is -CHO.

Compounds (XVII) where R₈ is (IIa.1) with m = 0 and N₀ = -COO- or R₈ is (IIb.1) or (IIc.1) with N₀ = -COO- can be obtained from the corresponding acids by reaction with compounds of formula (IX) as already described.

The corresponding acids of (IIa.1) above defined, are obtained from compounds (IIa.2) wherein m is 0 and Nₒₒ is -CHO by known protection and oxidation reactions.

The corresponding acids of (IIb.1) and (IIc.1) above defined, are obtained from commercially available (IIb.2) and (IIc.2): 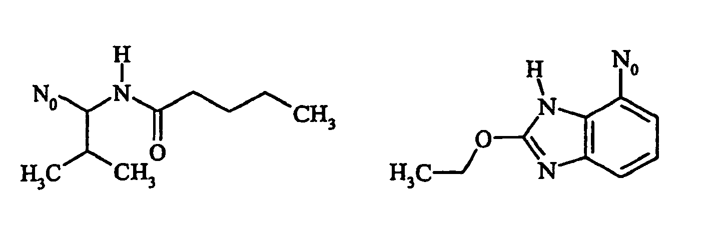 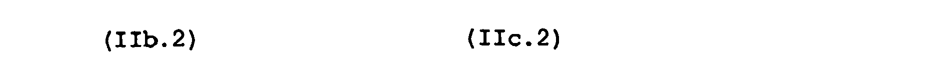 wherein N₀ is -COOH by known protection reations.

The following examples are to further illustrate the invention without limiting it.

### Example 1

### 2-butyl-4-chloro-1-[[2'-(1H-tetrazol-5-yl)[1,1'-biphenyl]-4-yl]methyl]-1H-imidazole-5-methanol 4-(nitrooxymethyl) benzoic acid ester (corresponding to compound (4))

Triphenylmethyl chloride (4.68 g, 16.8 mmol) was added in portions to a solution of Losartan potassium salt (7.0 g; 15.2 mmol) in THF (150 ml). The resulting mixture was stirred at room temperature for 24 hours. Then the reaction was adsorbed on silica gel and purified by flash chromatography (n-Hexane/AcOEt 6:4) affording **2-butyl-4-chloro-1-[[2'-(1-triphenylmethyltetrazol-5-yl)[1,1'-biphenyl]-4-yl]methyl]-1H-imidazole-5-methanol** (6.7 g, 66%).
From this compound the title compound **(4)** can be achieved through two different synthetic procedure:

### Synthetic procedure A

To a solution of 2-butyl-4-chloro-1-[[2'-(1-triphenylmethyltetrazol-5-yl)[1,1'-biphenyl]-4-yl]methyl]-1H-imidazole-5-methanol (1.7 g, 2.6 mmol), 4-(nitrooxymethyl)benzoic acid (0.66 g, 3.38 mmol) and *N,N-*dimethylaminopyridine (0.049 g, 0.4 mmol) in CH₂Cl₂ (20 ml) and THF (6 ml) cooled to 0° C, a solution of dicyclohexylcarbodiimide (0.722 g, 3.50 mmol) in CH₂Cl₂ (5 ml) was slowly added and the reaction was stirred at room temperature for 24 hours. Then the formed dicyclohexylurea was filtered off, and the organic phase was concentrated. The crude material was purified by silica gel chromatography (n-Hexane/AcOEt 75:25) affording **2-butyl-4-chloro-1-[[2'-(1-triphenylmethyltetrazol-5-y1)[1,1'-biphenyl]-9-yl]methyl]-1H-imidazole-5-methanol 4-(nitrooxymethyl)benzoic acid ester** (1.2 g, 55%) as a white solid.

2-butyl-4-chloro-1-[[2'-(1-triphenylmethyltetrazol-5-yl) [1,1'-biphenyl]-4-yl]methyl]-1H-imidazole-5-methanol 4-(nitrooxymethyl)benzoic acid ester (1.2 g, 1.42 mmol) was dissolved in CH₂Cl₂ (10 ml) and HCl was bubbled into the solution for 20 min. The mixture was the then concentrated and purified by flash chromatography (CH₂Cl₂/Acetone 8:2 then Acetone) affording a crude compound that was dissolved in H₂O/CH₃CN and freeze-dried affording **2-butyl-4-chloro-1-[[2'-(1H-tetrazol-5-yl)[1,1-biphenyl]-4-yl]methyl]-1H-imidazole-5-methanol 4-(nitrooxymethyl)benzoic acid ester** as a white solid (0.304 g, 36 %).
¹H-NMR (DMSO-*d*₆) : 7.73-7.56 (7H,m); 7.24 (1H,d); 7.00 (4H,m); 5.60 (2H,s); 5.39(2H,s) ; 5.28(2H,s); 2.61(2H,t); 1.53 (2H,m); *1.28(2H,m) ; 0.82(3H,t).

### Synthetic procedure B

To a solution of 2-butyl-4-chloro-1-[[2'-(1-triphenylmethyltetrazol-5-yl)[1,1'-biphenyl]-4-yl]rnethyl]-1H-imidazole-5-methanol (1.7 g, 2.6 mmol), 4-(chloromethyl)benzoic acid (0.571 g, 3.35 mmol) and *N,N-*dimethylaminopyridine (0.049 g, 0.4 mmol) in CH₂Cl₂ (20 ml) and THF (6 ml) cooled to 0 °C, dicyclohexylcarbodiimide (0.644 g, 3.12 mmol) was slowly added and the reaction was stirred at room temperature for 24 hours. Then the formed dicyclohexylurea was filtered off, and the organic phase was concentrated. The crude material was purified by flash chromatography (n-Hexane/AcOEt 75:25) affording **2-butyl-4-chloro-1-[[2'-(1-triphenylmethyltetrazol-5-yl)[1,1'-biphenyl]-4-yl]methyl]-1H-imidazole-5-methanol 4-(chloromethyl)benzoic acid ester** (1.56 g, yield 73%).

2-butyl-4-chloro-1-[[2'-(1-triphenylmethyltetrazol-5-yl)[1,1'-biphenyl]-4-yl]methyl]-1H-imidazole-5-methanol 4-(chloromethyl)benzoic acid ester (0.807 g, 0.98 mmol) was dissolved in CH₃CN (15 ml) and AgNO₃ (0.305 g, 1.8 mmol) was added, in the dark and under nitrogen. The mixture was stirred at 60 °C for 6 hours. Then the precipitated silver salts were filtered off and the organic phase was diluted with ACOEt and washed with NaH₂PO₄ (5%, 2 x 10 ml) and brine (2 x 10 ml), dried over Na₂SO₄ and concentrated. The crude material was purified by flash chromatography (n-Hexane/AcOEt 75:25) affording **2-butyl-4-chloro-1-[[2'-(1-triphenylmethyltetrazol-5-yl)[1,1'-biphenyl]-4-yl]methyl]-1H-imidazole-5-methanol 4-(nitrooxymethyl)benzoic acid ester** (0.553 g, 66%).

From 2-butyl-4-chloro-1-[[2'-(1-triphenylmethyltetrazol-5-yl)[1,1'-biphenyl]-4-yl]methyl]-1H-imidazole-5-methanol 4-(nitrooxymethyl)benzoic acid ester the title compound **2-butyl-4-chloro-1-[[2'-(1H-tetrazol-5-yl)[1,1'-biphenyl]-4-yl]methyl]-1H-imidazole-5-methanol 4-(nitrooxymethyl)benzoic acid ester** was obtained by acid hydrolysis as described in Procedure A.

### Example 2

### 2-butyl-4-chloro-1-[[2'-(1H-tetrazol-5-yl)[1,1'-biphenyl]-4-yl]methyl]-1H-imidazole-5-methanol 4-nitrooxybutanoic acid ester (corresponding to compound (2))

This compound can be achieved through four different synthetic procedure:

### Synthetic procedure A

To a solution of 2-butyl-4-chloro-1-[(2'-(1-triphenylmethyltetrazol-5-yl)[1,1'-biphenyl]-4-yl]methyl]-1H-imidazole-5-methanol (1.7 g, 2.6 mmol) (obtained in Example 1), 4-nitrooxybutanoic acid (0.536 g, 3.6 mmol) and

N,N-dimethylaminopyridine (0.05 g, 0.4 mmol) in CH₂Cl₂ (20 ml) and THF (6 ml) cooled to 0° C, a solution of dicyclohexylcarbodiimide (DCC) (0.722 g, 3.50 mmol) in CH₂Cl₂ (5 ml) was slowly added and the reaction was stirred at room temperature for 24 hours. Then the formed dicyclohexylurea was filtered off, and the organic phase was concentrated. The crude material was purified by flash chromatography (n-Hexane/EtOAc 7:3) affording **2-butyl-4-chloro-1-[[2'-(1-triphenylmethyltetrazol-5-yl)[1,1'-biphenyl]-4-yl]methyl]-1H-imidazole-5-methanol 4-nitrooxybutanoic acid ester** (1.45 g, 70%).

2-butyl-4-chloro-1-[[2'-(1-triphenylmethyltetrazol-5-yl)[1,1'-biphenyl]-4-yl]methyl]-1H-imidazole-5-methanol 4-nitrooxybutanoic acid ester (1.0 g, 1.25 mmol) was dissolved in CH₂Cl₂ (20 ml) and HCl was bubbled into the solution for 20 min. The reaction was then concentrated and purified by flash chromatography (CH₂Cl_{2/} Acetone 8:2 then Acetone) affording crude compound as a white foam. That was dissolved in H₂O/CH₃CN and freeze dried to give **2-butyl-4-chloro-1-[[2'-(1H-tetrazol-5-yl)[1,1'-biphenyl]-4-yl] methyl]-1H-imidazole-5-methanol 4-nitrooxybutanoic acid ester** (0.507 g, yield 71%)as a white solid.
¹H-NMR (DMSO-*d*₆): 7.66 (2H, d); 7.57 (1H,d); 7.49 (1H,d); 7.09 (2H,d); 6.95 (2H,d); 5.25 (2H,s); 4.99 (2H,s); 4.49 (2H,t); 2.54 (2H,t); 2.01 (2H,t); 1.60 (2H,m); 1.49 (2H,m); 1.32 (4H,m); 0.84 (3H,t).

### Synthetic procedure B

To a solution of 2-butyl-4-chloro-1-[[2'-(1-triphenylmethyltetrazol-5-yl)[1,1'-biphenyl]-4-yl]methyl]-1H-imidazole-5-methanol (obtained in Example 1) (1.7 g, 2.6 mmol), 4-bromobutanoic acid (0.561 g, 3.36 mmol) and N,N-dimethylaminopyridine (0.05 g, 0.4 mmol) in CH2C12 (20 ml) and THF (6 ml) cooled to 0° C, a solution of dicyclohexylcarbodiimide (0.722 g, 3.50 mmol) in CH2Cl2 (5 ml) was slowly added and the reaction was stirred at room temperature for 24 hours. Then the formed dicyclohexylurea was filtered off, and the organic phase was concentrated. The crude material was purified by silica gel chromatography (n-Hexane/ETOAc 75: 25) affording **2-butyl-4-chloro-1-[[2'-(1-triphenylmethyltetrazol-5-yl)[1,1'-biphenyl]-4-yl]methyl]-1H-imidazole-5-methanol 4-bromobutanoic acid ester** (1.27 g, yield 60%).

2-butyl-4-chloro-1-[[2'-(1-triphenylmethyltetrazol-5-yl)[1,1'-biphenyl]-4-yl]methyl]-1H-imidazole-5-methanol 4-bromobutanoic acid ester (1.2 g, 1.47 mmol) was dissolved in CH3CN (20 ml) and AgN03 (0.475 g, 2.8 mmol) was added in the dark and under nitrogen. The mixture was stirred at 60° C for 8 hours. Then it was partitioned between EtOAc and phosphate buffer (pH=3, 40 ml). The organic phase was washed with phosphate buffer (pH=3, 2 x 25 ml), brine, (3 x 25 ml), dried over Na2SO4 and concentrated. The crude material was purified by flash chromatography (n-Hexane/AcOEt 7:3) affording **2-butyl-4-chloro-1-[[2'-(1-triphenylmethyltetrazol-5-yl)[1,1'-biphenyl]-4-yl]methyl]-1H-imidazole-5-methanol 4-nitrooxybutanoic acid ester** (0.819 g, yield 70%) as a foam.

From 2-butyl-4-chloro-1-[[2'-(1-triphenylmethyltetrazol-5-yl) [1,1'-biphenyl]-4-yl]methyl]-1H-imidazole-5-methanol 4-nitrooxybutanoic acid ester the title compound **2-butyl-4-chloro-1-[[2'-(1H-tetrazol-5-yl)[1,1'-biphenyl]-9-yl]methyl]-1H-imidazole-5 -methanol 4-nitrooxybutanoic acid ester** was obtained by acid hydrolysis as described in Example 2, Procedure A (0.507 g, 71 %).

### Synthetic procedure C

To a solution of 2-butyl-4-chloro-1-[[2'-(1H-tetrazol-5-yl)[1,1'-biphenyl]-4-yl]methyl]-1H-imidazole-5-methanol (3.6 g, 8.5 mmol), N,N-dimethylaminopyridine (0.1 g, 0.85 mmol) and TEA (1.18 ml, 0.85 mmol) in THF (60 ml) cooled to 0 °C and under nitrogen a solution of 4-bromobutanoyl chloride (0.98 ml, 8.5 mmol) in THF (1 ml) was slowly added and the reaction was stirred at room temperature for 1.5 hours. Then it was partitioned between EtOAc and phosphate buffer (pH=3, 40 ml) and extracted with EtOAc (3 x 15 ml). The organic phase was dried over Na₂SO₄ and concentrated. The crude material was purified by flash chromatography (CH₂Cl₂/Acetone 8:2) affording **2-butyl-9-chloro-1-[[2'-(1H-tetrazol-5-yl)[1,1'-biphenyl]-4-yl]methyl]-1H-imidazole-5-methanol 4-bromobutanoic acid ester** (2.5 g, yield 51%) as a white solid.

2-butyl-4-chloro-1-[[2'-(1H-tetrazol-5-yl)[1,1'-biphenyl]-4-yl]methyl]-1H-imidazole-5-methanol 4-bromobutanoic acid ester (0.56 g, 0.98 mmol) was dissolved in CH₃CN (15 ml) and AgNO₃ (0.83 g, 4.9 mmol) was added in the dark and under nitrogen. The mixture was stirred at 60° C for 8 hours. Then it was cooled and poured into a phosphate buffer solution (pH=3, 40 ml). NaCl solid was added and the mixture was extracted with EtOAc. The organic phase was washed with phosphate buffer (pH=3, 2 x 25 ml), brine, (3 x 25 ml), dried over Na₂SO₄ and concentrated. The crude material was purified by flash chromatography (CH2C12/acetone 8:2 then acetone) affording crude compound as a white foam. That was dissolved in H₂O/CH₃CN and freeze dried to give **2-butyl-4-chloro-1-[[2'-(1H-tetrazol-5-yl) [1,1'-biphenyl]-4-yl]methyl]-1H-imidazole-5-methanol 4-nitrooxybutanoic acid ester** (0.3 g, yield 55%) as a white solid.

### Synthetic procedure D

To a solution of 4-bromobutyric acid (0.91 g, 5.4 mmol), pentafluorophenol (1.00 g, 5.4 mmol) and DMAP (0.13 g, 1.1 mmol) in CH₂Cl₂ (10 ml) cooled to 0 °C under nitrogen, N,N-dicyclohexylcarbodiimide (1.70 g, 8.1 mmol) was added in portions. After 1 h the reaction was slowly warmed to room temperature and stirred for 5 hours. The diciclohexylurea was filtered off and the mother liquor was concentrated and purified by flash chromatography (n-Hexane/EtOAc 98:2) affording **4-bromobutyric acid pentafluorophenyl ester** as a colourless oil (1.40 g, 78%).

A mixture of 4-bromobutyric acid pentafluorophenyl ester (0.65 g, 1.9 mmol) and AgNO₃ (0.83 g, 4.9 mmol) in CH₃CN (8 ml) was warmed at 70 °C for 20 minutes at the microwave. The formed salts were filtered off, the solvent was concentrated and the residue purified by flash chromatography (n-Hexane/EtOAc 95:5) affording **4-nitrooxybutyric acid pentafluorophenyl ester** as a clear oil (0.38 g, 62 %).

To a solution of 2-butyl-4-chloro-1-[[2'-(1H-tetrazol-5-yl)[1,1'-biphenyl]-4-yl]methyl]-1H-imidazole-5-methanol (0.48 g, 1.1 mmol), TEA (0.16 ml, 1.1 mmol) and DMAP (0.14 mg, 1.1 mmol) in DMF (3 ml), cooled to 0 °C, a solution of 4-nitrooxybutyric acid pentafluorophenyl ester (0.36 g, 1.1 mmol) in DMF (3 ml) was added. The reaction was slowly warmed to room temperature and stirred for 3 hours. Then the solvent was evaporated under reduced pressure. The residue was dissolved in EtOAc (10 ml) and washed with a buffer solution (pH=3) then with brine. The organic layer was dried over Na₂SO₄, concentrated and purified by flash chromatography (CH₂Cl₂/ MeOH 98:2) to afford the title compound (0.41 g, 66%).

### Example 3

### 2-butyl-4-chloro-1-[[2'-(1H-tetrazol-5-yl)[1,1'-biphenyl]-4-yl]methyl]-1H-imidazole-5-methanol 11-nitrooxyundecanoic acid ester (corresponding to compound (68))

Using The procedure **A** described in **Example 2** but starting from 2-butyl-4-chloro-1-[[2'-(1-triphenylmethyltetrazol-5-yl)[1,1'-biphenyl]-4-yl]methyl]-1H-imidazole-5-methanol (1.7 g, 2.6 mmol) and 11-nitrooxyundecanoic acid (0.78 g, 3.36 mmol), **2-butyl-4-chloro-1-[[2'-(1-triphenylmethyltetrazol-5-yl)[1,1'-biphenyl]-4-yl]atethyl]-1H-imidazole-5-methanol 11-nitrooxyundecanoic acid ester** (1.65 g, 80%) was obtained.

From acid hydrolysis of this compound (1.6 g, 2.0 mmol) **2-butyl-4-chloro-1-[[2'-(1-triphenylmethyltetrazol-5-yl)[1,1'-biphenyl]-4-yl]methyl]-1H-imidazole-5-methanol 11-nitrooxyundecanoic acid ester** (0.91 g,70%) was obtained after crystallization from Et₂O/n-Hexane. (DMSO): 7.66 (2H, d); 7.57 (1H,d); 7.59 (1H, d); 7.09 (2H, d); 6.95(2H,d); 5.25(2H,s); 4.99(2H,s); 4.49 (2H, t);2 .59(2H,t); 2.01(2H,t); 1.62 (2H,m); 1.49 (2H,m); 1.35-1.14 (16H,m); 0.84(3H,t).

### Example 4

### 2-butyl-4-chloro-1-[[2'-(1H-tetrazol-5-yl)[1,1'-biphenyl]-4-yl]methyl]-1H-imidazole-5-methanol 3-(nitrooxymethyl) benzoic acid ester (corresponding to compound (5))

2-butyl-4-chloro-1-[[2'-(1-triphenylmethyl-tetrazol-5-yl)[1,1'-biphenyl]-4-yl]methyl]-1H-imidazole-5-methanol (Prapared in Example 1) (1.0 g, 1.5 mmol), triethylamine (0.42 ml, 3.0 mmol) and *N,N*-dimethylaminopyridine (36 mg, 0.30 mmol) were dissolved in CH₂Cl₂ (10 ml). Then 3-(chloromethyl)benzoyl chloride (0.24 ml, 1.7 mmol) was added and the reaction was stirred at room temperature for 4 hours. The mixture was diluted with EtOAC (50 ml) and the organic phase was washed with NaH₂PO₄ (5 %, 2 x 25 ml), NaHCO₃ (5 %, 2 x 25 ml), brine (2 x 25 ml), dried over Na₂SO₄ and concentrated. The crude material was purified by flash chromatography (n-Hexane/EtOAC 75:25) affording **2-butyl-4-chloro-1-[[2'-(1-triphenylmethyl-tetrazol-5-yl)[1,1'-biphenyl]-4-yl]methyl]-1H-imidazole-5-methanol 3-(chloromethyl)benzoic acid ester** (1.0 g, 81 %) as an oil.

2-butyl-4-chloro-1-[[2'-(1-triphenylmethyl-tetrazol-5-yl)[1,1'-biphenyl]-4-yl]methyl]-1H-imidazole-5-methanol 3-(chloromethyl)benzoic acid ester (0.66 g, 0.20 mmol) was suspended in CH₃CN (10 ml) and NaI (0.24 g, 1.6 mmol) was added. The reaction was refluxed for 1 hour, then diluted with EtOAc (25 ml). The organic phase was washed with H₂O (3 x 25 ml), dried over NaSO₄ and concentrated. The crude material was dissolved in CH₃CN (4 ml) and AgNO₃ (0.34 g, 2 mmol) was added in the dark and under nitrogen. The reaction was stirred at room temperature for 2 hours, then it was diluted with EtOAC (10 ml). The organic phase was washed with NaH₂PO₄ (5 %, 2 x 10 ml) and brine (2 x 10 ml), dried over NaSO₄ and concentrated. The crude material was purified by flash chromatography (Hexane/EtOAc 75:25), affording **2-butyl-9-chloro-1-[[2'-(1-triphenylmethyltetrazol-5-yl)[1,1'-biphenyl]-4-yl]methyl]-1H-imidazole-5-methanol 3-(nitrooxymethyl)benzoic acid ester** (230 mg, 33 %).

2-butyl-4-chloro-1-[[2'-(1-triphenylmethyl-tetrazol-5-yl)[1,1'-biphenyl]-4-yl]methyl]-1H-imidazole-5-methanol 3-(nitrooxymethyl)benzoic acid ester (0.23 g, 0.27 mmol) was dissolved in CH₂Cl₂ (5 ml) and HCl was bubbled in the solution. 10 minutes later the reaction was concentrated and purified by flash chromatography (CH₂Cl₂/acetone 8:2 and then acetone). The yellow foam obtained was treated over decolorizing carbon, dissolved in H₂O/CH₃CN and freeze-dried affording **2-butyl-4-chloro-1-[[2'-(1H-tetrazol-5-yl)[1,1'-biphenyl]-4-yl]methyl]-1H-imidazole-5-methanol m-nitrobenzylbenzoic acid ester** as a white solid (0.11 g, 63 %).
(CDCl₃) : 7.90 (2H,m); 7.78 (1H,d) ; 7.56 (3H,m) : 7.40 (1H,m) ; 7 .19 (1H,d) ; 7.06 (2H,d); 6.83 (2H,d); 5.40 (2H, s) ; 5.24 (2H,s); 5.14 (2H,s); 2.47 (2H,t); 1.61 (2H,m); 1.32 (2H,m); 0.87 (3H,m).

### Example 5

### 2-butyl-4-chloro-1-[[2'-(1H-tetrazol-5-yl)[1,1'-biphenyl]-4-yl]methyl]-1H-imidazole-5-methanol 6-nitrooxyhexanoic acid ester (corresponding to compound (69)

2-butyl-4-chloro-1-[[2'-(1-triphenylmethyl-tetrazol-5-yl) [1,1'-biphenyl]-4-yl]methyl]-1H-imidazole-5-methanol (prepared in **Example 1**) (2.0 g, 3.0 mmol), 6-bromohexanoic acid (0. 90 g, 4 . 6 mmol), *N,N*-dimethylaminopyridine (38 mg, 0.3 mmol), triethylamine (1.3 ml, 9.3 mmol) were dissolved in CH₂Cl₂ (20 ml) and the solution was cooled to 0°C. Then 1-(3-dimethylaminopropyl)-3-ethylcarbodiimide hydrochloride (EDAC) (0.94 g, 9.3 mmol) was added and the reaction was slowly warmed to room temperature and stirred overnight. The organic phase was washed with NaH₂PO₄ (5 %, 20 ml) and brine (20 ml), dried over Na₂SO₄ and purified by flash chromatography (n-Hexane/EtOAc 7:3) affording **2-butyl-4-chloro-1-[[2'-(1-triphenylmethyl-tetrazol-5-yl)[1,1'-biphenyl]-4-yl]methyl]-1H-imidazole-5-methanol 6-bromohexanoic acid ester** as an oil (1.94 g, 76 %).

2-butyl-4-chloro-1-[[2'-(1-triphenylmethyl-tetrazol-5-yl)[1,1'-biphenyl]-4-yl]methyl]-1H-imidazole-5-methanol 6-bromohexanoic acid ester (0.77 g, 0.90 mmol) and NaI (0.30 g, 2.0 mmol) were dissolved in CH₃CN (10 ml) and the mixture was refluxed for 1 hour. Then it was diluted with EtOAc (50 ml) and the organic phase was washed with H₂O (2 x 25 ml), dried over Na₂SO₄ and concentrated. The crude was suspended in CH₃CN (7 ml) and AgNO₃ (0.60 g, 3.5 mmol) was added. The reaction was stirred at room temperature, in the dark and under nitrogen, for 3 hours. Then it was partitioned between EtOAc. (30 ml) and phosphate buffer (pH=3, 25 ml). The organic phase was washed with phosphate buffer (pH=3, 2 x 25 ml) and brine (3 x 25 ml), dried over Na₂SO₄ and concentrated. The crude material was purified by flash chromatography (n-Hexane/EtOAc 7:3) affording **2-butyl-4-chloro-1-[[2'-(1-triphenylmethyl-tetrazol-5-yl)[1,1'-biphenyl]-4-yl]methyl]-1H-imidazole-5-methanol 6-nitrooxyhexanoic acid ester** as a foam (0.69 g, 64 %).

2-butyl-4-chloro-1-[[2'-(1-triphenylmethyl-tetrazol-5-yl)[1,1'-biphenyl]-4-yl]methyl]-1H-imidazole-5-methanol 6-nitrooxyhexanoic acid ester (0.88 g) was dissolved in CH₂Cl₂ (20 ml) and HCl was bubbled into the solution for 20 minutes. The mixture was then concentrated and purified by flash chromatography (CH₂Cl₂/acetone 8:2 and then acetone) affording the product as a yellow foam. That was treated with decolorizing carbon, dissolved in H₂O/CH₃CN and freeze-dried to give product **2-butyl-4-chloro-1-[[2'-(1H-tetrazol-5-yl)[1,1'-biphenyl]-4-yl]methyl]-1H-imidazole-5-methanol 6-nitrooxyhexanoic acid ester** as a white solid (0.41 g, 68 %).
(CDCl₃): 7.79 (1H, d); 7.63-7.49 (2H, m); 7.41 (1H, d); 7.08 (2H, d); 6.77 (2H, d); 5.14 (2H, s); 4.88 (2H, s); 4.38 (2H, t); 2.38 (2H, t); 2.06 (2H, m); 1.70-1.50 (6H, m); 1.37-1.30 (4H, m); 0.85 (3H, t).

### Example 6

### 2-butyl-4-chloro-1-[[2'-(1H-tetrazol-5-yl)[1,1'-biphenyl]-4-yl]methyl]-1H-imidazole-5-carboxylic acid (3-nitrooxy)propyl ester (corresponding to compound (7))

To a solution of 2-butyl-4-chloro-5-formyl imidazole (1.2 g, 6.4 mmol) in t-ButOH (35 ml) and 5% aqueous Na₂HPO₄ solution (25 ml), a solution of KMnO₄ (6.1 g, 38.6 mmol)in water (40 ml) was added. After 6 minutes at room temperature, the mixture was quenched by addition of 40% aqueous NaHSO₃ solution. The suspension was filtered, washed with H₂O and the filtrate was freeze-dried. The residue was taken up with H₂O (50 ml) acidified to pH 2.5 with HCl 3N and extracted with EtOAc (3 ×70 ml). The combined organic extracts were dried over Na₂SO₄ and evaporated to dryness to give **2-butyl-4-chloro-imidazole 5-carboxylic acid** (1.07 g, 83%) as a white solid.

To a solution of 2-butyl-4-chloro-imidazole 5-carboxylic acid (0.61 g, 3 mmol), 3-bromopropanol (0.52 g, 3.74 mmol ) and N,N-dimethylaminopyridine (0.08 g, 0.65 mmol) in THF (12 ml) cooled to 0° C, dicyclohexylcarbodiimide (0.91 g, 4.4 mmol) was slowly added in portions and the reaction was stirred at room temperature for 4 hours. Then the formed dicyclohexylurea was filtered off, and the organic phase was concentrated. The crude material was purified by silica gel chromatography (n-Hexane/AcOEt 8:2) affording **2-butyl-4-chloro-imidazole 5-carboxylic acid 3-bromopropyl ester** (0.5 g, 50%) as a white foam.

2-butyl-4-chloro-imidazole 5-carboxylic acid 3-bromopropyl ester (0.807 g, 2.47 mmol) was dissolved in CH₃CN (15 ml) and AgNO₃ (0.63 g, 3.7 mmol) was added. The mixture was stirred at room temperature for 8 h. Then the precipitated silver salts were filtered off and the organic phase was diluted with ACOEt and washed with NaH₂PO₄ (5%, 2 x 10 ml) and brine (2 x 10 ml), dried over Na₂SO₄ and concentrated. The crude material was purified by flash chromatography (n-Hexane/AcOEt 70:30) affording **2-butyl-4-chloro-imidazole 5-carboxylic acid 3-nitrooxypropyl ester** (0.377 g, 50%).

To a solution of 2-butyl-4-chloro-imidazole 5-carboxylic acid 3-nitrooxypropyl ester (0.76 g, 2.5 mmol) in dimethylacetamide (DMA) (13 ml) cooled to 0 °C and under nitrogen, potassium tert-butylate (0.28 g, 2.5 mmol) was slowly added in portions. After 10 min stirring a solution of N-(triphenylmethyl)-5-(4'-bromomethylbiphenyl-2-yl-)tetrazole (1.7 g, 3 mmol) in DMA (10 ml) was added and the mixture was stirred at room temperature for 1 h. Then the mixture was partitioned between water and EtOAc. The organic phase was separated, dried over Na₂SO₄ and concentrated. The crude material was purified by flash chromatography (n-Hexane/EtOAc 7:3) **affording 2-butyl-4-chloro-1-[[2'-(1-triphenylmethyltetrazol-5-yl)[1,1'-biphenyl]-4-yl]methyl]-1H-imidazole-5-carboxylic acid 3-nitrooxypropyl ester** (1.56 g, 80%).

From 2-butyl-4-chloro-1-[[2'-(1-triphenylmethyltetrazol-5-yl)[1,1'-biphenyl]-4-yl]methyl]-1H-imidazole-5-carboxylic acid 3-nitrooxypropyl ester ( 1 g, 1.28 mmol) the title compound (white solid) was achieved through acid hydrolysis as described for analogous compound in **Example 1** procedure A (0.28 g, 40%).
¹H-NMR (DMSO-*d*₆) : 7.60-7.20 (4H,m); 7.12 (2H, d) ; 6.92 (2H,d) ; 5.72 (2H, s) ; 4.58 (2H, t) ; 4.50 (2H,t) ; 2.54 (2H,t); 2.31 (2H,m) ; 1.49 (2H,m) ; 1.32 (2H,m) ; 0.84 (3H,t). **Studies on vascular tone**

The ability of the nitroderivatives of ARB to induce vasorelaxation in comparison to native ARB, was tested in vitro in isolated rabbit thoracic aorta preparations (Wanstall J.C. et al., Br. J. Pharmacol., 134:463-472, 2001). Male New Zealand rabbits were anaesthetized with thiopental-Na (50 mg/kg, iv), sacrificed by exsanguinations and then the thorax was opened and the aorta dissected. Aortic ring preparations (4 mm in length) were set up in physiological salt solution (PSS) at 37°C in small organ chambers (5 ml). The composition of PSS was (mM): NaCl 130, NaHCO₃14.9, KH₂PO₄ 1.2, MgSO₄ 1.2, HEPES 10, CaCl₂, ascorbic acid 170 and glucose 1.1 (95% O₂ /5% CO₂ ; pH 7.4). Each ring was mounted under 2 g passive tension. Isometric tension was recorded with a Grass transducer (Grass FT03) attached to a BIOPAC MP150 System. Preparations were allowed to equilibrate for 1h, and then contracted submaximally with noradrenaline (NA, 1 µM) and, when the contraction was stable, acetylcholine (ACh, 10 µM) was added. A relaxant response to ACh indicated the presence of a functional endothelium. Vessels that were unable to contract NA or showed no relaxation to Ach were discarded. When a stable precontraction was reached, a cumulative concentration-response curve to either of the vasorelaxant agents was obtained in the presence of a functional endothelium. Each arterial ring was exposed to only one combination of inhibitor and vasorelaxant. Moreover, the effect of the soluble guanylyl cyclase inhibitor ODQ (1-H-(1,2,9)-oxadiazol(4,3-a)quinoxalin-1-one) on vasorelaxation elicited by the compounds was examined preincubating the aortic rings with ODQ (10 µM) for 20 min.

Responses to relaxing agents are expressed as a percentage of residual contraction and plotted against concentration of test compound. IC₅₀ values (where IC₅₀ is the concentration producing 50% of the maximum relaxation to the test compound) were interpolated from these plots.

During the experimental period, the plateau obtained with NA was stable without significant spontaneous loss of contraction in the aortic rings. Under these experimental conditions, the ARB losartan, did not produce relaxation at any of the concentration tested, the curve being not different from that built up in the presence of vehicle alone.

As shown in Table 1, the nitroderivatives of the invention were able to induce relaxation in a concentration-dependent manner. Furthermore, in experiments performed in the presence of ODQ (10 µM), the vasorelaxant responses to tested compounds were inhibited.

**Table 1**

| **Compound** | **IC₅₀ (µM)±** sem |
|---|---|
| Losartan | no effect up to 100 µM |
| Compound of **EX.1** | 33 ± 12 |
| Compound of **EX.2** | 15 ± 3 |
| Compound of **EX.4** | 54 ± 16 |
| Compound of **EX.5** | 18 ± 6 |

| | |
|---|---|
| **IC₅₀** is the concentration which inhibits 50% of the response. | |

### Effect of losartan nitroderivative on inflammatory pathways in vitro

The experiments were performed using RAW 264.7 monocyte macrophage cell line. Cells were stimulated in the presence of lipopolysaccharide (LPS) (1 pg/ml) for 16 hrs. At the end of the incubation, the culture media were collected and analyzed for nitrite content using a standard Griess reaction.

The results reported in Table 2 are expressed as % of nitrite content for each treatment vs. LPS-treated samples.

**Table 2**

| Study of inhibition of LPS-induced nitrite accumulation in RAW 264.7 macrophages | | |
|---|---|---|
| **Compound** | **Concentration (µM)** | **Nitrite (% *vs* vehicle)** |
| Losartan | 25 | 99± 9 |
| Compound of **EX.4** | 25 | 61± 3 |

As shown in Table 2, differently from the parent compound, the nitroderivative (compound of Ex.4) was able to inhibit the accumulation of nitrites induced by LPS.

### Study of antiplatelet activity of losartan nitroderivatives in vitro

The ability of losartan nitroderivatives to inhibit platelet aggregation was evaluated *in vitro* in human platelets. Platelet aggregation was measured in 0.25 ml platelet reach plasma (PRP) samples according to Born method (Gresele P, Arnout J, Deckmyn H, et al., J Clin Invest. 1987;80:1435-45). Aggregating agent used was U46619, a TxA₂ analog, based on the evidence that this agonist is sensitive to the effects of nitric oxide. Compounds were incubated at 37°C for 2 min before adding the aggregating agent. Aggregation was followed for 5 min and the maximal amplitude (cm) was measured. DMSO (0.05% final concentration) was used as vehicle. Compounds were tested at concentrations ranging from 10 to 100µM.

**Table 3**

| Study of antiplatelet activity of losartan nitroderivatives vs losartan in human platelets | |
|---|---|
| **Compound** | **Platelet aggregation (PRP)** (incubation time: 2 min) IC₅₀ µM |
| Losartan | 33 |
| Compound of **EX.1** | 5 |
| Compound of **EX.2** | 11 |

As shown in Table 3, the nitroderivatives were able to significantly inhibit platelet aggregation induced by U46619. Losartan showed a weak effect.

### Study of antihypertensive activity of losartan nitroderivative in vivo

The ability of losartan nitroderivative (compound of Ex.2) to decrease blood pressure was evaluated in conscious spontaneously hypertensive rats (SHRs). Two groups of SHRs (250-300 g) received a daily oral dose of either losartan (10 mg/kg po) or losartan nitroderivative (equimolar dose) for 3 days. Systolic blood pressure (SBP) and heart rate were monitored by telemetry at different time points after dosing.

**Table 4**

| **Systolic blood pressure (mmHg)** | | | | |
|---|---|---|---|---|
| **Compound** | **Baseline** | **30 min** | **12 hrs** | **24 hrs** |
| Losartan (10 mg/kg po) | 143 | 133 | 135 | 136 |
| Compound of **EX.2** (12 mg/kg po) | 143 | 115 | 126 | 128 |

As shown in Table 4, differently from the parent compound, the nitroderivative (compound of Ex.2) was able to induce a clear reduction in blood pressure levels over the treatment period.

## Claims

1. A compound of general formula (I) or a pharmaceutically acceptable salt or stereoisomer thereof:
R-(Y-ONO₂)ₛ (I)
wherein:
s is an integer equal to 1 or 2;
R is selected from the following Angiotensin II Receptor Blocker residues of formula (II) or (III): 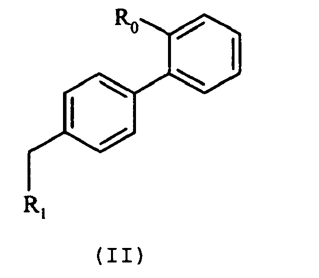 wherein:
R₀ is 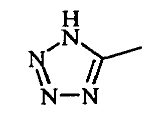
or -No which is a group capable to bind to Y, having one of the following meaning:
-COO-, -O-, -CONH-, -OCO-, -OCOO- or 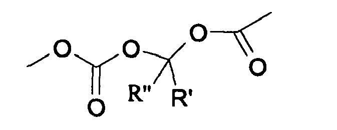 wherein R' and R" are the same or different, and are H or straight or branched C₁-C₄ alkyl;
R₁ is selected from the group consisting of: 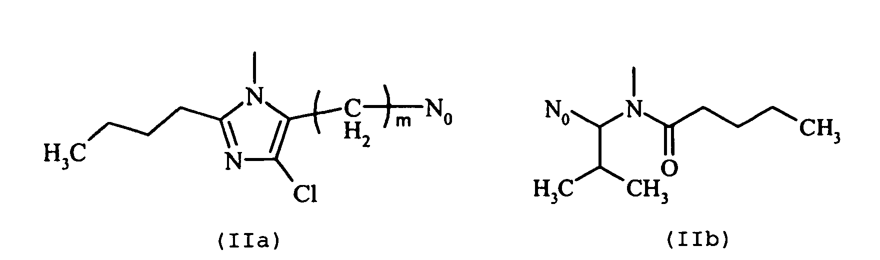 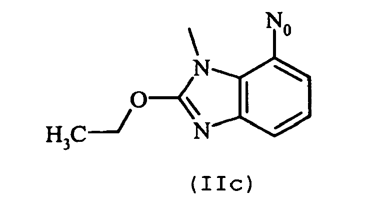 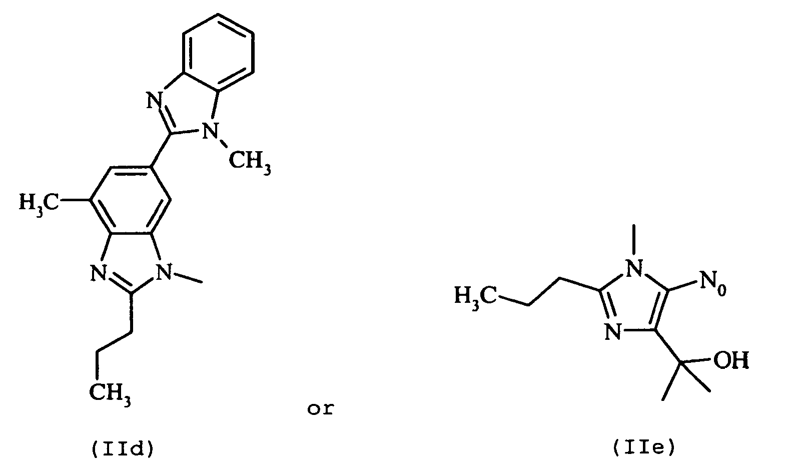 wherein m is an integer equal to 0 or 1 and N₀ is as above defined; 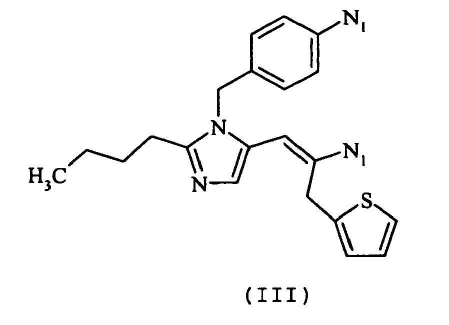 wherein N₁ has the same meaning as N₀ or is equal to -COOH; with the proviso that at least one of the groups N₁ is equal to -COO- or -CONH-, i.e. it is a group capable to bind to Y;
Yis a bivalent radical having the following meaning:
a)
- straight or branched C₁-C₂₀ alkylene, preferably C₁-C₁₀, being optionally substituted with one or more of the substituents selected from the group consisting of: halogen atoms, hydroxy, -ONO₂ or T₀, wherein T₀ is
- OC(O) (C₁-C₁₀ alkyl) -ONO₂ or -O(C₁-C₁₀ alkyl) -ONO₂;
- cycloalkylene with 5 to 7 carbon atoms into cycloalkylene ring, the ring being optionally substituted with side chains T, wherein T is straight or branched alkyl with from 1 to 10 carbon atoms, preferably CH₃;
b) 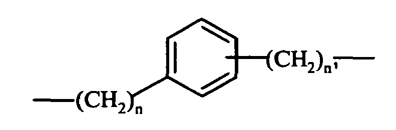
c) 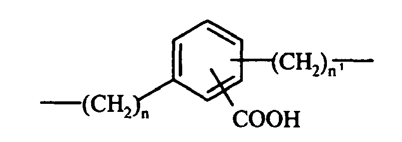 wherein n is an integer from 0 to 20, and n¹ is an integer from 1 to 20;
d) 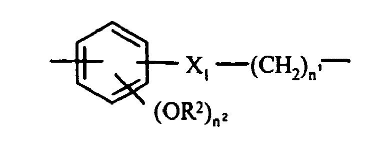 wherein:
n¹ is as defined above and n² is an integer from 0 to 2;
X₁ = -OCO- or -COO- and R² is H or CH₃;
e) 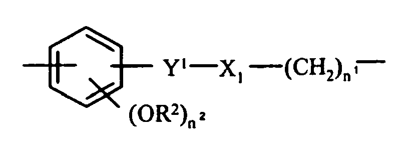 wherein:
n¹, n², R² and X₁ are as defined above;
Y¹ is -CH₂-CH₂- or -CH=CH-(CH₂)ₙ²-;
f) 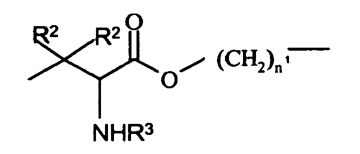 wherein:
n¹ and R² are as defined above, R³ is H or -COCH₃;
with the proviso that when Y is selected from the bivalent
radicals mentioned under b)-f), the -ONO₂ group is linked to a -(CH₂)ₙ¹ group;
g) 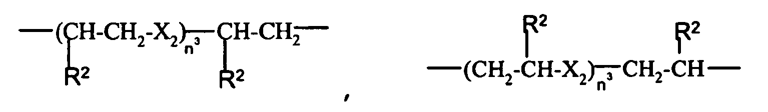 wherein X₂ is -O- or -S-, n³ is an integer from 1 to 6, preferably from 1 to 4, R² is as defined above;
h) 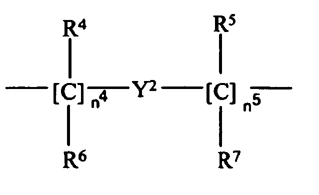 wherein:
n⁴ is an integer from 0 to 10;
n⁵ is an integer from 1 to 10;
R⁴, R⁵, R⁶, R⁷ are the same or different, and are H or straight or branched C₁-C₄ alkyl, preferably R⁴, R⁵, R⁶, R⁷ are H;
wherein the -ONO₂ group is linked to 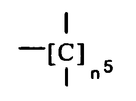 wherein n⁵ is as defined above;
Y² is an heterocyclic saturated, unsaturated or aromatic 5 or 6 members ring, containing one or more heteroatoms selected from nitrogen, oxygen, sulfur,
and is selected from

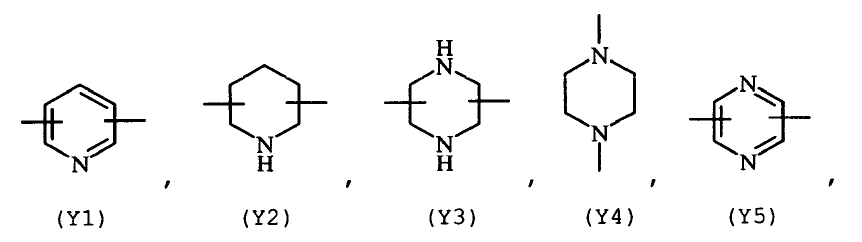

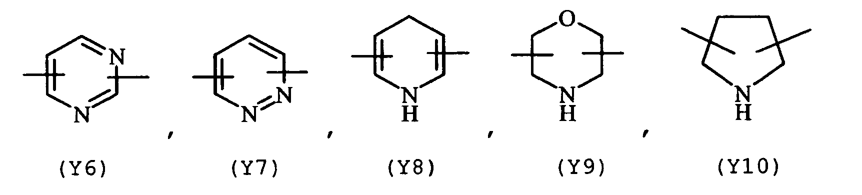

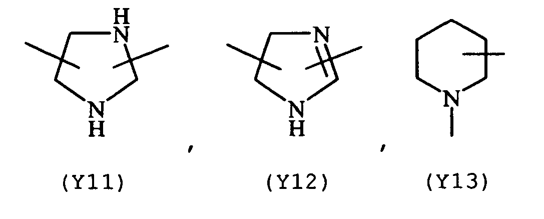

2. A compound of general formula (I) or a pharmaceutically acceptable salt or stereoisomer thereof according to claim 1 wherein Y is a bivalent radical having the following meaning:
a) straight or branched C₁-C₁₀ alkylene, being optionally substituted with T₀, wherein T₀ is as above defined;
b) 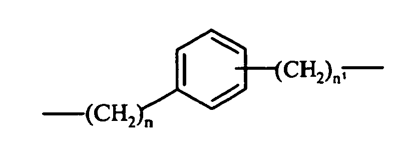 wherein n is an integer equal to 0 or 1, and n¹ is an integer equal to 1; with the proviso the -ONO₂ group is linked to a -(CH2)ₙ¹ group;
g) 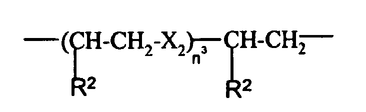 wherein X₂ is -O- or -S-, n³ is an integer equal to 1 and R² is H.

3. A compound selected from the group consisting of: 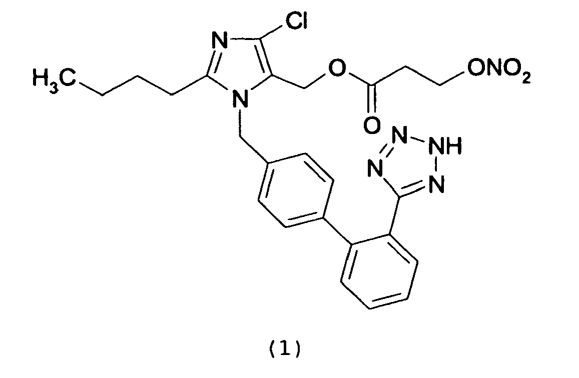 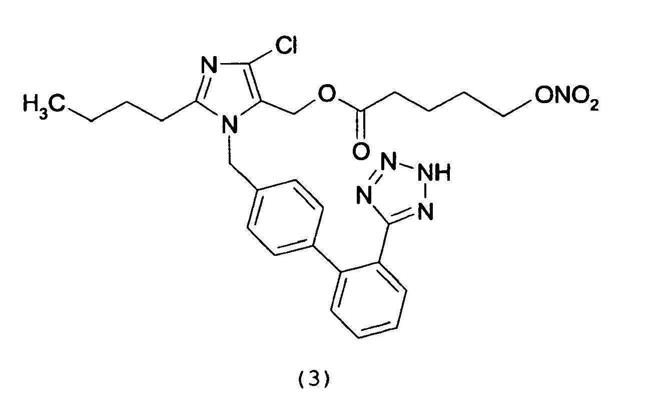 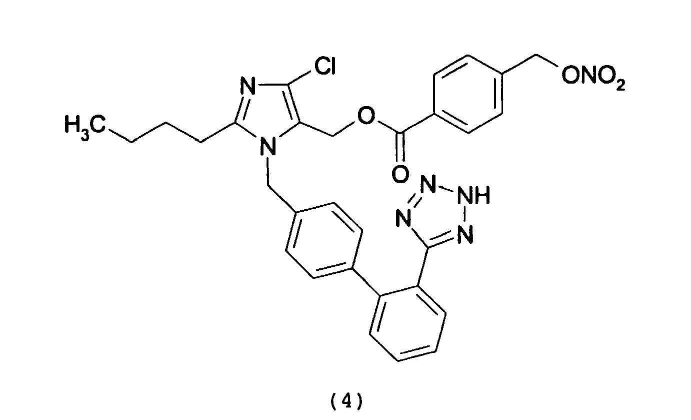 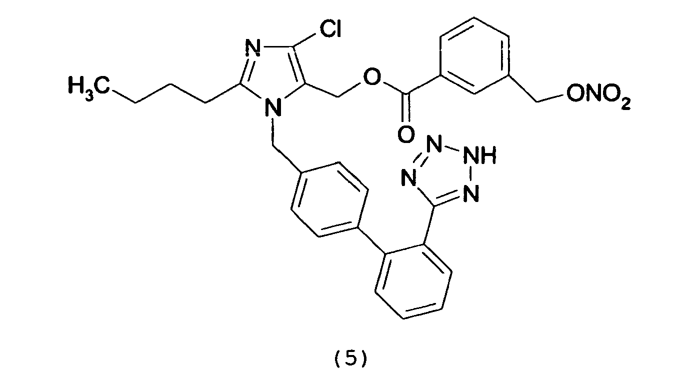 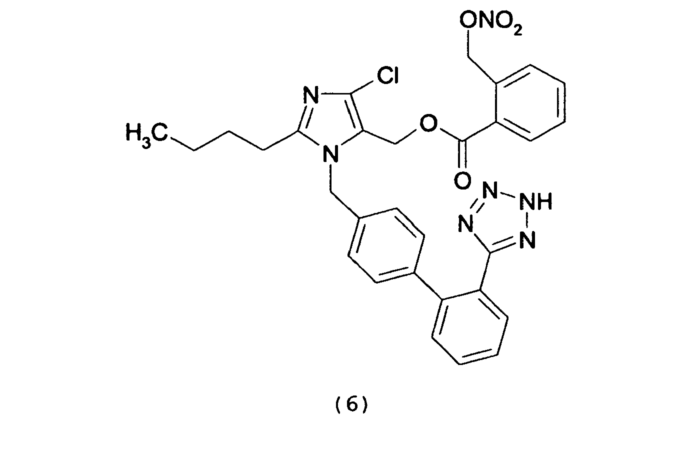 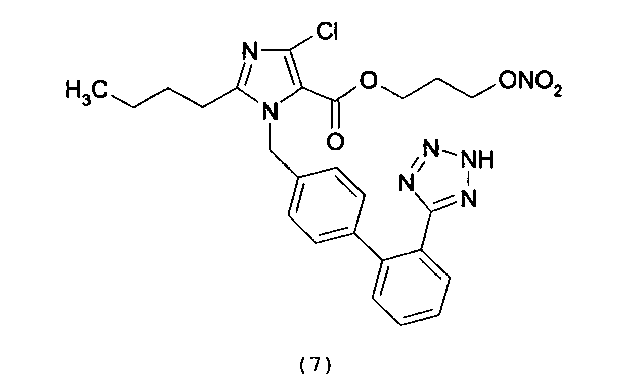 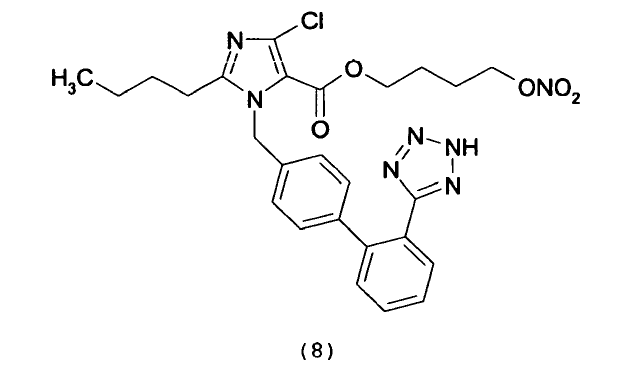 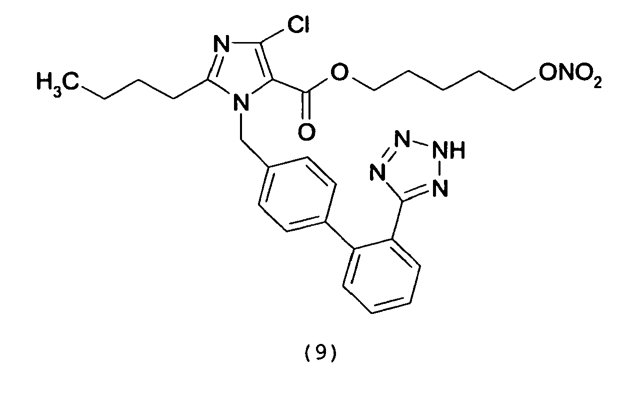 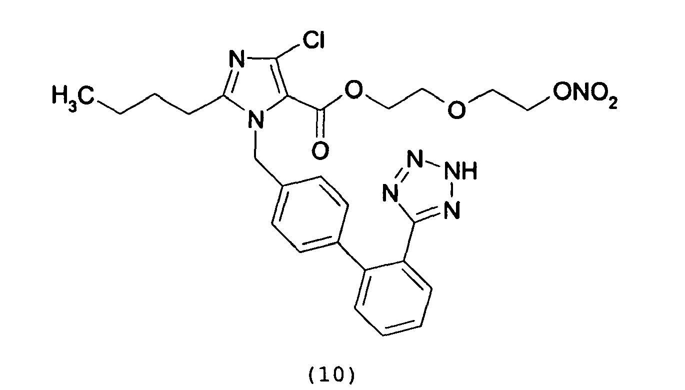 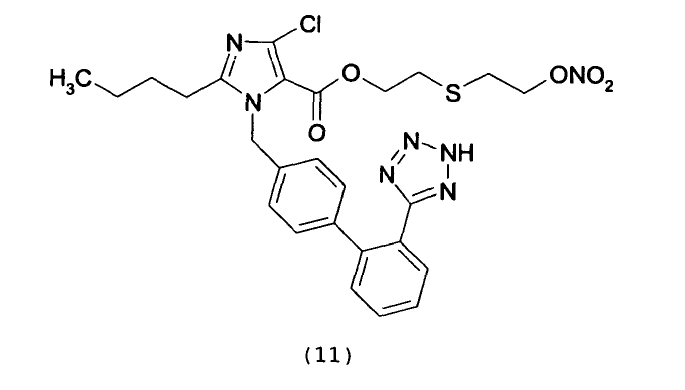 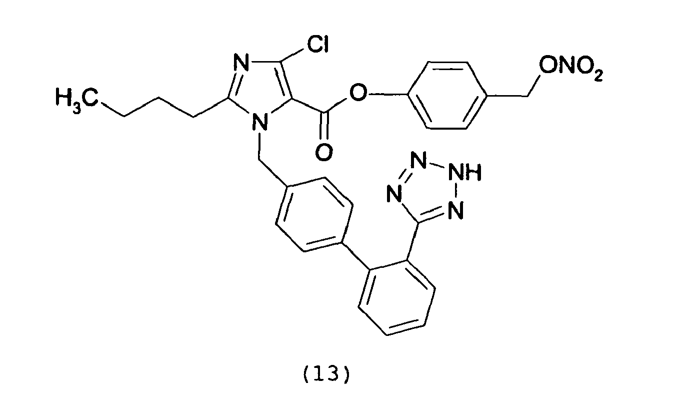 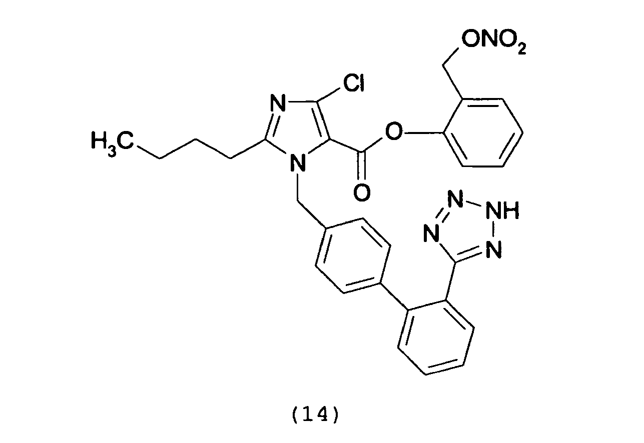 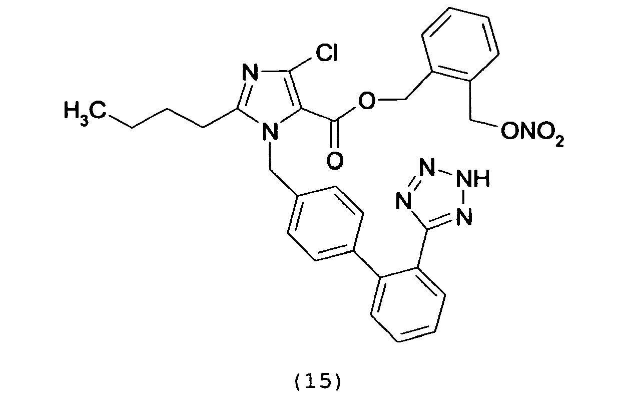 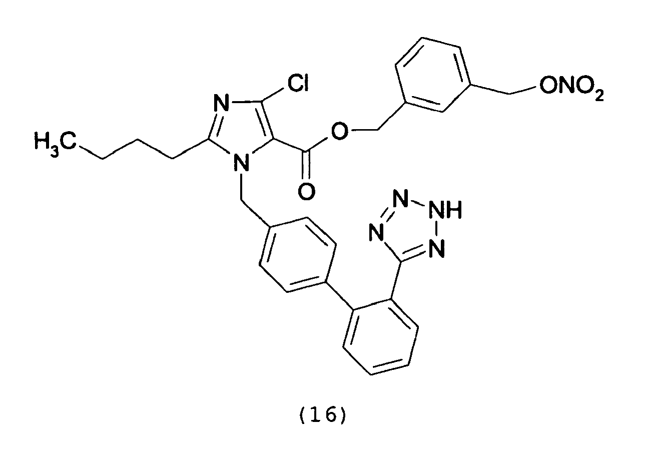 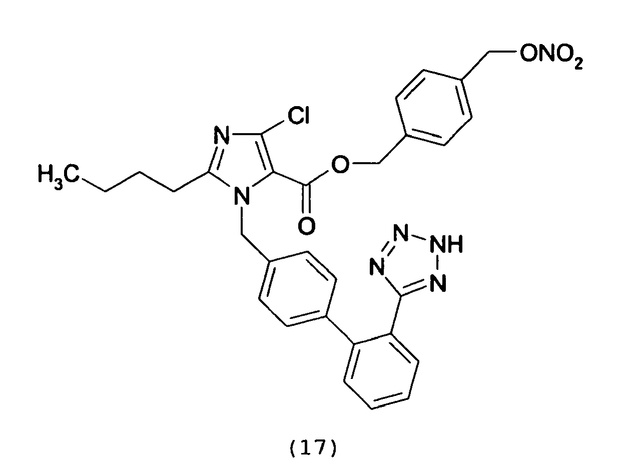 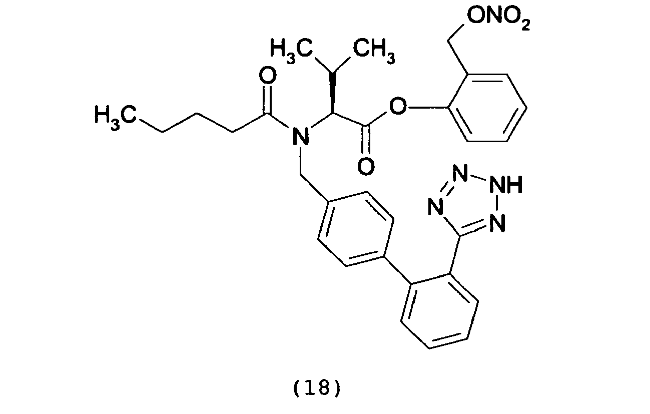 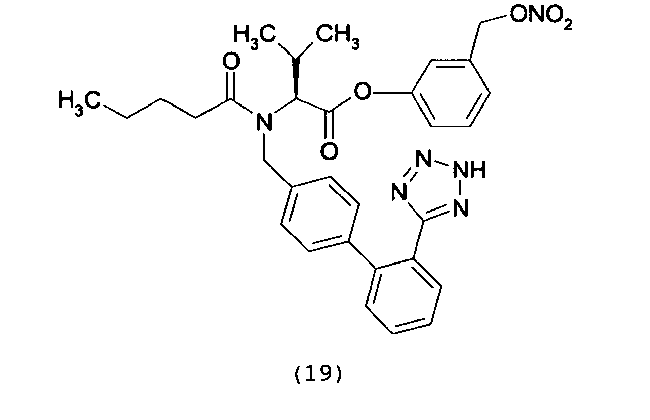 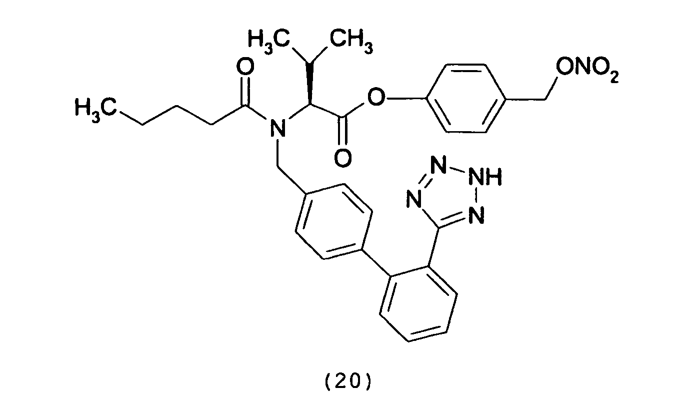 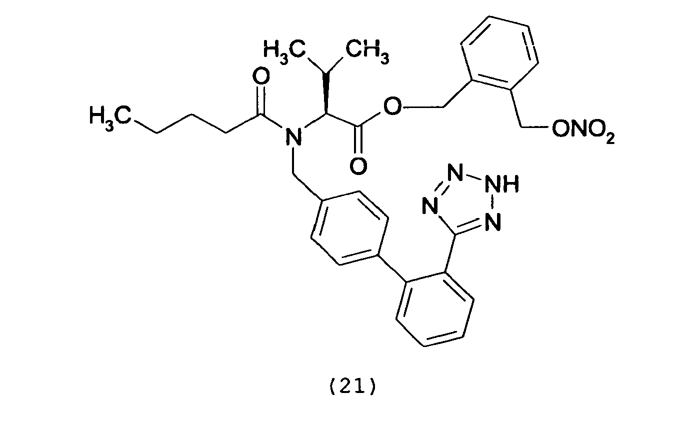 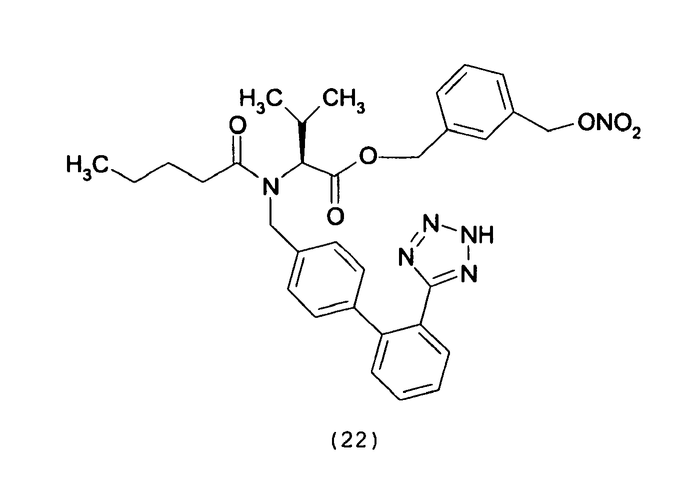 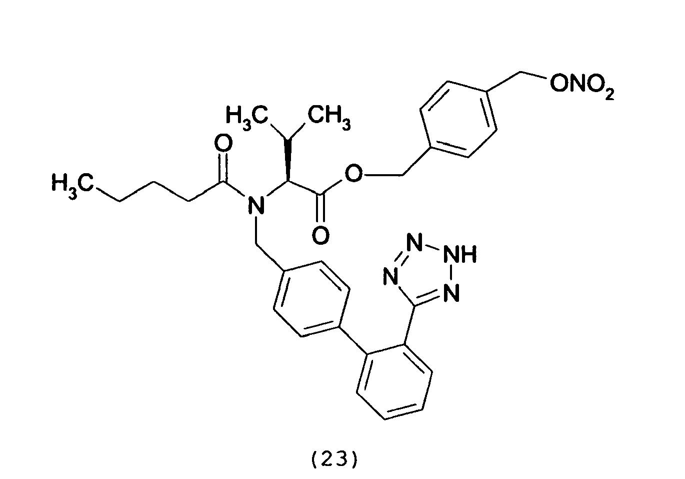 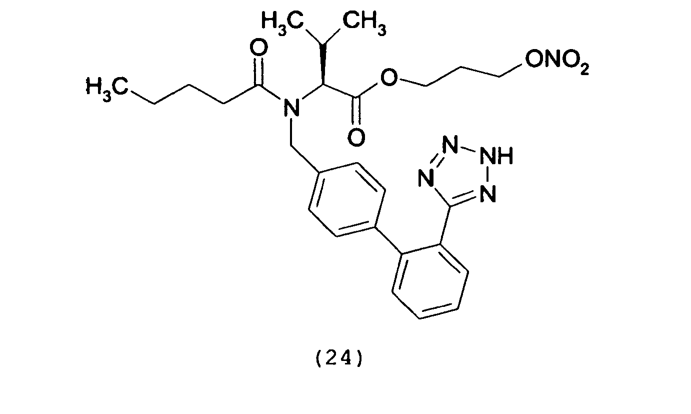 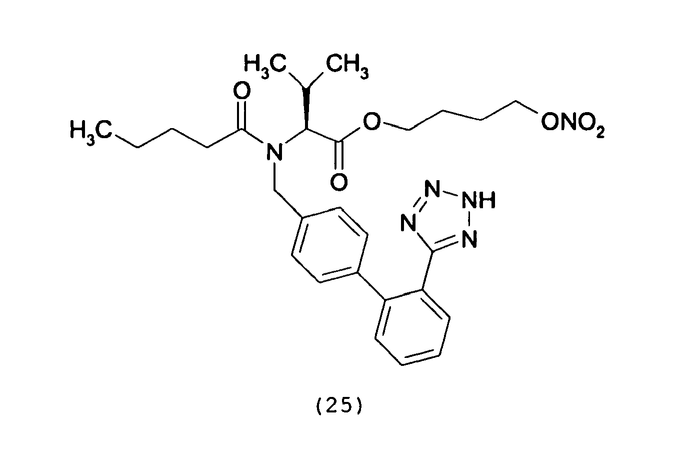 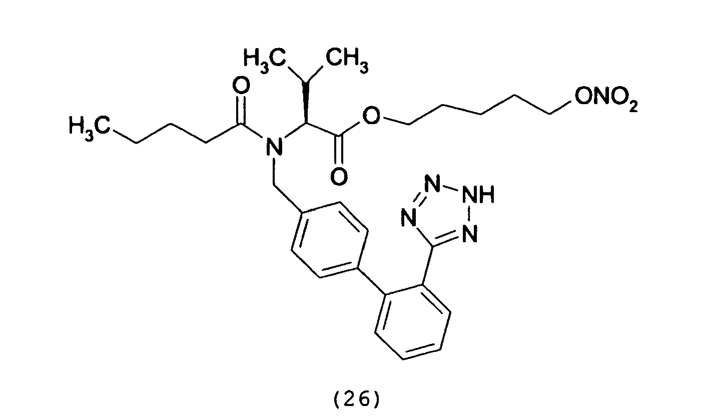 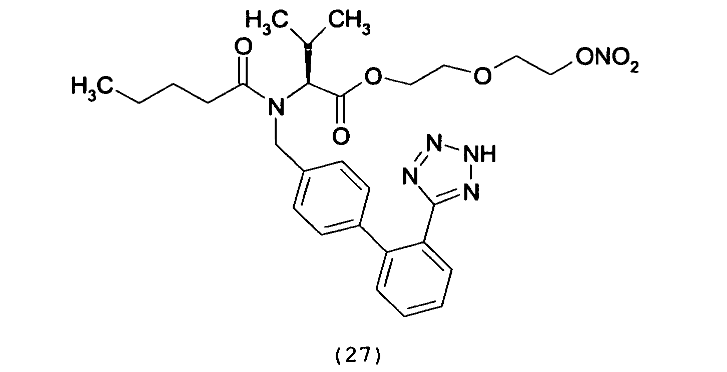 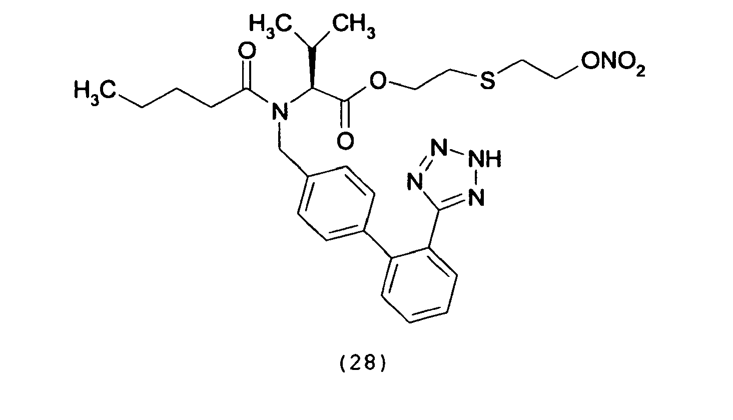 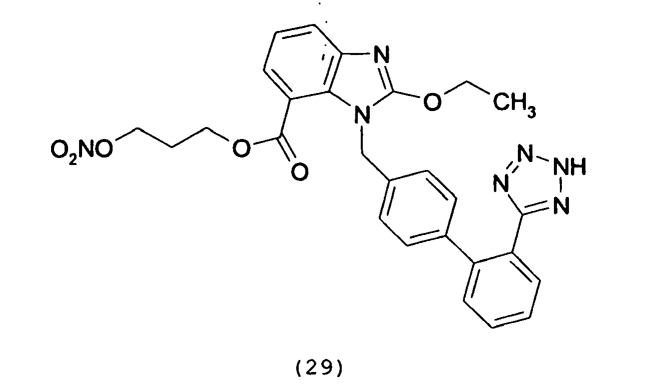 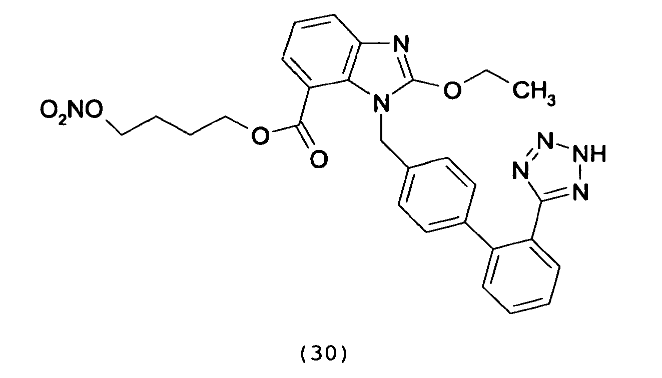 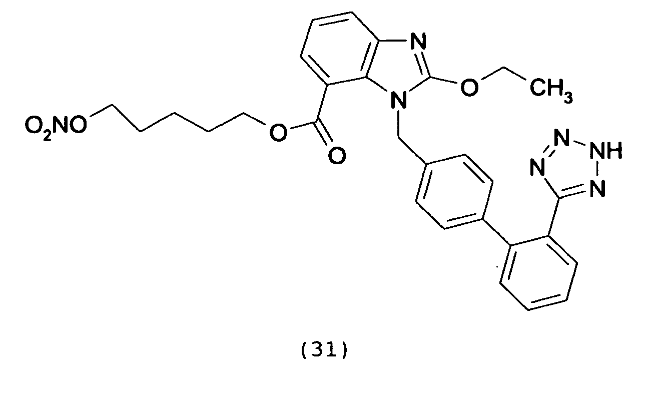 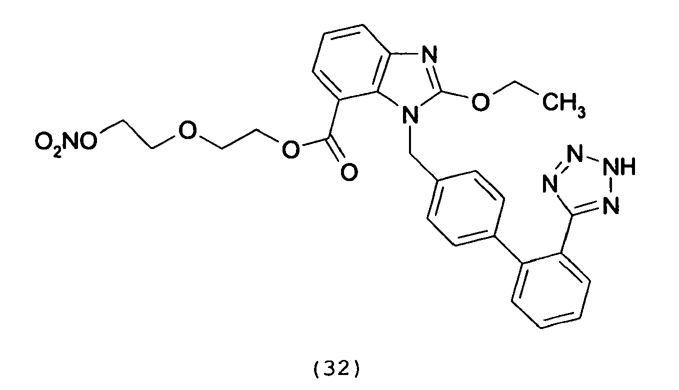 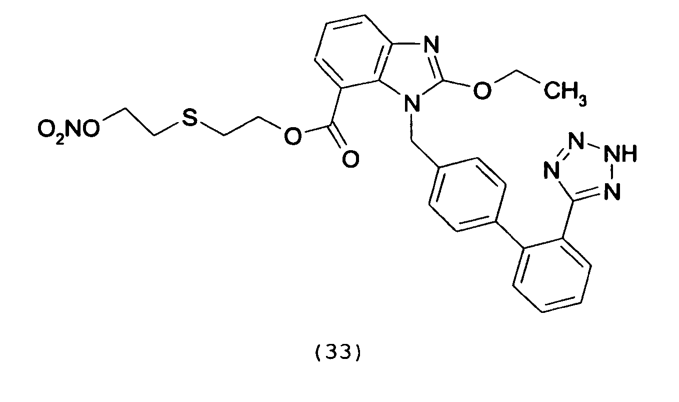 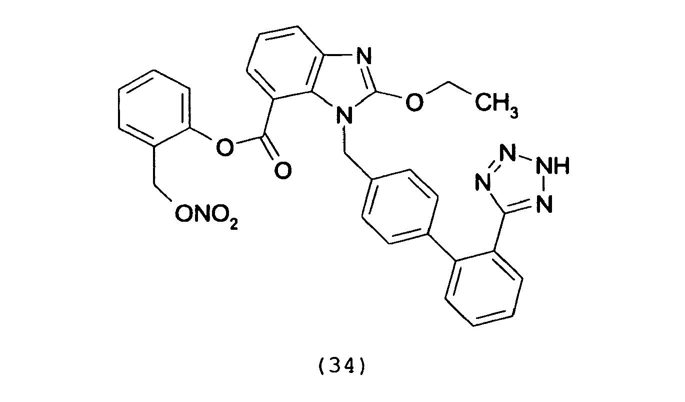 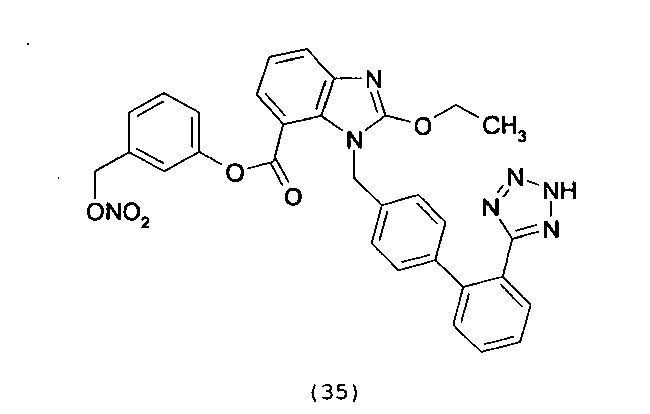 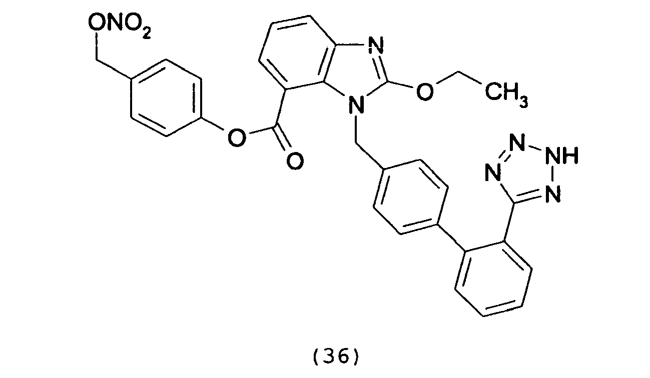 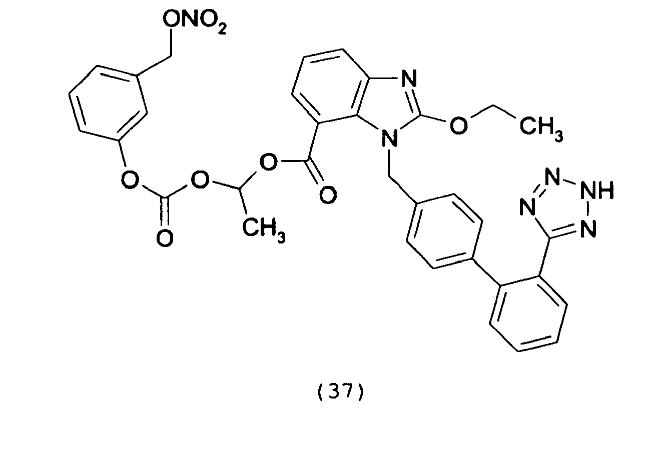 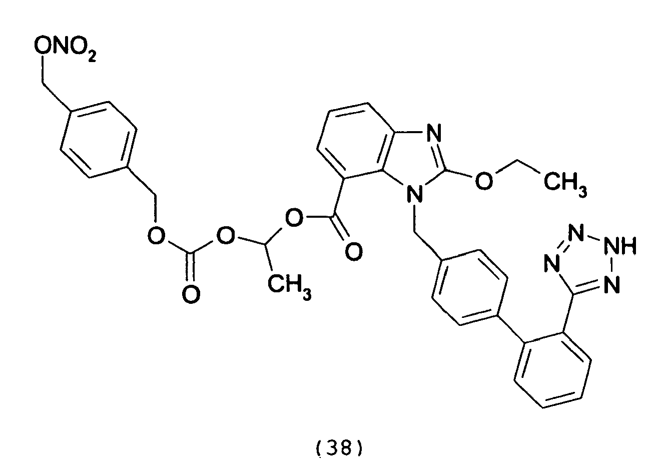 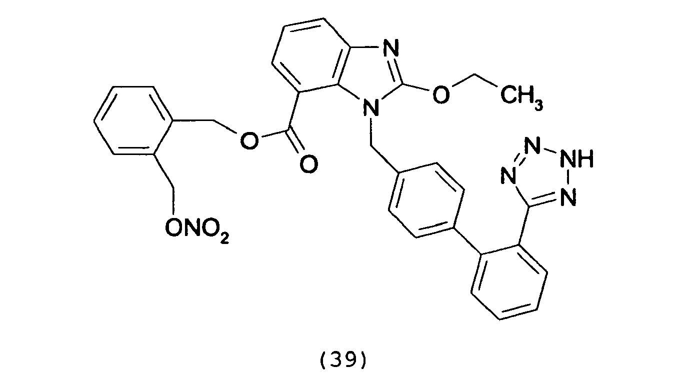 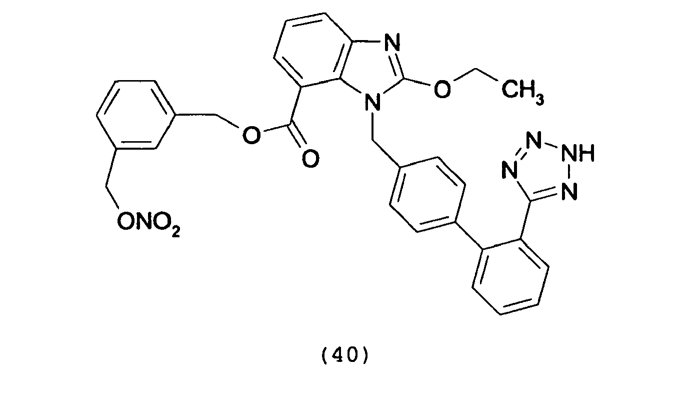 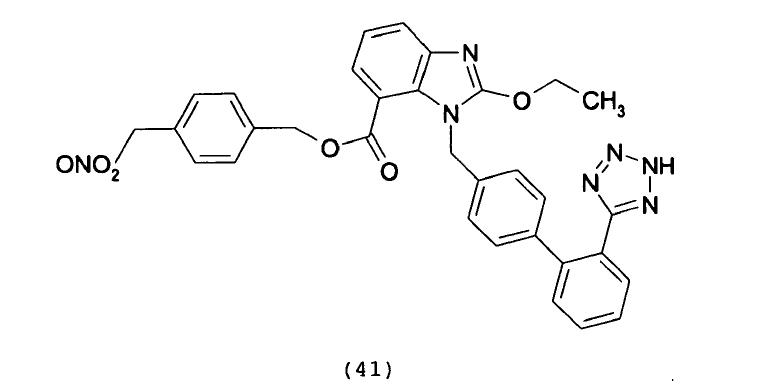 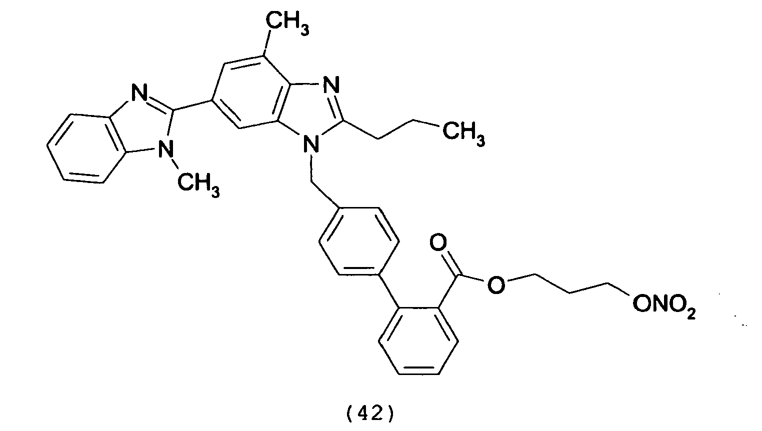 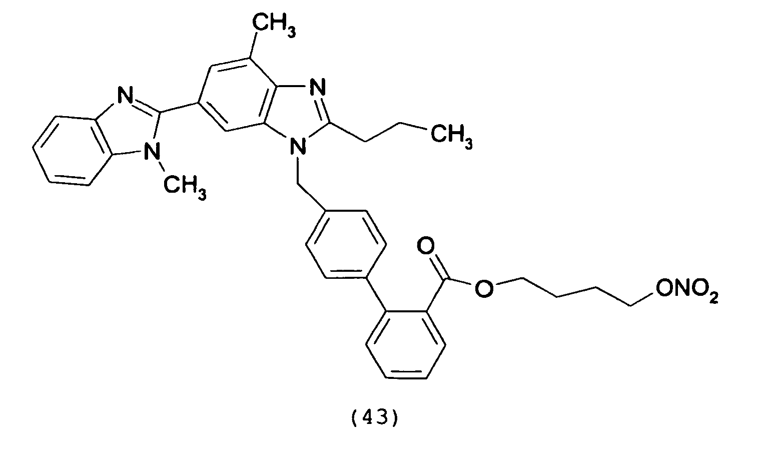 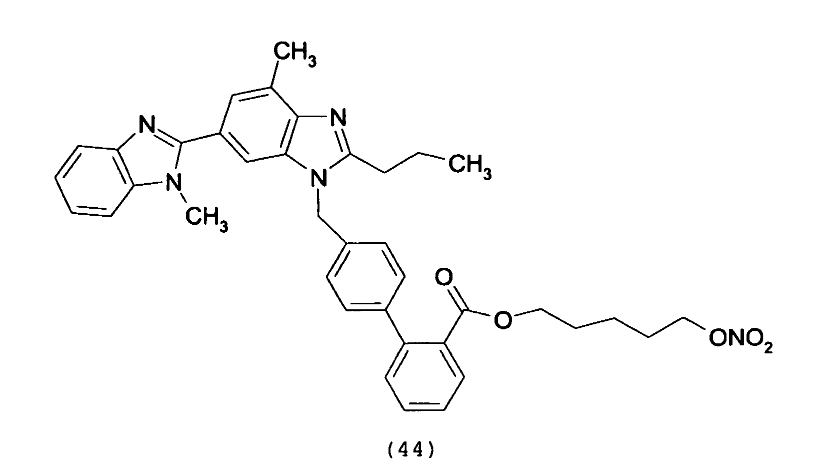 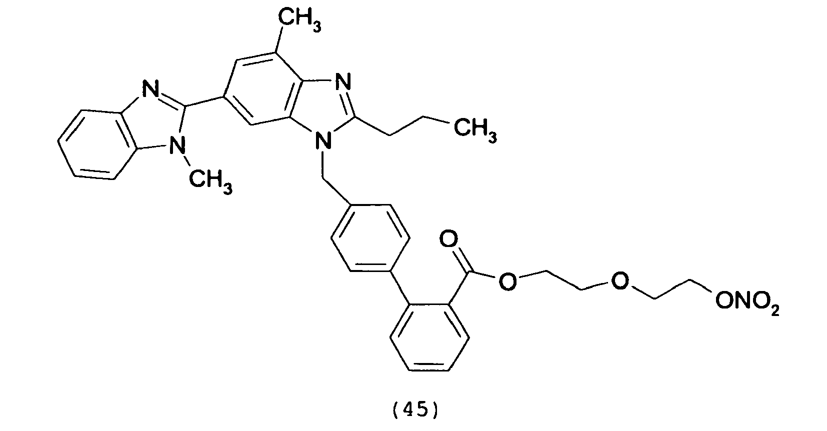 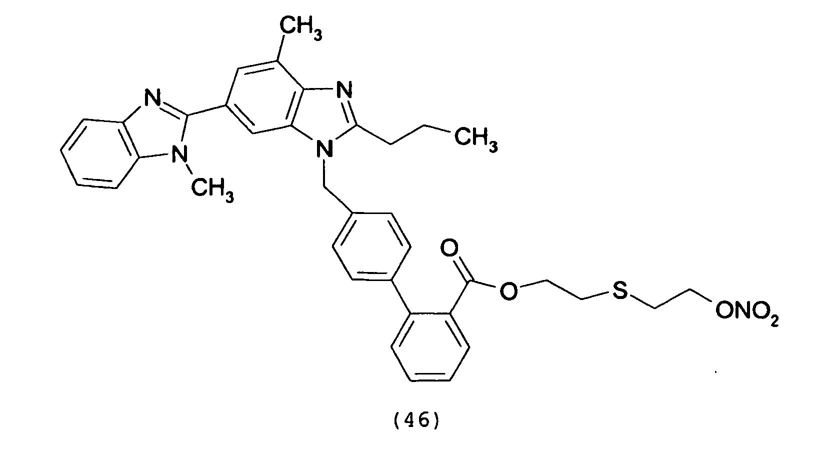 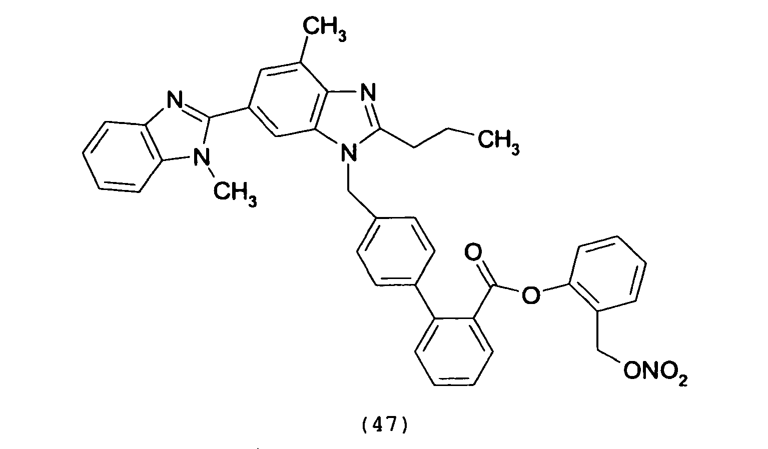 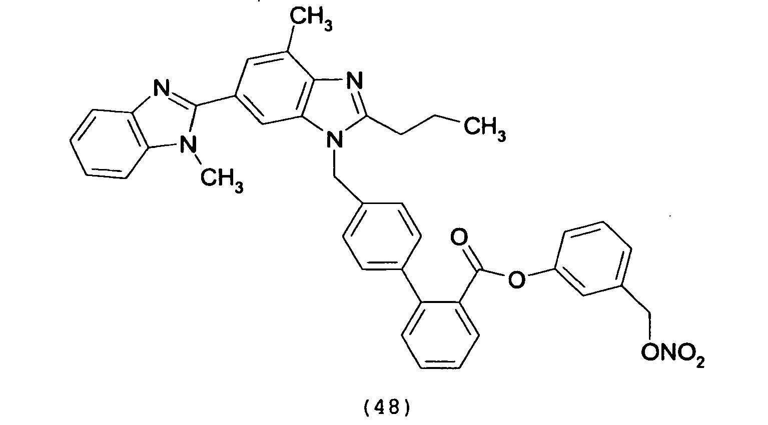 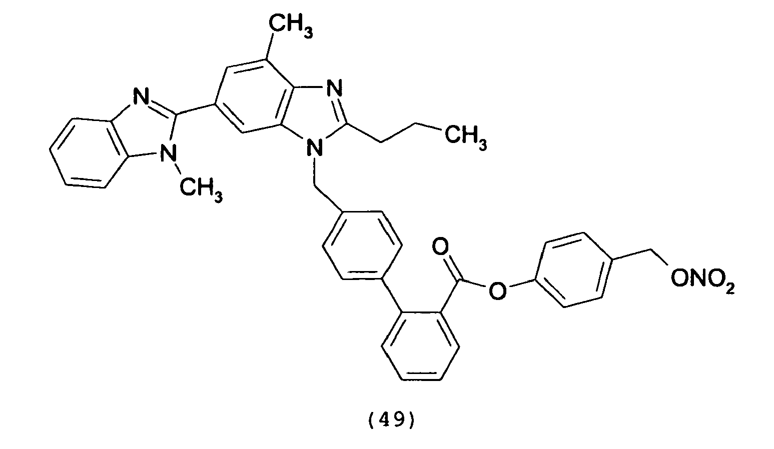 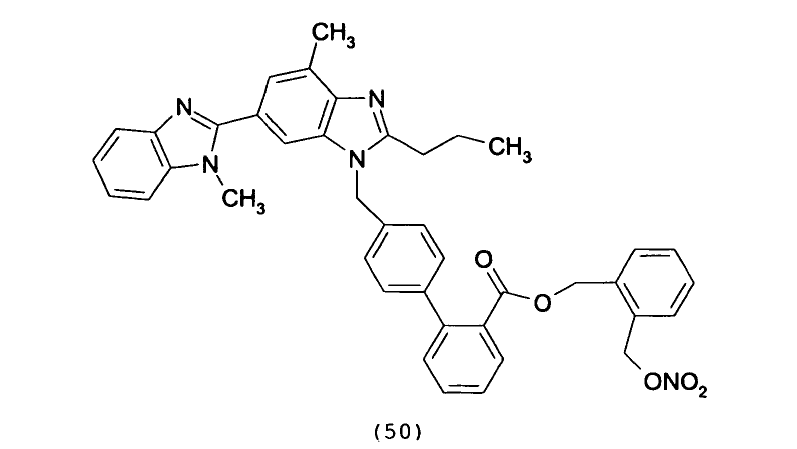 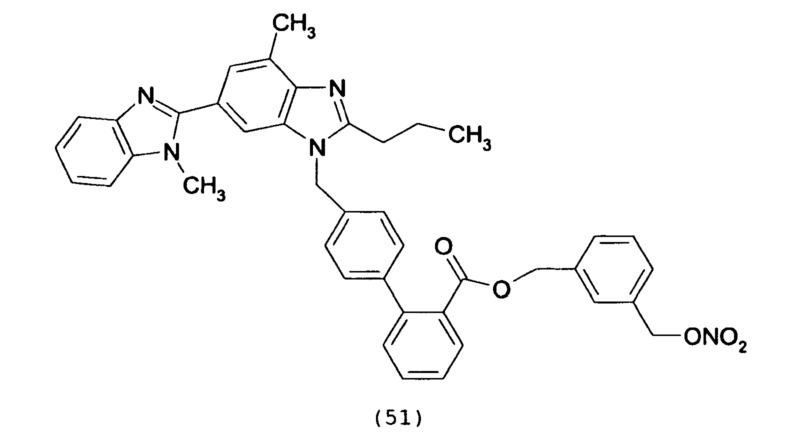 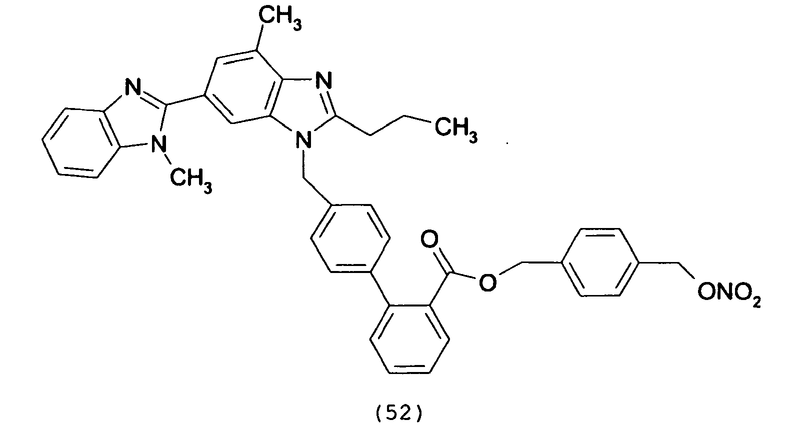 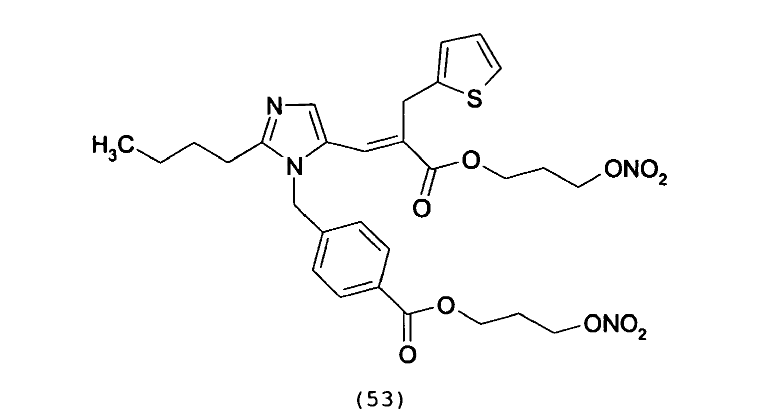 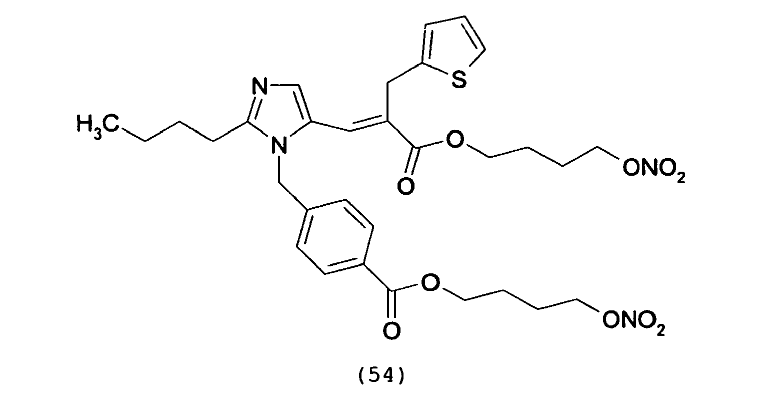 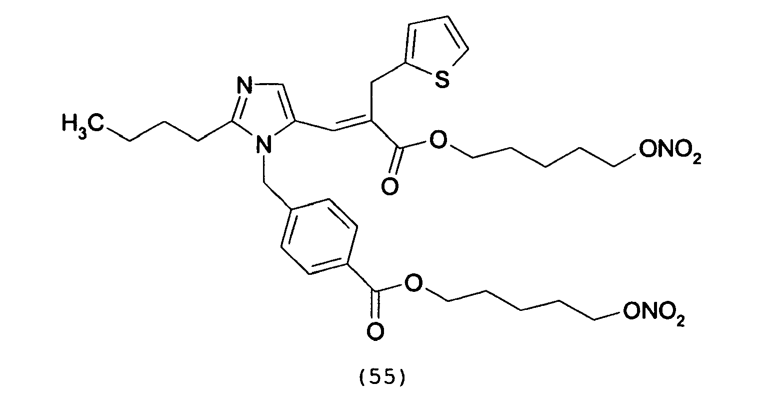 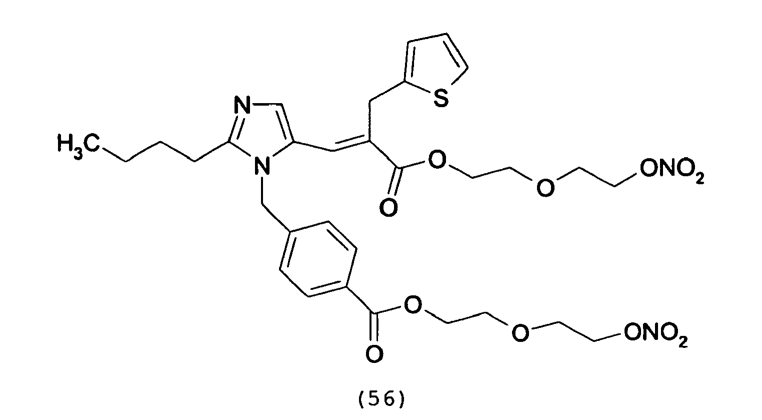 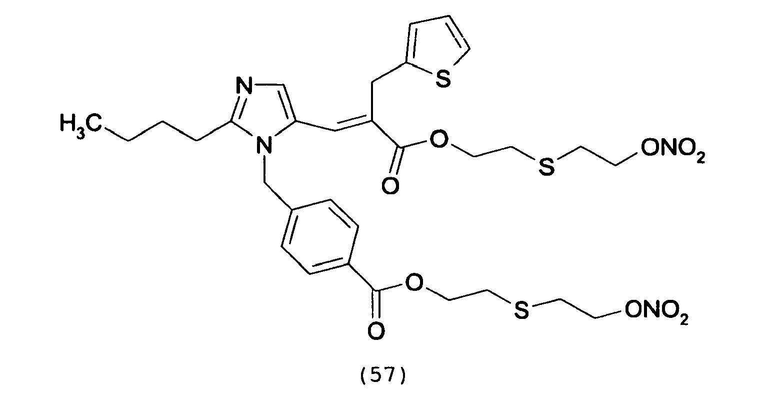 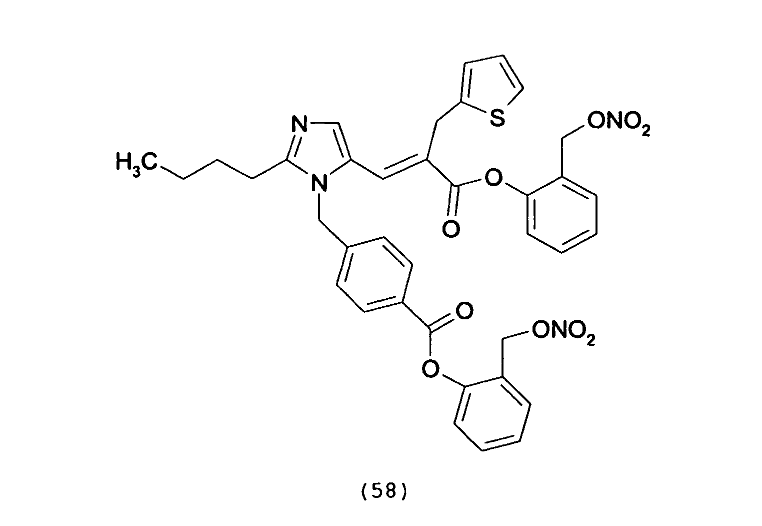 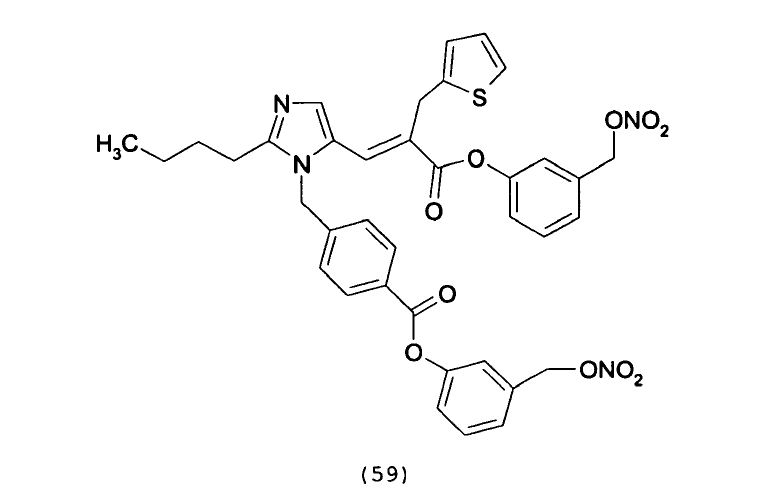 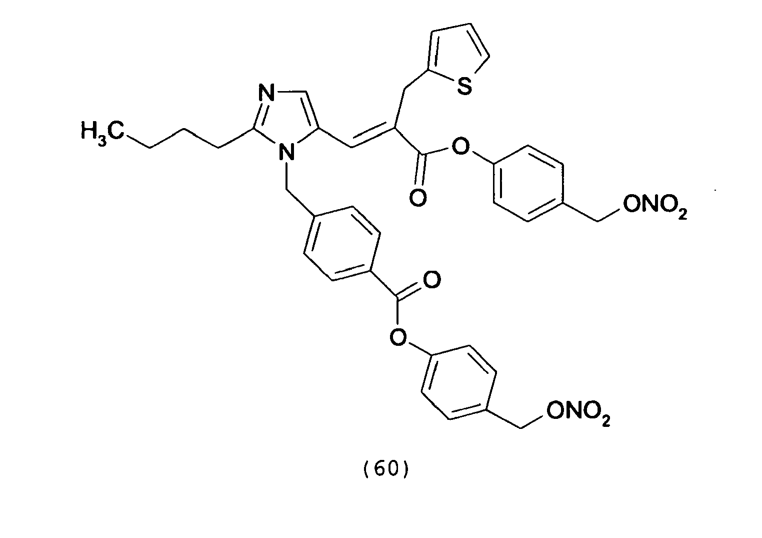 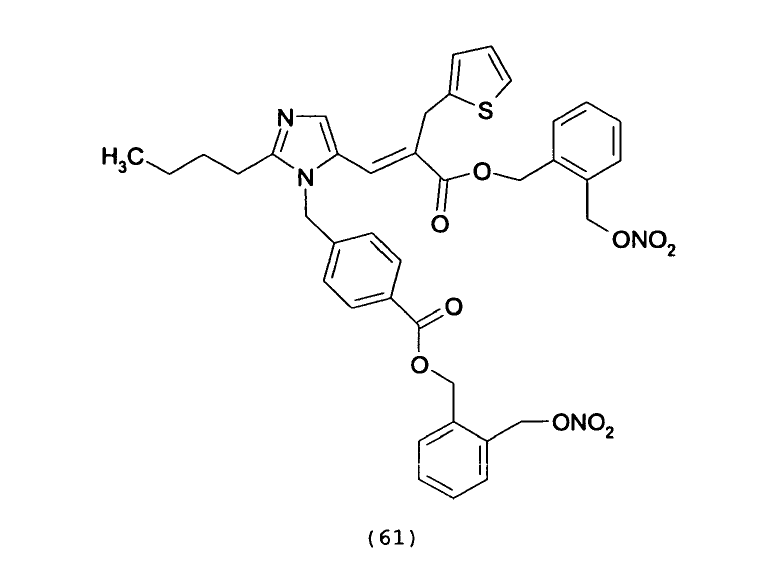 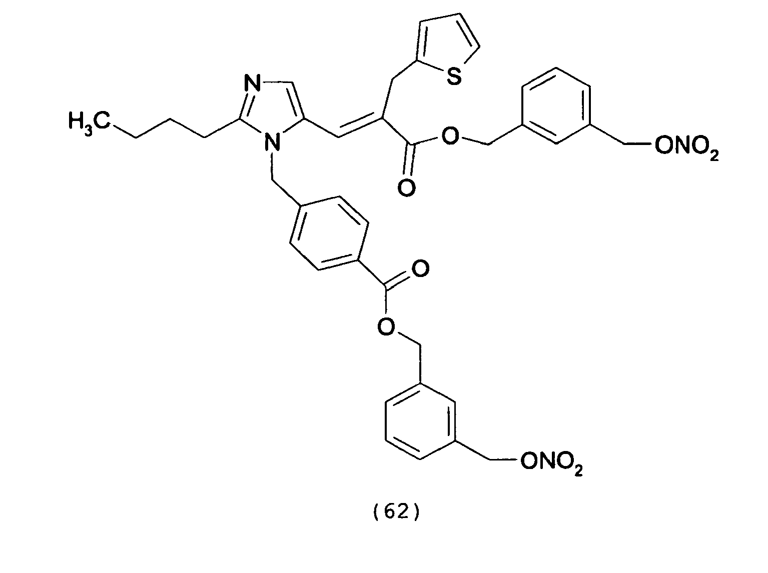 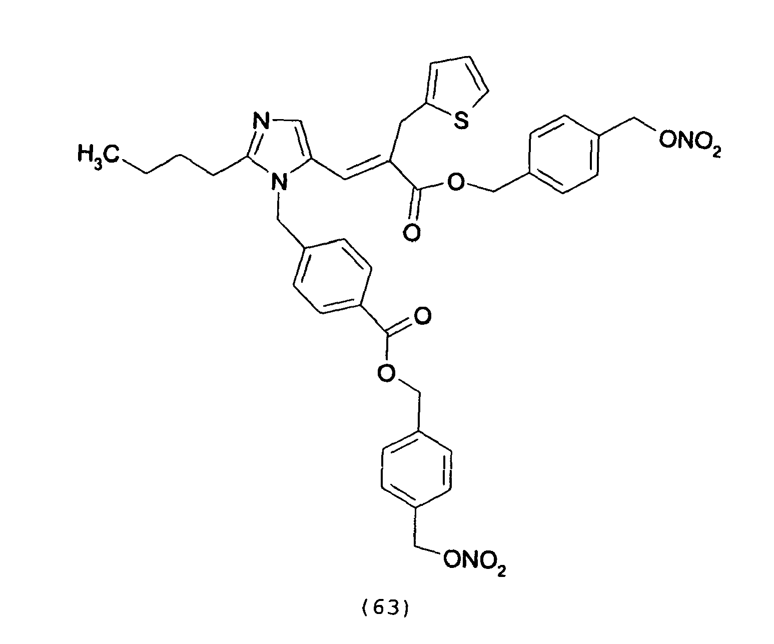 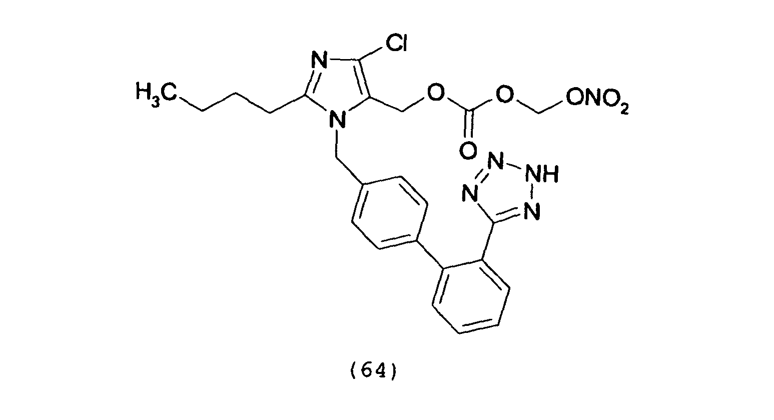 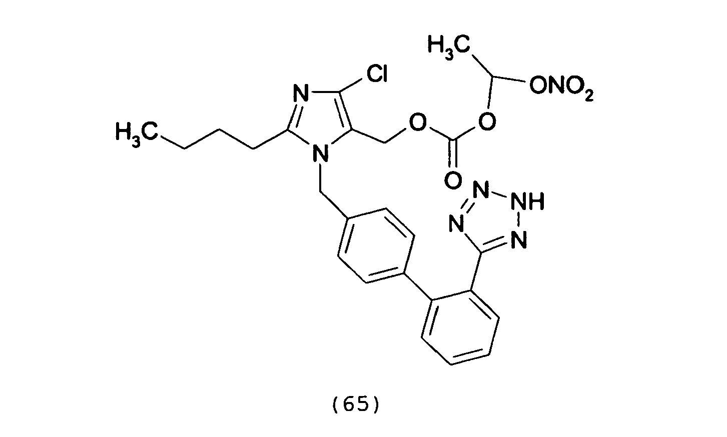 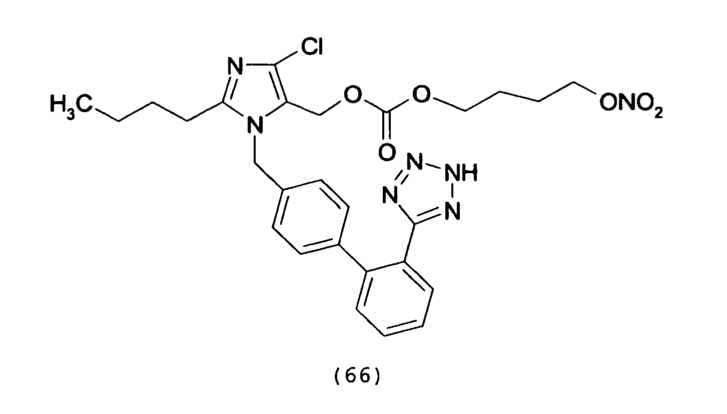 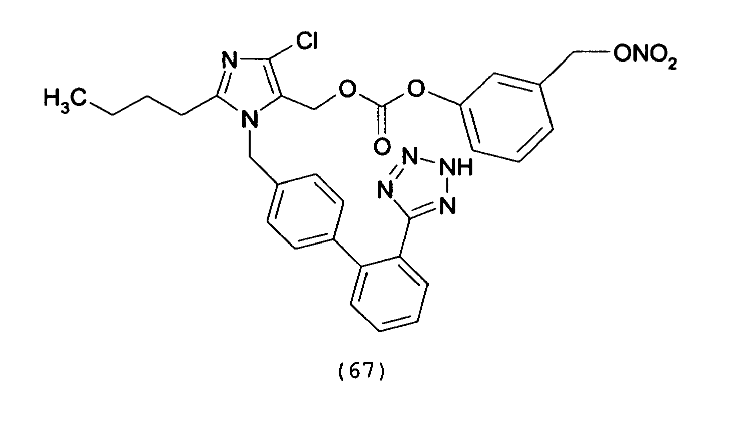 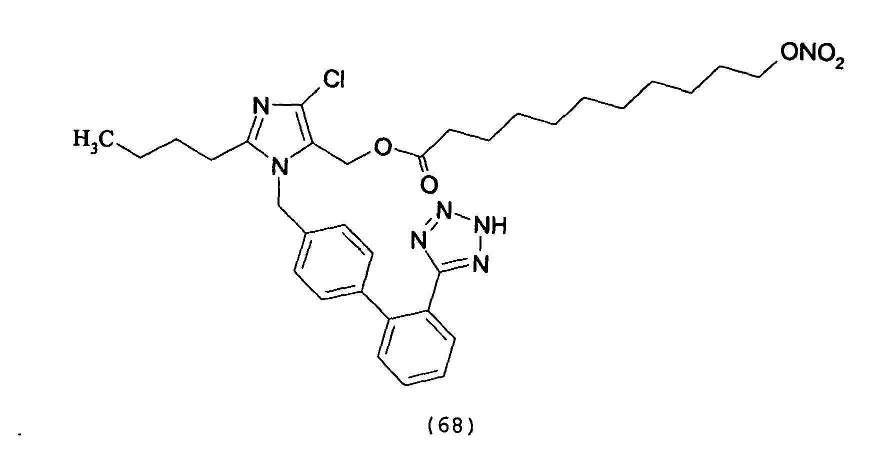 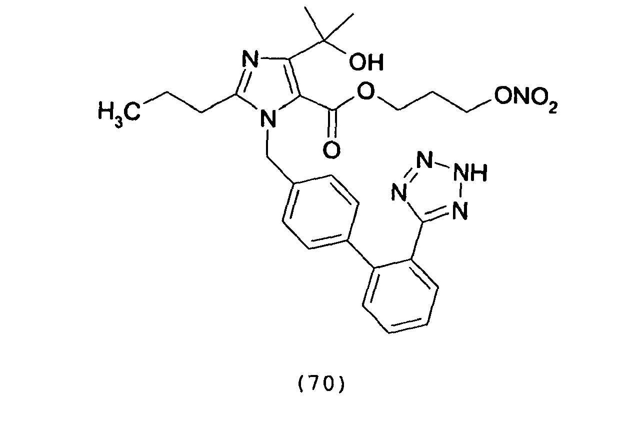 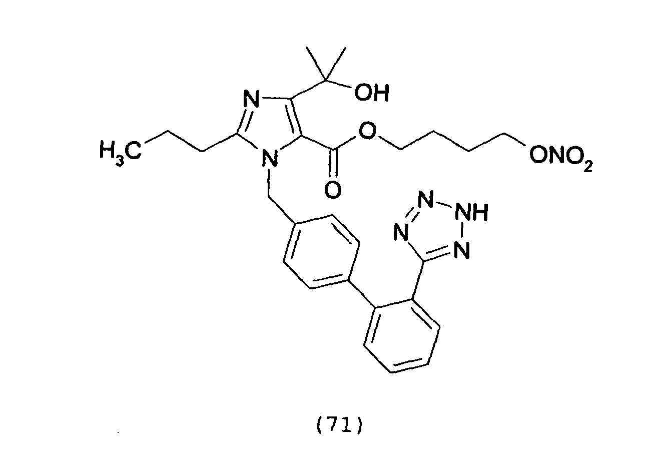 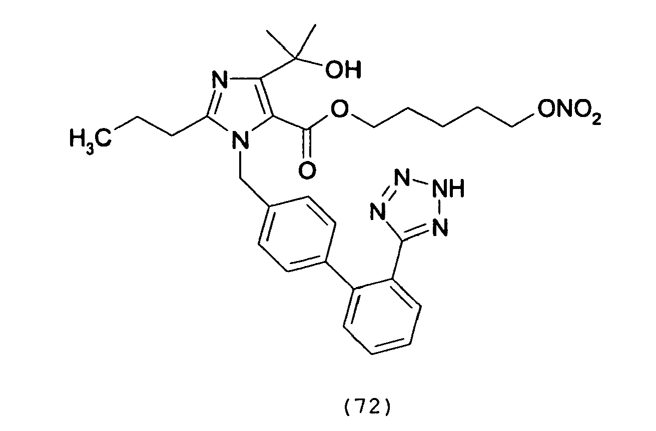 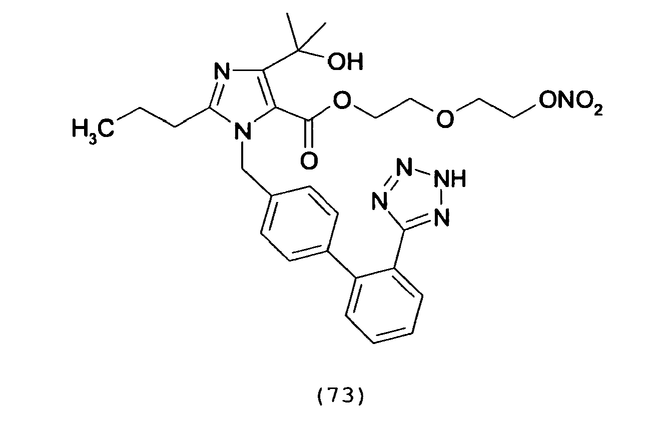 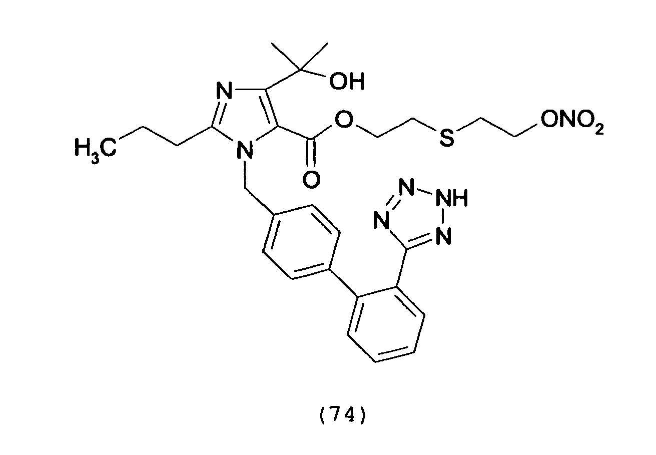 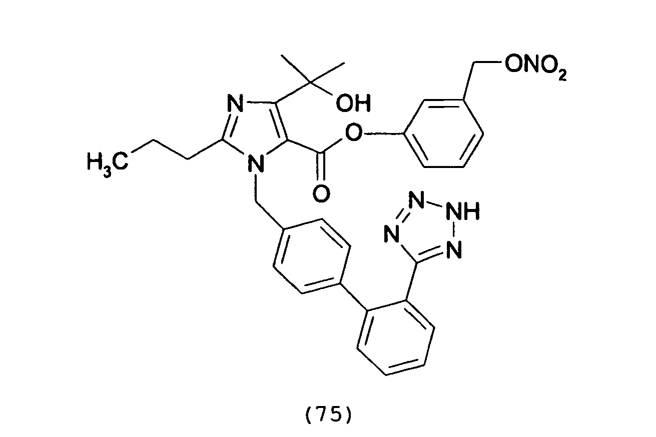 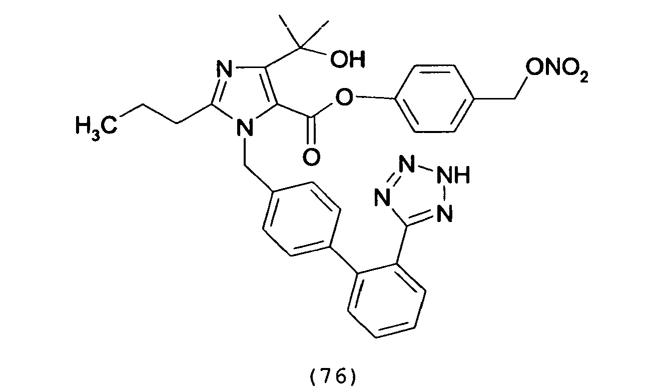 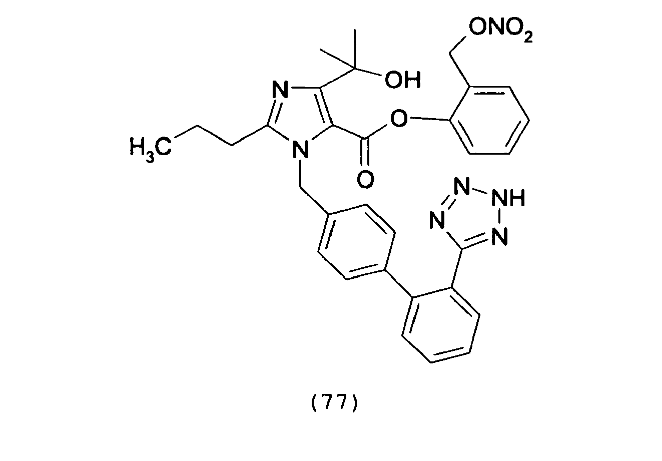 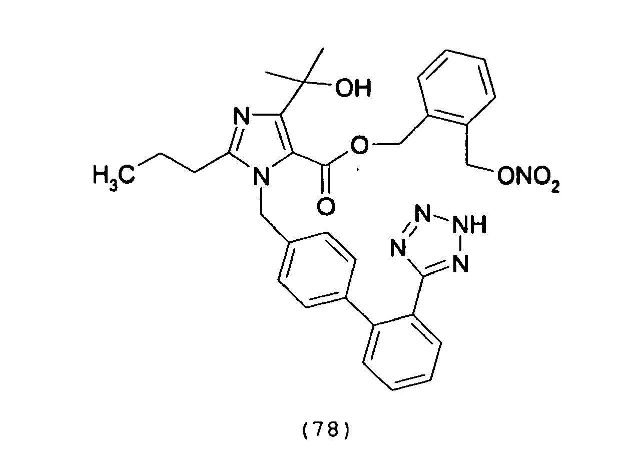 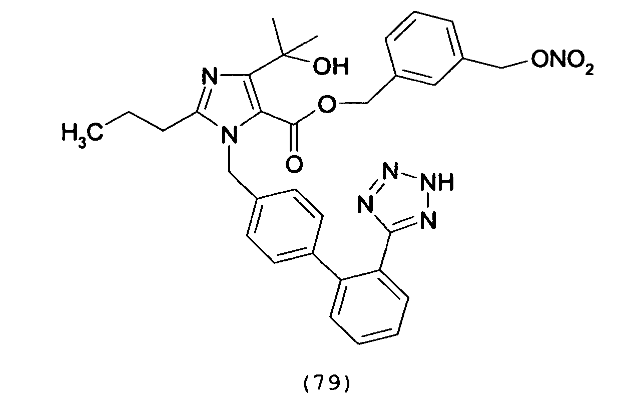 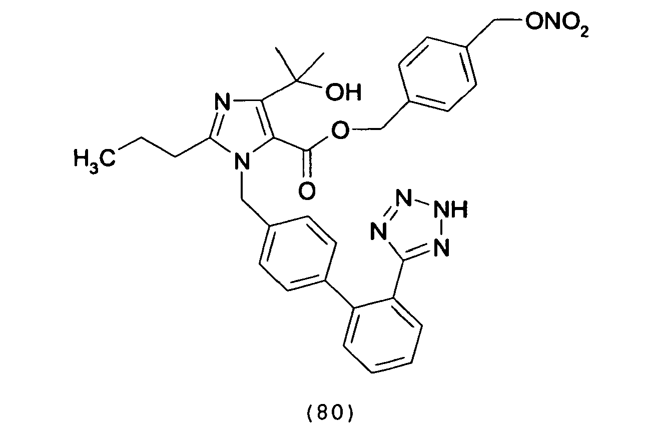 or a pharmaceutically acceptable salt or stereoisomer thereof.

4. A compound of formula (2): 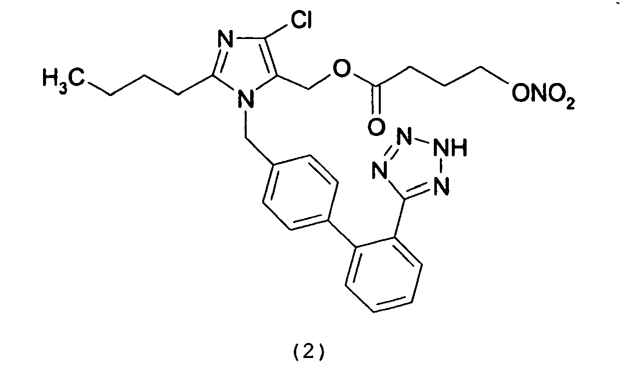 or a pharmaceutically acceptable salt or stereoisomer thereof.

5. A compound of formula (12): 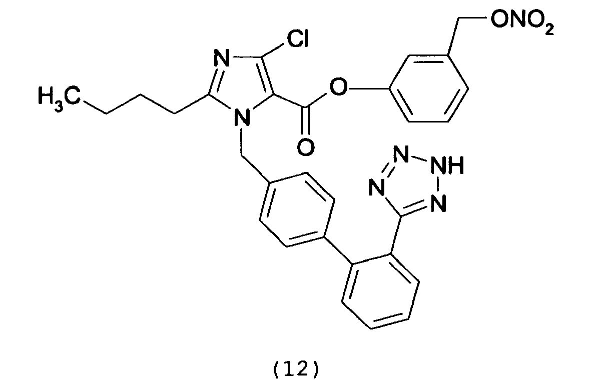 or a pharmaceutically acceptable salt or stereoisomer thereof.

6. A compound of formula (69): 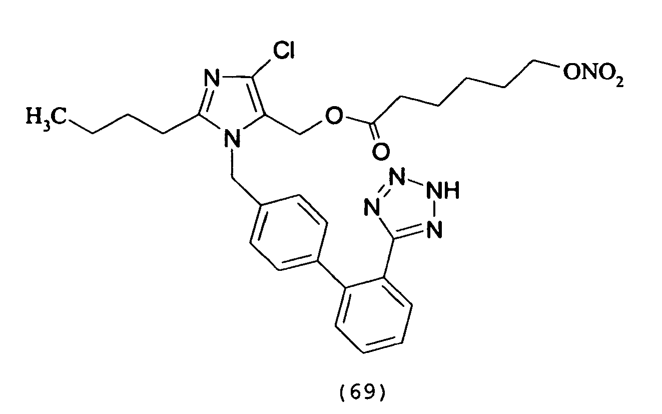 or a pharmaceutically acceptable salt or stereoisomer thereof.

7. A compound of general formula (I) according to claims 1-6 for use as a medicament.

8. Use of a compound according to claims 1-6 for preparing a drug having anti-inflammatory, antithrombotic and antiplatelet activity.

9. Use of a compound according to claims 1-6, for preparing a drug that can be employed in the treatment or prophylaxis of cardiovascular, renal and chronic liver diseases, inflammatory processes and metabolic syndromes.

10. Use of a compound according to claim 9, for preparing a drug that can be employed in the treatment or prophylaxis of heart failure, myocardial infarction, ischemic stroke, atherosclerosis, ocular and pulmonary hypertension, hypertension, diabetic nephropathy, peripheral vascular diseases, left ventricular dysfunction and hypertrophy, liver fibrosis and portal hypertension.

11. A pharmaceutical composition comprising a pharmaceutically acceptable carrier and a pharmaceutically effective amount of a compound of general formula (I) or a salt or stereoisomer thereof according to claims 1-6.

12. A pharmaceutical composition according to claim 11 in a suitable form for the oral, parenteral, rectal, topic and transdermic administration, by inhalation spray or aerosol or iontophoresis devices.

13. Liquid or solid pharmaceutical composition for oral, parenteral, rectal, topic and transdermic administration or inhalation in the form of tablets, capsules and pills eventually with enteric coating, powders, granules, gels, emulsions, solutions, suspensions, syrups, elixir, injectable forms, suppositories, in transdermal patches or liposomes, containing a compound of formula (I) or a salt or stereoisomer thereof according to claims 1-6 and a pharmaceutically acceptable carrier.

14. A pharmaceutical composition comprising a compound of general formula (I), at least a compound used to treat cardiovascular disease and a pharmaceutically acceptable carrier.

15. Pharmaceutical composition according to claim 14
wherein the compound used to treat cardiovascular disease is selected from the group consisting of: ACE inhibitors, HMGCoA reductase inhibitors, beta-adrenergic blockers, calcium channel blockers, diuretics, antithrombotics such as aspirin, nitrosated ACE inhibitors, nitrosated HMGCoA reductase inhibitors, nitrosated beta-adrenergic blockers, nitrosated aspirin and nitrosated diuretics.

16. A pharmaceutical kit comprising a compound of general formula (I) as defined in claim 1, a compound used to treat cardiovascular disease as combined preparation for simultaneous, separated, sequential use for the treatment of cardiovascular disease.

17. A pharmaceutical kit according to claim 16 wherein the compound used to treat cardiovascular disease is selected from the group consisting of: ACE inhibitors, HMGCoA reductase inhibitors, beta-adrenergic blockers, calcium channel blockers, diuretics, antithrombotics such as aspirin, nitrosated ACE inhibitors, nitrosated HMGCoA reductase inhibitors, nitrosated beta-adrenergic blockers, nitrosated aspirin and nitrosated diuretics.

## Patentansprüche

1. Verbindung der allgemeinen Formel (I) oder ein pharmazeutisch verträgliches Salz oder Stereoisomer davon:
R-(Y-ONO₂)ₛ (I)
worin:
s eine ganze Zahl ist, die 1 oder 2 entspricht;
R aus den folgenden Angiotensin-II-Rezeptorblockerresten der Formel (II) oder (III) ausgewählt ist: 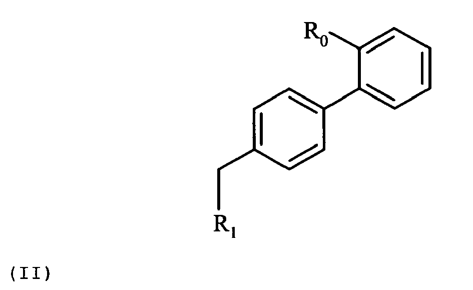 worin:
R₀ 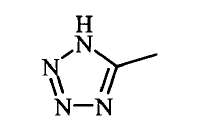 ist,
oder -N₀, was eine Gruppe ist, die befähigt ist, an Y zu binden, mit einer der folgenden Bedeutungen:
-COO-, -O-, -CONH-, -OCO-, -OCOO- oder 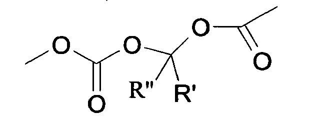 worin R' und R" gleich oder verschieden sind, und H oder ein geradkettiges oder verzweigtes C₁-C₄-Alkyl sind;
R₁ aus der Gruppe ausgewählt ist, die besteht aus: 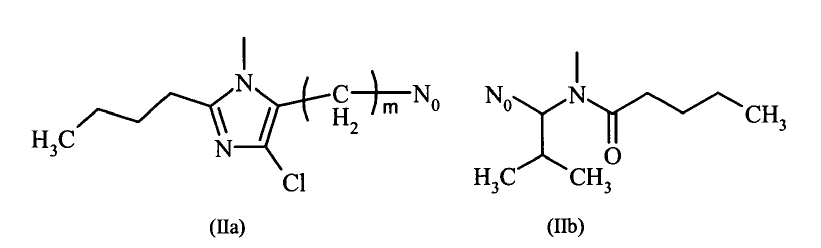 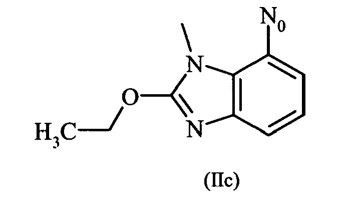 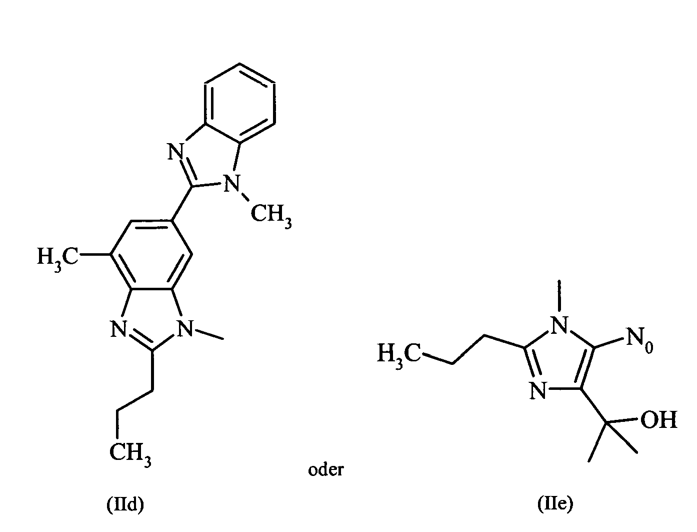 worin m eine ganze Zahl ist, die 0 oder 1 entspricht, und N₀ wie oben definiert ist; 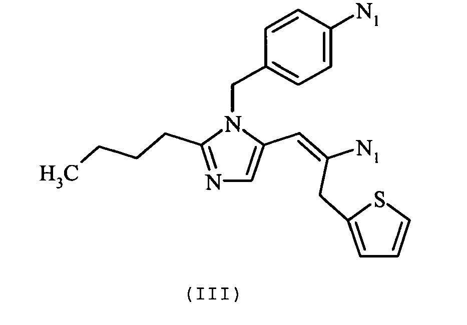 worin N₁ die gleiche Bedeutung wie N₀ aufweist oder -COOH entspricht; mit der Maßgabe, dass wenigstens eine der Gruppen N₁ -COO- oder -CONHentspricht, d.h., es ist eine Gruppe, die befähigt ist, an Y zu binden;
Y ist ein bivalenter Rest mit der folgenden Bedeutung:
a)
- geradkettiges oder verzweigtes C₁-C₂₀-Alkylen, vorzugsweise C₁-C₁₀, gegebenenfalls mit ein oder mehreren der Substituenten substituiert, ausgewählt aus der Gruppe, die aus: Halogenatomen, Hydroxy, -ONO₂ oder T₀ besteht, worin T₀
- OC(O)(C₁-C₁₀-Alkyl)-ONO₂ oder -O(C₁-C₁₀-Alkyl)-ONO₂;
- Cycloalkylen mit 5 bis 7 Kohlenstoffatomen in einem Cycloalkylring ist, wobei der Ring gegebenenfalls mit Seitenketten T substituiert ist, worin T geradkettiges oder verzweigtes Alkyl mit 1 bis 10 Kohlenstoffatomen, vorzugsweise CH₃ ist;
b) 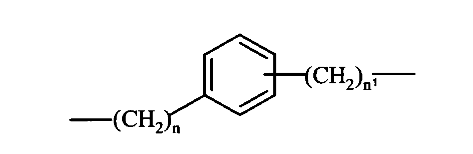
c) 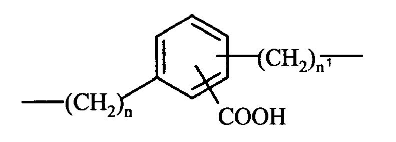 worin n eine ganze Zahl von 0 bis 20 ist, und n¹ eine ganze Zahl von 1 bis 20 ist;
d) 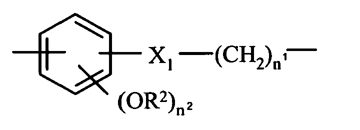 worin:
n¹ wie oben definiert ist, und n² eine ganze Zahl von 0 bis 2 ist;
X₁ = -OCO- oder -COO- und R² H oder CH₃ ist;
e) 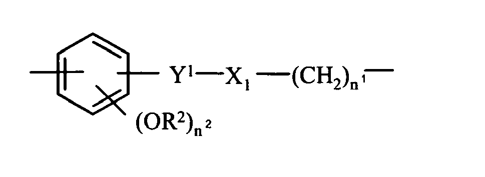 worin:
n¹, n², R² und X₁ wie oben definiert sind;
Y¹ -CH₂-CH₂- oder -CH=CH-(CH₂)ₙ²- ist;
f) 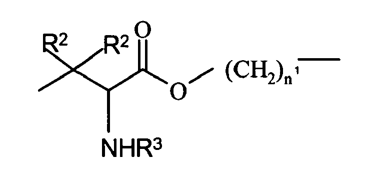 worin:
n¹ und R² wie oben definiert sind, R³ H oder -COCH₃ ist;
mit der Maßgabe, dass, wenn Y aus den bivalenten Resten ausgewählt ist, die unter b)-f) angegeben sind, die
-ONO₂-Gruppe mit einer -(CH₂)ₙ¹-Gruppe verbunden ist;
g) 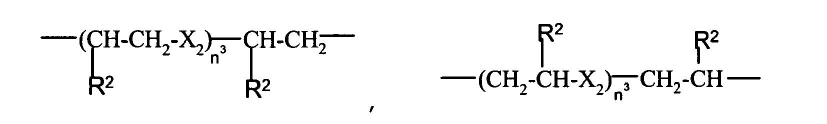 worin X₂ -O- oder -S- ist, n³ eine ganze Zahl von 1 bis 6 ist,
vorzugsweise von 1 bis 4, R² wie oben definiert ist;
h) 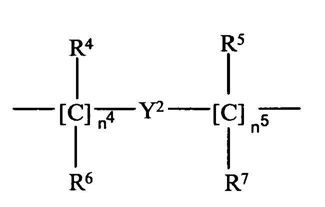 worin:
n⁴eine ganze Zahl von 0 bis 10 ist;
n⁵ eine ganze Zahl von 1 bis 10 ist;
R⁴, R⁵, R⁶, R⁷ gleich oder verschieden sind, und H oder ein geradkettiges oder verzweigtes C₁-C₄-Alkyl sind, vorzugsweise sind R⁴, R⁵, R⁶, R⁷ H;
worin die -ONO₂-Gruppe mit 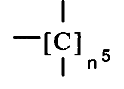 verbunden ist, worin n⁵ wie oben definiert ist;
Y² ist ein heterocyclischer, gesättigter, ungesättigter oder aromatischer 5- oder 6-gliedriger Ring, enthaltend ein oder mehrere Heteroatome, ausgewählt aus Stickstoff, Sauerstoff, Schwefel, und ist ausgewählt aus:

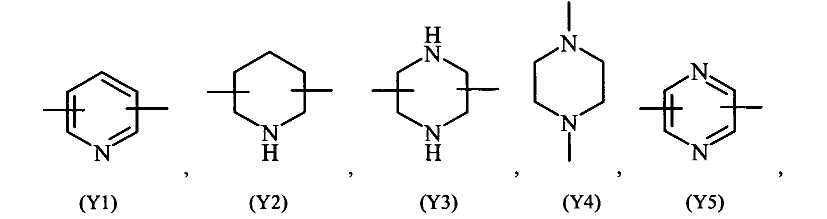

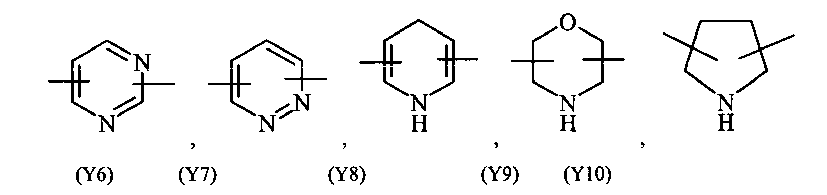

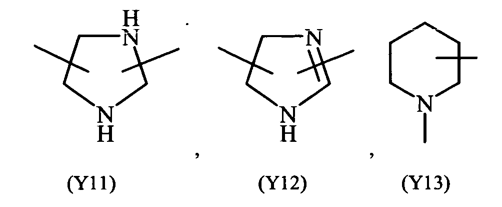

2. Verbindung der allgemeinen Formel (I) oder ein pharmazeutisch verträgliches Salz oder Stereoisomer davon nach Anspruch 1, worin Y ein bivalenter Rest mit der folgenden Bedeutung ist:
a) geradkettiges oder verzweigtes C₁-C₁₀-Alkylen, gegebenenfalls mit T₀ substituiert, worin T₀ wie oben definiert ist;
b) 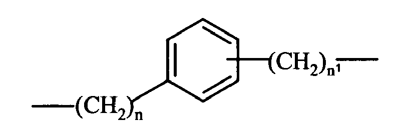 worin n eine ganze Zahl ist, die 0 oder 1 entspricht, und n¹ eine ganze Zahl ist, die 1 entspricht; mit der Maßgabe, dass die -ONO₂-Gruppe mit einer -(CH₂)ₙ¹-Gruppe verbunden ist;
g) 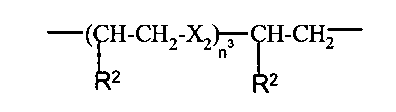 worin X₂ -O- oder -S- ist, n³ eine ganze Zahl ist, die 1 entspricht, und R² H ist.

3. Verbindung, ausgewählt aus der Gruppe, die besteht aus: 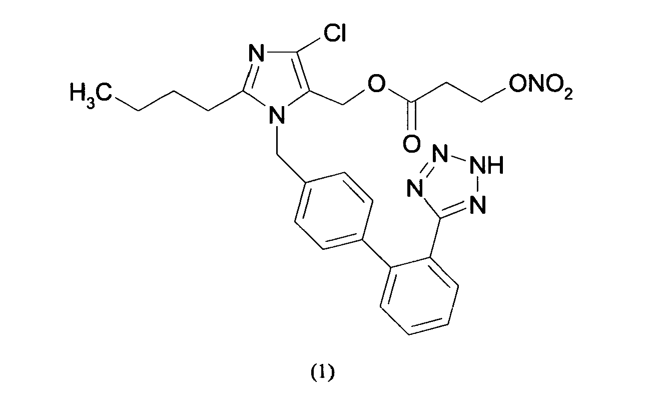 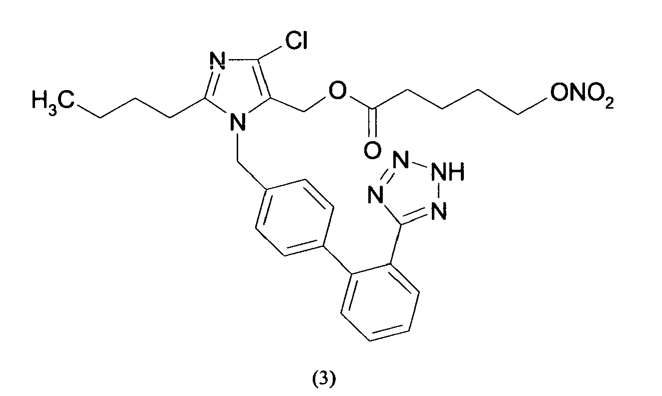 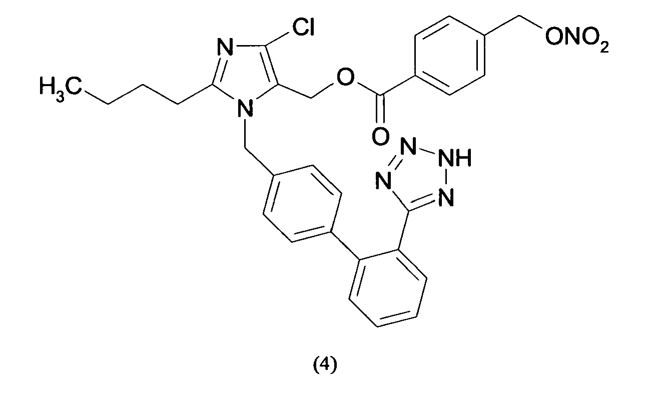 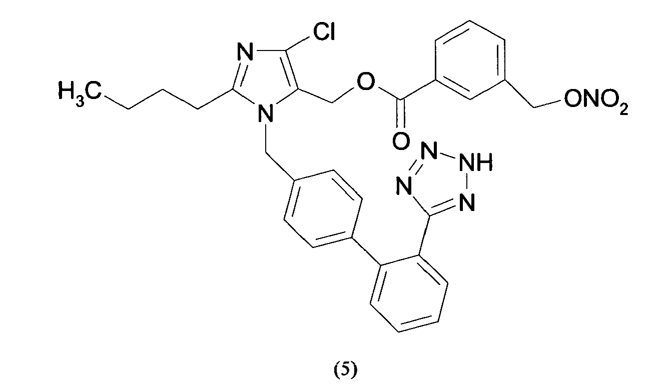 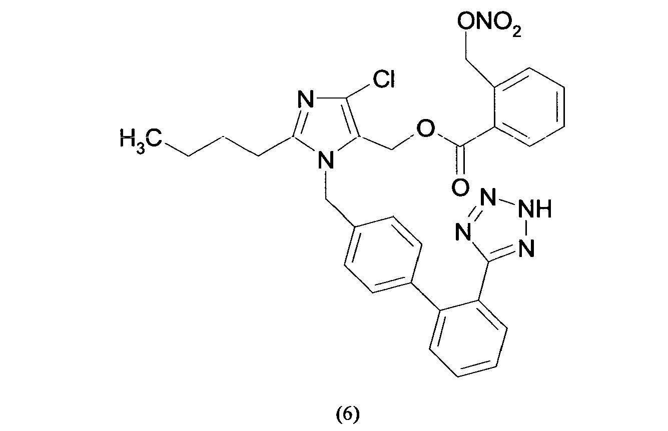 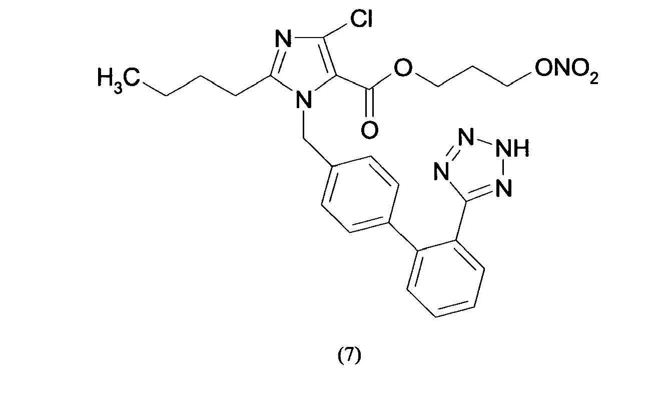 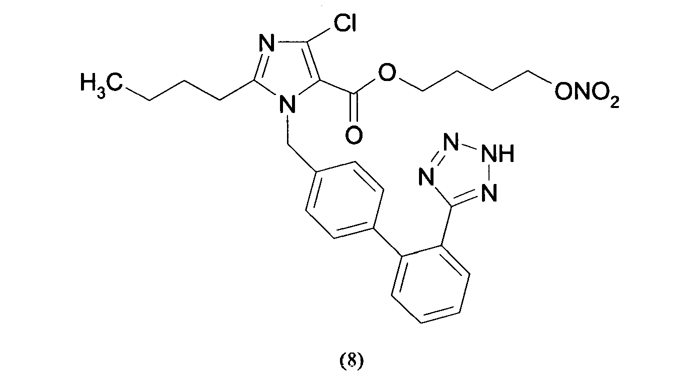 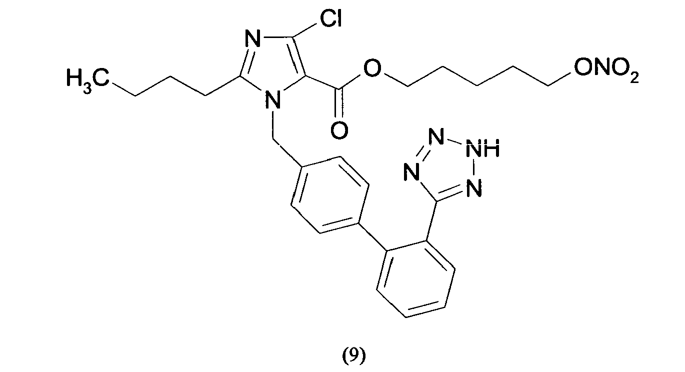 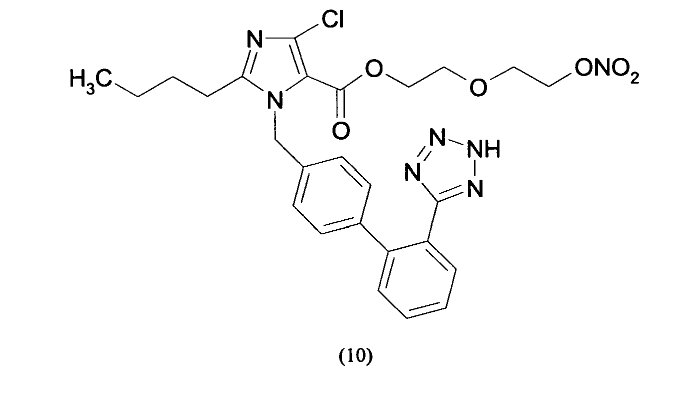 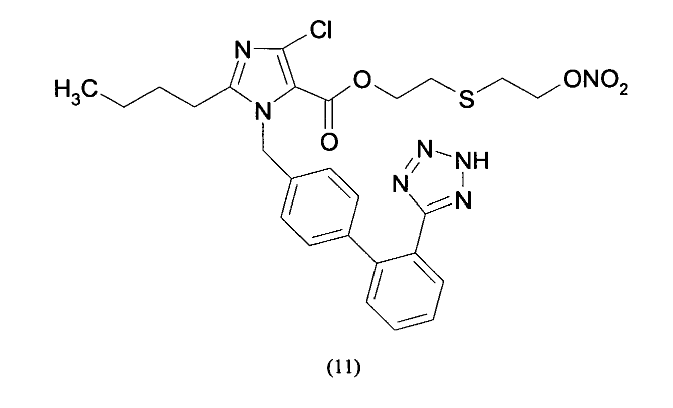 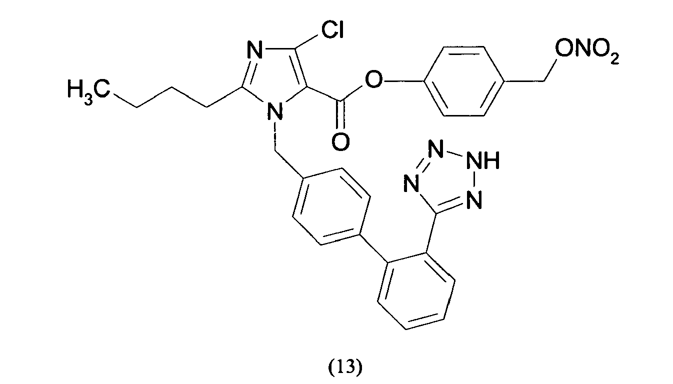 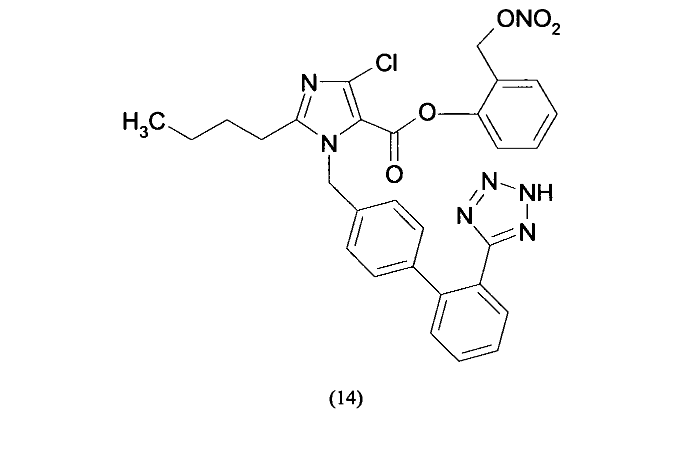 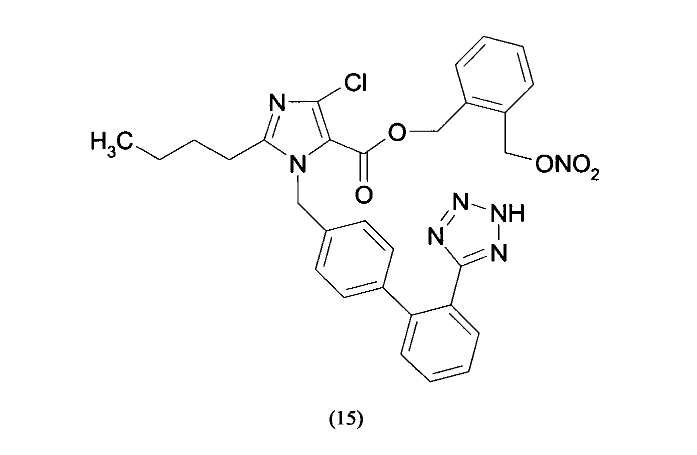 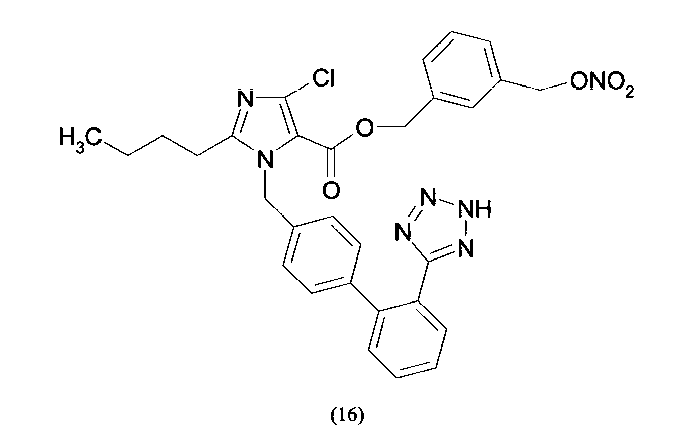 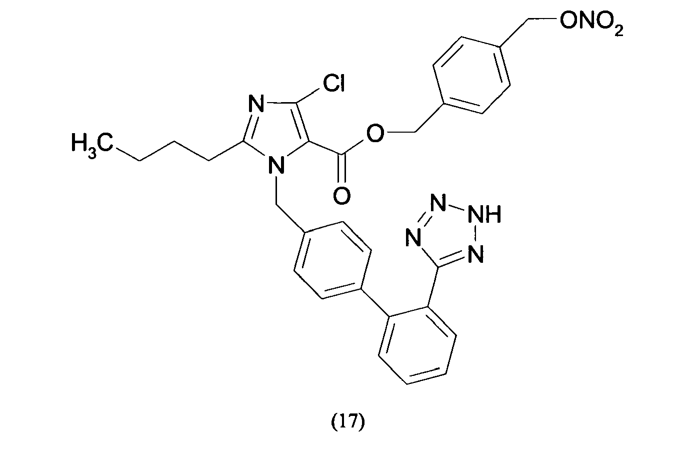 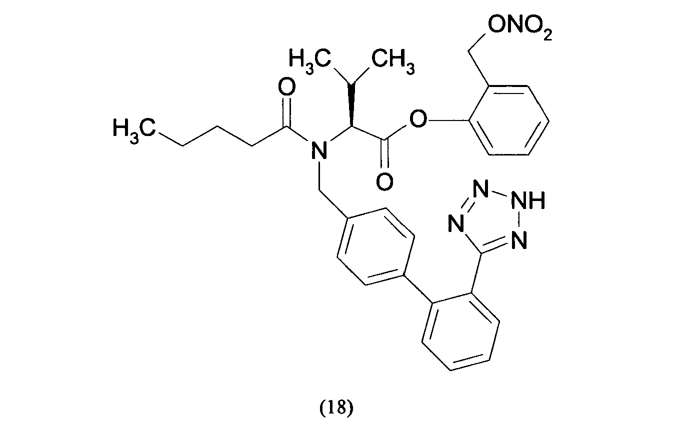 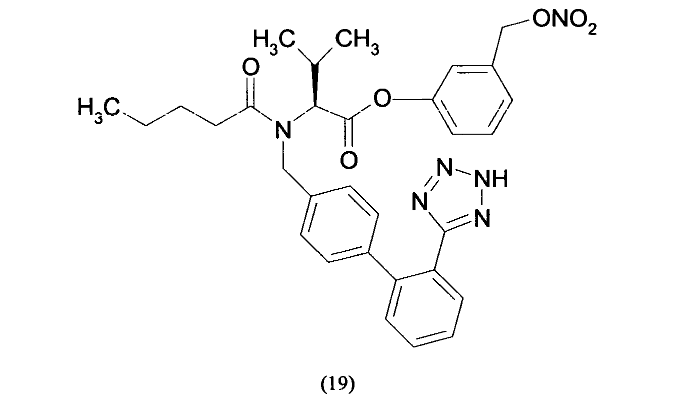 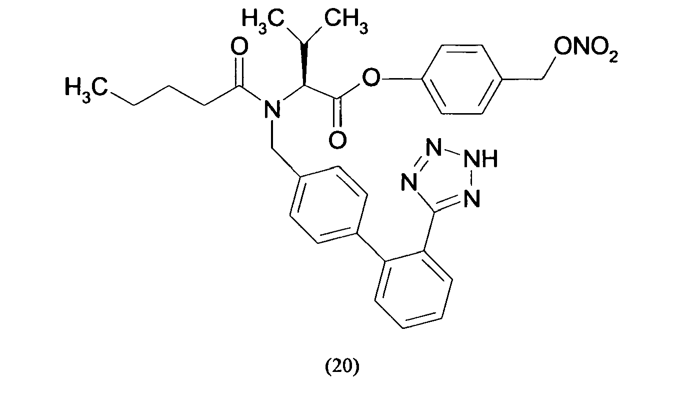 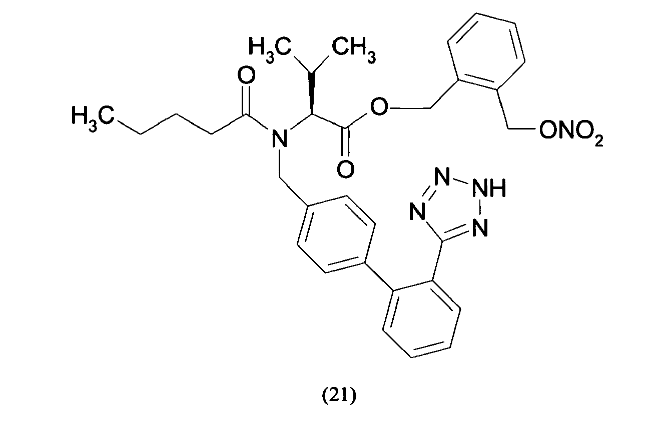 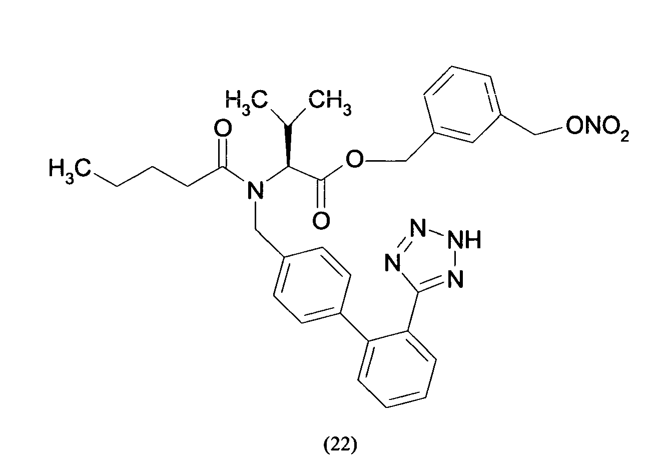 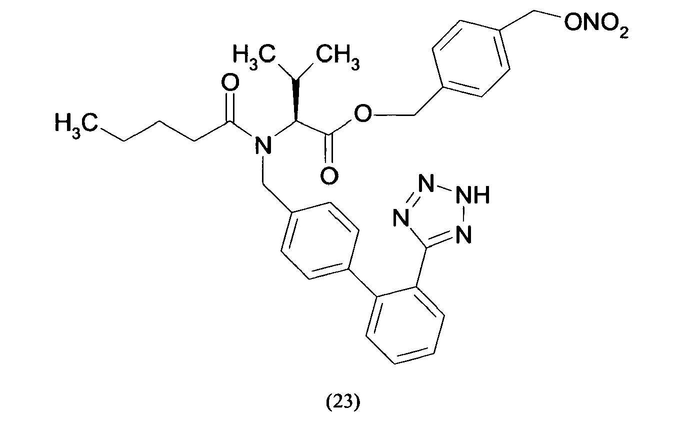 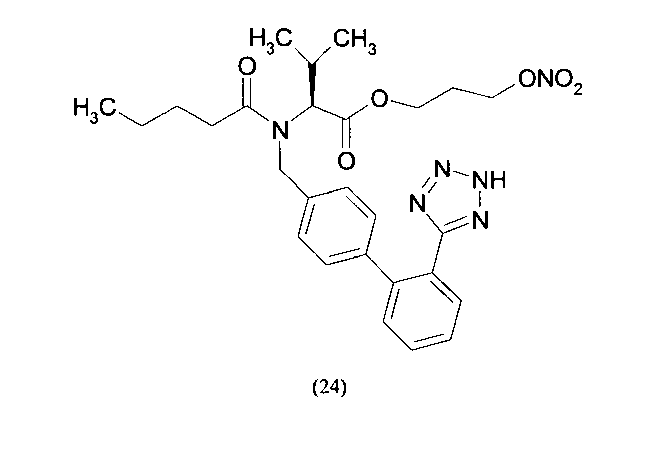 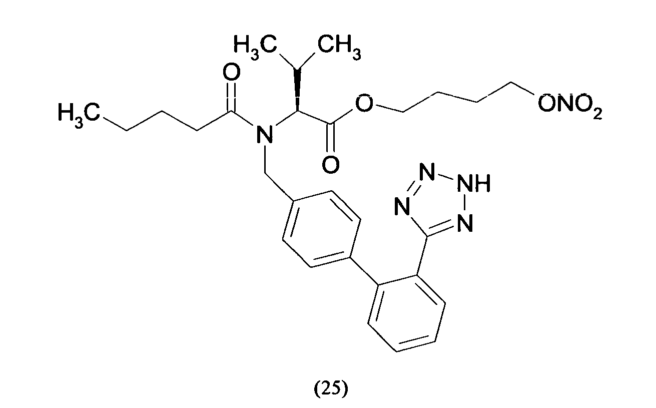 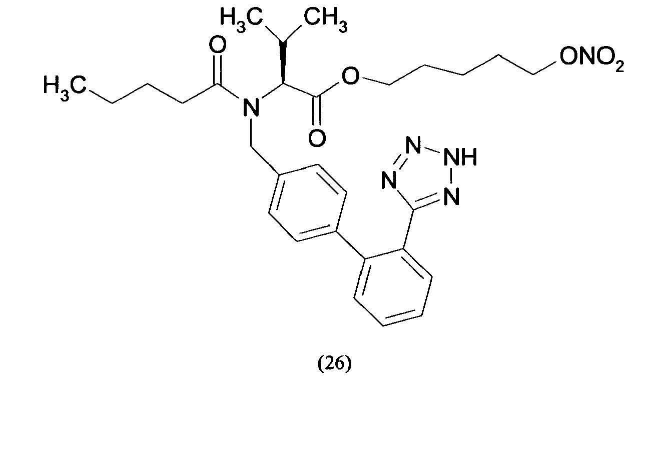 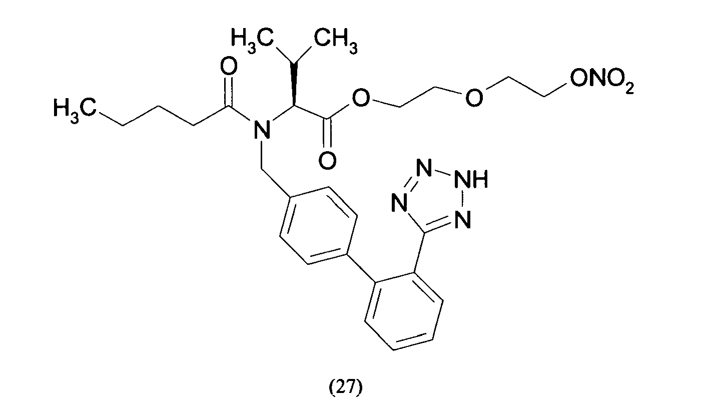 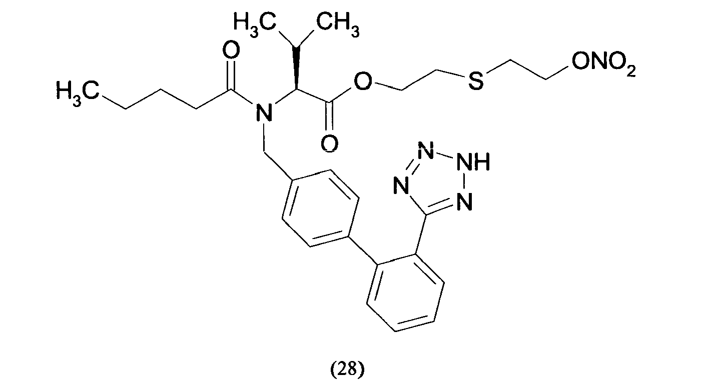 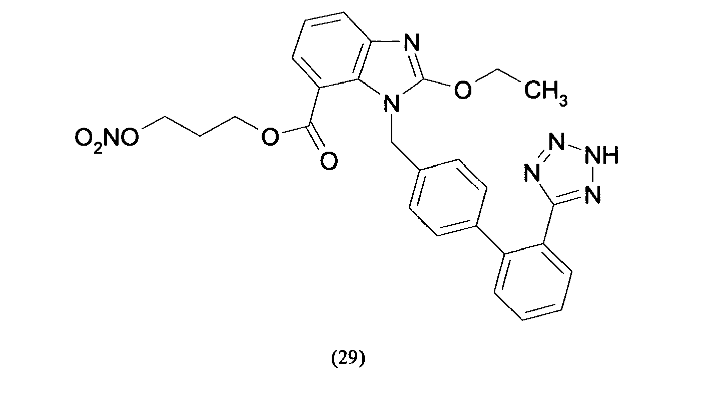 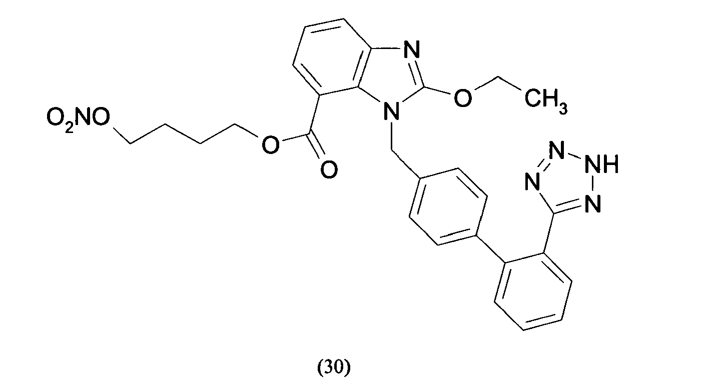 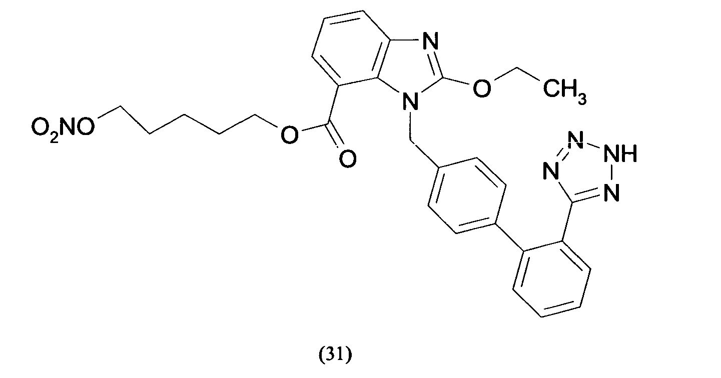 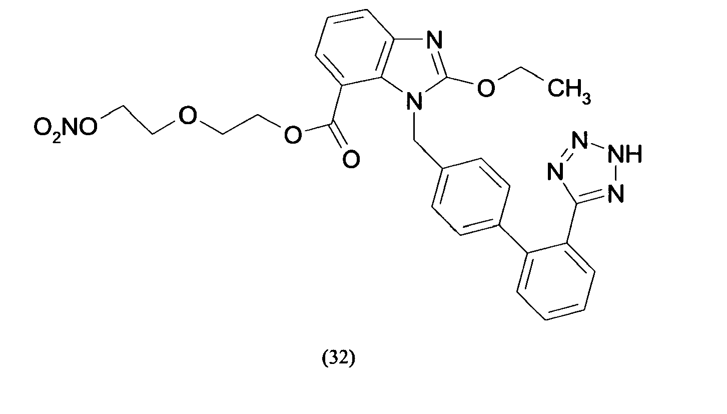 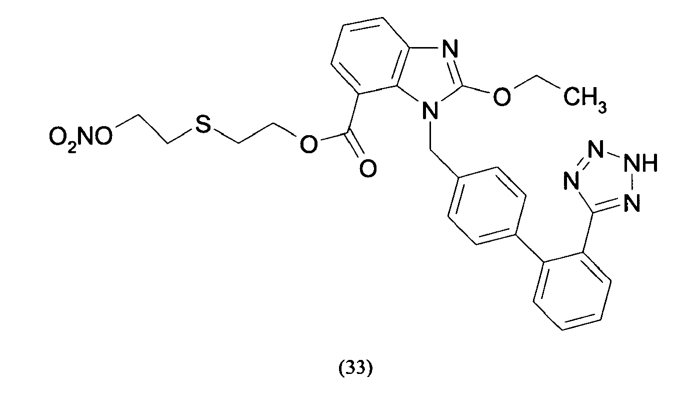 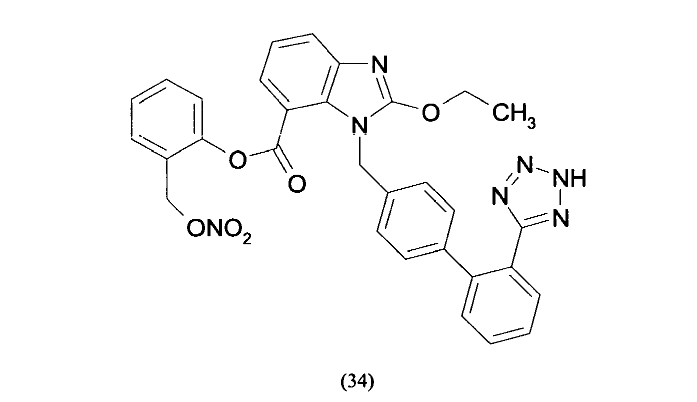 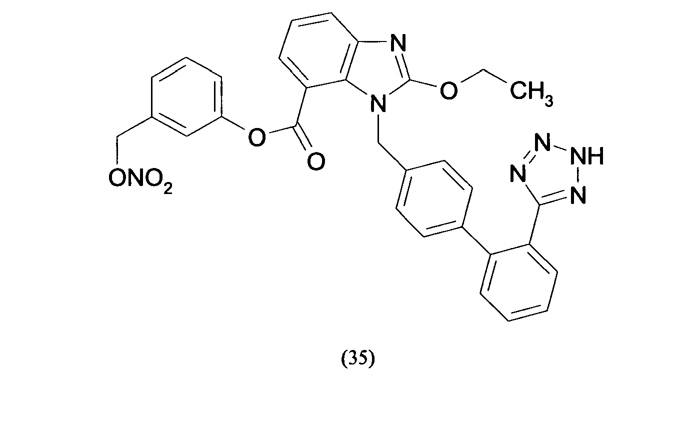 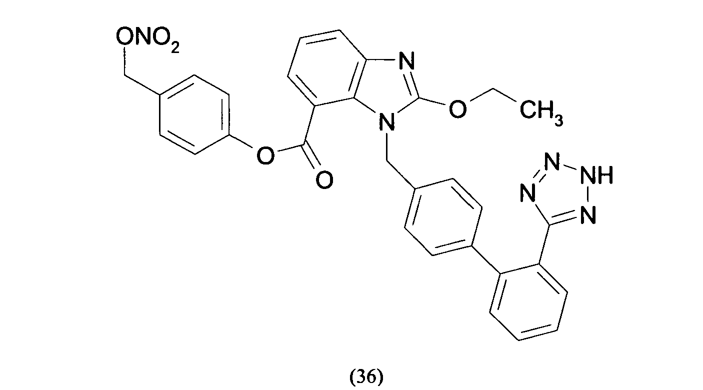 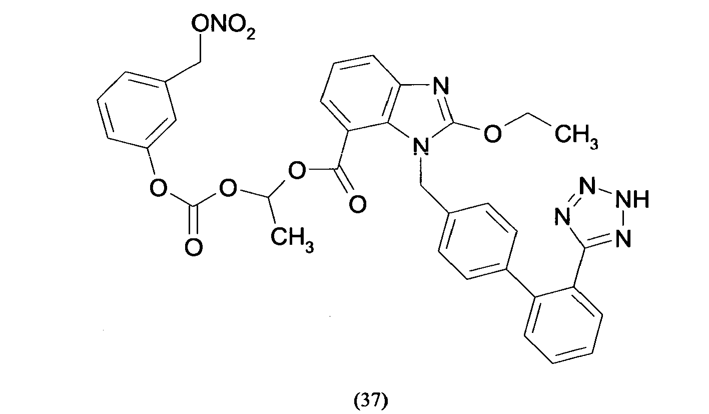 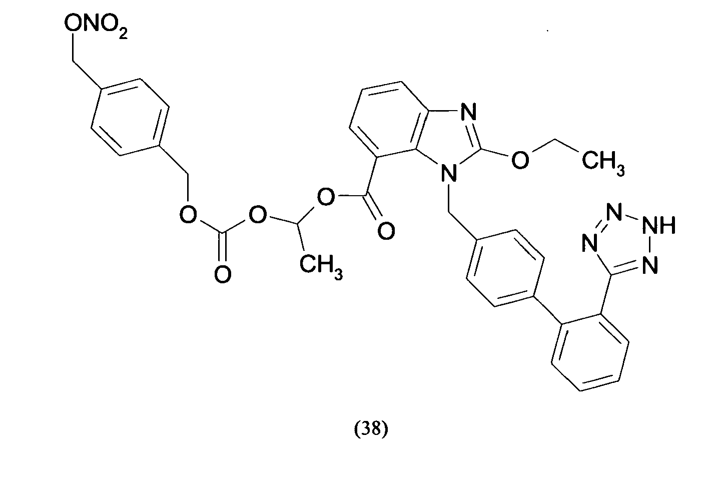 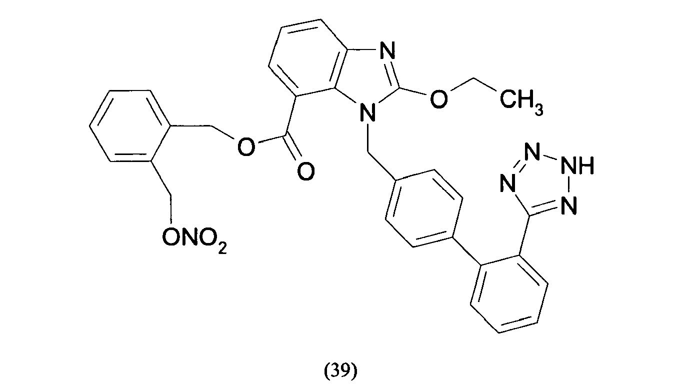 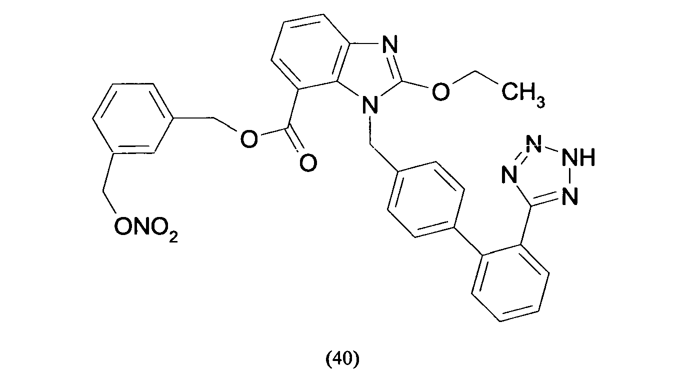 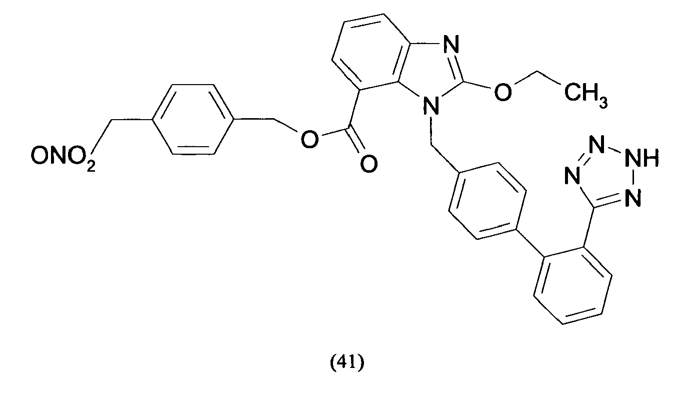 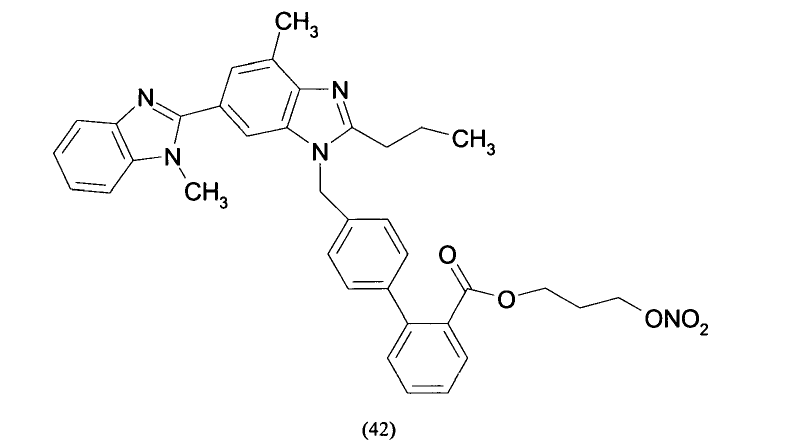 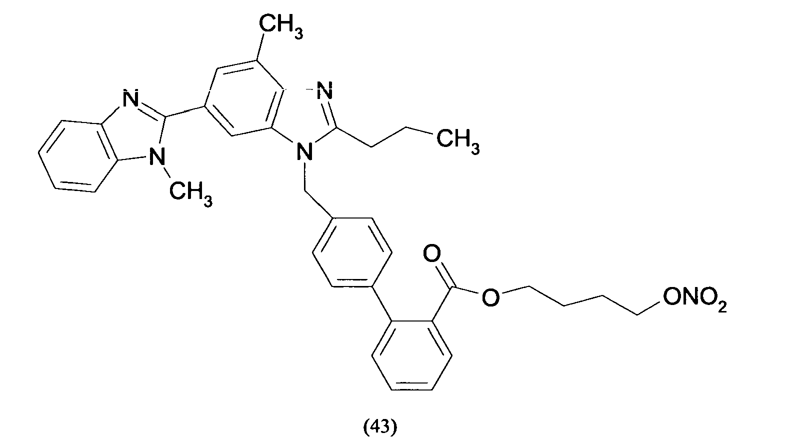 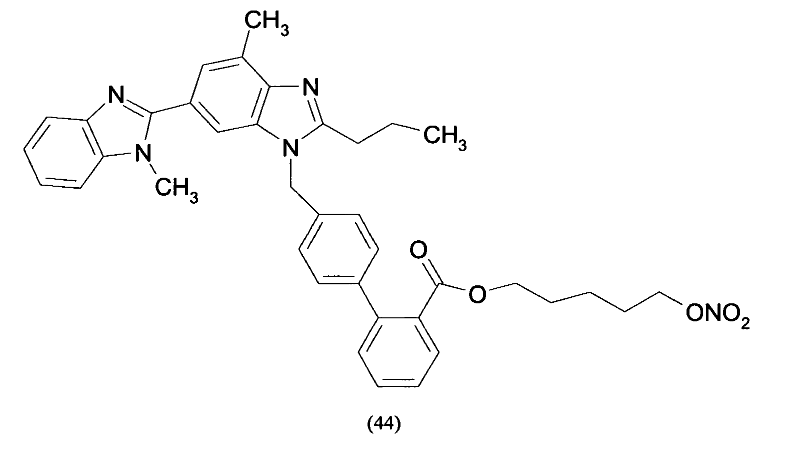 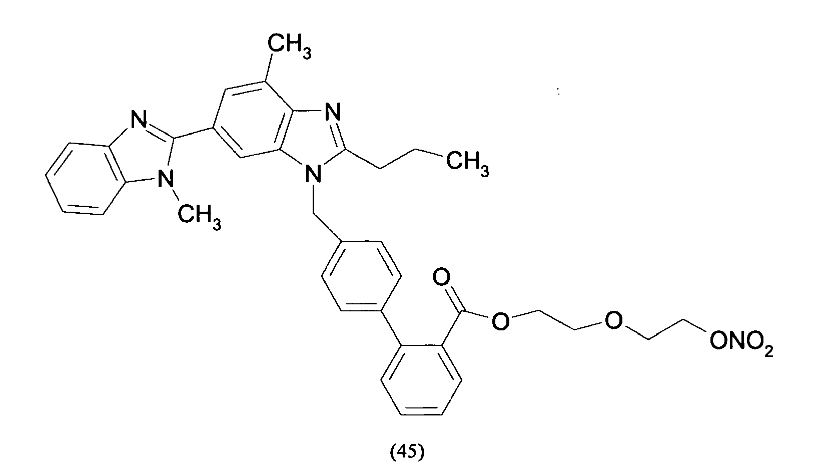 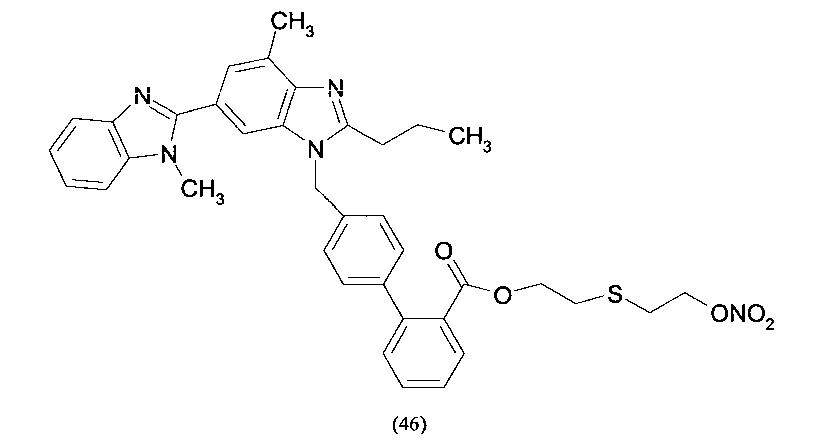 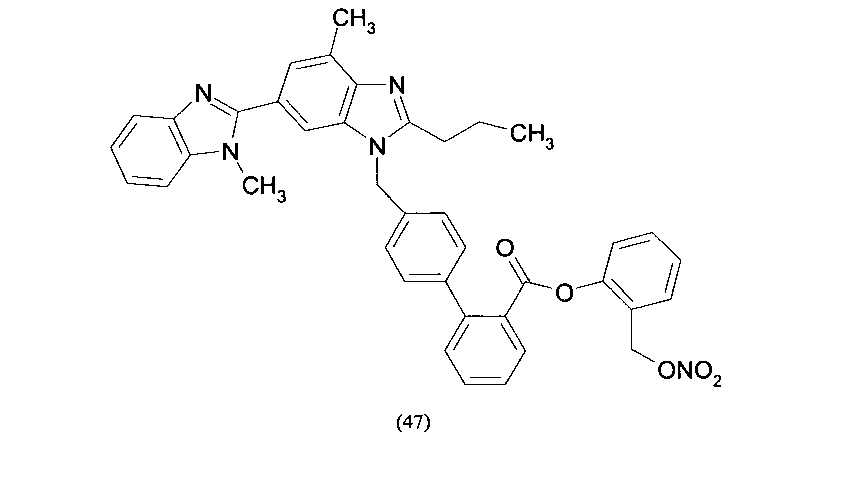 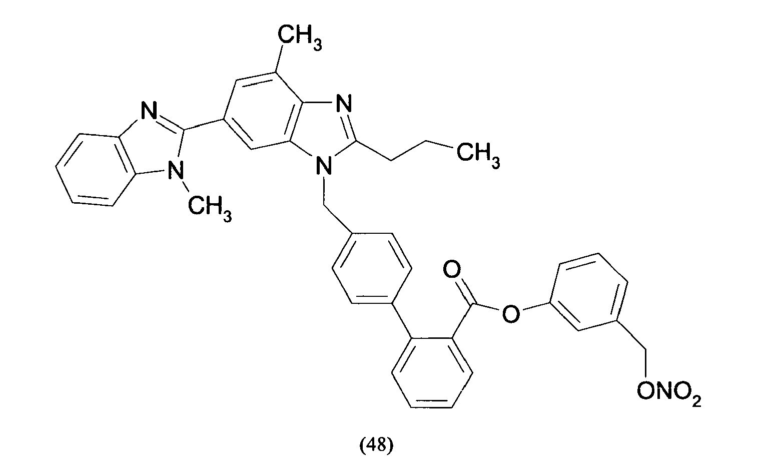 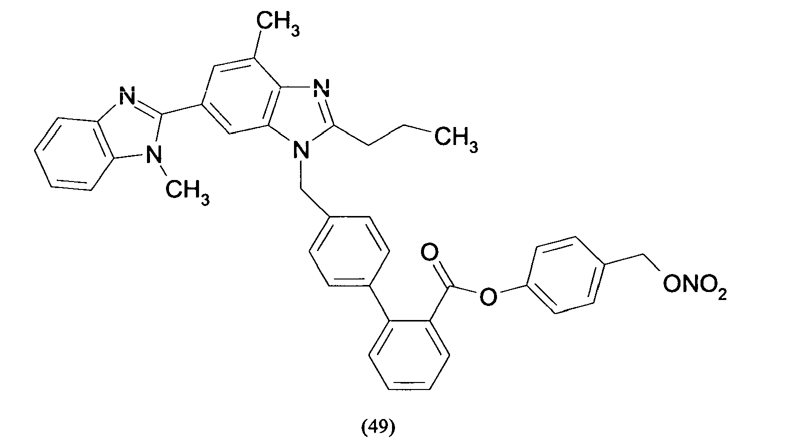 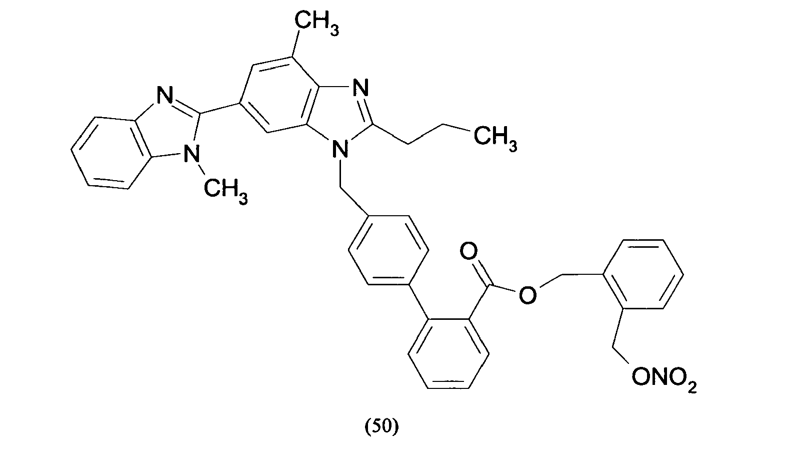 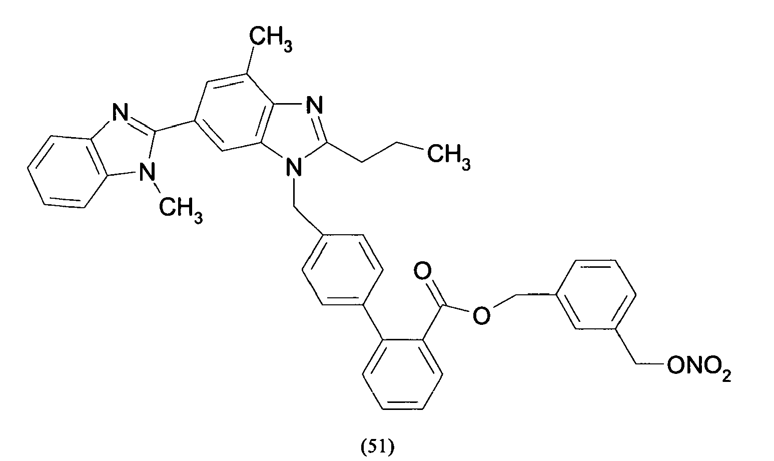 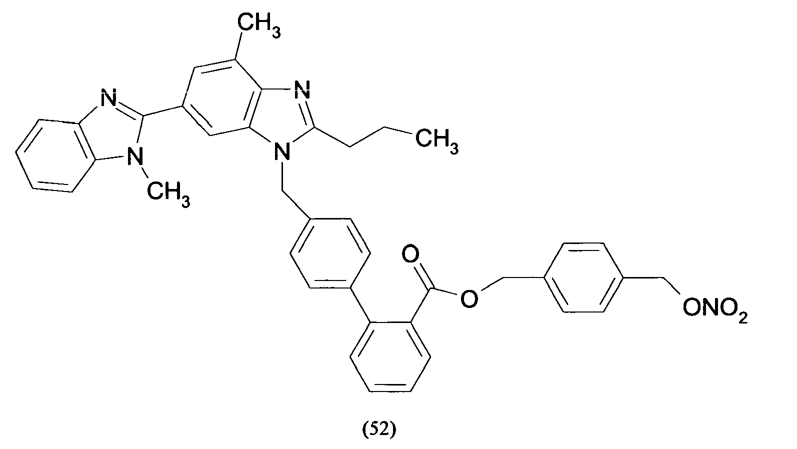 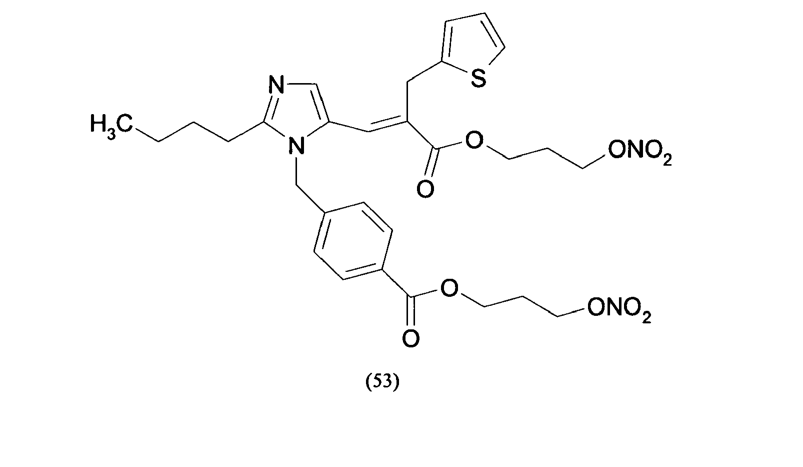 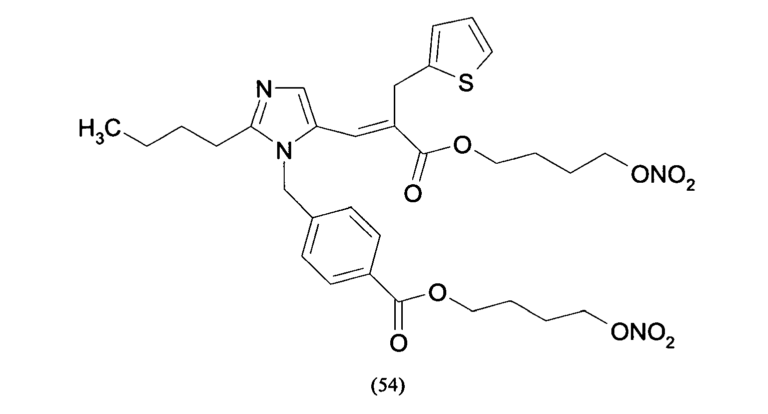 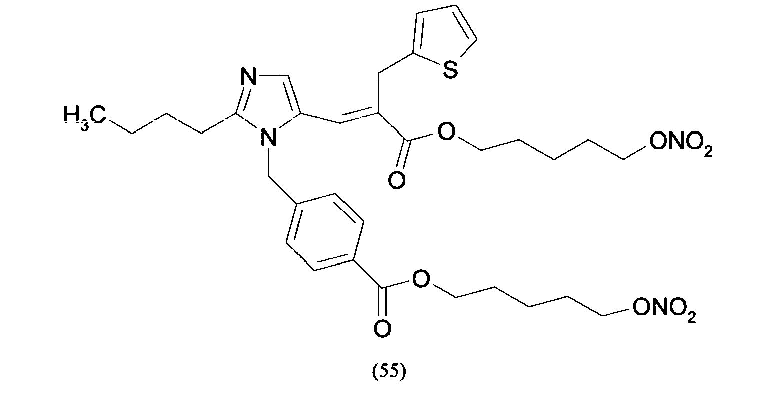 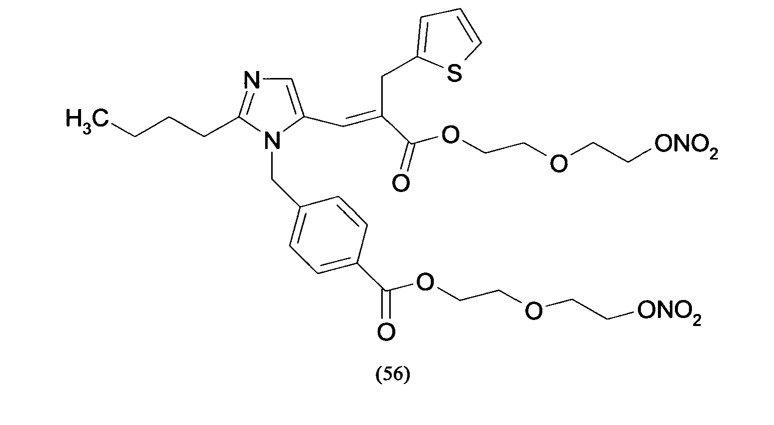 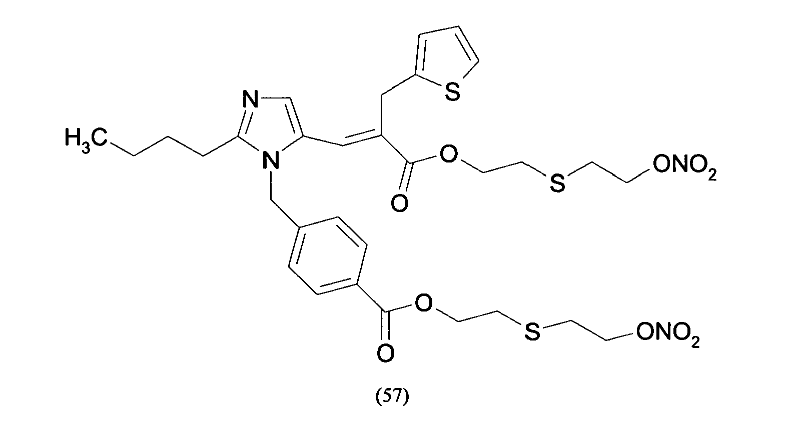 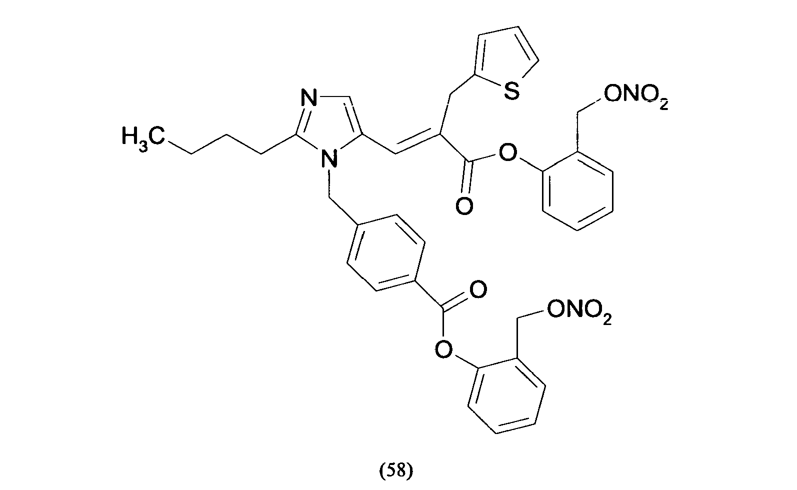 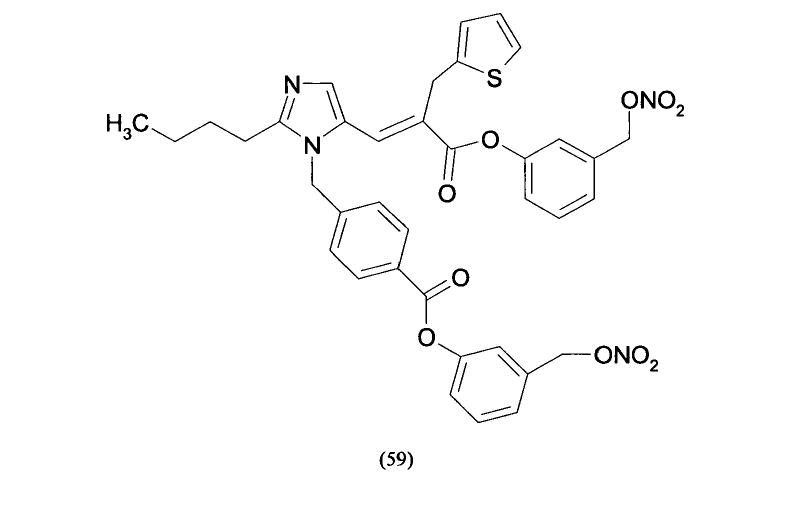 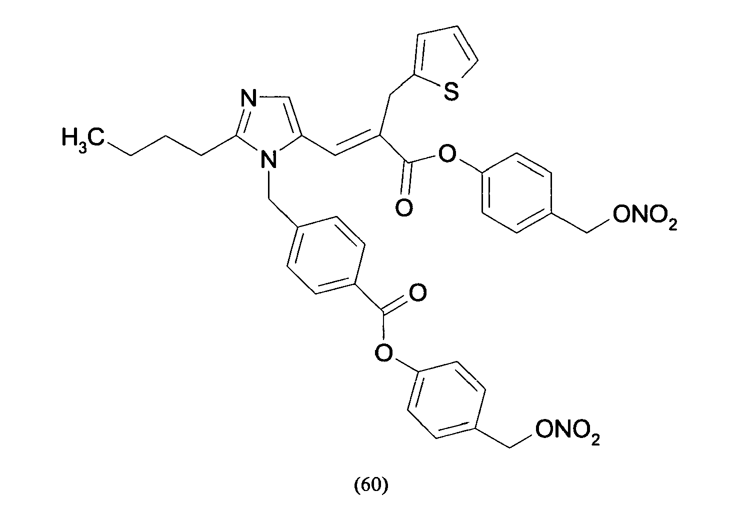 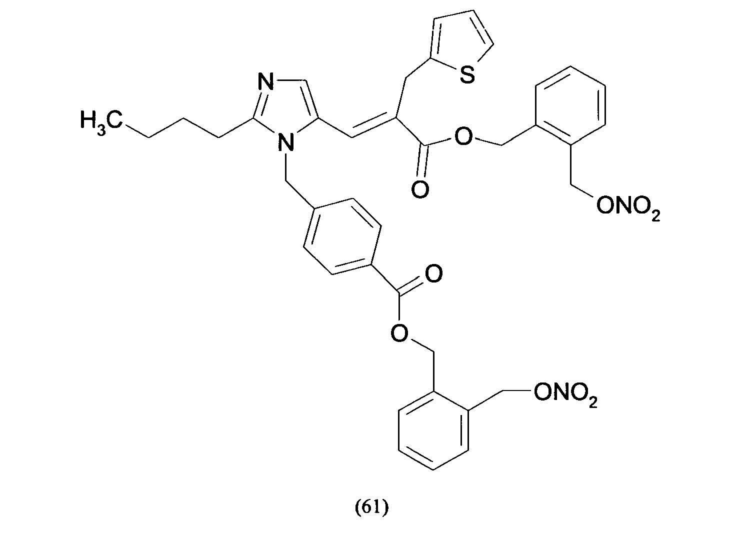 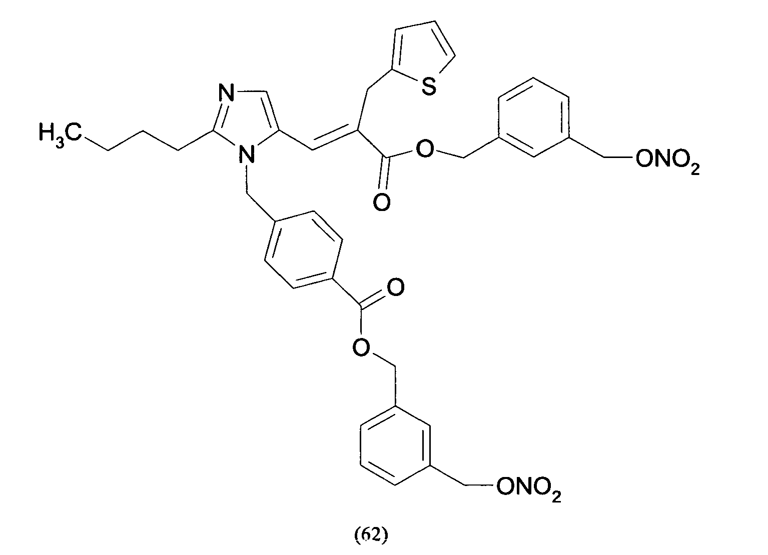 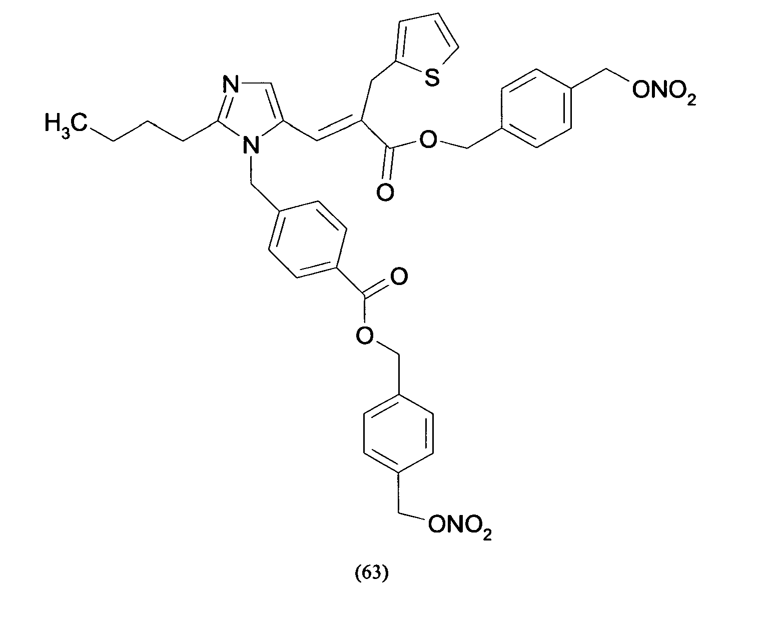 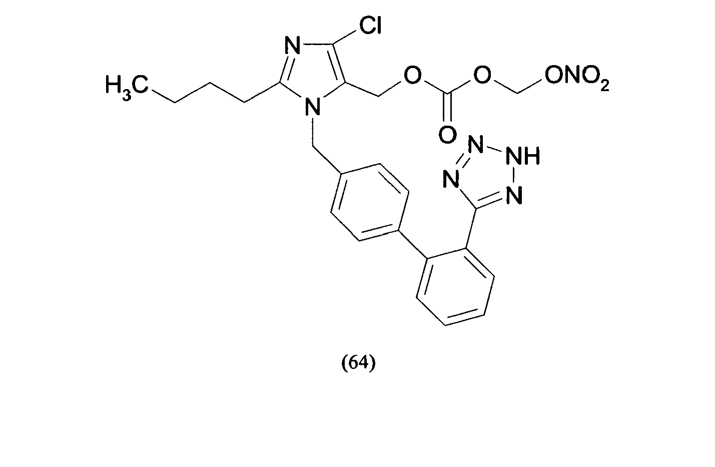 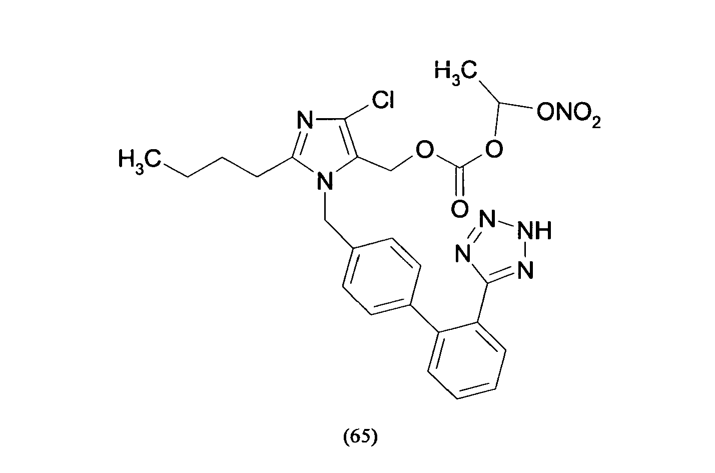 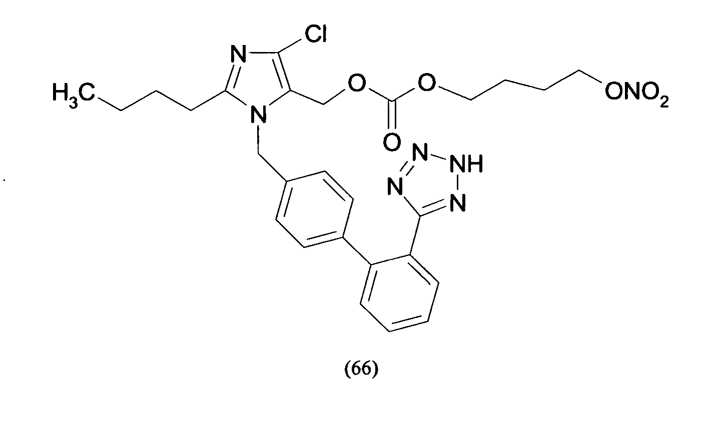 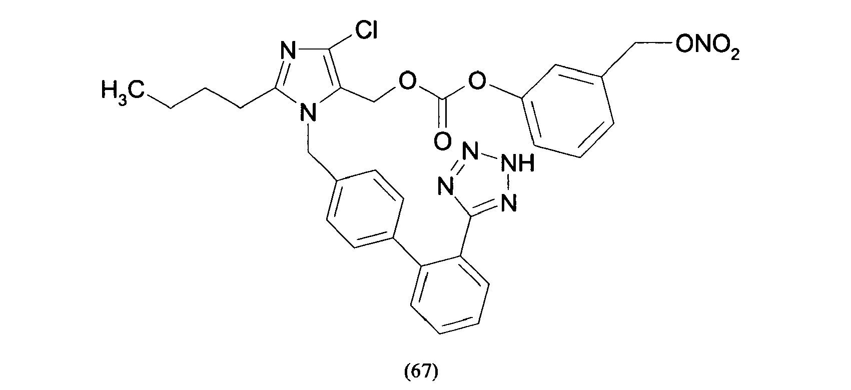 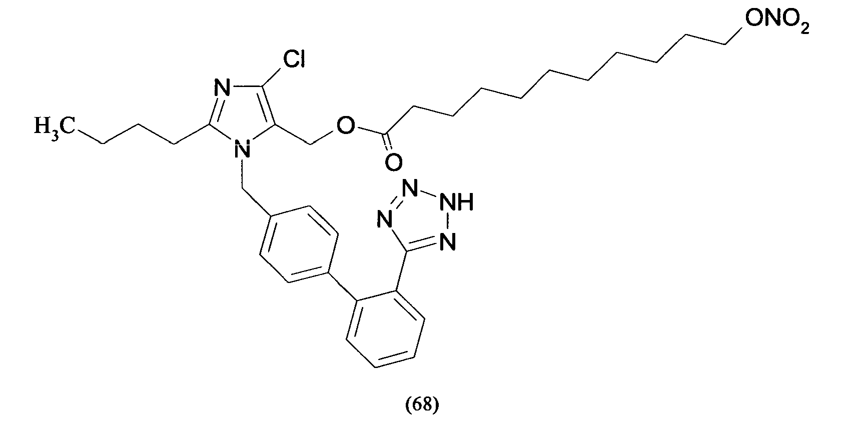 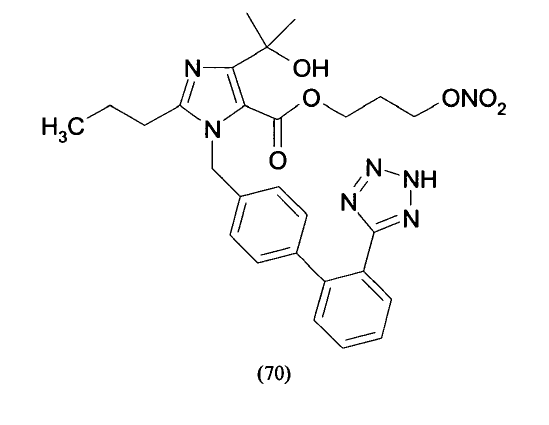 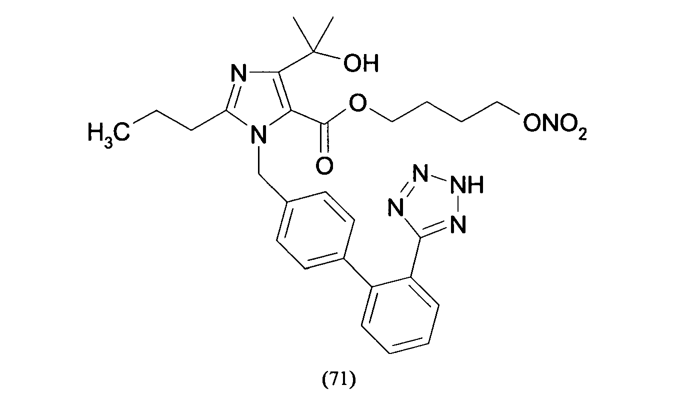 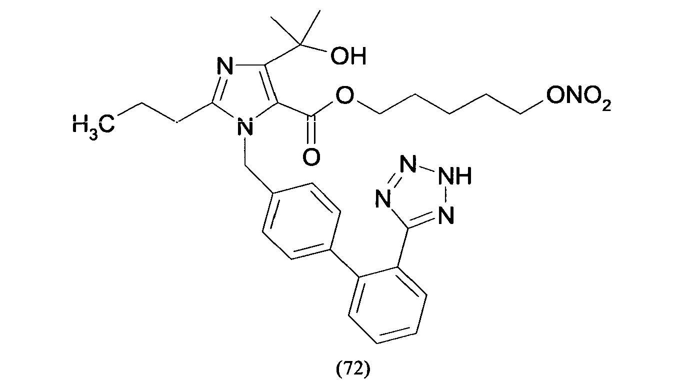 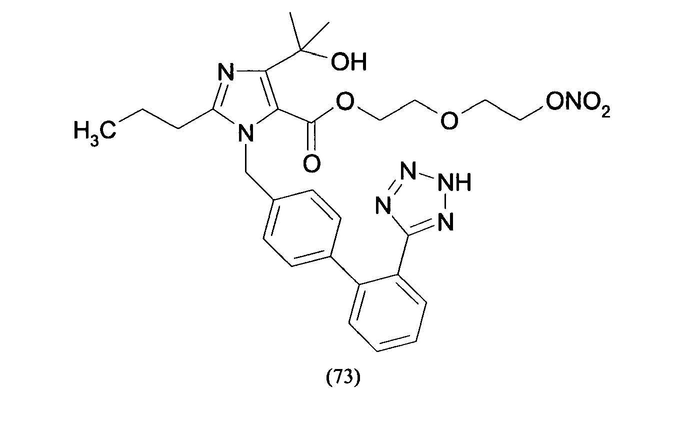 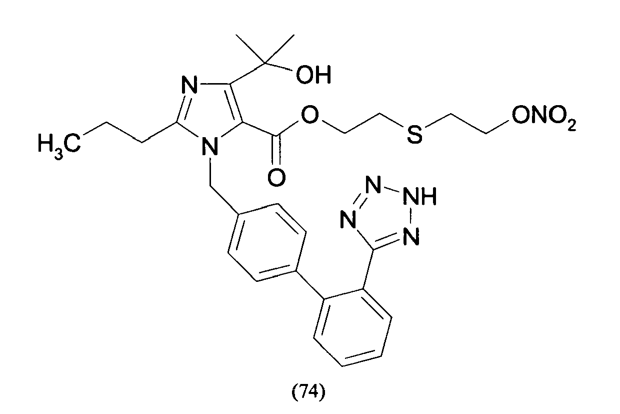 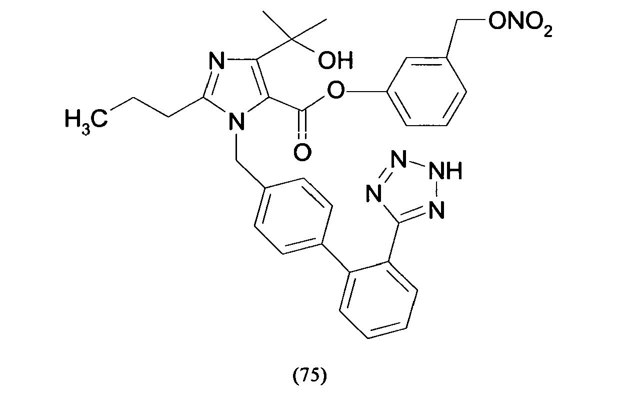 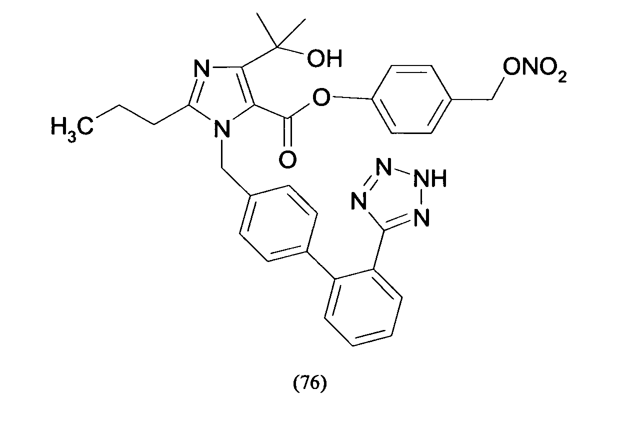 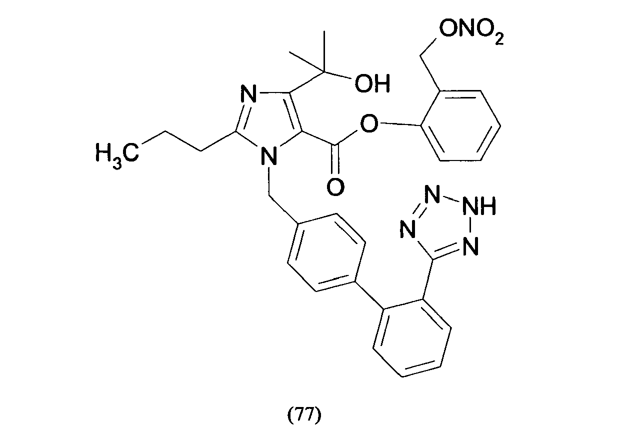 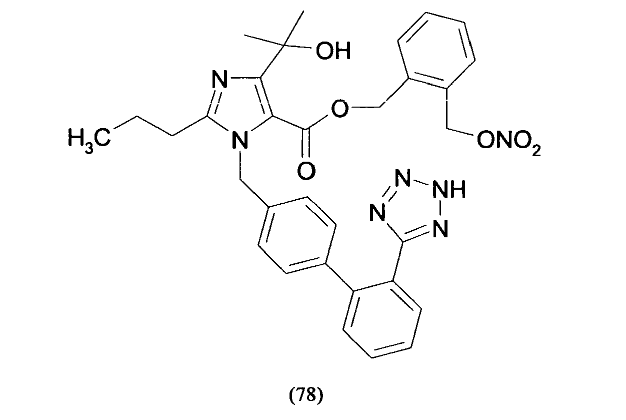 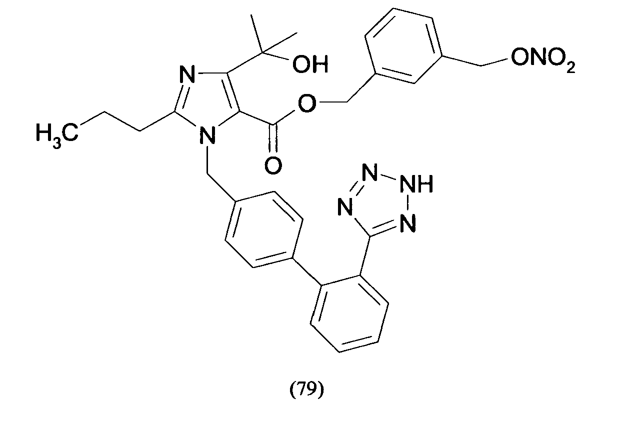 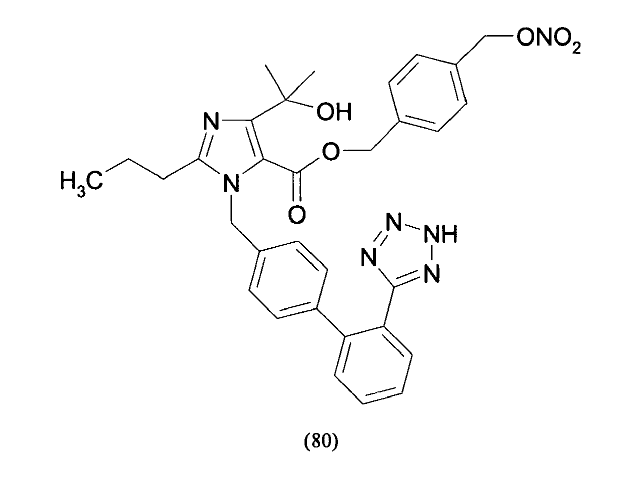 oder ein pharmazeutisch verträgliches Salz oder Stereoisomer davon.

4. Verbindung der Formel (2): 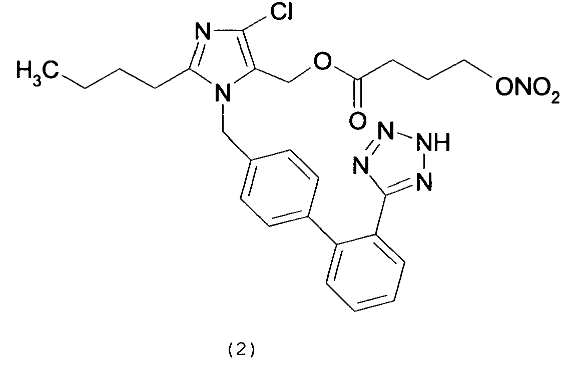 oder ein pharmazeutisch verträgliches Salz oder Stereoisomer davon.

5. Verbindung der Formel (12): 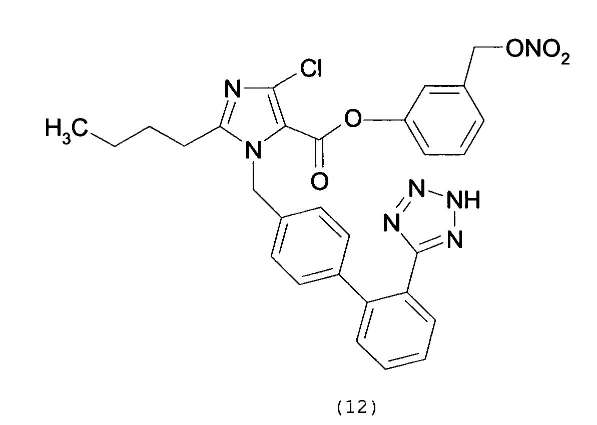 oder ein pharmazeutisch verträgliches Salz oder Stereoisomer davon.

6. Verbindung der Formel (69): 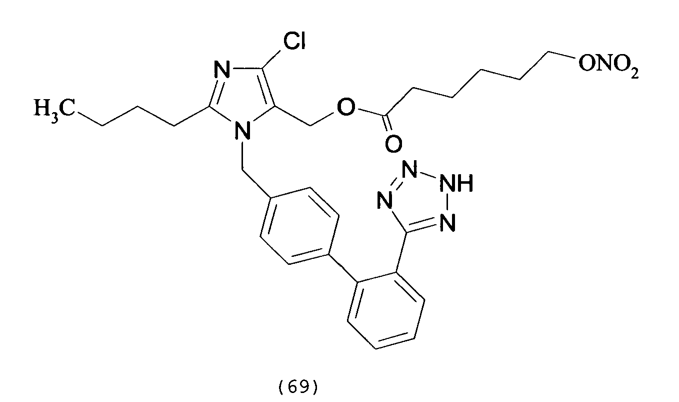 oder ein pharmazeutisch verträgliches Salz oder Stereoisomer davon.

7. Verbindung der allgemeinen Formel (I) nach den Ansprüchen 1 - 6 für die Verwendung als ein Medikament.

8. Verwendung einer Verbindung nach den Ansprüchen 1 - 6 zur Herstellung eines Medikaments mit einer anti-entzündlichen, anti-thrombotischen und anti-thrombozytären Aktivität.

9. Verwendung einer Verbindung nach den Ansprüchen 1 - 6 zur Herstellung eines Medikaments, das bei der Behandlung oder Prophylaxe von kardiovaskulären, Nieren- und chronischen Leber-Erkrankungen, entzündlichen Prozessen und metabolischen Syndromen verwendet werden kann.

10. Verwendung einer Verbindung nach Anspruch 9 zur Herstellung eines Medikaments, das bei der Behandlung oder Prophylaxe von Herzversagen, Myokardinfarkt, Hirnschlag, Atherosklerose, Augen- und Lungenhypertension, Hypertension, diabetischer Nephropathie, peripheralen vaskulären Krankheiten, links-ventrikulärer Dysfunktion und Hypertrophie, Leberfibrose und Pfortaderhypertension verwendet werden kann.

11. Pharmazeutische Zusammensetzung, die einen pharmazeutisch verträglichen Träger und eine pharmazeutisch wirksame Menge einer Verbindung der allgemeinen Formel (I) oder ein Salz oder Stereoisomer davon nach den Ansprüchen 1-6 umfasst.

12. Pharmazeutische Zusammensetzung nach Anspruch 11 in einer geeigneten Form für die orale, parenterale, rektale, topische und transdermale Verabreichung, über ein Inhalationsspray oder Aerosol oder über iontophoretische Vorrichtungen.

13. Flüssige oder feste pharmazeutische Zusammensetzung für die orale, parenterale, rektale, topische und transdermale Verabreichung oder Inhalation in der Form von Tabletten, Kapseln und Pillen, schließlich mit einer enterischen Beschichtung, Pulvern, Granulaten, Gelen, Emulsionen, Lösungen, Suspensionen, Sirupen, Elixieren, injizierbare Formen, Zäpfchen, in transdermalen Pflastern oder Liposomen, enthaltend eine Verbindung der Formel (I) oder ein Salz oder Stereoisomer davon nach den Ansprüchen 1-6 und einen pharmazeutisch verträglichen Träger.

14. Pharmazeutische Zusammensetzung, umfassend eine Verbindung der allgemeinen Formel (I), wenigstens eine Verbindung, die verwendet wird, um eine kardiovaskuläre Krankheit zu behandeln, und einen pharmazeutisch verträglichen Träger.

15. Pharmazeutische Zusammensetzung nach Anspruch 14, worin die Verbindung, die verwendet wird, um eine kardiovaskuläre Krankheit zu behandeln, ausgewählt ist aus der Gruppe, bestehend aus: ACE-Inhibitoren, HMGCoA-Reduktaseinhibitoren, β-adrenergen Blockern, Calcium-Kanalblockern, Diuretika, Antithrombotika, wie Aspirin, nitrosierten ACE-Inhibitoren, nitrosierten HMGCoA-Reduktaseinhibitoren, nitrosierten β-adrenergen Blockern, nitrosiertem Aspirin und nitrosierten Diuretika.

16. Pharmazeutischer Kit, umfassend eine Verbindung der allgemeinen Formel (I), wie in Anspruch 1 definiert, eine Verbindung, die verwendet wird, um eine kardiovaskuläre Krankheit zu behandeln, als kombinierte Präparation für die gleichzeitige, getrennte, nach einander folgende Verwendung für die Behandlung einer kardiovaskulären Krankheit.

17. Pharmazeutischer Kit nach Anspruch 16, worin die Verbindung, die verwendet wird, um eine kardiovaskuläre Krankheit zu behandeln, aus der Gruppe ausgewählt ist, bestehend aus: ACE-Inhibitoren, HMGCoA-Reduktaseinhibitoren, β-adrenergen Blockern, Calcium-Kanalblockern, Diuretika, Antithrombotika, wie Aspirin, nitrosierten ACE-Inhibitoren, nitrosierten HMGCoA-Reduktaseinhibitoren, nitrosierten β-adrenergen Blockern, nitrosiertem Aspirin und nitrosierten Diuretika.

## Revendications

1. Composé de formule générale (I) ou sel pharmaceutiquement acceptable ou stéréoisomère de celui-ci:
R-(Y-ONO₂)ₛ (I)
dans laquelle :
s est un entier égal à 1 ou 2 ;
R est choisi parmi les résidus d'agents bloquants du récepteur de l'Angiotensine II de formule (II) ou (III) : 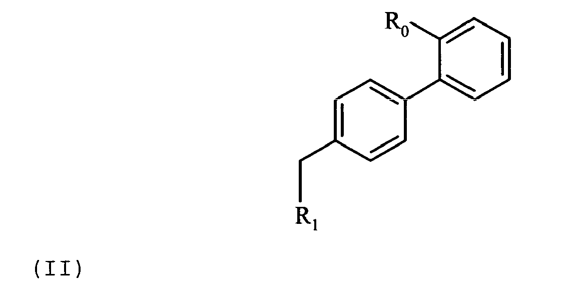 dans laquelle:
R₀ est 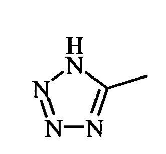 ou -N₀ qui est un groupe capable de se lier à Y, ayant une des significations suivantes :
-COO-, -O-, -CONH-, -OCO-, -OCOO- ou 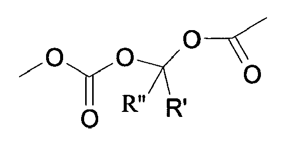 où R' et R" sont identiques ou différents, et sont H ou alkyle en C₁-C₄ linéaire ou ramifié ;
R₁ est choisi dans le groupe constitué par : 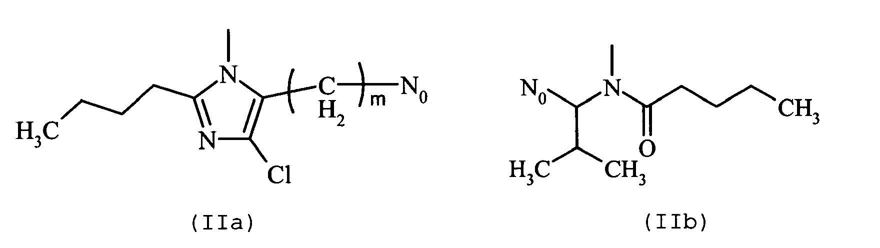 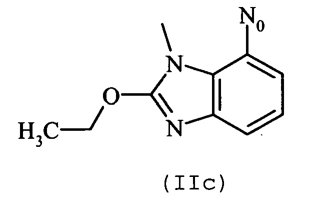 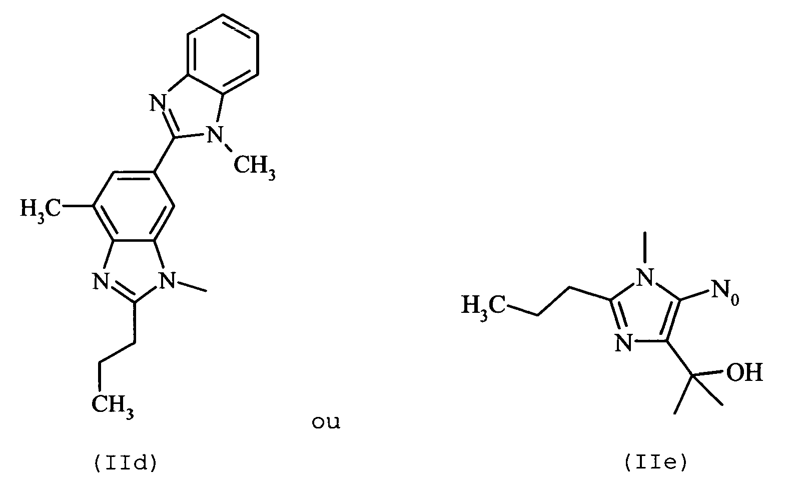 où m est un entier égal à 0 ou 1 et N₀ est tel que défini ci-dessus ; 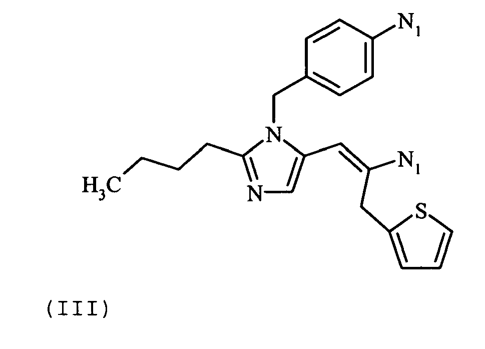 où N₁ a la même signification que N₀ ou est égal à -COOH; avec la réserve qu'au moins un des groupes N₁ est égal à -COO- ou -CONH-, c'est-à-dire, que c'est un groupe capable de se lier à Y ;
Y est un radical bivalent ayant la signification suivante
a)
- alkylène en C₁-C₂₀ linéaire ou ramifié, de préférence en C₁-C₁₀, éventuellement substitué par un ou plusieurs des substituants choisis dans le groupe constitué par : les atomes d'halogène, hydroxy, -ONO₂ et T₀, où T₀ est
- OC(O) (alkyl en C₁-C₁₀)-ONO₂ ou -O(alkyl en C₁-C₁₀)-ONO₂ ;
- cycloalkylène avec 5 à 7 atomes de carbone dans le cycle cycloalkylène, le cycle étant éventuellement substitué par des chaînes latérales T, où T est alkyle linéaire ou ramifié avec de 1 à 10 atome(s) de carbone, de préférence CH₃;
b) 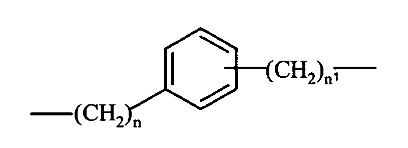
c) 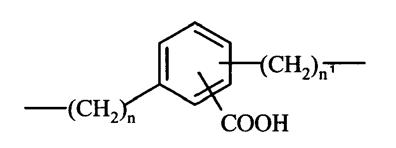 où n est un entier de 0 à 20, et n¹ est un entier de 1 à 20 ;
d) 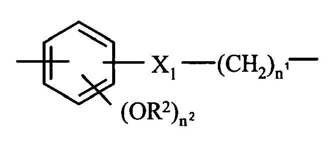 où :
n¹ est tel que défini ci-dessus et n² est un entier de 0 à 2 ;
X₁ = -OCO- ou -COO- et R² est H ou CH₃ ;
e) 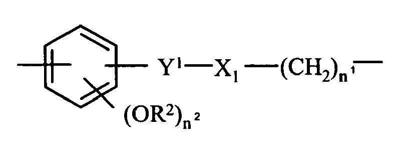 où :
n¹, n², R² et X₁ sont tels que définis ci-dessus;
Y¹ est -CH₂-CH₂- ou -CH=CH-(CH²)ₙ²- ;
f) 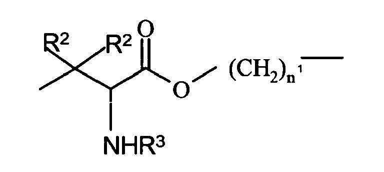 dans laquelle:
n¹ et R² sont tels que définis ci-dessus, R³ est H ou -COCH₃ ;
avec la réserve que lorsque Y est choisi parmi les radicaux bivalents mentionnés sous b)-f), le groupe -ONO₂ est lié à un groupe -(CH₂)ₙ¹ ;
g) 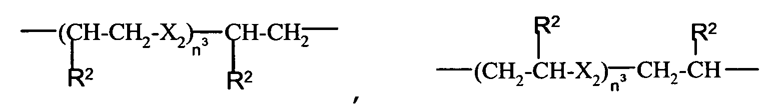 où X₂ est -O- ou -S-, n³ est un entier de 1 à 6, de préférence de 1 à 4, R² est tel que défini ci-dessus ;
h) 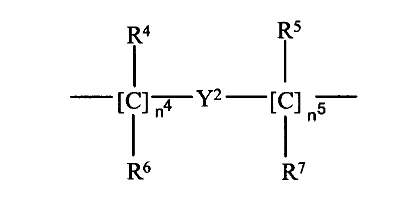 où :
n⁴ est un entier de 0 à 10 ;
n⁵ est un entier de 1 à 10 ;
R⁴, R⁵, R⁶, R⁷ sont identiques ou différents, et sont H ou alkyle en C₁-C₄ linéaire ou ramifié, de préférence R⁴, R⁵, R⁶, R⁷ sont H ;
où le groupe -ONO₂ est lié à 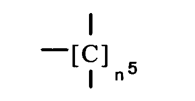 où n⁵ est tel que défini ci-dessus ;
Y² est un cycle hétérocyclique à 5 ou 6 chaînons, saturé, insaturé ou aromatique, contenant un ou plusieurs hétéroatome(s) choisi(s) parmi azote, oxygène, soufre, et est choisi parmi

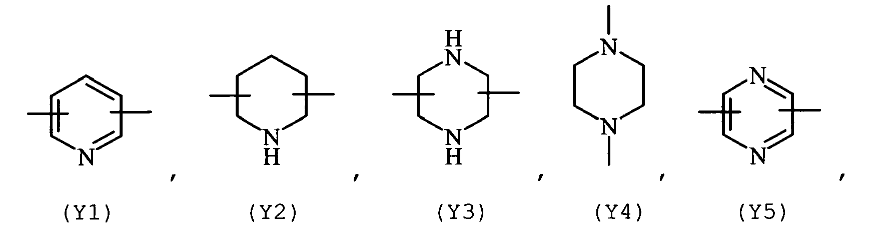

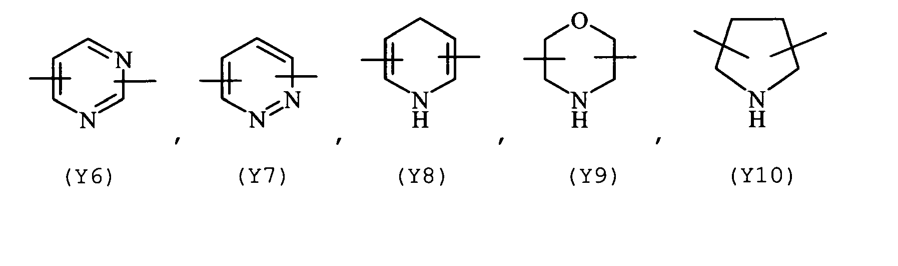

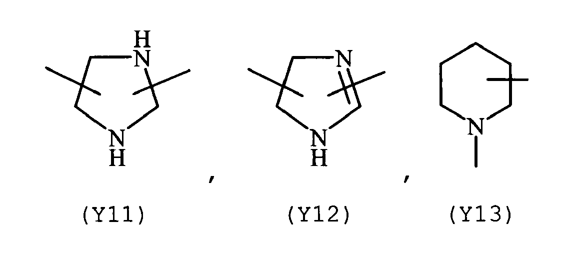

2. Composé de formule générale (I) ou sel pharmaceutiquement acceptable ou stéréoisomère de celui-ci selon la revendication 1, formule dans laquelle Y est un radical bivalent ayant la signification suivante :
a) alkylène en C₁-C₁₀ linéaire ou ramifié, éventuellement substitué par T₀, où T₀ est tel que défini ci-dessus ;
b) 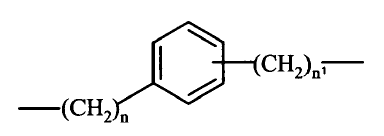 où n est un entier égal à 0 ou 1, et n¹ est un entier égal à 1 ; avec la réserve que le groupe -ONO₂ est lié à un groupe -(CH₂)ₙ¹ ;
g) 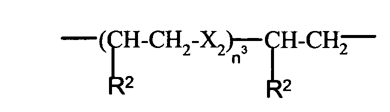
où X₂ est -O- ou -S-, n³ est un entier égal à 1 et R² est H.

3. Composé choisi dans le groupe constitué par: 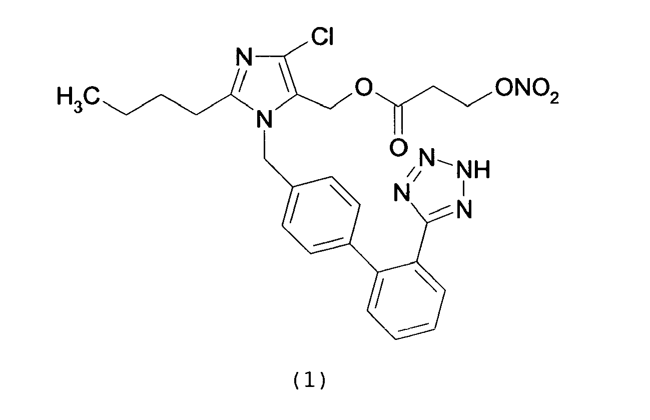 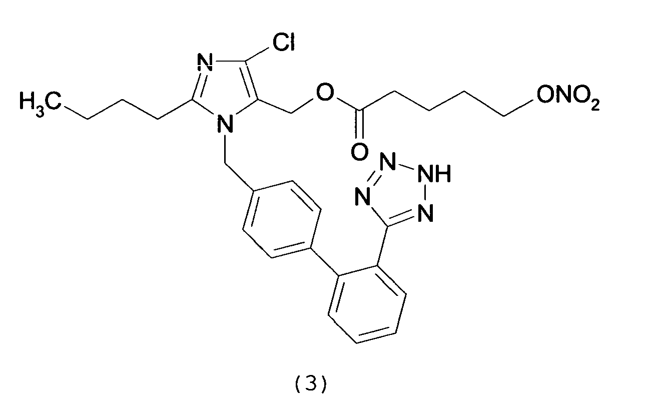 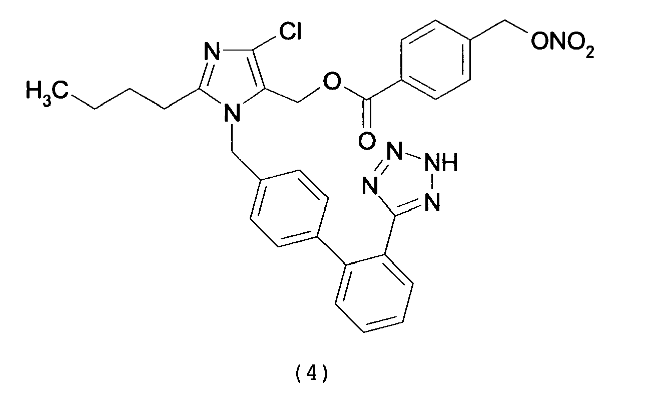 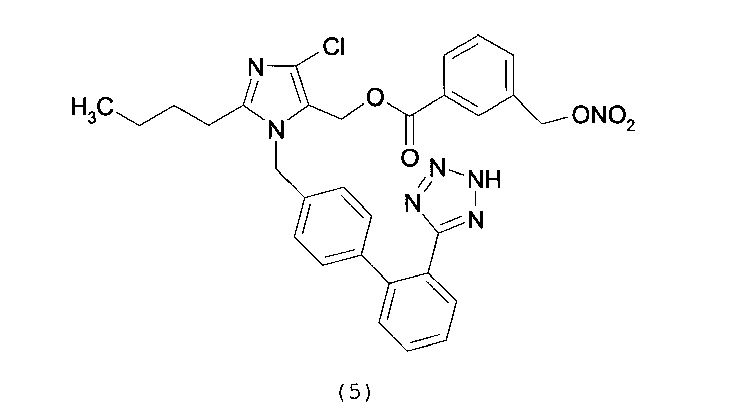 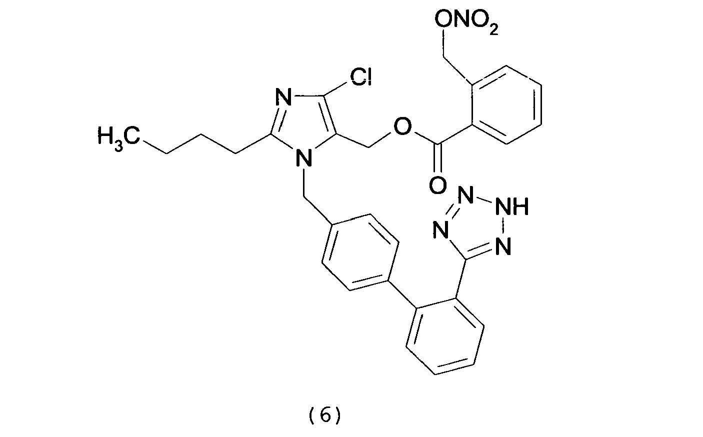 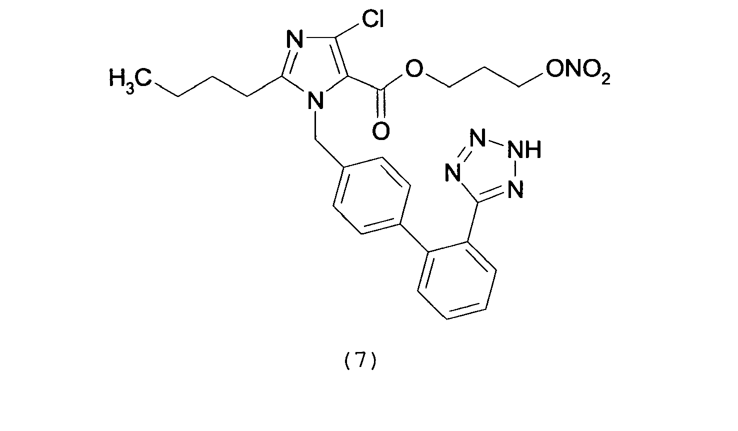 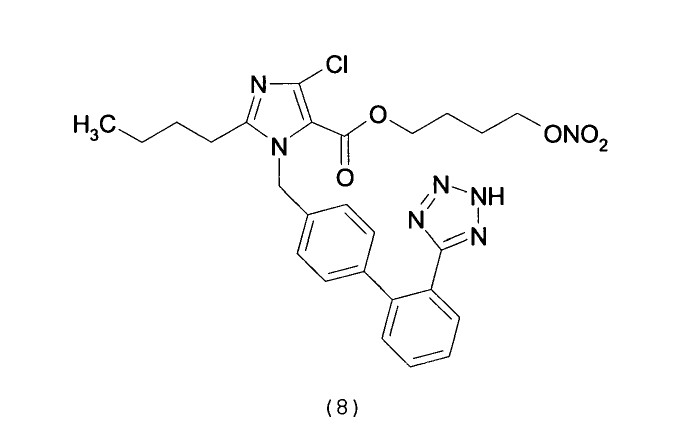 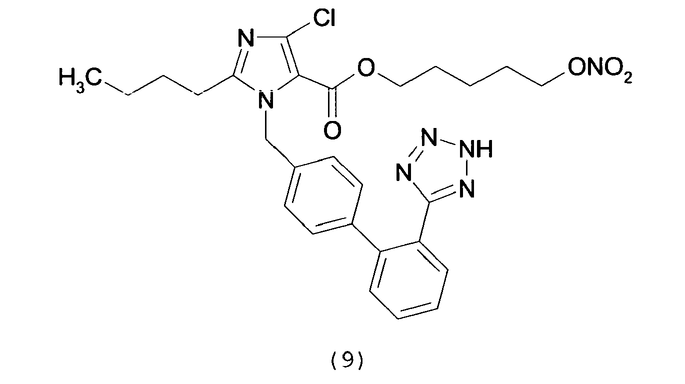 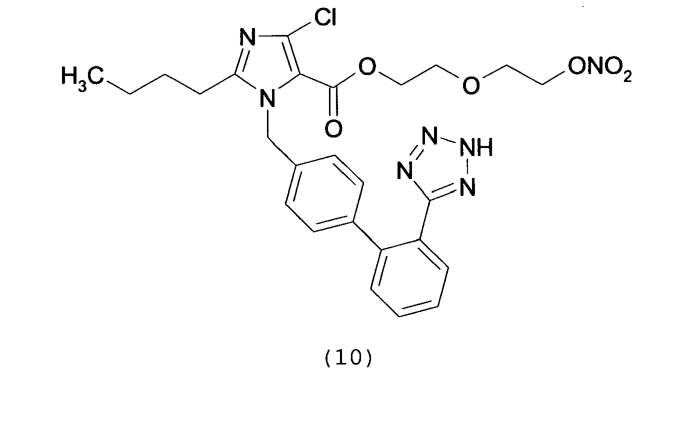 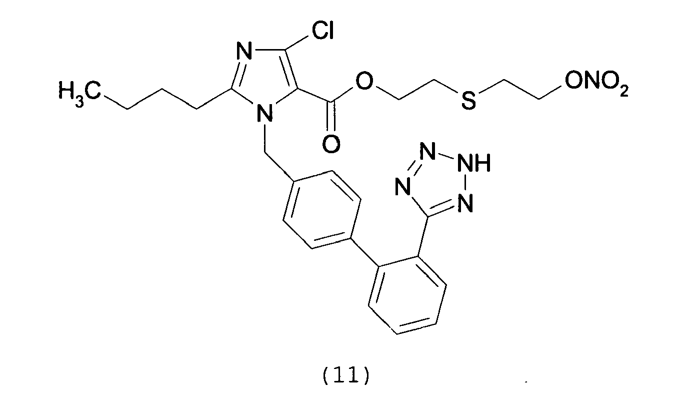 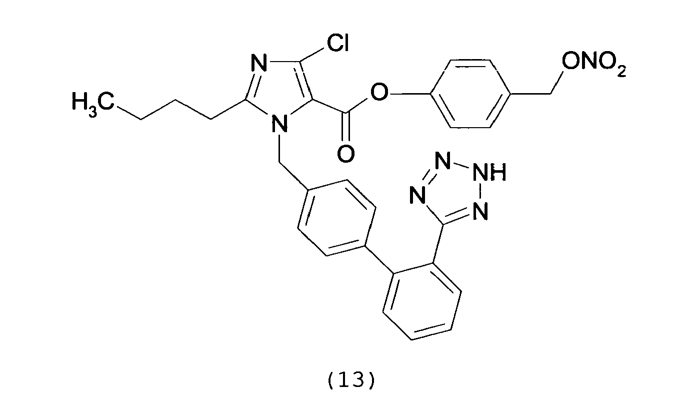 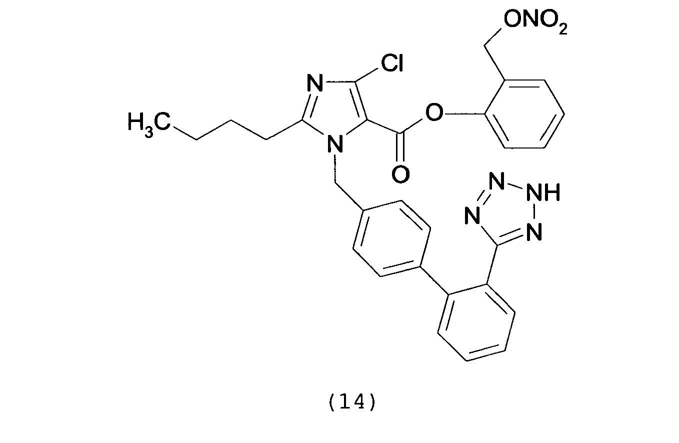 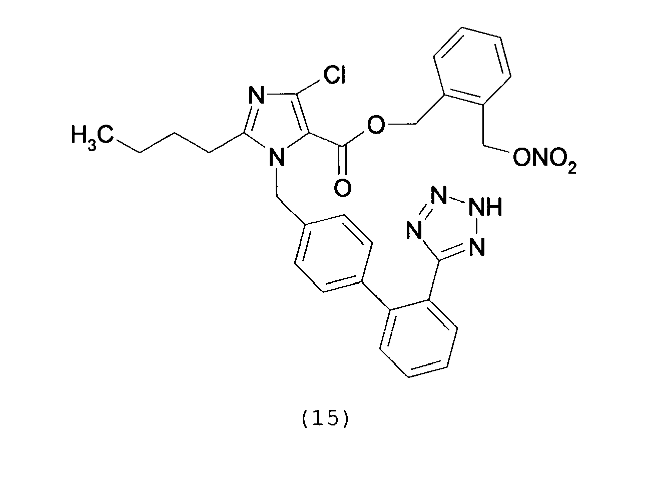 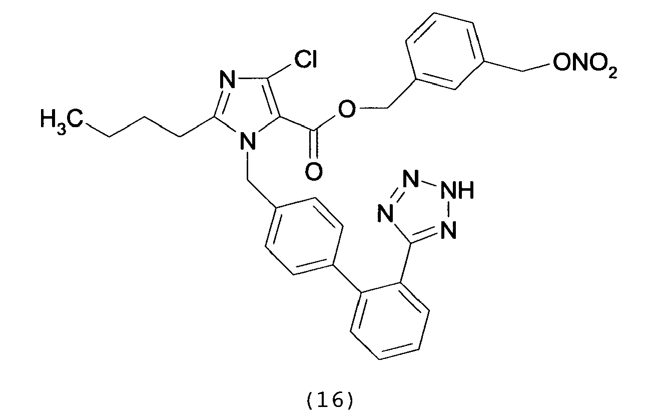 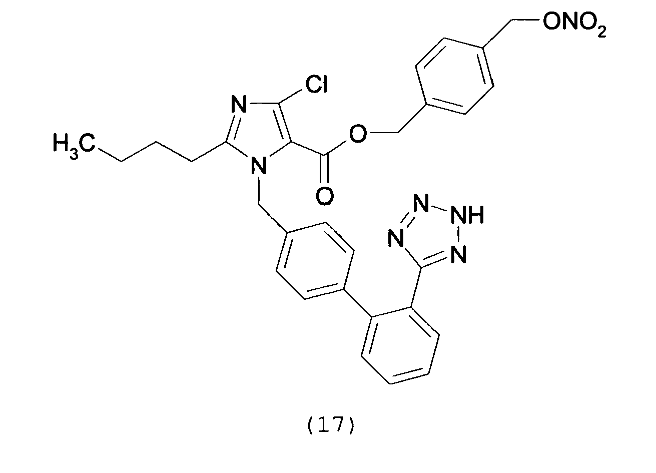 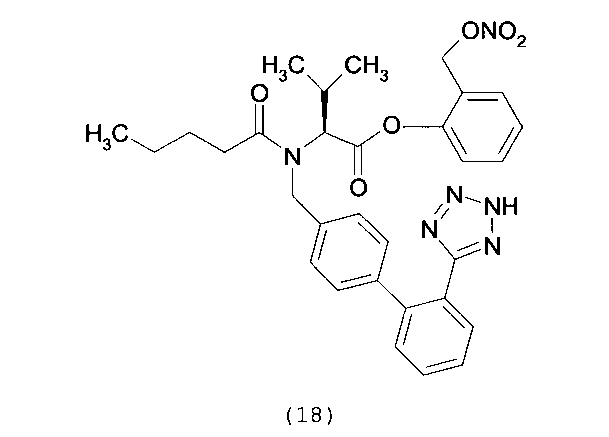 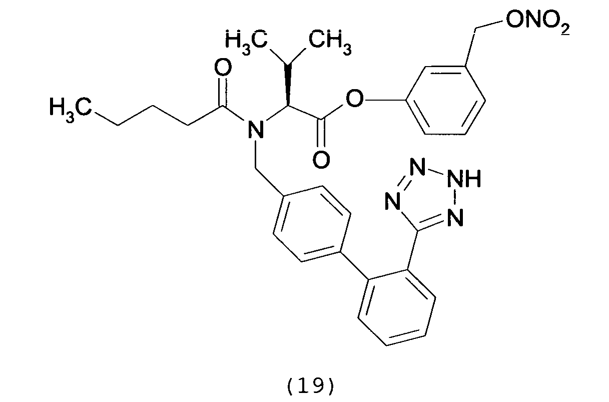 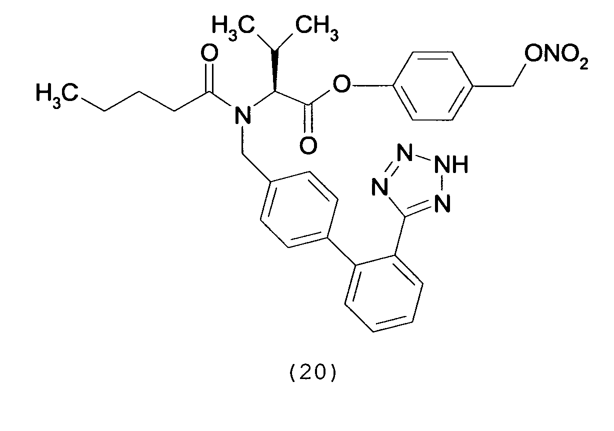 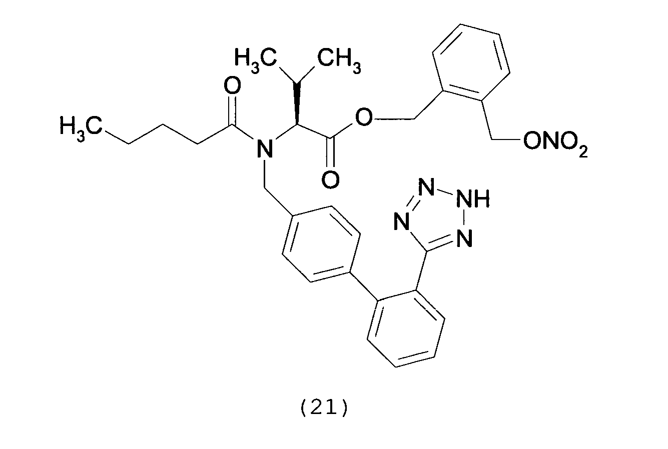 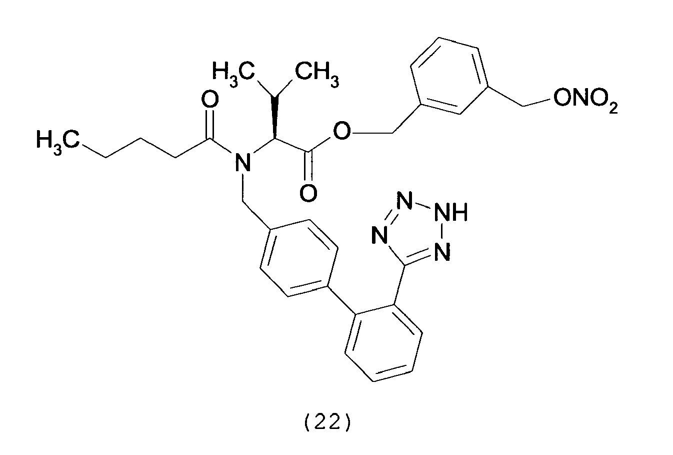 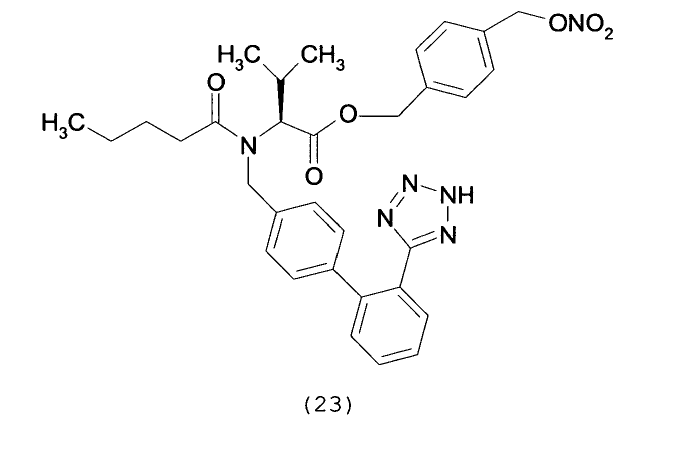 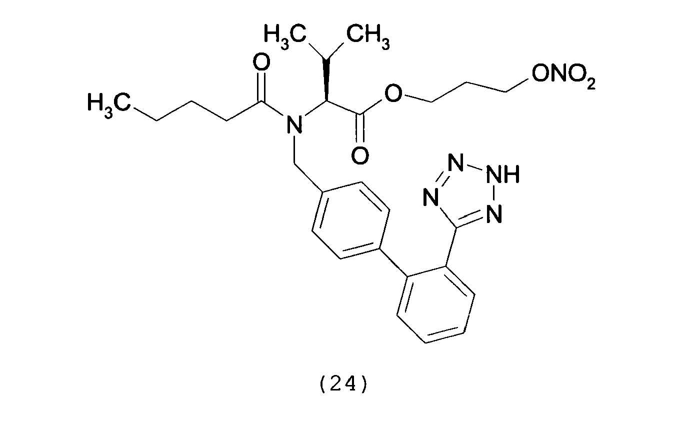 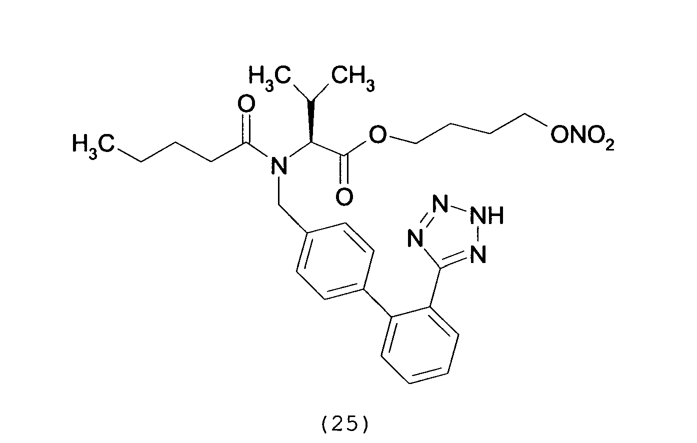 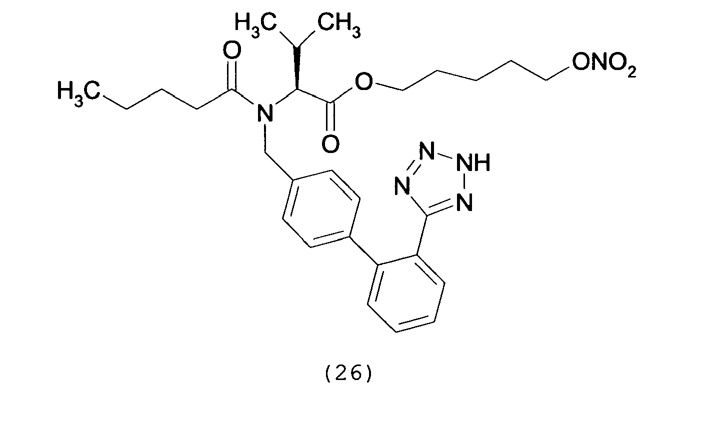 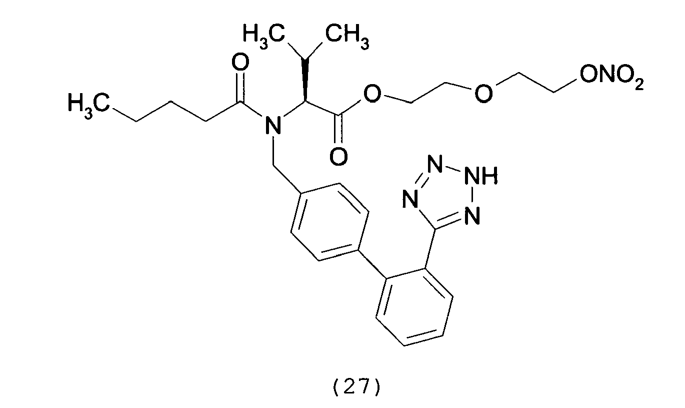 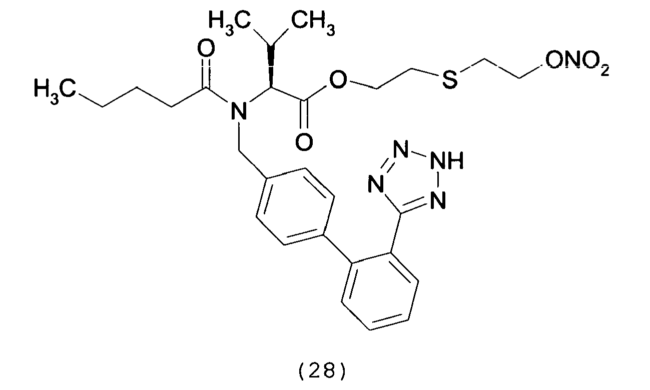 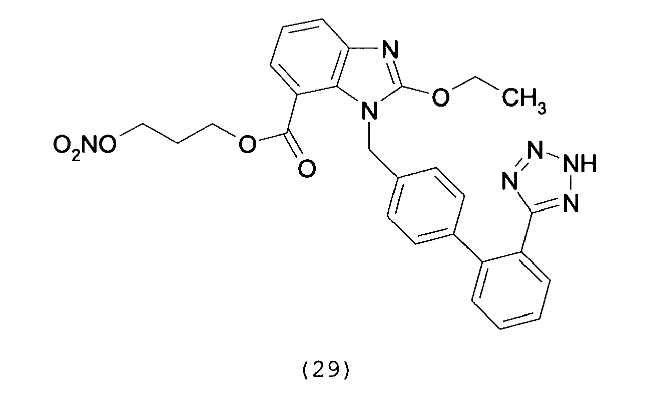 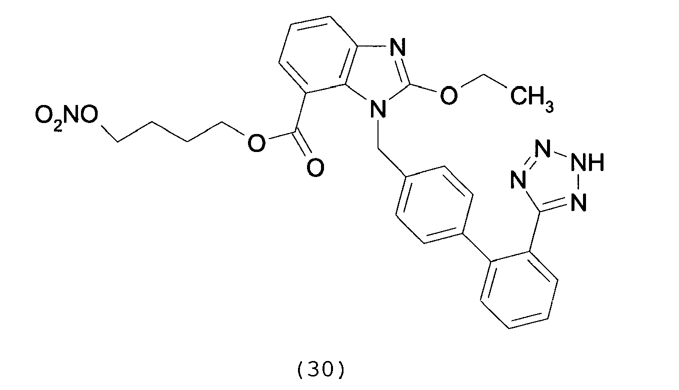 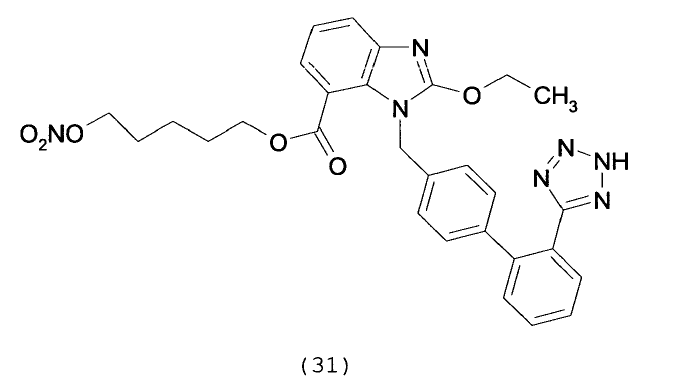 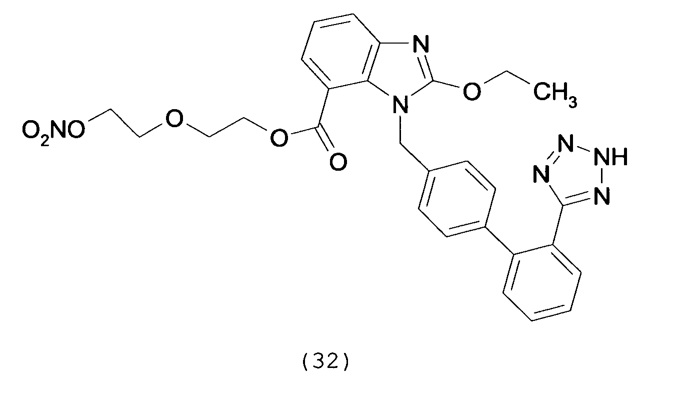 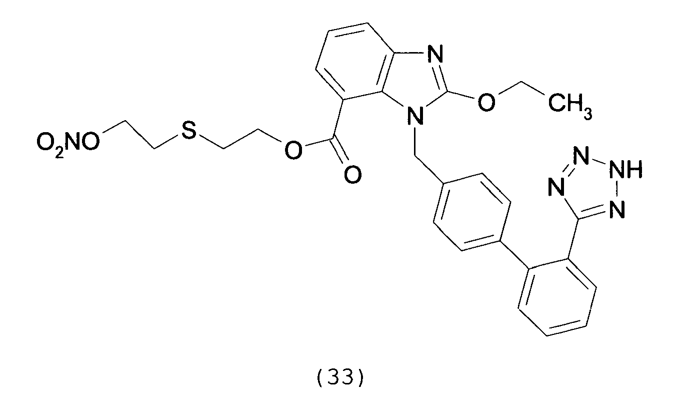 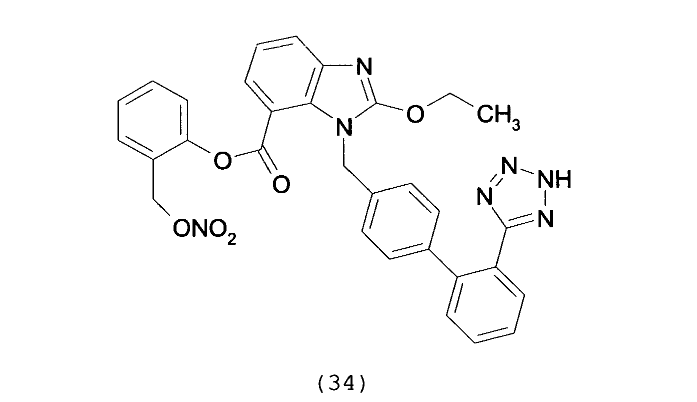 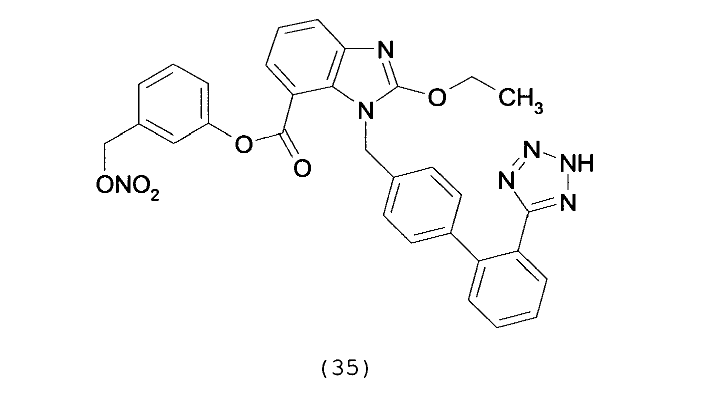 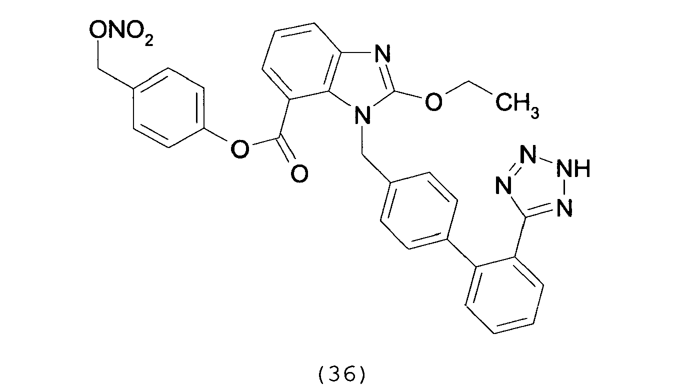 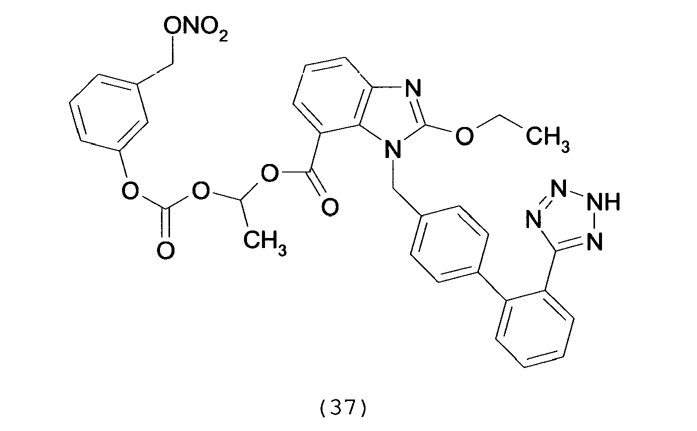 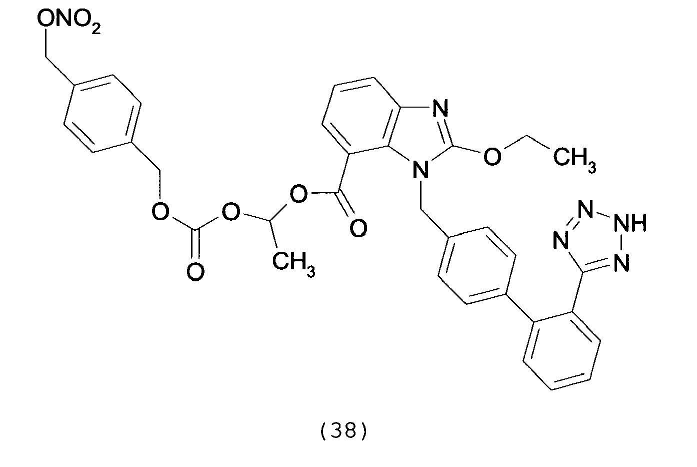 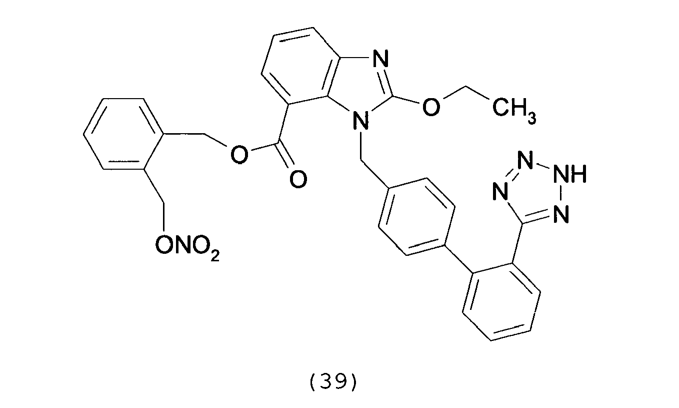 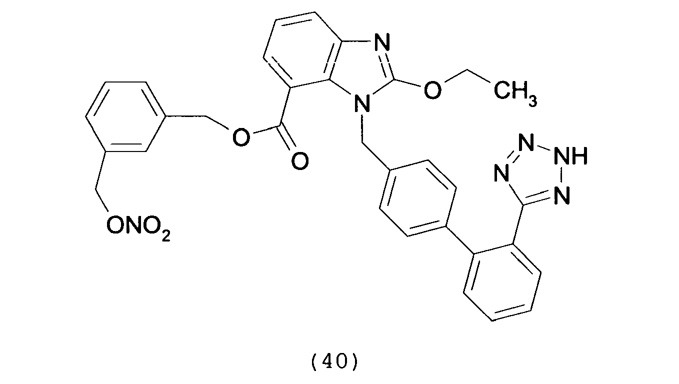 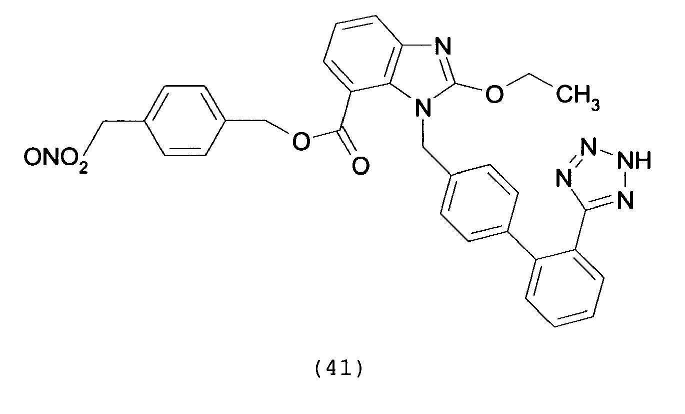 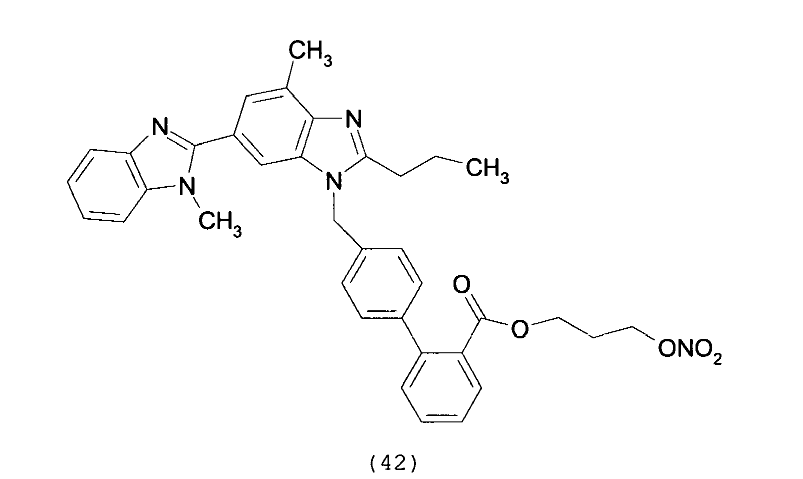 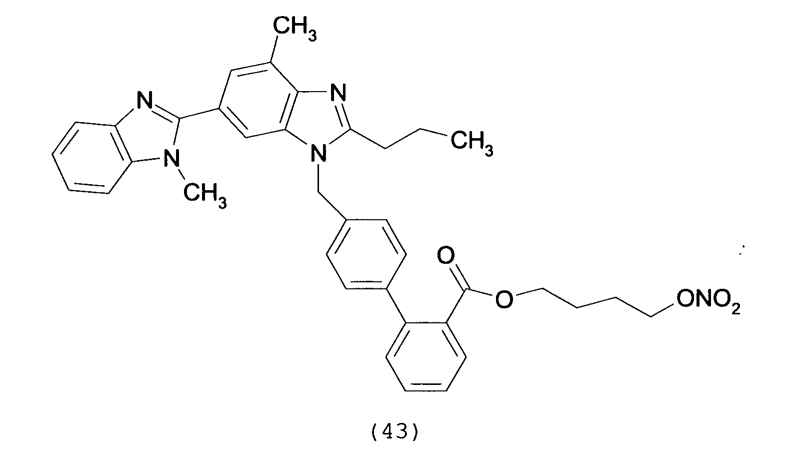 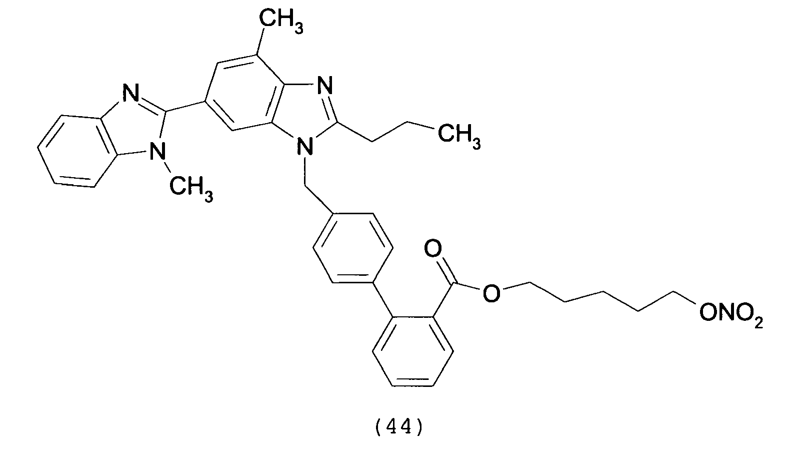 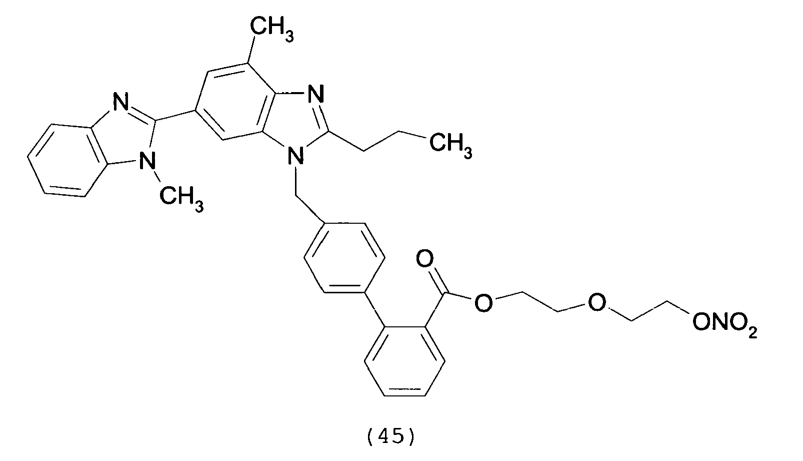 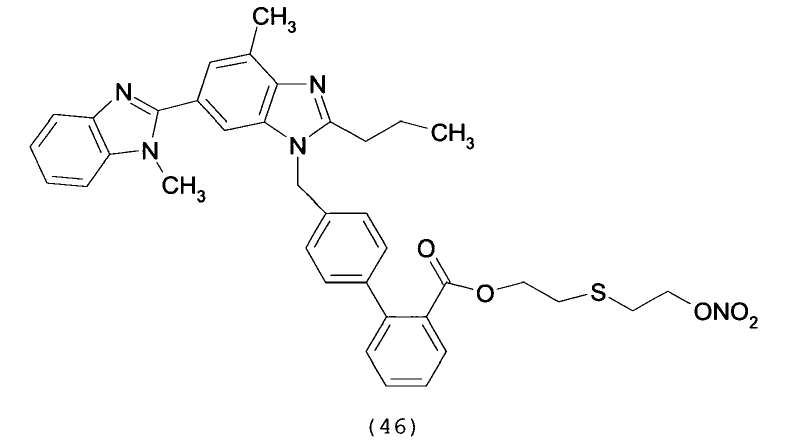 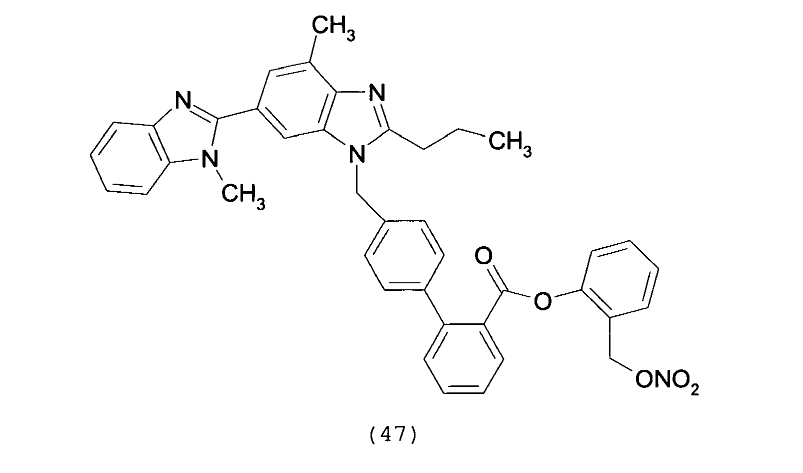 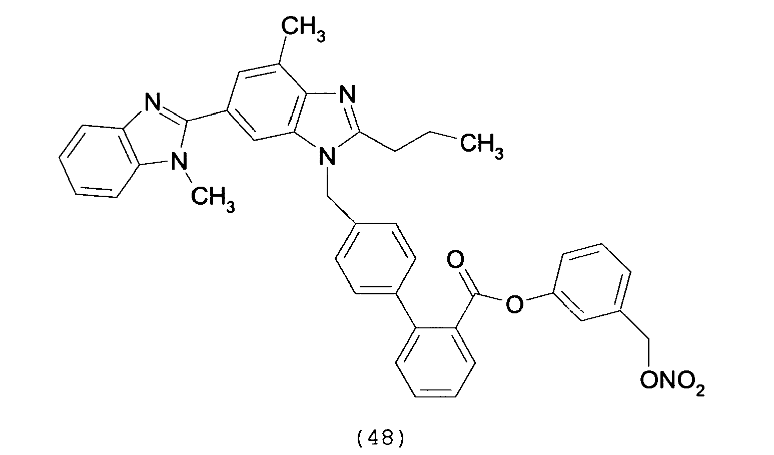 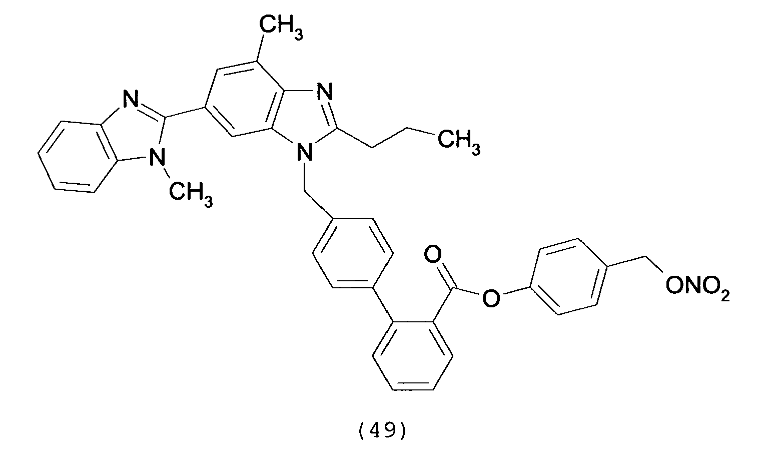 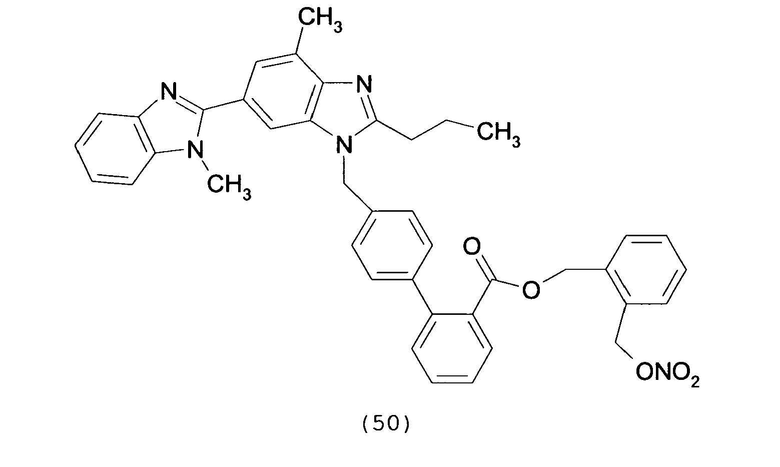 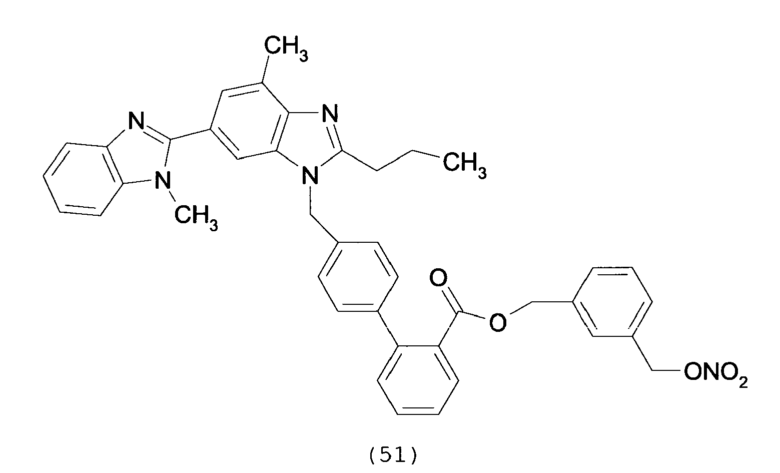 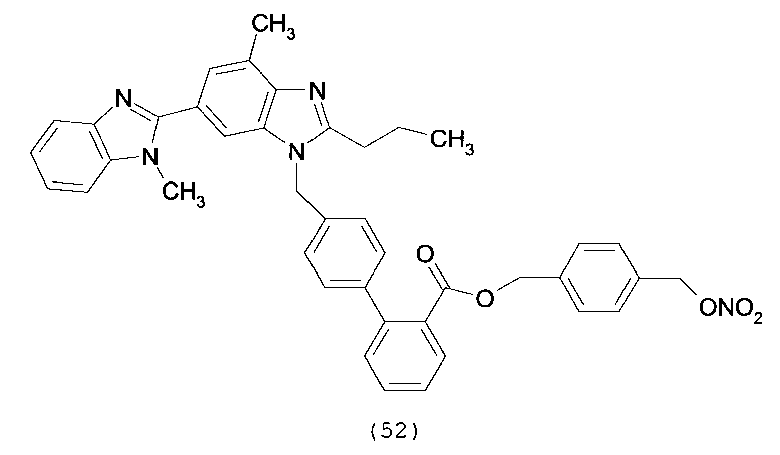 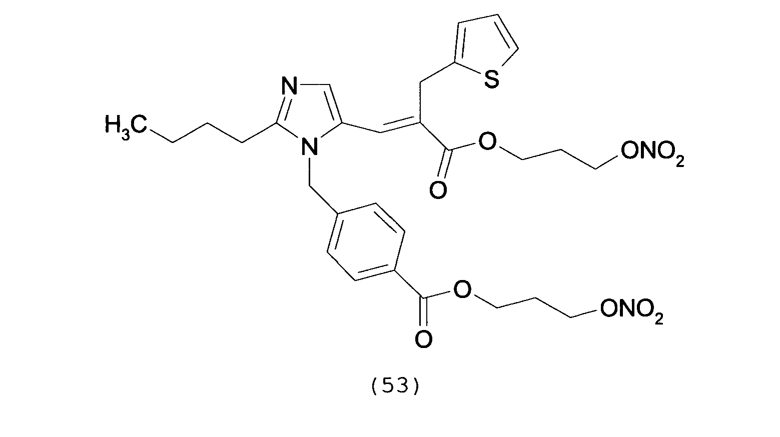 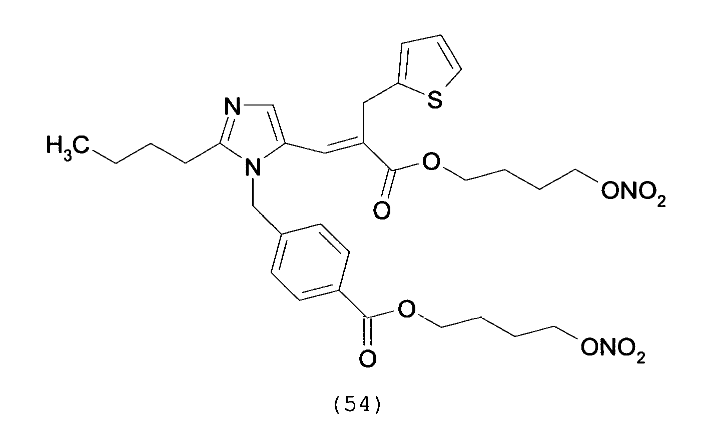 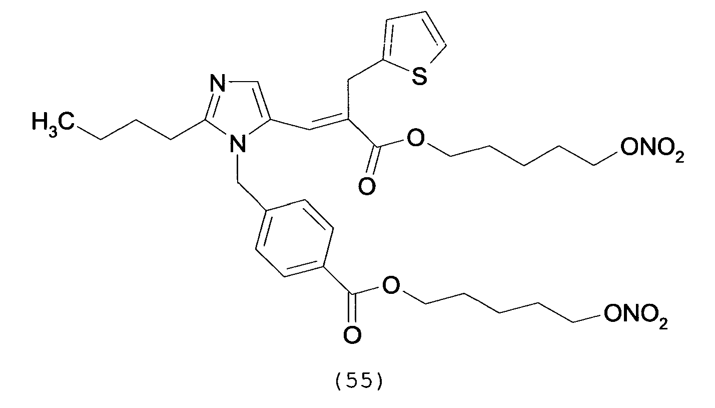 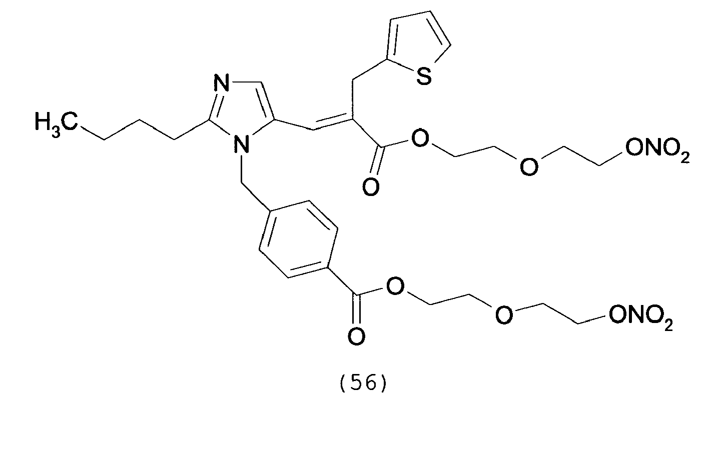 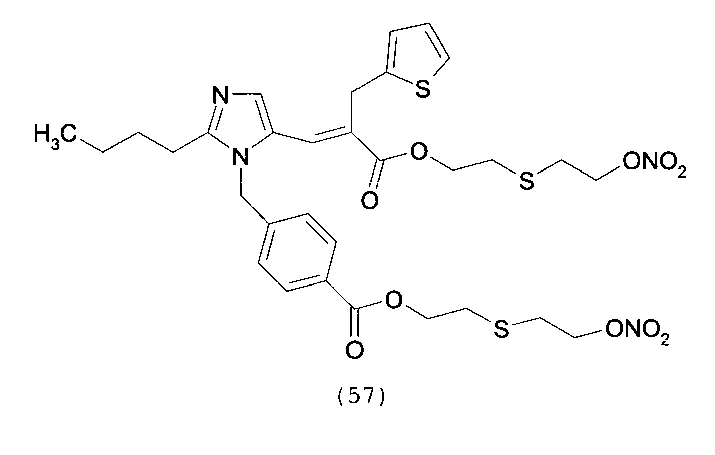 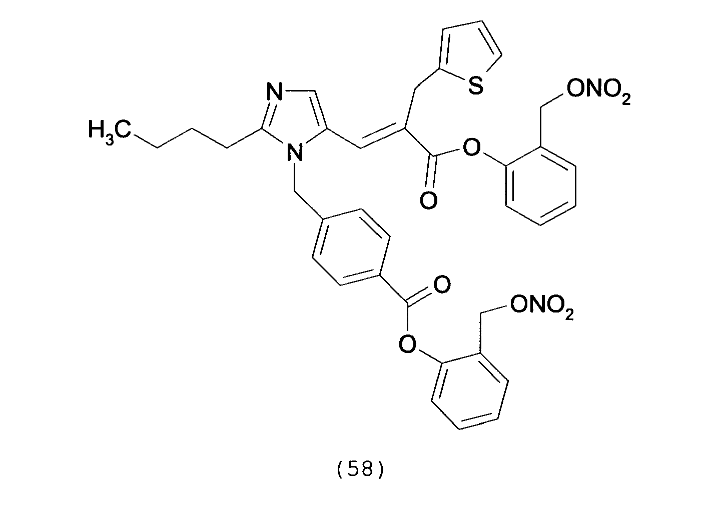 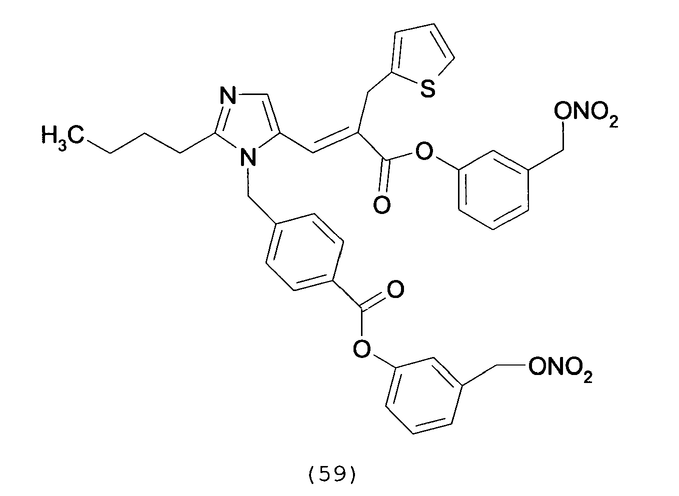 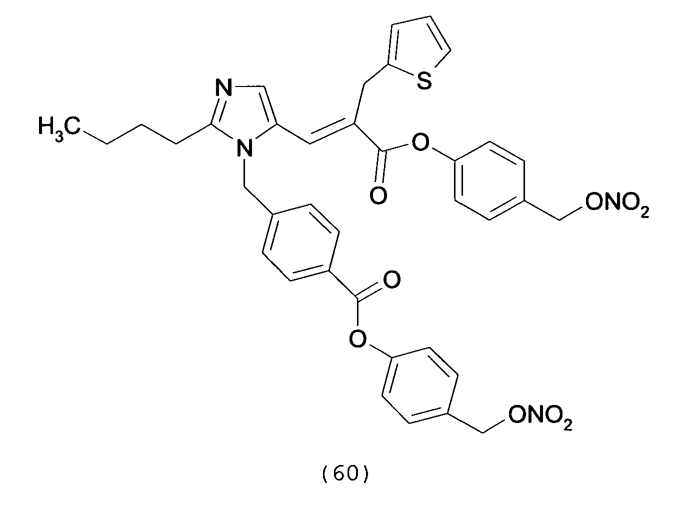 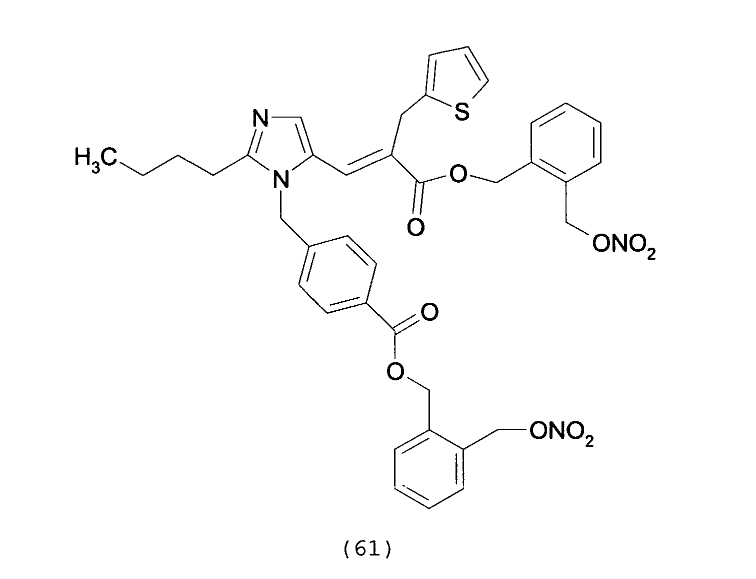 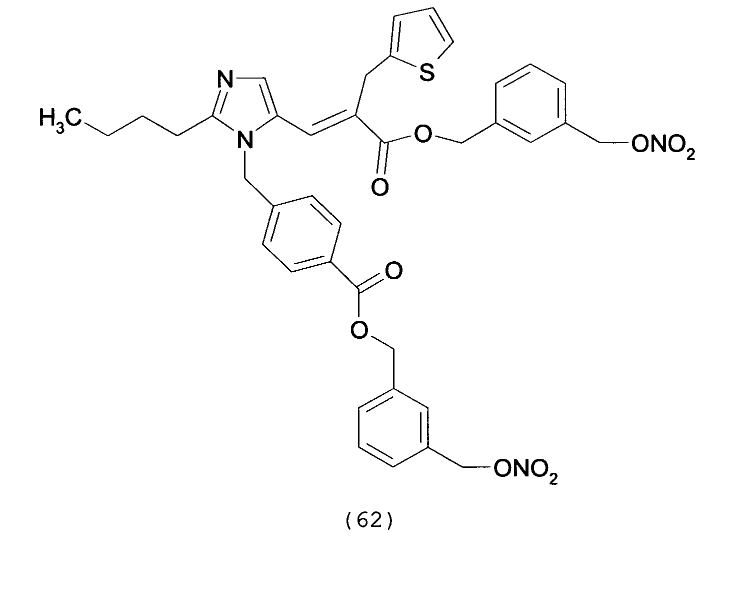 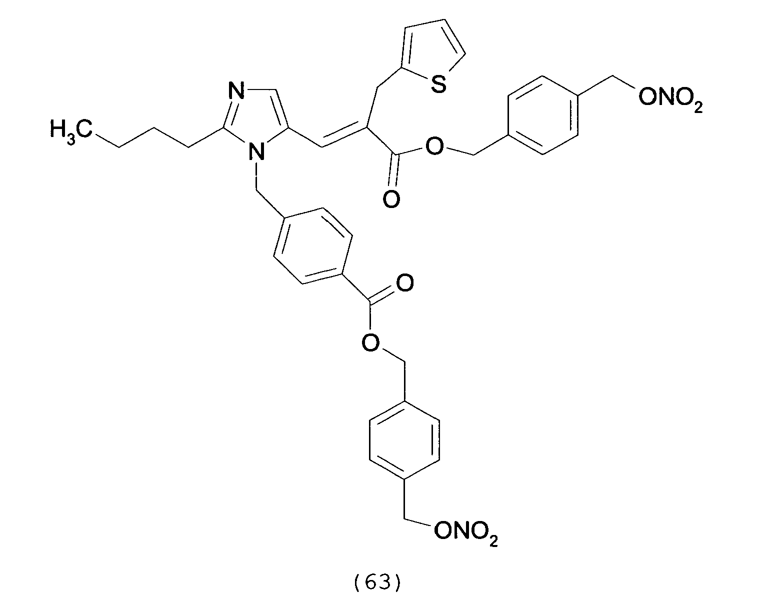 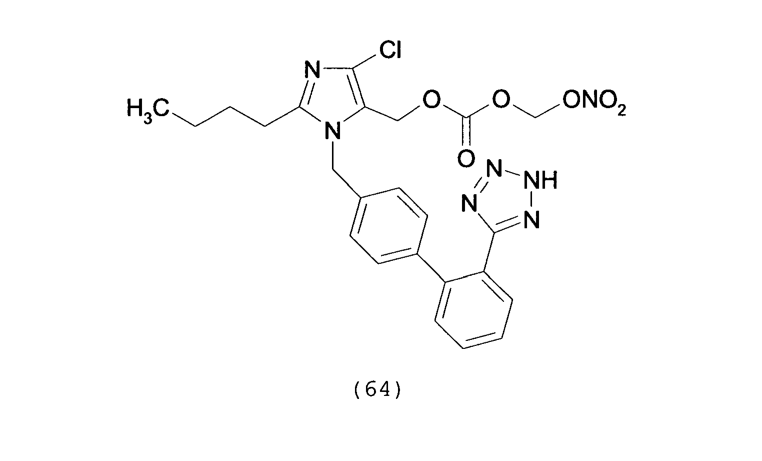 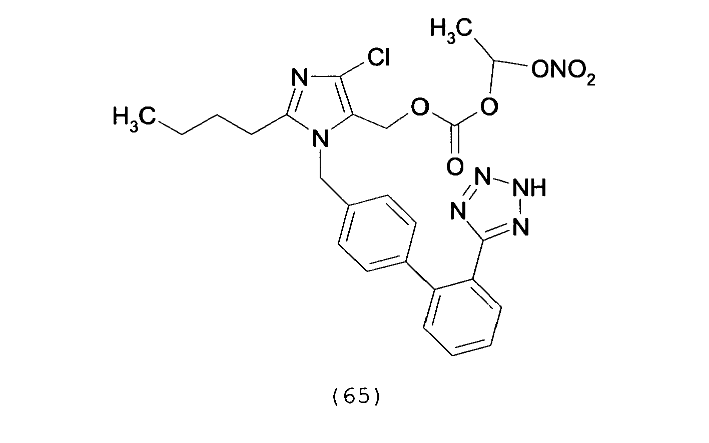 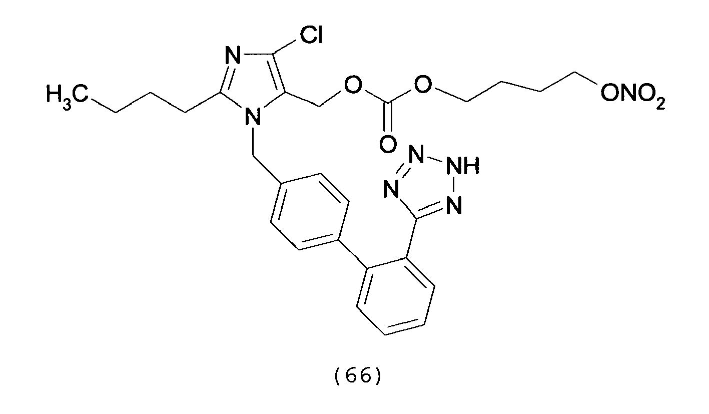 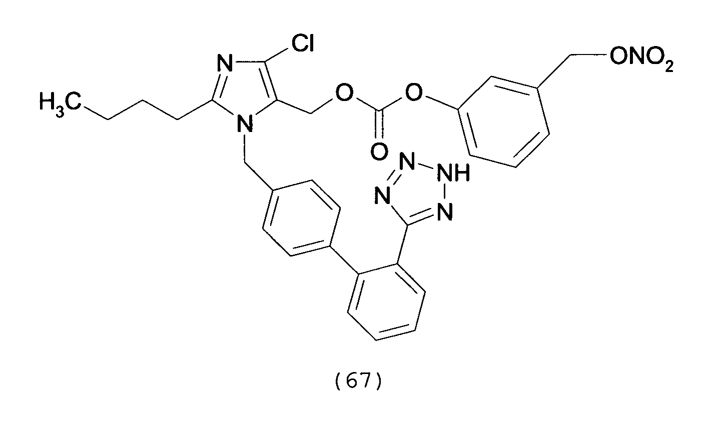 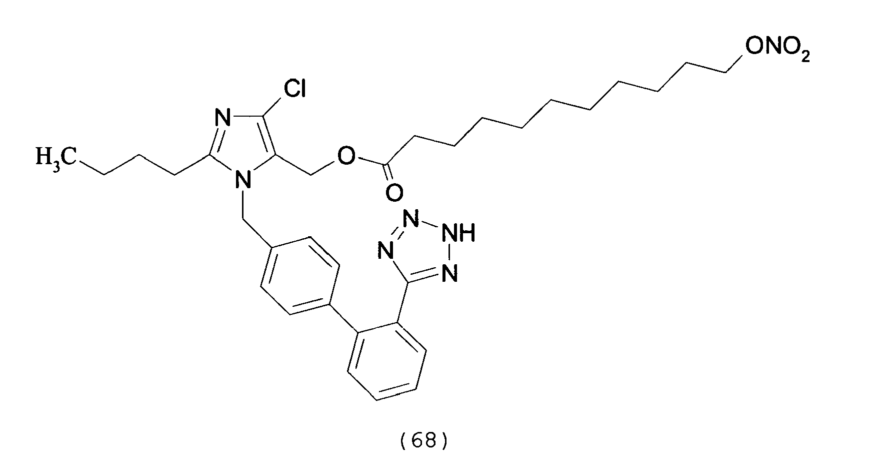 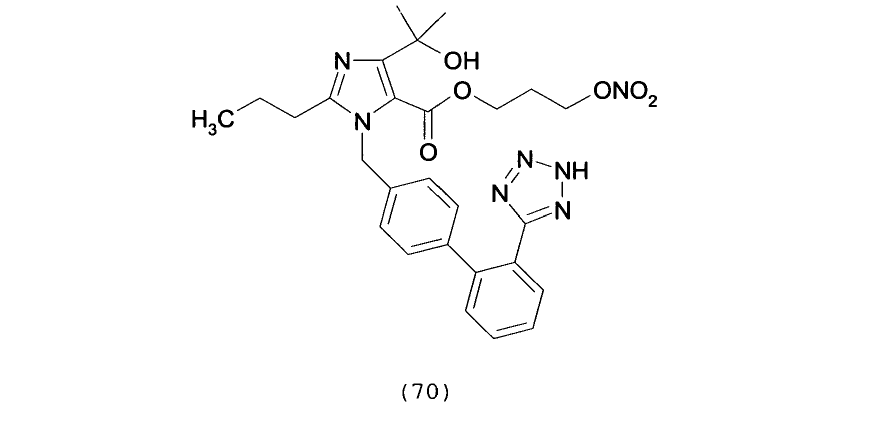 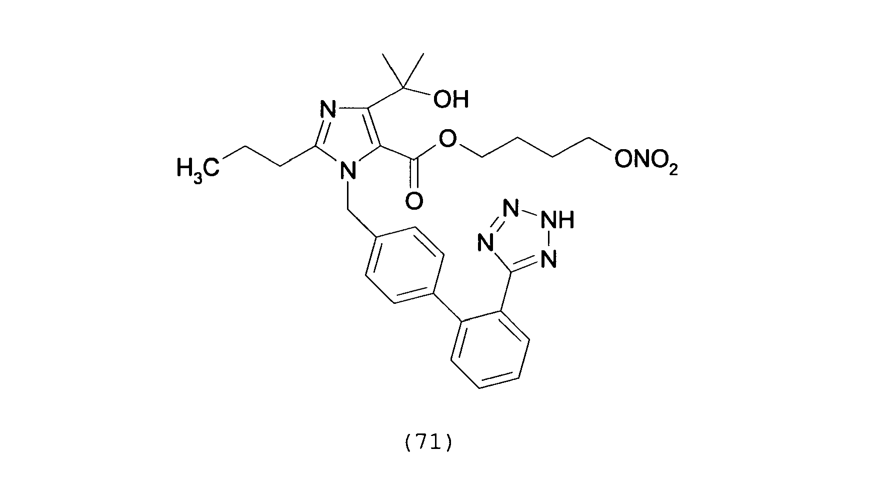 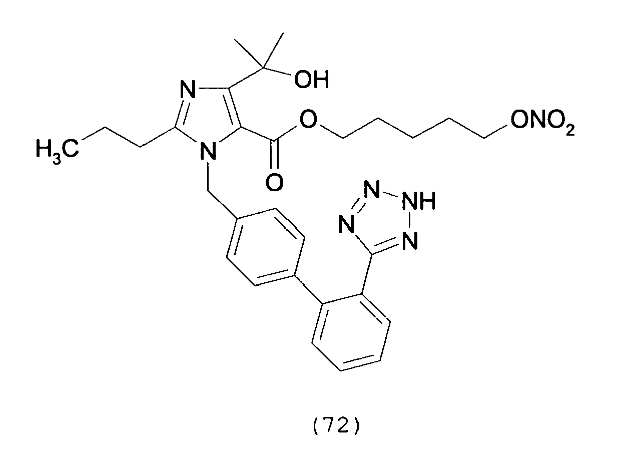 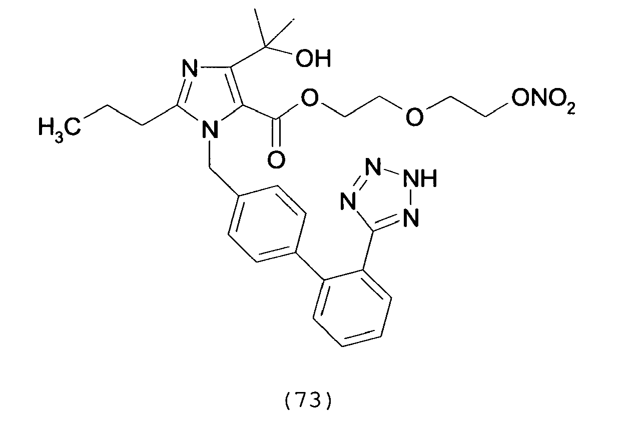 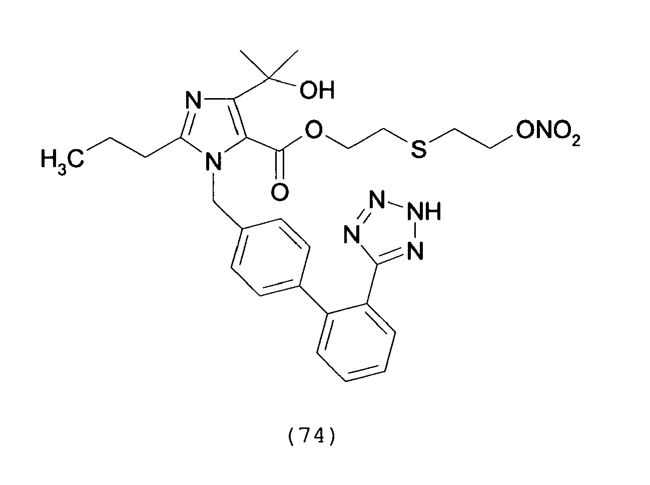 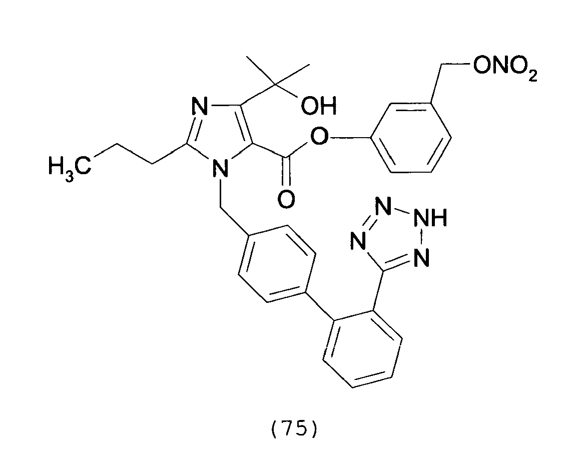 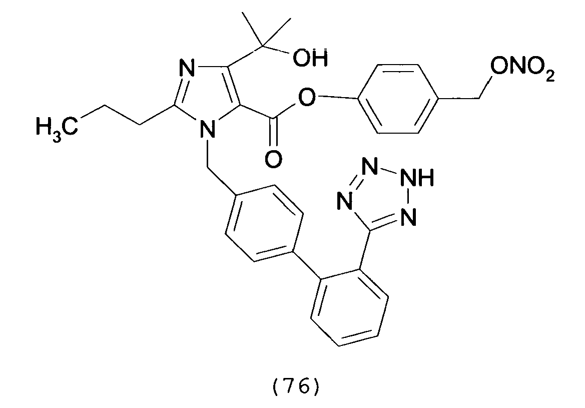 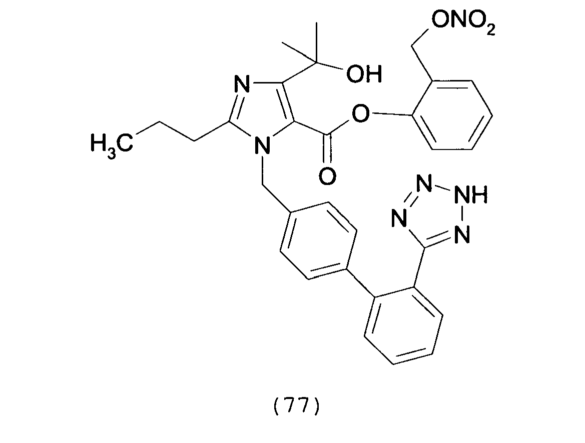 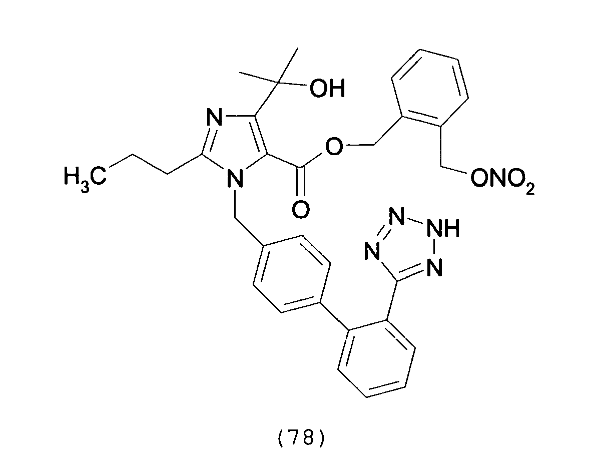 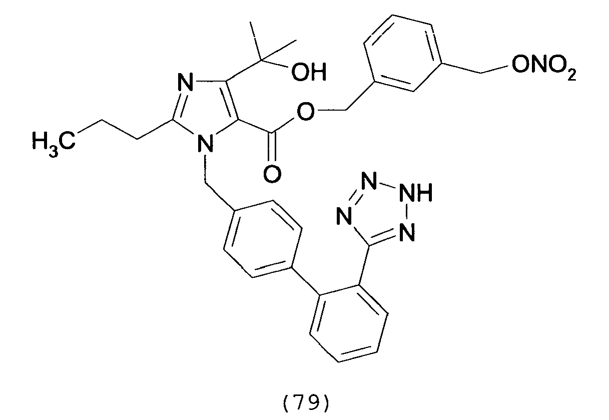 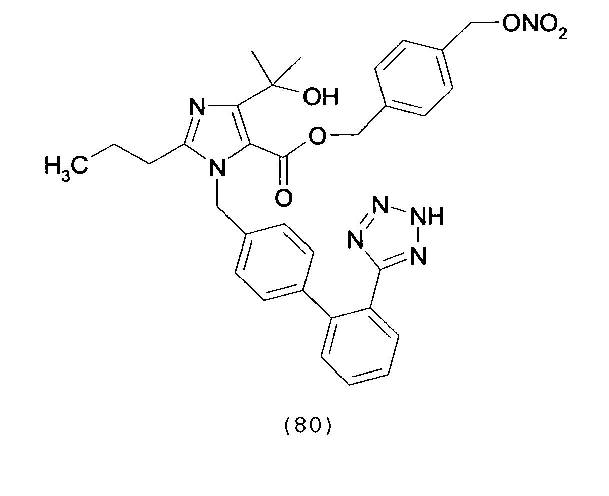 ou sel pharmaceutiquement acceptable ou stéréoisomère de celui-ci.

4. Composé de formule (2) : 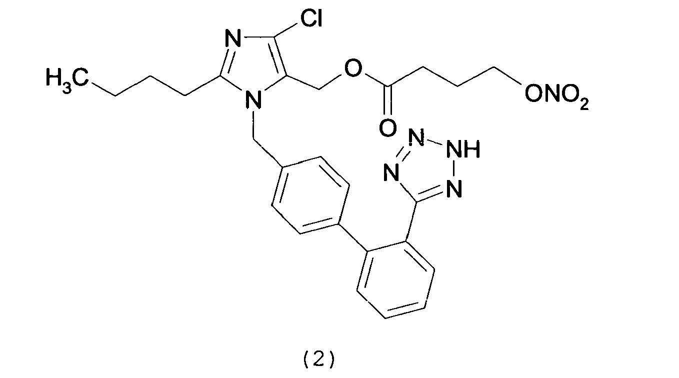 ou sel pharmaceutiquement acceptable ou stéréoisomère de celui-ci.

5. Composé de formule (12) : 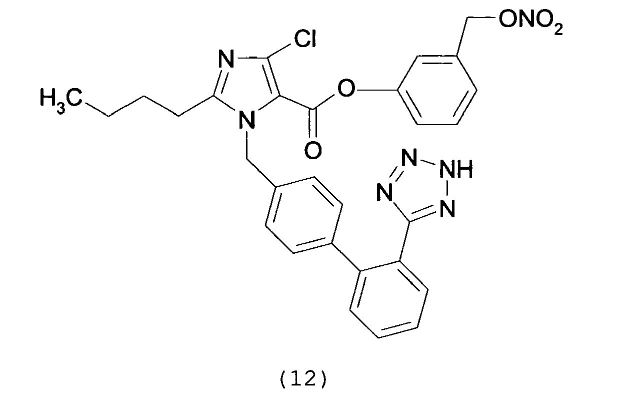 ou sel pharmaceutiquement acceptable ou stéréoisomère de celui-ci.

6. Composé de formule (69) : 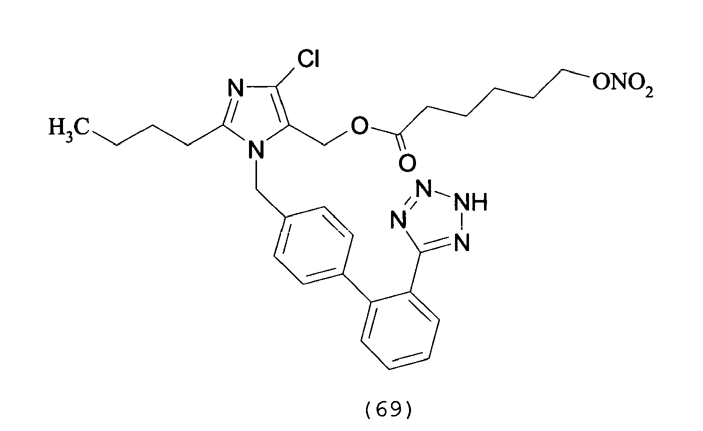 ou sel pharmaceutiquement acceptable ou stéréoisomère de celui-ci.

7. Composé de formule générale (I) selon les revendications 1-6 pour une utilisation en tant que médicament.

8. Utilisation d'un composé selon les revendications 1-6 pour préparer un médicament ayant une activité anti-inflammatoire, anti-thrombotique et anti-plaquettes.

9. Utilisation d'un composé selon les revendications 1-6, pour préparer un médicament qui peut être employé dans le traitement ou la prophylaxie des maladies cardiovasculaires, rénales et chroniques du foie, les processus inflammatoires et les syndromes métaboliques.

10. Utilisation d'un composé selon la revendication 9, pour préparer un médicament qui peut être employé dans le traitement ou la prophylaxie de la crise cardiaque, de l'infarctus du myocarde, de l'attaque ischémique, de l'athérosclérose, de l'hypertension oculaire et pulmonaire, de l'hypertension, de la néphropathie diabétique, des maladies vasculaires périphériques, du dysfonctionnement et de l'hypertrophie ventriculaire gauche, de la fibrose du foie et de l'hypertension portale.

11. Composition pharmaceutique comprenant un véhicule pharmaceutiquement acceptable et une quantité pharmaceutiquement efficace d'un composé de formule générale (I) ou d'un sel ou stéréoisomère de celui-ci selon les revendications 1-6.

12. Composition pharmaceutique selon la revendication 11 sous une forme appropriée pour l'administration par voie orale, parentérale, rectale, topique et transdermique, par spray d'inhalation ou aérosol ou dispositifs d'iontophorèse.

13. Composition pharmaceutique liquide ou solide pour une administration par voie orale, parentérale, rectale, topique et transdermique ou par inhalation, sous la forme de comprimés, de capsules et de pilules, éventuellement avec un revêtement entérique, de poudres, de granulés, de gels, d'émulsions, de solutions, de suspensions, de sirops, d'élixir, de formes injectables, de suppositoires, de patches transdermiques ou de liposomes, contenant un composé de formule (I) ou un sel ou stéréoisomère de celui-ci selon les revendications 1-6 et un véhicule pharmaceutiquement acceptable.

14. Composition pharmaceutique comprenant un composé de formule générale (I), au moins un composé utilisé pour traiter une maladie cardiovasculaire et un véhicule pharmaceutiquement acceptable.

15. Composition pharmaceutique selon la revendication 14 dans laquelle le composé utilisé pour traiter une maladie cardiovasculaire est choisi dans le groupe constitué par : les inhibiteurs de l'ACE, les inhibiteurs de la HMGCoA réductase, les agents bloquants béta-adrénergiques, les agents bloquants du canal du calcium, les diurétiques, les agents anti-thrombotiques tels que l'aspirine, les inhibiteurs de l'ACE nitrosés, les inhibiteurs de la HMGCoA réductase nitrosés, les agents bloquants béta-adrénergiques nitrosés, l'aspirine nitrosée et les diurétiques nitrosés.

16. Kit pharmaceutique comprenant un composé de formule générale (I) tel que défini dans la revendication 1, un composé utilisé pour traiter une maladie cardiovasculaire, sous forme d'une préparation combinée pour une utilisation simultanée, séparée, en séquence pour le traitement d'une maladie cardiovasculaire.

17. Kit pharmaceutique selon la revendication 16 dans lequel le composé utilisé pour traiter une maladie cardiovasculaire est choisi dans le groupe constitué par : les inhibiteurs de l'ACE, les inhibiteurs de la HMGCoA réductase, les agents bloquants béta-adrénergiques, les agents bloquants du canal du calcium, les diurétiques, les agents anti-thrombotiques tels que l'aspirine, les inhibiteurs de l'ACE nitrosés, les inhibiteurs de la HMGCoA réductase nitrosés, les agents bloquants béta-adrénergiques nitrosés, l'aspirine nitrosée et les diurétiques nitrosés.
